(19) **Europäisches Patentamt / European Patent Office / Office européen des brevets**

(11) **EP 2 240 510 B1**

(12) **EUROPEAN PATENT SPECIFICATION**

(45) Date of publication and mention of the grant of the patent:
**10.05.2017 Bulletin 2017/19**

(21) Application number: **08869158.9**

(22) Date of filing: **23.12.2008**

(51) Int Cl.:
*C07K 14/415* (2006.01)  *C12N 15/82* (2006.01)
*A01H 5/00* (2006.01)

(86) International application number:
**PCT/IL2008/001657**

(87) International publication number:
**WO 2009/083958 (09.07.2009 Gazette 2009/28)**

(54) **ISOLATED POLYPEPTIDES, POLYNUCLEOTIDES USEFUL FOR MODIFYING SALINITY STRESS TOLERANCE IN PLANTS**

ISOLIERTE POLYPEPTIDE, POLYNUKLEOTIDE ZUR VERÄNDERUNG DER SALZRESISTENZ BEI PFLANZEN

PEPTIDES ISOLÉS, POLYNUCLÉOTIDES UTILES POUR MODIFIER LA TOLÉRANCE AUX STRESS DE SALINITÉ DANS LES PLANTES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.12.2007 US 9166 P**
**20.08.2008 US 136238 P**

(43) Date of publication of application:
**20.10.2010 Bulletin 2010/42**

(73) Proprietor: **Evogene Ltd.**
**76121 Rechovot (IL)**

(72) Inventors:
• **RONEN, Gil**
**38870 Emek Hefer (IL)**
• **VINOCUR, Basia Judith**
**76291 Rechovot (IL)**
• **DIBER, Alex**
**75502 Rishon-lezion (IL)**
• **AYAL, Sharon**
**76951 Kiryat-ekron (IL)**
• **KARCHI, Hagai**
**76834 Doar-na Emek Soreq (IL)**
• **HERSCHKOVITZ, Yoav**
**53400 Givataim (IL)**

(74) Representative: **Dennemeyer & Associates S.A.**
**Postfach 70 04 25**
**81304 München (DE)**

(56) References cited:
**WO-A2-2006/076423    US-A1- 2003 233 670**

• **DATABASE EMBL [Online] 12 May 2004 (2004-05-12), "Lycopersicon esculentum clone 133453R, mRNA sequence." XP002529190 retrieved from EBI accession no. EMBL:BT014251 Database accession no. BT014251**
• **DATABASE UniProt [Online] 28 November 2006 (2006-11-28), "SubName: Full=Major intrinsic protein;" XP002529191 retrieved from EBI accession no. UNIPROT:A0FI89 Database accession no. A0FI89**
• **FRAY RUPERT G ET AL: "Nucleotide sequence and expression of a ripening and water stress-related cDNA from tomato with homology to the MIP class of membrane channel proteins" PLANT MOLECULAR BIOLOGY, [Online] vol. 24, no. 3, 1994, pages 539-543, XP009117320 & DATABASE UniProt 1 October 1994 (1994-10-01), "RecName: Full=Probable aquaporin PIP-type pTOM75; AltName: Full=Ripening-associated membrane protein; Short=RAMP;"**
• **HACHEZ ET AL: "Modulating the expression of aquaporin genes in planta: A key to understand their physiological functions?" BIOCHIMICA ET BIOPHYSICA ACTA. BIOMEMBRANES, AMSTERDAM, NL, vol. 1758, no. 8, 1 August 2006 (2006-08-01), pages 1142-1156, XP005655605 ISSN: 0005-2736**

- **MAUREL ET AL: "Plant aquaporins: Novel functions and regulation properties" FEBS LETTERS, ELSEVIER, AMSTERDAM, NL, vol. 581, no. 12, 25 May 2007 (2007-05-25), pages 2227-2236, XP022078418 ISSN: 0014-5793 cited in the application**

Remarks:
The complete document including Reference Tables and the Sequence Listing can be downloaded from the EPO website

Remarks:
The complete document including Reference Tables and the Sequence Listing can be downloaded from the EPO website

**Description**

This application is continuation of U.S. Patent Application No. 12/810,855 filed on June 28, 2010, which is a National Phase of PCT Patent Application No. PCT/IL2008/001657 having International filing date of December 23, 2008, which claims the benefit of priority of U.S. Provisional Patent Application Nos. 61/136,238 filed on August 20, 2008 and 61/009,166 filed on December 27, 2007.

FIELD AND BACKGROUND OF THE INVENTION

The present invention, relates to a method of increasing abiotic stress tolerance of a plant according to claim 1.

Abiotic stress conditions such as salinity, drought, flood, suboptimal temperature and toxic chemical pollution, cause substantial damage to agricultural plants. Most plants have evolved strategies to protect themselves against these conditions. However, if the severity and duration of the stress conditions are too great, the effects on plant development, growth and yield of most crop plants are profound. Furthermore, most of the crop plants are highly susceptible to abiotic stress (ABS) and thus necessitate optimal growth conditions for commercial crop yields. Continuous exposure to stress causes major alterations in the plant metabolism which ultimately leads to cell death and consequently yield losses.

The global shortage of water supply is one of the most severe agricultural problems affecting plant growth and crop yield and efforts are made to mitigate the harmful effects of desertification and salinization of the world's arable land. Thus, Agbiotech companies attempt to create new crop varieties which are tolerant to different abiotic stresses focusing mainly in developing new varieties that can tolerate water shortage for longer periods.

Studies have shown that plant adaptations to adverse environmental conditions are complex genetic traits with polygenic nature. When water supply is limited, the plant WUE is critical for the survival and yield of crop. Since water scarcity is increasing and water quality is reducing worldwide it is important to increase water productivity and plant WUE. Many of the environmental abiotic stresses, such as drought, low temperature or high salt, decrease root hydraulic conductance, affect plant growth and decrease crop productivity.

Genetic improvement of FUE in plants can be generated either via traditional breeding or via genetic engineering. Attempts to improve FUE in transgenic plants are described in U.S. Patent Applications 20020046419 to Choo, et al.; U.S. Pat. Appl. 20030233670 to Edgerton et al.; U.S. Pat. Appl. 20060179511 to Chomet et al.; Yanagisawa et al. [Proc. Natl. Acad. Sci. U.S.A. 2004, 101(20):7833-8]; Good AG et al. [Trends Plant Sci. 2004, 9(12):597-605]; and U.S. Pat. No. 6,084,153 to Good et al.

Aquaporins (AQPs), the water channel proteins, are involved in transport of water through the membranes, maintenance of cell water balance and homeostasis under changing environmental and developmental conditions [Maurel C. Plant aquaporins: Novel functions and regulation properties. FEBS Lett. 2007, 581(12):2227-36]. These proteins are considered to be the main passage enabling transport of water and small neutral solutes such as urea and $CO_2$ through the membrane [Maurel C. Plant aquaporins: Novel functions and regulation properties. FEBS Lett. 2007 Jun 12; 581(12):2227-36]. In plants, AQPs are present as four subfamilies of intrinsic proteins: plasma membrane (PIP), tonoplast (TIP), small and basic (SIP) and NOD26-like (NIP). The total number of AQP members in plants, as compared to animals, appears to be surprisingly high [Maurel C., 2007 (Supra)]. For instance, 35 AQP genes have been identified in the Arabidopsis genome [Quigley F, et al., "From genome to function: the Arabidopsis aquaporins". Genome Biol. 2002, 3(1):RESEARCH0001.1-1.17], 36 in maize [Chaumont F, et al., 2001, "Aquaporins constitute a large and highly divergent protein family in maize. Plant Physiol", 125(3):1206-15], and 33 in rice [Sakurai, J., et a., 2005, Identification of 33 rice aquaporin genes and analysis of their expression and function. Plant Cell Physiol. 46, 1568-1577]. The high number of AQPs in plants suggests a diverse role and differential regulation under variable environmental conditions [Maurel C., 2007 (Supra)].

WO2004/104162 to the present inventors teaches polynucleotide sequences and methods of utilizing same for increasing the tolerance of a plant to abiotic stresses and/or increasing the biomass of a plant.

WO2007/020638 to the present inventors teaches polynucleotide sequences and methods of utilizing same for increasing the tolerance of a plant to abiotic stresses and/or increasing the biomass, vigor and/or yield of a plant.

Lian HL, et al., 2006 (Cell Res. 16: 651-60) over-expressed members of the PIP1 subgroup of AQPs in rice. Aharon R., et al. 2003 (Plant Cell, 15: 439-47) over-expressed the Arabidopsis plasma membrane aquaporin, PIP1b, in transgenic tobacco plants.

US 2003233670 provides polynucleotides and proteins encoded by the polypeptides. The disclosed polynucleotides and polypeptides find use in production of transgenic plants to produce plants having improved properties. It further provides methods of producing fertile transgenic plants, preferably maize, with desirable phenotypes and progeny of any generation derived from the fertile transgenic plants.

SUMMARY OF THE INVENTION

The present invention concerns a method of increasing abiotic stress tolerance of a plant as compared to a non-transformed plant of the same species which is grown under the same growth conditions, comprising over-expressing within the plant an exogenous polynucleotide encoding a polypeptide comprising an amino acid sequence at least 85 % homologous to the amino acid sequence set forth in SEQ ID NO: 33, wherein said polypeptide is capable of transporting water in a plant, thereby increasing the abiotic stress tolerance of the plant as compared to the non-transformed plant of the same species which is grown under the same growth conditions, wherein the abiotic stress condition is salinity stress.

Preferably, said exogenous polynucleotide comprises the nucleic acid sequence set forth in SEQ ID NO: 7 or 2757.

Preferably, said polypeptide comprises an amino acid sequence at least 90 % homologous to the amino acid sequence set forth in SEQ ID NO: 33.

Preferably, said polypeptide comprises an amino acid sequence at least 95 % homologous to the amino acid sequence set forth in SEQ ID NO: 33.

Preferably, said polypeptide comprises the amino acid sequence set forth by SEQ ID NO:33.

The method of the present invention further comprises growing the plant expressing said exogenous polynucleotide under the abiotic stress.

The method of the present invention wherein the plant is a dicotyledonous plant.

The method of the present invention wherein the plant is a monocotyledonous plant.

BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

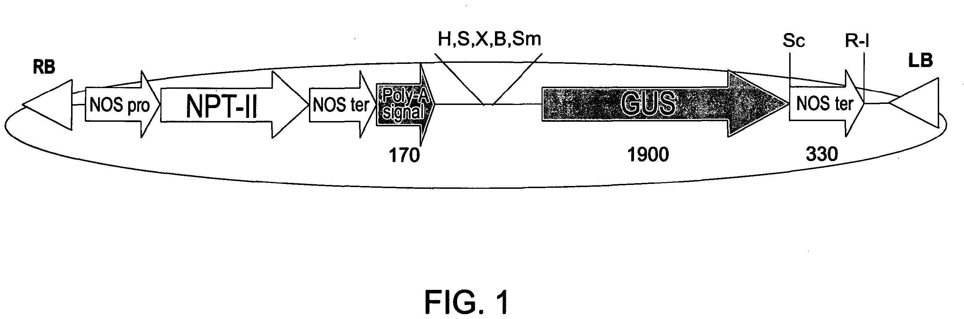

FIG. 1 is a schematic illustration of the pGI binary plasmid used for expressing the isolated polynucleotide sequences of the invention. RB - T-DNA right border; LB - T-DNA left border; H- *Hind*III restriction enzyme; X - *Xba*I restriction enzyme; B - *Bam*HI restriction enzyme; S - *Sal*I restriction enzyme; Sm - *Sma*I restriction enzyme; R-I - *Eco*RI restriction enzyme; Sc - *Sac*I/*Sst*I/*Ecl*136II; (numbers) - Length in base-pairs; NOS pro = nopaline synthase promoter; NPT-II = neomycin phosphotransferase gene; NOS ter = nopaline synthase terminator; Poly-A signal (polyadenylation signal); GUSintron - the GUS reporter gene (coding sequence and intron). The isolated polynucleotide sequences of some embodiments of the invention were cloned into the vector while replacing the GUSintron reporter gene.

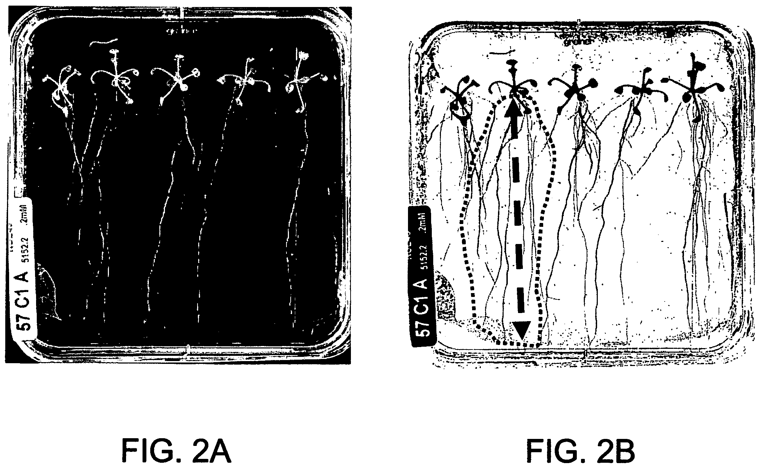

FIGs. 2A-B are images depicting root development of plants grown in transparent agar plates. The different transgenes were grown in transparent agar plates for 10-15 days and the plates were photographed every 2-5 days starting at day 1. FIG. 2A - An exemplary image of plants taken following 12 days on agar plates. FIG. 2B - An exemplary image of root analysis in which the length of the root measured is represented by a red arrow.

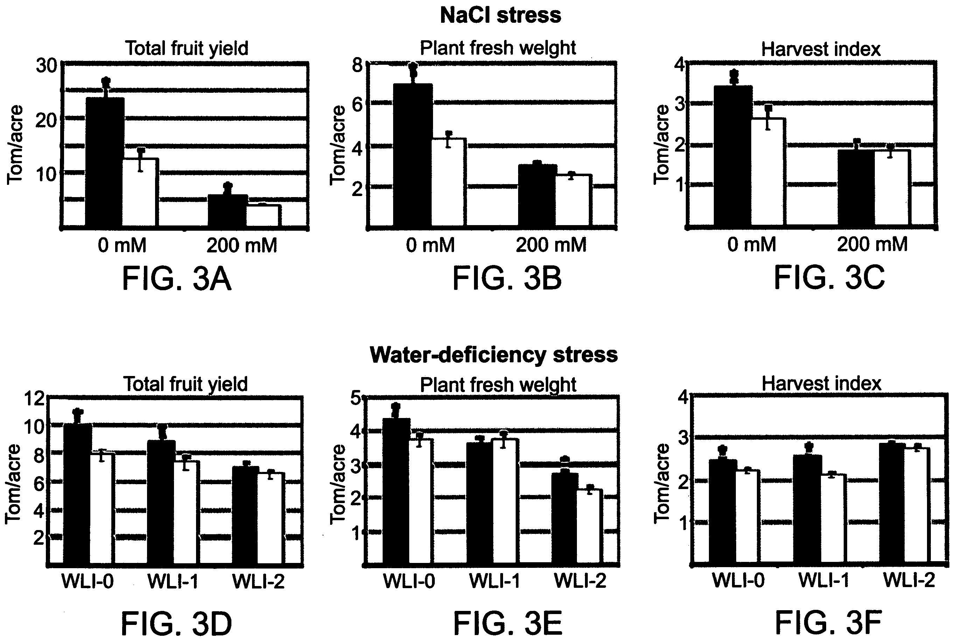

FIGs. 3A-F are histograms depicting the total economic fruit yield, plant biomass and harvest index for TOM-ABST36 (black bar) vs. control (white bar) plants growing in the commercial greenhouse under a 200 mM sodium chloride (NaCl) irrigation regime (FIG. 3A-C, respectively), or under two different water-stress regimes (WLI-1 and WLI-2; FIG. 3D-F, respectively). Yield performance was compared to plants growing under standard irrigation conditions (0 mM NaCl and WLI-0). Results are the average of the four independent events. *Significantly different at $P \leq 0.05$.

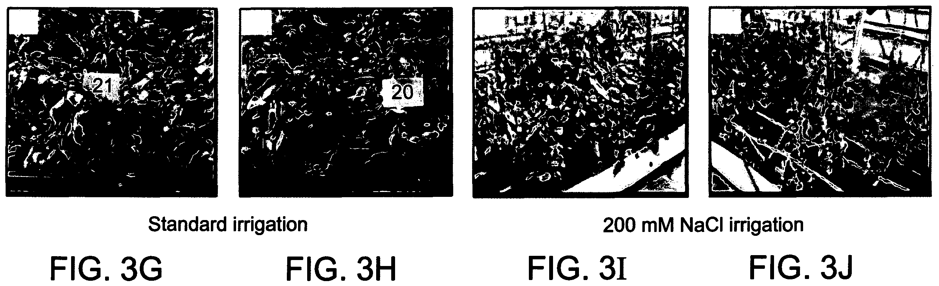

FIGs. 3G-J are photographs of transgenic tomato plants or control plants grown under various conditions. FIG. 3G - TOM-ABST36 plants growing under regular irrigation conditions; FIG. 3H - control plants growing under regular irrigation conditions; FIG. 3I - TOM-ABST36 plants after growing under a 200-mM NaCl-irrigation regime during the entire growing season; FIG. 3J - control plants after growing under a 200-mM NaCl-irrigation regime during the entire growing season.

DESCRIPTION OF SPECIFIC EMBODIMENTS OF THE INVENTION

The present disclosure, in some embodiments thereof, relates to novel aquaporin polynucleotides and polypeptides, and more particularly, but not exclusively, to methods of using same for increasing abiotic stress tolerance, water use efficiency, fertilizer use efficiency, biomass, vigor and/or yield of a plant.

While reducing the invention to practice, the present inventors have identified novel aquaporin (AQP) polynucleotides and polypeptides encoded thereby.

Thus, as shown in the Examples section which follows, the present inventors have employed a bioinformatics approach which combines digital expression analysis and cross-species comparative genomics and screened 7.2 million expressed sequence tags (ESTs) from 1,195 relevant EST's libraries of both monocot and dicot plant species. Using this approach 1,114 different AQP genes have been identified and were further classified to 11 subgroups (Table 1). Further analysis revealed that ESTs of the TIP2 subgroup are significantly over-represented in both plants' roots and in plants exposed to abiotic stress (ABS), and that polypeptides (e.g., SEQ ID NOs: 27-28, 45-48, Table 2) encoded by polynucleotides of the TIP2 subgroup (e.g., SEQ ID NOs:1, 2, 19-22, Table 2) share a common consensus sequence TLXFXFAGVGS (SEQ ID NO:2826). Based on over-representation in roots, ABS conditions and tissues with low water levels (such as seed and pollen) additional polynucleotides of the aquaporin gene family were identified (SEQ ID NOs: 3-18, 23-26, Table 2), as well as homologues or orthologues thereof (SEQ ID NOs:53-1400, 2844-3051 for polynucleotides and SEQ ID NOs:1401-2746, 3052-3259 for polypeptides; Table 3). Moreover, quantitative RT-PCR analysis demonstrated increased expression of representative AQP genes (e.g., SEQ ID NOs:5, 6 and 7) under salt stress, which was higher in plants exhibiting salt tolerance as compared to plants which are sensitive to salt stress (Table 5, Example 2 of the Examples section which follows). As is further described in Examples 3-4 of the Examples section which follows, representative AQP polynucleotides were cloned (Tables 7, 8 and 9) and transgenic plants over-expressing same were generated (Example 4). These plants were shown to exhibit increased tolerance to various abiotic stresses such as osmotic stress (Tables 10-14; Example 5) and salinity stress (Tables 30-44; Example 6), increased fertilizer use efficiency (under nitrogen limiting conditions, Tables 60-69, Example 7) and increased growth, biomass and yield under normal [Tables 15-29 (Example 5), 45-59 (Example 6)] or abiotic stress conditions conditions (Examples 5-8). Altogether, these results suggest the use of the AQP polynucleotides and polypeptides of the disclosure for increasing abiotic stress tolerance, water use efficiency, fertilizer use efficiency, biomass, vigor and/or yield of a plant.

It should be noted that polypeptides or polynucleotides which affect (e.g., increase) plant metabolism, growth, reproduction and/or viability under stress, can also affect the plant growth, biomass, yield and/or vigor under optimal conditions.

Thus, according to one aspect of the disclosure, there is provided a method of increasing abiotic stress tolerance, water use efficiency, fertilizer use efficiency, growth, biomass, yield and/or vigor of a plant. The method is effected by expressing within the plant an exogenous polynucleotide encoding a polypeptide comprising the amino acid consensus sequence TLXFXFAGVGS as set forth by SEQ ID NO:2826, wherein expression of the polypeptide promotes plants' biomass/ vigor and/or yield under normal or stress conditions.

It is suggested that the polypeptide's activity is structurally associated with the integrity of the above consensus sequence (SEQ ID NO:2826). In some embodiments of this aspect of the present disclosure, the activity is a water channel activity which typically resides in the vacuaolar membrane (tonoplast) and/or the plasma membrane of the plant cell and enables the transport of water and/or small neutral solutes such as urea, nitrates and carbon dioxide ($CO_2$) through the membrane.

The phrase "abiotic stress" as used herein refers to any adverse effect on metabolism, growth, reproduction and/or viability of a plant. Accordingly, abiotic stress can be induced by suboptimal environmental growth conditions such as, for example, salinity, water deprivation, flooding, freezing, low or high temperature, heavy metal toxicity, anaerobiosis, nutrient deficiency, atmospheric pollution or UV irradiation. The implications of abiotic stress are discussed in the Background section.

The phrase "abiotic stress tolerance" as used herein refers to the ability of a plant to endure an abiotic stress without suffering a substantial alteration in metabolism, growth, productivity and/or viability.

As used herein the phrase "water use efficiency (WUE)" refers to the level of organic matter produced per unit of water consumed by the plant, *i.e.,* the dry weight of a plant in relation to the plant's water use, e.g., the biomass produced per unit transpiration.

As used herein the phrase "fertilizer use efficiency" refers to the uptake, spread, absorbent, accumulation, relocation (within the plant) and use of one or more of the minerals and organic moieties absorbed from the soil, such as nitrogen, phosphates and/or potassium.

As used herein the phrase "plant biomass" refers to the amount (measured in grams of air-dry tissue) of a tissue produced from the plant in a growing season, which could also determine or affect the plant yield or the yield per growing area.

As used herein the phrase "plant yield" refers to the amount (as determined by weight/size) or quantity (numbers) of tissue produced per plant or per growing season. Hence increased yield could affect the economic benefit one can obtain from the plant in a certain growing area and/or growing time.

As used herein the phrase "plant vigor" refers to the amount (measured by weight) of tissue produced by the plant in a given time. Hence increase vigor could determine or affect the plant yield or the yield per growing time or growing area.

As used herein the term "increasing" refers to at least about 2 %, at least about 3 %, at least about 4 %, at least about 5 %, at least about 10 %, at least about 15 %, at least about 20 %, at least about 30 %, at least about 40 %, at least about 50 %, at least about 60 %, at least about 70 %, at least about 80 %, increase in plant abiotic stress tolerance, water use efficiency, fertilizer use efficiency, growth, biomass, yield and/or vigor as compared to a native plant [*i.e.,* a plant not modified with the biomolecules (polynucleotide or polypeptides) of the disclosure, e.g., a non-transformed plant of the same species which is grown under the same growth conditions).

As used herein, the phrase "exogenous polynucleotide" refers to a heterologous nucleic acid sequence which may not be naturally expressed within the plant or which overexpression in the plant is desired. The exogenous polynucleotide may be introduced into the plant in a stable or transient manner, so as to produce a ribonucleic acid (RNA) molecule and/or a polypeptide molecule. It should be noted that the exogenous polynucleotide may comprise a nucleic acid sequence which is identical or partially homologous to an endogenous nucleic acid sequence of the plant.

According to some embodiments of the disclosure, the exogenous polynucleotide of the disclosure encodes a polypeptide having an amino acid sequence at least about 60 %, at least about 65 %, at least about 70 %, at least about 75 %, at least about 80 %, at least about 81 %, at least about 82 %, at least about 83 %, at least about 84 %, at least about 85 %, at least about 86 %, at least about 87 %, at least about 88 %, at least about 89 %, at least about 90 %, at least about 91 %, at least about 92 %, at least about 93 %, at least about 94 %, at least about 95 %, at least about 96 %, at least about 97 %, at least about 98 %, at least about 99 %, or more say 100 % homologous to the amino acid sequence selected from the group consisting of SEQ ID NOs: 27-28, 45-48, 1401-1403, 1405-1435, 1437-1494, 1496-1542, 1544-1553, 1555-1559, 1561, 2449-2450, 2453-2458, 2460-2463, 2465-2481, 2483, 2484 and 2765.

Homology (e.g., percent homology) can be determined using any homology comparison software, including for example, the BlastP or TBLASTN software of the National Center of Biotechnology Information (NCBI) such as by using default parameters, when starting from a polypeptide sequence; or the tBLASTX algorithm (available via the NCBI) such as by using default parameters, which compares the six-frame conceptual translation products of a nucleotide query sequence (both strands) against a protein sequence database.

Homologous sequences include both orthologous and paralogous sequences. The term "paralogous" relates to gene-duplications within the genome of a species leading to paralogous genes. The term "orthologous" relates to homologous genes in different organisms due to ancestral relationship.

One option to identify orthologues in monocot plant species is by performing a reciprocal blast search. This may be done by a first blast involving blasting the sequence-of-interest against any sequence database, such as the publicly available NCBI database which may be found at: Hypertext Transfer Protocol://World Wide Web (dot) ncbi (dot) nlm (dot) nih (dot) gov. If orthologues in rice were sought, the sequence-of-interest would be blasted against, for example, the 28,469 full-length cDNA clones from Oryza sativa Nipponbare available at NCBI. The blast results may be filtered. The full-length sequences of either the filtered results or the non-filtered results are then blasted back (second blast) against the sequences of the organism from which the sequence-of-interest is derived. The results of the first and second blasts are then compared. An orthologue is identified when the sequence resulting in the highest score (best hit) in the first blast identifies in the second blast the query sequence (the original sequence-of-interest) as the best hit. Using the same rational a paralogue (homolog to a gene in the same organism) is found. In case of large sequence families, the ClustalW program may be used [Hypertext Transfer Protocol://World Wide Web (dot) ebi (dot) ac (dot) uk/Tools/clustalw2/index (dot) html], followed by a neighbor-joining tree (Hypertext Transfer Protocol://en (dot) wikipedia (dot) org/wiki/Neighbor-joining) which helps visualizing the clustering.

According to some embodiments of the disclosure, the exogenous polynucleotide encodes a polypeptide consisting of the amino acid sequence set forth by SEQ ID NO:27-28, 45-48, 1401-1403, 1405-1435, 1437-1494, 1496-1542, 1544-1553, 1555-1559, 1561, 2449-2450, 2453-2458, 2460-2463, 2465-2481, 2483, 2484 or 2765.

According to some embodiments of the disclosure the exogenous polynucleotide comprises a nucleic acid sequence which is at least about 60 %, at least about 65 %, at least about 70 %, at least about 75 %, at least about 80 %, at least about 81 %, at least about 82 %, at least about 83 %, at least about 84 %, at least about 85 %, at least about 86 %, at least about 87 %, at least about 88 %, at least about 89 %, at least about 90 %, at least about 91 %, at least about 92 %, at least about 93 %, at least about 93 %, at least about 94 %, at least about 95 %, at least about 96 %, at least about 97 %, at least about 98 %, at least about 99 %, e.g., 100 % identical to the nucleic acid sequence selected from the group consisting of SEQ ID NOs:1, 2, 19, 20-22, 53-55, 57-87, 89-141, 143-147, 149-195, 197-206, 208-212, 214, 1102-1103, 1106-1111, 1113-1116, 1118-1134, 1136, 2751-2752 and 2748-2750.

Identity (e.g., percent homology) can be determined using any homology comparison software, including for example, the BlastN software of the National Center of Biotechnology Information (NCBI) such as by using default parameters.

According to some embodiments of the disclosure the exogenous polynucleotide is set forth by SEQ ID NO:1, 2, 19, 20-22, 53-55, 57-87, 89-141, 143-147, 149-195, 197-206, 208-212, 214, 1102-1103, 1106-1111, 1113-1116, 1118-1134, 1136, 2751-2752, 2748-2749, or 2750.

Notwithstanding the above, additional AQP polynucleotides and polypeptides encoded thereby are contemplated by the present teachings.

According to some embodiments of the disclosure, the exogenous polynucleotide encodes a polypeptide having an amino acid sequence at least about 60 %, at least about 65 %, at least about 70 %, at least about 75 %, at least about 80 %, at least about 85 %, at least about 86 %, at least about 87 %, at least about 88 %, at least about 89 %, at least about 90 %, at least about 91 %, at least about 92 %, at least about 93 %, at least about 94 %, at least about 95 %, at least about 96 %, at least about 97 %, at least about 98 %, at least about 99 %, e.g., 100 % homologous to SEQ ID NO:33, 34, 30, 27-29, 31, 32, 35-52, 1401-1403, 1405-1435, 1437-1494, 1496-1542, 1544-1553, 1555-1559, 1561-1827, 1829-1866, 1868-2450, 2453-2458, 2460-2463, 2465-2481, 2483, 2485-2746, 2765-2769, 3052-3065, 3067-3258 or 3259.

According to some embodiments of the disclosure, the exogenous polynucleotide encodes a polypeptide consisting of the amino acid sequence set forth by SEQ ID NO:33, 34, 30, 27-29, 31, 32, 35-52, 1401-1403, 1405-1435, 1437-1494, 1496-1542, 1544-1553, 1555-1559, 1561-1827, 1829-1866, 1868-2450, 2453-2458, 2460-2463, 2465-2481, 2483, 2485-2746, 2765-2769, 3052-3065, 3067-3258 or 3259.

In an exemplary embodiment the exogenous polynucleotide does not encode a polypeptide having the amino acid sequence selected from the group consisting of SEQ ID NOs: 1828, 1867, 1404, 1436, 1495, 1543, 1554, 1560, 2451, 2452, 2459, 2464, 2482, 2484 and 3066.

According to some embodiments of the disclosure, the exogenous polynucleotide is at least at least about 60 %, least at least about 65 %, least at least about 70 %, least at least about 75 %least at least about 80 %, at least about 85 %, at least about 86 %, at least about 87 %, at least about 88 %, at least about 89 %, at least about 90 %, at least about 91 %, at least about 92 %, at least about 93 %, at least about 94 %, at least about 95 %, at least about 96 %, at least about 97 %, at least about 98 %, at least about 99 %, e.g., 100 % identical to SEQ ID NO:7, 8, 4, 1-3, 5, 6, 9-26, 53-55, 57-87, 89-147, 149-195, 197-206, 208-212, 214-480, 482-519, 521-1103, 1106-1111, 1113-1116, 1118-1134, 1136, 1138-1400, 2748-2764, 2843-2857, 2859-3050 or 3051.

According to some embodiments of the disclosure, the polynucleotide is set forth by SEQ ID NO:7, 8, 4, 1-3, 5, 6, 9-26, 53-55, 57-87, 89-147, 149-195, 197-206, 208-212, 214-480, 482-519, 521-1103, 1106-1111, 1113-1116, 1118-1134, 1136, 1138-1400, 2748-2764, 2843-2857, 2859-3050 or 3051.

In an exemplary embodiments the exogenous polynucleotide is not the polynucleotide set forth by SEQ ID NO: 481, 520, 56, 88, 148, 196, 207, 213, 1104, 1105, 1112, 1117, 1135, 1137 or 2858.

As used herein the term "polynucleotide" refers to a single or double stranded nucleic acid sequence which is isolated and provided in the form of an RNA sequence, a complementary polynucleotide sequence (cDNA), a genomic polynucleotide sequence and/or a composite polynucleotide sequences (e.g., a combination of the above).

As used herein the phrase "complementary polynucleotide sequence" refers to a sequence, which results from reverse transcription of messenger RNA using a reverse transcriptase or any other RNA dependent DNA polymerase. Such a sequence can be subsequently amplified *in vivo* or *in vitro* using a DNA dependent DNA polymerase.

As used herein the phrase "genomic polynucleotide sequence" refers to a sequence derived (isolated) from a chromosome and thus it represents a contiguous portion of a chromosome.

As used herein the phrase "composite polynucleotide sequence" refers to a sequence, which is at least partially complementary and at least partially genomic. A composite sequence can include some exonal sequences required to encode the polypeptide of the present disclosure, as well as some intronic sequences interposing therebetween. The intronic sequences can be of any source, including of other genes, and typically will include conserved splicing signal sequences. Such intronic sequences may further include cis acting expression regulatory elements.

According to some embodiments of the disclosure, the polynucleotide of the disclosure comprises no more than 5000 nucleic acids in length. According to some embodiments of the disclosure, the polynucleotide of the disclosure comprises no more than 4000 nucleic acids in length, e.g., no more than 3000 nucleic acids, e.g., no more than 2500 nucleic acids.

Nucleic acid sequences encoding the polypeptides of the present disclosure may be optimized for expression. A non-limiting example of an optimized nucleic acid sequence is provided in SEQ ID NO:2751, which encodes an optimized polypeptide comprising the amino acid sequence set forth by SEQ ID NO:27. Examples of such sequence modifications include, but are not limited to, an altered G/C content to more closely approach that typically found in the plant species of interest, and the removal of codons atypically found in the plant species commonly referred to as codon optimization.

The phrase "codon optimization" refers to the selection of appropriate DNA nucleotides for use within a structural gene or fragment thereof that approaches codon usage within the plant of interest. Therefore, an optimized gene or nucleic acid sequence refers to a gene in which the nucleotide sequence of a native or naturally occurring gene has been modified in order to utilize statistically-preferred or statistically-favored codons within the plant. The nucleotide sequence typically is examined at the DNA level and the coding region optimized for expression in the plant species determined using any suitable procedure, for example as described in Sardana et al. (1996, Plant Cell Reports 15:677-681). In this method, the standard deviation of codon usage, a measure of codon usage bias, may be calculated by first finding the squared proportional deviation of usage of each codon of the native gene relative to that of highly expressed plant genes, followed by a calculation of the average squared deviation. The formula used is: $1 SDCU = n = 1 N [(Xn - Yn) / Yn] 2 / N$, where Xn refers to the frequency of usage of codon n in highly expressed plant genes, where Yn to the frequency of usage of codon n in the gene of interest and N refers to the total number of codons in the gene of interest. A Table of codon usage from highly expressed genes of dicotyledonous plants is compiled using the data of Murray et al. (1989, Nuc Acids Res. 17:477-498).

One method of optimizing the nucleic acid sequence in accordance with the preferred codon usage for a particular plant cell type is based on the direct use, without performing any extra statistical calculations, of codon optimization Tables such as those provided on-line at the Codon Usage Database through the NIAS (National Institute of Agrobiological Sciences) DNA bank in Japan (Hypertext Transfer Protocol://World Wide Web (dot) kazusa (dot) or (dot) jp/codon/). The Codon Usage Database contains codon usage tables for a number of different species, with each codon usage Table having been statistically determined based on the data present in Genbank.

By using the above Tables to determine the most preferred or most favored codons for each amino acid in a particular species (for example, rice), a naturally-occurring nucleotide sequence encoding a protein of interest can be codon optimized for that particular plant species. This is effected by replacing codons that may have a low statistical incidence in the particular species genome with corresponding codons, in regard to an amino acid, that are statistically more favored. However, one or more less-favored codons may be selected to delete existing restriction sites, to create new ones at potentially useful junctions (5' and 3' ends to add signal peptide or termination cassettes, internal sites that might be used to cut and splice segments together to produce a correct full-length sequence), or to eliminate nucleotide sequences that may negatively effect mRNA stability or expression.

The naturally-occurring encoding nucleotide sequence may already, in advance of any modification, contain a number of codons that correspond to a statistically-favored codon in a particular plant species. Therefore, codon optimization of the native nucleotide sequence may comprise determining which codons, within the native nucleotide sequence, are not statistically-favored with regards to a particular plant, and modifying these codons in accordance with a codon usage table of the particular plant to produce a codon optimized derivative. A modified nucleotide sequence may be fully or partially optimized for plant codon usage provided that the protein encoded by the modified nucleotide sequence is produced at a level higher than the protein encoded by the corresponding naturally occurring or native gene. Construction of synthetic genes by altering the codon usage is described in for example PCT Patent Application 93/07278.

Thus, the disclosure encompasses nucleic acid sequences described hereinabove; fragments thereof, sequences hybridizable therewith, sequences homologous thereto, sequences encoding similar polypeptides with different codon usage, altered sequences characterized by mutations, such as deletion, insertion or substitution of one or more nucleotides, either naturally occurring or man induced, either randomly or in a targeted fashion.

As mentioned, the present inventors have uncovered previously uncharacterized polypeptides which share the amino acid consensus sequence set forth by SEQ ID NO:2826.

Thus, the disclosure provides an isolated polypeptide having an amino acid sequence at least about 60 %, at least about 65 %, at least about 70 %, at least about 75 %, at least about 80 %, at least about 81 %, at least about 82 %, at least about 83 %, at least about 84 %, at least about 85 %, at least about 86 %, at least about 87 %, at least about 88 %, at least about 89 %, at least about 90 %, at least about 91 %, at least about 92 %, at least about 93 %, at least about 93 %, at least about 94 %, at least about 95 %, at least about 96 %, at least about 97 %, at least about 98 %, at least about 99 %, or more say 100 % homologous to an amino acid sequence selected from the group consisting of SEQ ID NO: 27-28, 45-48, 1401-1403, 1405-1435, 1437-1494, 1496-1542, 1544-1553, 1555-1559, 1561, 2449-2450, 2453-2458, 2460-2463, 2465-2481, 2483, 2484 and 2765.

According to some embodiments of the disclosure, the disclosure provides an isolated polypeptide having an amino acid sequence at least about 60 %, at least about 65 %, at least about 70 %, at least about 75 %, at least about 80 %, at least about 81 %, at least about 82 %, at least about 83 %, at least about 84 %, at least about 85 %, at least about 86 %, at least about 87 %, at least about 88 %, at least about 89 %, at least about 90 %, at least about 91 %, at least about 92 %, at least about 93 %, at least about 93 %, at least about 94 %, at least about 95 %, at least about 96 %, at least about 97 %, at least about 98 %, at least about 99 %, or more say 100 % homologous to an amino acid sequence selected from the group consisting of SEQ ID NOs:33, 34, 30, 27-29, 31, 32, 35-52, 1401-1403, 1405-1435, 1437-1494, 1496-1542, 1544-1553, 1555-1559, 1561-1827, 1829-1866, 1868-2450, 2453-2458, 2460-2463, 2465-2481, 2483, 2485-2746, 2765-2769, 3052-3065 and 3067-3259.

According to some embodiments of the disclosure, the polypeptide is set forth by SEQ ID NO: 33, 34, 30, 27-29, 31, 32, 35-52, 1401-1403, 1405-1435, 1437-1494, 1496-1542, 1544-1553, 1555-1559, 1561-1827, 1829-1866, 1868-2450, 2453-2458, 2460-2463, 2465-2481, 2483, 2485-2746, 2765-2769, 3052-3065, 3067-3258 or 3259.

In an exemplary embodiment the polypeptide is not the polypeptide set forth by SEQ ID NO: 1828, 1867, 1404, 1436, 1495, 1543, 1554, 1560, 2451, 2452, 2459, 2464, 2482, 2484 or 3066.

The disclosure also encompasses fragments of the above described polypeptides and polypeptides having mutations, such as deletions, insertions or substitutions of one or more amino acids, either naturally occurring or man induced, either randomly or in a targeted fashion.

The term "'plant" as used herein encompasses whole plants, ancestors and progeny of the plants and plant parts, including seeds, shoots, stems, roots (including tubers), and plant cells, tissues and organs. The plant may be in any form including suspension cultures, embryos, meristematic regions, callus tissue, leaves, gametophytes, sporophytes, pollen, and microspores. Plants that are particularly useful in the methods of the disclosure include all plants which belong to the superfamily Viridiplantae, in particular monocotyledonous and dicotyledonous plants including a fodder or forage legume, ornamental plant, food crop, tree, or shrub selected from the list comprising Acacia spp., Acer spp., Actinidia spp., Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chacoomeles spp., Cinnamomum cassia, Coffea arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Dibeteropogon amplectens, Dioclea spp, Dolichos spp., Dorycnium rectum, Echinochloa pyramidalis, Ehraffia spp., Eleusine coracana, Eragrestis spp., Erythrina spp., Eucalypfus spp., Euclea schimperi, Eulalia vi/losa, Pagopyrum spp., Feijoa sellowlana, Fragaria spp., Flemingia spp, Freycinetia banksli, Geranium thunbergii, GinAgo biloba, Glycine javanica, Gliricidia spp, Gossypium hirsutum, Grevillea spp., Guibourtia coleosperma, Hedysarum spp., Hemaffhia altissima, Heteropogon contoffus, Hordeum vulgare, Hyparrhenia rufa, Hypericum erectum, Hypeffhelia dissolute, Indigo incamata, Iris spp., Leptarrhena pyrolifolia, Lespediza spp., Lettuca spp., Leucaena leucocephala, Loudetia simplex, Lotonus bainesli, Lotus spp., Macrotyloma axillare, Malus spp., Manihot esculenta, Medicago saliva, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativam, Podocarpus totara, Pogonarthria fleckii, Pogonaffhria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys vefficillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp., Vitis vinifera, Watsonia pyramidata, Zantedeschia aethiopica, Zea mays, amaranth, artichoke, asparagus, broccoli, Brussels sprouts, cabbage, canola, carrot, cauliflower, celery, collard greens, flax, kale, lentil, oilseed rape, okra, onion, potato, rice, soybean, straw, sugar beet, sugar cane, sunflower, tomato, squash tea, maize, wheat, barely, rye, oat, peanut, pea, lentil and alfalfa, cotton, rapeseed, canola, pepper, sunflower, tobacco, eggplant, eucalyptus, a tree, an ornamental plant, a perennial grass and a forage crop. Alternatively algae and other non-Viridiplantae can be used for the methods of the present disclosure.

According to some embodiments of the disclosure, the plant used by the method of the disclosure is a crop plant such as rice, maize, wheat, barley, peanut, potato, sesame, olive tree, palm oil, banana, soybean, sunflower, canola, sugarcane, alfalfa, millet, leguminosae (bean, pea), flax, lupinus, rapeseed, tobacco, poplar and cotton.

Expressing the exogenous polynucleotide of the disclosure within the plant can be effected by transforming one or more cells of the plant with the exogenous polynucleotide, followed by generating a mature plant from the transformed cells and cultivating the mature plant under conditions suitable for expressing the exogenous polynucleotide within the mature plant.

According to some embodiments of the disclosure, the transformation is effected by introducing to the plant cell a nucleic acid construct which includes the exogenous polynucleotide of some embodiments of the disclosure and at least one promoter capable of directing transcription of the exogenous polynucleotide in the plant cell. Further details of suitable transformation approaches are provided hereinbelow.

As used herein, the term "promoter" refers to a region of DNA which lies upstream of the transcriptional initiation site of a gene to which RNA polymerase binds to initiate transcription of RNA. The promoter controls where (e.g., which portion of a plant) and/or when (e.g., at which stage or condition in the lifetime of an organism) the gene is expressed.

Any suitable promoter sequence can be used by the nucleic acid construct of the present disclosure. Preferably the promoter is a constitutive promoter, a tissue-specific, or an abiotic stress-inducible promoter.

Suitable constitutive promoters include, for example, CaMV 35S promoter (SEQ ID NO:2825; Odell et al., Nature 313:810-812, 1985); Arabidopsis At6669 promoter (SEQ ID NO:2823; see PCT Publication No. WO04081173A2); maize Ubi 1 (Christensen et al., Plant Sol. Biol. 18:675-689, 1992); rice actin (McElroy et al., Plant Cell 2:163-171, 1990); pEMU (Last et al., Theor. Appl. Genet. 81:581-588, 1991); CaMV 19S (Nilsson et al., Physiol. Plant 100:456-462, 1997); GOS2 (de Pater et al, Plant J Nov;2(6):837-44, 1992); ubiquitin (Christensen et al, Plant Mol. Biol. 18: 675-689, 1992); Rice cyclophilin (Bucholz et al, Plant Mol Biol. 25(5):837-43, 1994); Maize H3 histone (Lepetit et al, Mol. Gen. Genet. 231: 276-285, 1992); Actin 2 (An et al, Plant J. 10(1);107-121, 1996) and Synthetic Super MAS (Ni et al., The Plant Journal 7: 661-76, 1995). Other constitutive promoters include those in U.S. Pat. Nos. 5,659,026, 5,608,149; 5.608,144;

5,604,121; 5.569,597: 5.466,785; 5,399,680; 5,268,463; and 5,608,142.

Suitable tissue-specific promoters include, but not limited to, leaf-specific promoters [such as described, for example, by Yamamoto et al., Plant J. 12:255-265, 1997; Kwon et al., Plant Physiol. 105:357-67, 1994; Yamamoto et al., Plant Cell Physiol. 35:773-778, 1994; Gotor et al., Plant J. 3:509-18, 1993; Orozco et al., Plant Mol. Biol. 23:1129-1138, 1993; and Matsuoka et al., Proc. Natl. Acad. Sci. USA 90:9586-9590, 1993], seed-preferred promoters [e.g., from seed specific genes (Simon, et al., Plant Mol. Biol. 5. 191, 1985; Scofield, et al., J. Biol. Chem. 262: 12202, 1987; Baszczynski, et al., Plant Mol. Biol. 14: 633, 1990), Brazil Nut albumin (Pearson' et al., Plant Mol. Biol. 18: 235- 245, 1992), legumin (Ellis, et al. Plant Mol. Biol. 10: 203-214, 1988), Glutelin (rice) (Takaiwa, et al., Mol. Gen. Genet. 208: 15-22, 1986; Takaiwa, et al., FEBS Letts. 221: 43-47, 1987), Zein (Matzke et al Plant Mol Biol, 143).323-32 1990), napA (Stalberg, et al, Planta 199: 515-519, 1996), Wheat SPA (Albanietal, Plant Cell, 9: 171- 184, 1997), sunflower oleosin (Cummins, etal., Plant Mol. Biol. 19: 873-876, 1992)], endosperm specific promoters [e.g., wheat LMW and HMW, glutenin-1 (Mol Gen Genet 216:81-90, 1989; NAR 17:461-2), wheat a, b and g gliadins (EMBO3:1409-15, 1984), Barley ltrl promoter, barley B1, C, D hordein (Theor Appl Gen 98:1253-62, 1999; Plant J 4:343-55, 1993; Mol Gen Genet 250:750- 60, 1996), Barley DOF (Mena et al, The Plant Journal, 116(1): 53- 62, 1998), Biz2 (EP99106056.7), Synthetic promoter (Vicente-Carbajosa et al., Plant J. 13: 629-640, 1998), rice prolamin NRP33, rice -globulin Glb-1 (Wu et al, Plant Cell Physiology 39(8) 885- 889, 1998), rice alpha-globulin REB/OHP-1 (Nakase et al. Plant Mol. Biol. 33: 513-S22, 1997), rice ADP-glucose PP (Trans Res 6:157-68, 1997), maize ESR gene family (Plant J 12:235-46, 1997), sorgum gamma- kafirin (PMB 32:1029-35, 1996)], embryo specific promoters [e.g., rice OSH1 (Sato et al, Proc. Nati. Acad. Sci. USA, 93: 8117-8122), KNOX (Postma-Haarsma ef al, Plant Mol. Biol. 39:257-71, 1999), rice oleosin (Wu et at, J. Biochem., 123:386, 1998)], and flower-specific promoters [e.g., AtPRP4, chalene synthase (chsA) (Van der Meer, et al., Plant Mol. Biol. 15, 95-109, 1990), LAT52 (Twell et al Mol. Gen Genet. 217:240-245; 1989), apetala- 3].

Suitable abiotic stress-inducible promoters include, but not limited to, salt-inducible promoters such as RD29A (Yamaguchi-Shinozalei et al., Mol. Gen. Genet. 236:331-340, 1993); drought-inducible promoters such as maize rab17 gene promoter (Pla et. al., Plant Mol. Biol. 21:259-266, 1993), maize rab28 gene promoter (Busk et. al., Plant J. 11:1285-1295, 1997) and maize lvr2 gene promoter (Pelleschi et. al., Plant Mol. Biol. 39:373-380, 1999); heat-inducible promoters such as heat tomato hsp80-promoter from tomato (U.S. Pat. No. 5,187,267).

The nucleic acid construct of some embodiments of the disclosure can further include an appropriate selectable marker and/or an origin of replication. According to some embodiments of the disclosure, the nucleic acid construct utilized is a shuttle vector, which can propagate both in E. coli (wherein the construct comprises an appropriate selectable marker and origin of replication) and be compatible with propagation in cells. The construct according to the present disclosure can be, for example, a plasmid, a bacmid, a phagemid, a cosmid, a phage, a virus or an artificial chromosome.

The nucleic acid construct of some embodiments of the disclosure can be utilized to stably or transiently transform plant cells. In stable transformation, the exogenous polynucleotide is integrated into the plant genome and as such it represents a stable and inherited trait. In transient transformation, the exogenous polynucleotide is expressed by the cell transformed but it is not integrated into the genome and as such it represents a transient trait.

There are various methods of introducing foreign genes into both monocotyledonous and dicotyledonous plants (Potrykus, I., Annu. Rev. Plant. Physiol., Plant. Mol. Biol. (1991) 42:205-225; Shimamoto et al., Nature (1989) 338:274-276).

The principle methods of causing stable integration of exogenous DNA into plant genomic DNA include two main approaches:

(i) Agrobacterium-mediated gene transfer: Klee et al. (1987) Annu. Rev. Plant Physiol. 38:467-486; Klee and Rogers in Cell Culture and Somatic Cell Genetics of Plants, Vol. 6, Molecular Biology of Plant Nuclear Genes, eds. Schell, J., and Vasil, L. K., Academic Publishers, San Diego, Calif. (1989) p. 2-25; Gatenby, in Plant Biotechnology, eds. Kung, S. and Arntzen, C. J., Butterworth Publishers, Boston, Mass. (1989) p. 93-112.

(ii) Direct DNA uptake: Paszkowski et al., in Cell Culture and Somatic Cell Genetics of Plants, Vol. 6, Molecular Biology of Plant Nuclear Genes eds. Schell, J., and Vasil, L. K., Academic Publishers, San Diego, Calif. (1989) p. 52-68; including methods for direct uptake of DNA into protoplasts, Toriyama, K. et al. (1988) Bio/Technology 6:1072-1074. DNA uptake induced by brief electric shock of plant cells: Zhang et al. Plant Cell Rep. (1988) 7:379-384. Fromm et al. Nature (1986) 319:791-793. DNA injection into plant cells or tissues by particle bombardment, Klein et al. Bio/Technology (1988) 6:559-563; McCabe et al. Bio/Technology (1988) 6:923-926; Sanford, Physiol. Plant. (1990) 79:206-209; by the use of micropipette systems: Neuhaus et al., Theor. Appl. Genet. (1987) 75:30-36; Neuhaus and Spangenberg, Physiol. Plant. (1990) 79:213-217; glass fibers or silicon carbide whisker transformation of cell cultures, embryos or callus tissue, U.S. Pat. No. 5,464,765 or by the direct incubation of DNA with germinating pollen, DeWet et al. in Experimental Manipulation of Ovule Tissue, eds. Chapman, G. P. and Mantell, S. H. and Daniels, W. Longman, London, (1985) p. 197-209; and Ohta, Proc. Natl. Acad. Sci. USA (1986) 83:715-719.

The Agrobacterium system includes the use of plasmid vectors that contain defined DNA segments that integrate into the plant genomic DNA. Methods of inoculation of the plant tissue vary depending upon the plant species and the Agrobacterium delivery system. A widely used approach is the leaf disc procedure which can be performed with any tissue explant that provides a good source for initiation of whole plant differentiation. See, e.g., Horsch et al. in Plant Molecular Biology Manual A5, Kluwer Academic Publishers, Dordrecht (1988) p. 1-9. A supplementary approach employs the Agrobacterium delivery system in combination with vacuum infiltration. The Agrobacterium system is especially viable in the creation of transgenic dicotyledonous plants.

There are various methods of direct DNA transfer into plant cells. In electroporation, the protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals or tungsten particles, and the microprojectiles are physically accelerated into cells or plant tissues.

Following stable transformation plant propagation is exercised. The most common method of plant propagation is by seed. Regeneration by seed propagation, however, has the deficiency that due to heterozygosity there is a lack of uniformity in the crop, since seeds are produced by plants according to the genetic variances governed by Mendelian rules. Basically, each seed is genetically different and each will grow with its own specific traits. Therefore, it is preferred that the transformed plant be produced such that the regenerated plant has the identical traits and characteristics of the parent transgenic plant. Therefore, it is preferred that the transformed plant be regenerated by micropropagation which provides a rapid, consistent reproduction of the transformed plants.

Micropropagation is a process of growing new generation plants from a single piece of tissue that has been excised from a selected parent plant or cultivar. This process permits the mass reproduction of plants having the preferred tissue expressing the fusion protein. The new generation plants which are produced are genetically identical to, and have all of the characteristics of, the original plant. Micropropagation allows mass production of quality plant material in a short period of time and offers a rapid multiplication of selected cultivars in the preservation of the characteristics of the original transgenic or transformed plant. The advantages of cloning plants are the speed of plant multiplication and the quality and uniformity of plants produced.

Micropropagation is a multi-stage procedure that requires alteration of culture medium or growth conditions between stages. Thus, the micropropagation process involves four basic stages: Stage one, initial tissue culturing; stage two, tissue culture multiplication; stage three, differentiation and plant formation; and stage four, greenhouse culturing and hardening. During stage one, initial tissue culturing, the tissue culture is established and certified contaminant-free. During stage two, the initial tissue culture is multiplied until a sufficient number of tissue samples are produced to meet production goals. During stage three, the tissue samples grown in stage two are divided and grown into individual plantlets. At stage four, the transformed plantlets are transferred to a greenhouse for hardening where the plants' tolerance to light is gradually increased so that it can be grown in the natural environment.

According to some embodiments of the disclosure, the transgenic plants are generated by transient transformation of leaf cells, meristematic cells or the whole plant.

Transient transformation can be effected by any of the direct DNA transfer methods described above or by viral infection using modified plant viruses.

Viruses that have been shown to be useful for the transformation of plant hosts include CaMV, Tobacco mosaic virus (TMV), brome mosaic virus (BMV) and Bean Common Mosaic Virus (BV or BCMV). Transformation of plants using plant viruses is described in U.S. Pat. No. 4,855,237 (bean golden mosaic virus; BGV), EP-A 67,553 (TMV), Japanese Published Application No. 63-14693 (TMV), EPA 194,809 (BV), EPA 278,667 (BV); and Gluzman, Y. et al., Communications in Molecular Biology: Viral Vectors, Cold Spring Harbor Laboratory, New York, pp. 172-189 (1988). Pseudovirus particles for use in expressing foreign DNA in many hosts, including plants are described in WO 87/06261.

According to some embodiments of the disclosure, the virus used for transient transformations is avirulent and thus is incapable of causing severe symptoms such as reduced growth rate, mosaic, ring spots, leaf roll, yellowing, streaking, pox formation, tumor formation and pitting. A suitable avirulent virus may be a naturally occurring avirulent virus or an artificially attenuated virus. Virus attenuation may be effected by using methods well known in the art including, but not limited to, sub-lethal heating, chemical treatment or by directed mutagenesis techniques such as described, for example, by Kurihara and Watanabe (Molecular Plant Pathology 4:259-269, 2003), Gal-on et al. (1992), Atreya et al. (1992) and Huet et al. (1994).

Suitable virus strains can be obtained from available sources such as, for example, the American Type culture Collection (ATCC) or by isolation from infected plants. Isolation of viruses from infected plant tissues can be effected by techniques well known in the art such as described, for example by Foster and Tatlor, Eds. "Plant Virology Protocols: From Virus Isolation to Transgenic Resistance (Methods in Molecular Biology (Humana Pr), Vol 81)", Humana Press, 1998. Briefly, tissues of an infected plant believed to contain a high concentration of a suitable virus, preferably young leaves and flower petals, are ground in a buffer solution (e.g., phosphate buffer solution) to produce a virus infected sap which can be used in subsequent inoculations.

Construction of plant RNA viruses for the introduction and expression of non-viral exogenous polynucleotide sequences in plants is demonstrated by the above references as well as by Dawson, W. O. et al., Virology (1989) 172:285-292; Takamatsu et al. EMBO J. (1987) 6:307-311; French et al. Science (1986) 231:1294-1297; Takamatsu et al. FEBS Letters (1990) 269:73-76; and U.S. Pat. No. 5,316,931.

When the virus is a DNA virus, suitable modifications can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA, which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

In one embodiment, a plant viral polynucleotide is provided in which the native coat protein coding sequence has been deleted from a viral polynucleotide, a non-native plant viral coat protein coding sequence and a non-native promoter, preferably the subgenomic promoter of the non-native coat protein coding sequence, capable of expression in the plant host, packaging of the recombinant plant viral polynucleotide, and ensuring a systemic infection of the host by the recombinant plant viral polynucleotide, has been inserted. Alternatively, the coat protein gene may be inactivated by insertion of the non-native polynucleotide sequence within it, such that a protein is produced. The recombinant plant viral polynucleotide may contain one or more additional non-native subgenomic promoters. Each non-native subgenomic promoter is capable of transcribing or expressing adjacent genes or polynucleotide sequences in the plant host and incapable of recombination with each other and with native subgenomic promoters. Non-native (foreign) polynucleotide sequences may be inserted adjacent the native plant viral subgenomic promoter or the native and a non-native plant viral subgenomic promoters if more than one polynucleotide sequence is included. The non-native polynucleotide sequences are transcribed or expressed in the host plant under control of the subgenomic promoter to produce the desired products.

In a second embodiment, a recombinant plant viral polynucleotide is provided as in the first embodiment except that the native coat protein coding sequence is placed adjacent one of the non-native coat protein subgenomic promoters instead of a non-native coat protein coding sequence.

In a third embodiment, a recombinant plant viral polynucleotide is provided in which the native coat protein gene is adjacent its subgenomic promoter and one or more non-native subgenomic promoters have been inserted into the viral polynucleotide. The inserted non-native subgenomic promoters are capable of transcribing or expressing adjacent genes in a plant host and are incapable of recombination with each other and with native subgenomic promoters. Non-native polynucleotide sequences may be inserted adjacent the non-native subgenomic plant viral promoters such that the sequences are transcribed or expressed in the host plant under control of the subgenomic promoters to produce the desired product.

In a fourth embodiment, a recombinant plant viral polynucleotide is provided as in the third embodiment except that the native coat protein coding sequence is replaced by a non-native coat protein coding sequence.

The viral vectors are encapsidated by the coat proteins encoded by the recombinant plant viral polynucleotide to produce a recombinant plant virus. The recombinant plant viral polynucleotide or recombinant plant virus is used to infect appropriate host plants. The recombinant plant viral polynucleotide is capable of replication in the host, systemic spread in the host, and transcription or expression of foreign gene(s) (exogenous polynucleotide) in the host to produce the desired protein.

Techniques for inoculation of viruses to plants may be found in Foster and Taylor, eds. "Plant Virology Protocols: From Virus Isolation to Transgenic Resistance (Methods in Molecular Biology (Humana Pr), Vol 81)", Humana Press, 1998; Maramorosh and Koprowski, eds. "Methods in Virology" 7 vols, Academic Press, New York 1967-1984; Hill, S.A. "Methods in Plant Virology", Blackwell, Oxford, 1984; Walkey, D.G.A. "Applied Plant Virology", Wiley, New York, 1985; and Kado and Agrawa, eds. "Principles and Techniques in Plant Virology", Van Nostrand-Reinhold, New York.

In addition to the above, the polynucleotide of the present disclosure can also be introduced into a chloroplast genome thereby enabling chloroplast expression.

A technique for introducing exogenous polynucleotide sequences to the genome of the chloroplasts is known. This technique involves the following procedures. First, plant cells are chemically treated so as to reduce the number of chloroplasts per cell to about one. Then, the exogenous polynucleotide is introduced via particle bombardment into the cells with the aim of introducing at least one exogenous polynucleotide molecule into the chloroplasts. The exogenous polynucleotides selected such that it is integratable into the chloroplast's genome via homologous recombination which is readily effected by enzymes inherent to the chloroplast. To this end, the exogenous polynucleotide includes, in addition to a gene of interest, at least one polynucleotide stretch which is derived from the chloroplast's genome. In addition, the exogenous polynucleotide includes a selectable marker, which serves by sequential selection procedures to ascertain that all or substantially all of the copies of the chloroplast genomes following such selection will include the exogenous polynucleotide. Further details relating to this technique are found in U.S. Pat. Nos. 4,945,050; and 5,693,507. A polypeptide can thus be produced by the protein expression system of the chloroplast and become integrated into the chloroplast's inner membrane.

Since abiotic stress tolerance, water use efficiency, fertilizer use efficiency, growth, biomass, yield and/or vigor in plants can involve multiple genes acting additively or in synergy (see, for example, in Quesda et al., Plant Physiol. 130:951-063, 2002), the present disclosure also envisages expressing a plurality of exogenous polynucleotides in a single host plant to thereby achieve superior effect on abiotic stress tolerance, water use efficiency, fertilizer use efficiency, growth, biomass, yield and/or vigor.

Expressing a plurality of exogenous polynucleotides in a single host plant can be effected by co-introducing multiple nucleic acid constructs, each including a different exogenous polynucleotide, into a single plant cell. The transformed cell can than be regenerated into a mature plant using the methods described hereinabove.

Alternatively, expressing a plurality of exogenous polynucleotides in a single host plant can be effected by co-introducing into a single plant-cell a single nucleic-acid construct including a plurality of different exogenous polynucleotides. Such a construct can be designed with a single promoter sequence which can transcribe a polycistronic messenger RNA including all the different exogenous polynucleotide sequences. To enable co-translation of the different polypeptides encoded by the polycistronic messenger RNA, the polynucleotide sequences can be inter-linked via an internal ribosome entry site (IRES) sequence which facilitates translation of polynucleotide sequences positioned downstream of the IRES sequence. In this case, a transcribed polycistronic RNA molecule encoding the different polypeptides described above will be translated from both the capped 5' end and the two internal IRES sequences of the polycistronic RNA molecule to thereby produce in the cell all different polypeptides. Alternatively, the construct can include several promoter sequences each linked to a different exogenous polynucleotide sequence.

The plant cell transformed with the construct including a plurality of different exogenous polynucleotides, can be regenerated into a mature plant, using the methods described hereinabove.

Alternatively, expressing a plurality of exogenous polynucleotides in a single host plant can be effected by introducing different nucleic acid constructs, including different exogenous polynucleotides, into a plurality of plants. The regenerated transformed plants can then be cross-bred and resultant progeny selected for superior abiotic stress tolerance, water use efficiency, fertilizer use efficiency, growth, biomass, yield and/or vigor traits, using conventional plant breeding techniques.

Thus, the disclosure encompasses plants exogenously expressing (as described above) the polynucleotide(s) and/or polypeptide(s) of the disclosure. Once expressed within the plant cell or the entire plant, the level of the polypeptide encoded by the exogenous polynucleotide can be determined by methods well known in the art such as, activity assays, Western blots using antibodies capable of specifically binding the polypeptide, Enzyme-Linked ImmunoSorbent Assay (ELISA), radio-immuno-assays (RIA), immunohistochemistry, immunocytochemistry, immunofluorescence and the like.

Methods of determining the level in the plant of the RNA transcribed from the exogenous polynucleotide are well known in the art and include, for example, Northern blot analysis, reverse transcription polymerase chain reaction (RT-PCR) analysis (including quantitative, semi-quantitative or real-time RT-PCR) and RNA-*in situ* hybridization.

As mentioned, the polypeptide according to some embodiments of the disclosure, functions as a water channel. Thus, the disclosure according to some embodiments encompasses functional equivalents of the polypeptide (e.g., polypeptides capable of the biological activity of a water channel) which can be identified by functional assays (e.g., being capable of transporting water in a plant) using e.g., a cell-swelling assay (Meng, Q. X. et al. 2008. Cell Physiol Biochem, 21. pp. 123-128).

The polynucleotides and polypeptides described hereinabove can be used in a wide range of economical plants, in a safe and cost effective manner.

The effect of the transgene (the exogenous polynucleotide encoding the polypeptide) on abiotic stress tolerance, water use efficiency, fertilizer use efficiency, growth, biomass, yield and/or vigor can be determined using known methods.

*Abiotic stress tolerance -* Transformed (*i.e.,* expressing the transgene) and non-transformed (wild type) plants are exposed to an abiotic stress condition, such as water deprivation, suboptimal temperature (low temperature, high temperature), nutrient deficiency, nutrient excess, a salt stress condition, osmotic stress, heavy metal toxicity, anaerobiosis, atmospheric pollution and UV irradiation.

*Salinity tolerance assay -* Transgenic plants with tolerance to high salt concentrations are expected to exhibit better germination, seedling vigor or growth in high salt. Salt stress can be effected in many ways such as, for example, by irrigating the plants with a hyperosmotic solution, by cultivating the plants hydroponically in a hyperosmotic growth solution (e.g., Hoagland solution), or by culturing the plants in a hyperosmotic growth medium [e.g., 50 % Murashige-Skoog medium (MS medium)]. Since different plants vary considerably in their tolerance to salinity, the salt concentration in the irrigation water, growth solution, or growth medium can be adjusted according to the specific characteristics of the specific plant cultivar or variety, so as to inflict a mild or moderate effect on the physiology and/or morphology of the plants (for guidelines as to appropriate concentration see, Bernstein and Kafkafi, Root Growth Under Salinity Stress In: Plant Roots, The Hidden Half 3rd ed. Waisel Y, Eshel A and Kafkafi U. (editors) Marcel Dekker Inc., New York, 2002, and reference therein).

For example, a salinity tolerance test can be performed by irrigating plants at different developmental stages with increasing concentrations of sodium chloride (for example 50 mM, 100 mM, 200 mM, 400 mM NaCl) applied from the bottom and from above to ensure even dispersal of salt. Following exposure to the stress condition the plants are frequently monitored until substantial physiological and/or morphological effects appear in wild type plants. Thus, the external phenotypic appearance, degree of wilting and overall success to reach maturity and yield progeny are compared between control and transgenic plants. Quantitative parameters of tolerance measured include, but are not limited to, the average wet and dry weight, the weight of the seeds yielded, the average seed size and the number of seeds produced per plant. Transformed plants not exhibiting substantial physiological and/or morphological effects, or exhibiting higher biomass than wild-type plants, are identified as abiotic stress tolerant plants.

*Osmotic tolerance test* - Osmotic stress assays (including sodium chloride and mannitol assays) are conducted to determine if an osmotic stress phenotype was sodium chloride-specific or if it was a general osmotic stress related phenotype. Plants which are tolerant to osmotic stress may have more tolerance to drought and/or freezing. For salt and osmotic stress germination experiments, the medium is supplemented for example with 50 mM, 100 mM, 200 mM NaCl or 100 mM, 200 mM NaCl, 400 mM mannitol. See also Example 5 of the Examples section which follows.

*Drought tolerance assay/Osmoticum assay* - Tolerance to drought is performed to identify the genes conferring better plant survival after acute water deprivation. To analyze whether the transgenic plants are more tolerant to drought, an osmotic stress produced by the non-ionic osmolyte sorbitol in the medium can be performed. Control and transgenic plants are germinated and grown in plant-agar plates for 4 days, after which they are transferred to plates containing 500 mM sorbitol. The treatment causes growth retardation, then both control and transgenic plants are compared, by measuring plant weight (wet and dry), yield, and by growth rates measured as time to flowering.

Conversely, soil-based drought screens are performed with plants overexpressing the polynucleotides detailed above. Seeds from control Arabidopsis plants, or other transgenic plants overexpressing the polypeptide of the disclosure are germinated and transferred to pots. Drought stress is obtained after irrigation is ceased accompanied by placing the pots on absorbent paper to enhance the soil-drying rate. Transgenic and control plants are compared to each other when the majority of the control plants develop severe wilting. Plants are re-watered after obtaining a significant fraction of the control plants displaying a severe wilting. Plants are ranked comparing to controls for each of two criteria: tolerance to the drought conditions and recovery (survival) following re-watering.

*Cold stress tolerance -* To analyze cold stress, mature (25 day old) plants are transferred to 4 °C chambers for 1 or 2 weeks, with constitutive light. Later on plants are moved back to greenhouse. Two weeks later damages from chilling period, resulting in growth retardation and other phenotypes, are compared between both control and transgenic plants, by measuring plant weight (wet and dry), and by comparing growth rates measured as time to flowering, plant size, yield, and the like.

*Heat stress tolerance -* Heat stress tolerance is achieved by exposing the plants to temperatures above 34 °C for a certain period. Plant tolerance is examined after transferring the plants back to 22 °C for recovery and evaluation after 5 days relative to internal controls (non-transgenic plants) or plants not exposed to neither cold or heat stress.

*Germination tests* - Germination tests compare the percentage of seeds from transgenic plants that could complete the germination process to the percentage of seeds from control plants that are treated in the same manner. Normal conditions are considered for example, incubations at 22 °C under 22-hour light 2-hour dark daily cycles. Evaluation of germination and seedling vigor is conducted between 4 and 14 days after planting. The basal media is 50 % MS medium (Murashige and Skoog, 1962 Plant Physiology 15, 473-497).

Germination is checked also at unfavorable conditions such as cold (incubating at temperatures lower than 10 °C instead of 22 °C) or using seed inhibition solutions that contain high concentrations of an osmolyte such as sorbitol (at concentrations of 50 mM, 100 mM, 200 mM, 300 mM, 500 mM, and up to 1000 mM) or applying increasing concentrations of salt (of 50 mM, 100 mM, 200 mM, 300 mM, 500 mM NaCl).

*Water use efficiency* - can be determined as the biomass produced per unit transpiration. To analyze WUE, leaf relative water content can be measured in control and transgenic plants. Fresh weight (FW) is immediately recorded; then leaves are soaked for 8 hours in distilled water at room temperature in the dark, and the turgid weight (TW) is recorded. Total dry weight (DW) is recorded after drying the leaves at 60 °C to a constant weight. Relative water content (RWC) is calculated according to the following Formula I:

$$\textit{Formula I}$$

$$(FW - DW/TW - DW) \times 100$$

*Fertilizer use efficiency* - To analyze whether the transgenic plants are more responsive to fertilizers, plants are grown in agar plates or pots with a limited amount of fertilizer, as described, for example, in Example 6, hereinbelow and in Yanagisawa et al (Proc Natl Acad Sci USA. 2004; 101:7833-8). The plants are analyzed for their overall size, time to flowering, yield, protein content of shoot and/or grain. The parameters checked are the overall size of the mature plant, its wet and dry weight, the weight of the seeds yielded, the average seed size and the number of seeds produced per plant. Other parameters that may be tested are: the chlorophyll content of leaves (as nitrogen plant status and the degree of leaf verdure is highly correlated), amino acid and the total protein content of the seeds or other plant parts such as leaves or shoots, oil content, etc. Similarly, instead of providing nitrogen at limiting amounts, phosphate or potassium can be added at increasing concentrations. Again, the same parameters measured are the same as listed above. In this way, nitrogen use efficiency (NUE), phosphate use efficiency (PUE) and potassium use efficiency (KUE) are assessed, checking the ability of the transgenic plants to thrive under nutrient restraining conditions.

*Nitrogen determination* - The procedure for N (nitrogen) concentration determination in the structural parts of the plants involves the potassium persulfate digestion method to convert organic N to $NO_3^-$ (Purcell and King 1996 Argon. J. 88:111-113, the modified $Cd^-$ mediated reduction of $NO_3^-$ to $NO_2^-$ (Vodovotz 1996 Biotechniques 20:390-394) and the measurement of nitrite by the Griess assay (Vodovotz 1996, supra). The absorbance values are measured at 550 nm against a standard curve of $NaNO_2$. The procedure is described in details in Samonte et al. 2006 Agron. J. 98:168-176.

*Grain protein concentration -* Grain protein content (g grain protein $m^{-2}$) is estimated as the product of the mass of grain N (g grain N $m^{-2}$) multiplied by the N/protein conversion ratio of k-5.13 (Mosse 1990, supra). The grain protein concentration is estimated as the ratio of grain protein content per unit mass of the grain (g grain protein $kg^{-1}$ grain).

*Oil content -* The oil content of a plant can be determined by extraction of the oil from the seed or the vegetative portion of the plant. Briefly, lipids (oil) can be removed from the plant (e.g., seed) by grinding the plant tissue in the presence of specific solvents (e.g., hexane or petroleum ether) and extracting the oil in a continuous extractor. Indirect oil content analysis can be carried out using various known methods such as Nuclear Magnetic Resonance (NMR) Spectroscopy, which measures the resonance energy absorbed by hydrogen atoms in the liquid state of the sample [See for example, Conway TF. and Earle FR., 1963, Journal of the American Oil Chemists' Society; Springer Berlin / Heidelberg, ISSN: 0003-021X (Print) 1558-9331 (Online)]; the Near Infrared (NI) Spectroscopy, which utilizes the absorption of near infrared energy (1100-2500 nm) by the sample; and a method described in WO/2001/023884, which is based on extracting oil a solvent, evaporating the solvent in a gas stream which forms oil particles, and directing a light into the gas stream and oil particles which forms a detectable reflected light.

The plant vigor can be calculated by the increase in growth parameters such as leaf area, fiber length, rosette diameter, plant fresh weight and the like per time.

The growth rate can be measured using digital analysis of growing plants. For example, images of plants growing in greenhouse on plot basis can be captured every 3 days and the rosette area can be calculated by digital analysis. Rosette area growth is calculated using the difference of rosette area between days of sampling divided by the difference in days between samples.

Measurements of seed yield can be done by collecting the total seeds from 8-16 plants together, weighting them using analytical balance and dividing the total weight by the number of plants. Seed per growing area can be calculated in the same manner while taking into account the growing area given to a single plant. Increase seed yield per growing area could be achieved by increasing seed yield per plant, and/or by increasing number of plants capable of growing in a given area.

Evaluation of the seed yield per plant can be done by measuring the amount (weight or size) or quantity (*i.e.,* number) of dry seeds produced and harvested from 8-16 plants and divided by the number of plants.

Evaluation of growth rate can be done by measuring plant biomass produced, rosette area, leaf size or root length per time (can be measured in $cm^2$ per day of leaf area).

Fiber length can be measured using fibrograph. The fibrograph system was used to compute length in terms of "Upper Half Mean" length. The upper half mean (UHM) is the average length of longer half of the fiber distribution. The fibrograph measures length in span lengths at a given percentage point (Hypertext Transfer Protocol://World Wide Web (dot) cottoninc (dot) com/ClassificationofCotton/?Pg=4#Length).

Thus, the present disclosure is of high agricultural value for promoting the yield of commercially desired crops (e.g., biomass of vegetative organ such as poplar wood, or reproductive organ such as number of seeds or seed biomass).

As used herein the term "about" refers to $\pm$ 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions illustrate some embodiments of the disclosure in a non limiting fashion.

Generally, the nomenclature used herein and the laboratory procedures utilized in the present disclosure include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

## EXAMPLE 1

## IDENTIFICATION OF AQP GENES USING DIGITAL EXPRESSION ANALYSIS AND CROSS-SPECIES COMPARATIVE GENOMIC

The large number of AQPs in plants and the contradictory results obtained when AQPs were overexpressed in plants demonstrate the need to selectively identify the AQP genes which can improve water use efficiency (WUE) in plants, lead to increased yield and biomass under abiotic stress as well as under favorable conditions.

Under unfavorable stress conditions, some biological activities of the plant are stopped or reduced, while others, not earlier active, initiate. Still, some of the activities, which are vital for plant survival, are maintained. One hypothesis is that key genes needed for plants to maintain vital activities under unfavorable conditions would be active under broad spectrum of biotic and abiotic stresses.

To test this hypothesis and to identify the key AQP genes having the potential to improve plant performance under different biotic and/or abiotic stress conditions (e.g., salt or drought stress) a combination of digital expression analysis (also known as Electronic Northern blot) and cross-species comparative genomics was performed. The database used was available from NCBI (Hypertext Transfer Protocol://World Wide Web (dot) ncbi (dot) nlm (dot) nih (dot) gov/dbEST/) and included 7.2 million expressed sequence tags (ESTs) from 1,195 relevant EST's libraries originated from 15 different species, including both monocot and dicot species, namely: Arabidopsis, barley, Brassica rapa, cotton, grape, maize, medicago, poplar, potato, rice, sorghum, soybean, sugarcane, tomato and wheat.

Tomato plants were selected as a model plant based on the high quality tomato database from several tomato species which can be used for data-mining and the present inventors' experience in using the tomato genome as a model plant. In addition, the relatively high salt tolerance exhibited by various tomato species makes the tomato genome an excellent candidate for identifying new stress tolerance mechanisms. Moreover, tomato is not only used as a model plant for genetic studies, it is also used as an important crop with well-defined yield parameters, which can be used to distinguish between genes affecting abiotic-stress tolerance and genes preventing yield loss under abiotic-stress conditions.

*Gene analysis and data mining* - For gene analysis and data mining the bioinformatic filtering approach used had three phases:

*1. Clustering and assembly:* EST and mRNA sequences of each of the 15 species were extracted from GenBank versions 157, 160, 161, 162, 164, 165, 166, clustered and assembled using Compugen's LEADS clustering and assembly platform (Compugen Ltd., Tel Aviv, Israel; Yelin et. al. 2003, Nature Biotechnology 21, 379-85). Automatically extracted EST library annotations were manually accurated and classified by anatomy, developmental stage, abiotic/biotic stress treatment and cultivars. The results were loaded into Oracle database. The predicted proteins were then annotated using InterPro(2) (Hypertext Transfer Protocol://World Wide Web (dot) ebi (dot) ac (dot) uk/interpro/).

*2. Selection of clusters* - All clusters that contained the Major intrinsic protein domain (IPR000425) were selected for further analysis (n = 1,114).

*3. Obtaining expression profile of the clusters -* By digital expression approach the expression profile of all clusters was obtained in terms of plant anatomy (*i.e.,* in what tissues/organs the gene was expressed), developmental stage (*i.e.,* the developmental stages at which a gene can be found) and profile of treatment (provides the physiological conditions under which a gene is expressed such as drought, cold, pathogen infection, etc).

Digital expression computations was calculated as follows: over-expression fold was computed as $m/(n* M/N)$, where "N" is total number of ESTs of specific organism; "M is number of ESTs in a given library/tissue/category; "n" is total number of ESTs in a given contig; "m" is the number of ESTs from the library/tissue/category in the contig; P-value was computed using Exact Fisher Test statistic. The combined P-value for over-expression in both Root and Abiotic stresses conditions was computed as $1 - (1-p1) \times (1-p2)$. 1,114 different AQP genes were identified in the inter species transcriptional databases. For the data mining process, the present inventors used a combination of two approaches: selection of AQP clusters showing significant over expression (EST distribution versus normal is more than two folds; statistical significance of over-expression - p Value < 0.05) either in roots compared to shoots or under various abiotic stresses (including drought, cold, salinity, heat, chemical treatments, etc.), compared to non stress control. It was found that ESTs of about 9 % of the AQP genes were significantly overrepresented in roots and 3.5 % of them were induced under different abiotic stresses. AQP genes which are highly overrepresented in roots were selected since plants with an efficient root system are expected to capture more water from a drying soil. In addition, AQP genes which are overrepresented in various abiotic stresses such as nutrient deficiency, heat, salinity and heavy metal stresses and biotic stresses such as application of elicitors and pathogens were selected considering that they can provide high tolerance to a wide spectrum of stresses.

The same set of 1,114 AQPs was classified according to the accepted groups known in the literature: first into the four major sub-groups: PIPs, TIPs, NIPs and SIPs, and a second classification divided these four sub-groups into eleven sub-groups according to their homology in amino acid sequences. A Fisher's exact test was then used to identify subgroups having significant EST over-presentation both in roots and upon exposure to different abiotic stresses. As shown in Table 1, hereinbelow, from the eleven subgroups, only the TIP2 subgroup showed a significant EST overrepresentation both in roots and upon exposure to abiotic stresses (P-value $1.7 \times 10^{-5}$ and $1.6 \times 10^{-3}$, respectively).

*Table 1*

| | AQP type distribution and over-expression in roots and abiotic stresses | | | | | | |
|---|---|---|---|---|---|---|---|
| | | Roots | | | Exposure to abiotic stresses | | |
| AQP type | Total No. of genes in database | No. of over-expressed genes | % over-expressed/all | P-value | No. of over-expressed genes | % over-expressed/all | P-value |
| PIP1 | 243 | 26 | 10.7 | 0.13 | 10 | 4.1 | 0.34 |
| PIP2 | 336 | 25 | 7.4 | 0.87 | 12 | 3.6 | 0.53 |
| PIP3 | 11 | 0 | 0.0 | 1 | 0 | 0 | 1 |
| SIP1 | 39 | 0 | 0.0 | 1 | 0 | 0 | 1 |
| SIP2 | 16 | 0 | 0.0 | 1 | 0 | 0 | 1 |
| TIP1 | 152 | 11 | 7.2 | 0.8 | 3 | 2 | 0.92 |
| TIP2 | 101 | 22 | 21.8 | 1.70E-05 | 10 | 9.9 | 1.6E-0.3 |
| TIP3 | 29 | 0 | 0.0 | 1 | 0 | 0 | 1 |
| TIP4 | 48 | 5 | 10.4 | 0.41 | 1 | 2.1 | 0.83 |
| TIP5 | 3 | 0 | 0.0 | 1 | 0 | 0 | 1 |
| NIP | 136 | 8 | 5.9 | 0.93 | 3 | 2.2 | 0.88 |
| Total | 1114 | 97 | | | 39 | | |
| Table 1. | | | | | | | |

These results suggest that over-expression and/or protein over-accumulation of the Tip2 subgroup can improve plant water use efficiency, ABST and yield.

*Genes of the Tip2 subgroup are highly expressed in roots and in abiotic stresses* - As shown in Table 1, hereinabove, the TIP2 subgroup (or subfamily) is highly expressed in roots and in abiotic stresses. The TIP2 subgroup is found in 38 plant species and other organisms (nucleic acid SEQ ID NOs: 1, 2, 19, 20-22; Table 2), available in public databases [Hypertext Transfer Protocol://World Wide Web (dot) ncbi (dot) nlm (dot) nih (dot) gov/dbEST/]. In tomato, the TIP2 gene was highly expressed in roots (6 fold, $p \leq 1.01$ E-24) and in both biotic (2 fold, $p \leq 4.6$ E-02) and abiotic stresses (4.5 fold, $p \leq$ E-02) (data not shown).

*Identification of a short consensus sequence of the Tip2 sub-family* - While comparing the consensus amino-acid sequences of Aquaporins, a short consensus sequence was identified which is unique to proteins of the Tip2 sub-family. The present inventors have suggested that this motif has an important role in managing water use efficiency (WUE), and when over-expressed in a plant can confer ABST and improved yield. The amino-acid consensus sequence identified is TLXFXFAGVGS (SEQ ID NO:2826), wherein X stands for any amino acid.

In addition, other genes of the aquaporin gene family were identified by bioinformatics tools as improving ABST and yield, based on combined digital gene expression profile in roots, tissues with low water levels (such as seed and pollen) and under abiotic stress conditions. These include SEQ ID NOs: 3-18, 23-26 (Table 2).

*Table 2*

| Identified Aquaporin Genes | | | | |
|---|---|---|---|---|
| *SEQ ID NO: (Polynucleotide)* | *Gene name* | *Cluster name* | *Organism* | *SEQ ID NO: (Polypeptide)* |
| 1 | MAB54 | tomato\|gb164\|BG125449 | tomato | 27 |
| 2 | MAB55 | tomato\|gb164\|BG134896 | tomato | 28 |
| 3 | MAB56 | tomato\|gb164\|AW218990 | tomato | 29 |
| 4 | MAB57 | tomato\|gb164\|AA824812 | tomato | 30 |
| 5 | MAB58 | tomato\|gb164\|BP881534 | tomato | 31 |
| 7 | MAB69 | tomato\|gb164\|AI637360 | tomato | 33 |
| 8 | MAB70 | tomato\|gb164\|BG133531 | tomato | 34 |
| 9 | MAB71 | tomato\|gb164\|BG629975 | tomato | 35 |
| 10 | MAB72 | tomato\|gb164\|BG136017 | tomato | 36 |
| 11 | MAB73 | tomato\|gb164\|BG131871 | tomato | 37 |
| 12 | MAB74 | tomato\|gb164\|AI775489 | tomato | 38 |
| 13 | MAB75 | tomato\|gb164\|BG136239 | tomato | 39 |
| 14 | MAB76 | tomato\|gb164\|BG134058 | tomato | 40 |
| 15 | MAB77 | tomato\|gb164\|BG629900 | tomato | 41 |
| 16 | MAB78 | tomato\|gb164\|BG130774 | tomato | 42 |
| 17 | MAB79 | tomato\|gb164\|BG124486 | tomato | 43 |
| 18 | MAB80 | tomato\|gb164\|AI483521 | tomato | 44 |
| 19 | MAB81 | tomato\|gb164\|CO751453 | tomato | 45 |
| 20 | MAB115 | barley\|gb157.2\|BF626376 | barley | 46 |
| 22 | MAB117 | barley\|gb157.2\|BE412516 | barley | 48 |
| 23 | MAB119 | tomato\|gb164\|BG134199 | tomato | 49 |
| 24 | MAB176 | tomato\|gb164\|CO635830 | tomato | 50 |
| 25 | MAB177 | tomato\|gb164\|CO751496 | tomato | 51 |
| 26 | MAB178 | tomato\|gb164\|CO751374 | tomato | 52 |

**Table 2.**

Sequences which are homologous [showing at least 80 % protein sequence identity on 80 % of the global hit or query length, as calculated using BlastP and tBlastN algorithms of the National Center of Biotechnology Information (NCBI)] or orthologues of the AQP genes described in Table 2, and are expected to possess the same role in ABST and yield improvement in plants, are disclosed in Table 3 hereinbelow (SEQ ID NOs:6, 215-1101 and 1138-1400; Table 3). In addition, Table 3 also includes homologous and orthologues of the AQP TIP2 subfamily (SEQ ID NOs:21, 53-214, 1102-1137) and additional homologous and orthologues (SEQ ID NOs:2844-3051).

Table 3

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| | Polynucleotide and polypeptide sequences of AQP homologous and orthologous | | | | | | | |
| 53 | apple\|gb157.3\|C N883304_T1 | apple | 1401 | 27 | 84 | 92.3387 097 | 100 | blastp |
| 54 | apple\|gb157.3\|C N489003_T1 | apple | 1402 | 27 | 83 | 100 | 100 | blastp |
| 55 | aquilegia\|gb157. 3\|DR915168_T1 | aquilegia | 1403 | 27 | 85 | 100 | 100 | blastp |
| 56 | arabidopsis\|gb16 5\|AT3G16240_T 1 | arabidopsis | 1404 | 27 | 81 | 99.1935 484 | 98.8 | blastp |
| 57 | artemisia\|gb164\| EY035829_T1 | artemisia | 1405 | 27 | 80 | 98.7903 226 | 99.1902 834 | blastp |
| 58 | artemisia\|gb164\| EY113320_T1 | artemisia | 1406 | 27 | 85 | 62.9032 258 | 100 | blastp |
| 59 | artemisia\|gb164\| EY070770_T1 | artemisia | 1407 | 27 | 81 | 98.7903 226 | 99.1902 834 | blastp |
| 60 | avocado\|gb164\|C V002132_T1 | avocado | 1408 | 27 | 84 | 50 | 100 | blastp |
| 61 | b_juncea\|gb164\| EVGN00333108 491419_T1 | b_juncea | 1409 | 27 | 83 | 100 | 100 | blastp |
| 62 | b_juncea\|gb164\| EVGN00503709 641655_T1 | b_juncea | 1410 | 27 | 82 | 53.6290 323 | 97.0588 235 | blastp |
| 63 | b_juncea\|gb164\| EVGN01003711 220829_T1 | b_juncea | 1411 | 27 | 84 | 63.7096 774 | 100 | blastp |
| 64 | b_oleracea\|gb16 1\|AM059585_T1 | b_olerac ea | 1412 | 27 | 84 | 100 | 100 | blastp |
| 65 | b_oleracea\|gb16 1\|AM385334_T1 | b_olerac ea | 1413 | 27 | 82 | 100 | 100 | blastp |
| 66 | b_oleracea\|gb16 1\|AM385915_T1 | b_oleracea | 1414 | 27 | 81 | 100 | 100 | blastp |
| 67 | b_rapa\|gb162\|B G543171_T1 | b_rapa | 1415 | 27 | 82 | 100 | 100 | blastp |
| 68 | b_rapa\|gb162\|B G543223_T1 | b_rapa | 1416 | 27 | 83 | 100 | 100 | blastp |
| 69 | b_rapa\|gb162\|L3 7478_T1 | b_rapa | 1417 | 27 | 81 | 100 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| | **Polynucleotide and polypeptide sequences of AQP homologous and orthologous** | | | | | | | |
| 70 | banana\|gb160\|D N238689_T1 | banana | 1418 | 27 | 83 | 76.6129 032 | 100 | blastp |
| 71 | bean\|gb164\|CB5 40614_T1 | bean | 1419 | 27 | 83 | 98.7903 226 | 98.7903 226 | blastp |
| 72 | canola\|gb161\|EV 092237_T1 | canola | 1420 | 27 | 84 | 100 | 100 | blastp |
| 73 | canola\|gb161\|CN 828178_T1 | canola | 1421 | 27 | 81 | 100 | 100 | blastp |
| 74 | canola\|gb161\|CX 188169_T1 | canola | 1422 | 27 | 82 | 100 | 100 | blastp |
| 75 | canola\|gb161\|CD 840590_T1 | canola | 1423 | 27 | 81 | 100 | 100 | blastp |
| 76 | cassava\|gb164\|C K650415_T1 | cassava | 1424 | 27 | 85 | 100 | 100 | blastp |
| 77 | castorbean\|gb16 0\|EE254645_T1 | castorbean | 1425 | 27 | 87 | 100 | 100 | blastp |
| 78 | centaurea\|gb161\| EH725826_T1 | centaurea | 1426 | 27 | 84 | 95.9677 419 | 84.3971 631 | blastp |
| 79 | centaurea\|gb161\| EL932474_T1 | centaurea | 1427 | 27 | 88 | 89.1129 032 | 97.7876 106 | blastp |
| 80 | cherry\|gb157.2\|E E488049_T1 | cherry | 1428 | 27 | 80 | 69.3548 387 | 100 | blastp |
| 81 | cichorium\|gb161 \|DT211633_T1 | cichorium | 1429 | 27 | 80 | 100 | 92.8571 429 | blastp |
| 82 | cichorium\|gb161 \|EH672622_T1 | cichorium | 1430 | 27 | 87 | 100 | 100 | blastp |
| 83 | citrus\|gb157.2\|C X663669_T1 | citrus | 1431 | 27 | 88 | 98.7903 226 | 99.1902 834 | blastp |
| 84 | citrus\|gb157.2\|C F417983_T1 | citrus | 1432 | 27 | 88 | 98.7903 226 | 99.1902 834 | blastp |
| 85 | citrus\|gb157.2\|C K665344_T1 | citrus | 1433 | 27 | 88 | 98.7903 226 | 99.1902 834 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| **Polynucleotide and polypeptide sequences of AQP homologous and orthologous** | | | | | | | | |
| 86 | citrus\|gb157.2\|C K665344 T2 | citrus | 1434 | 27 | 87 | 82.2580 645 | 100 | blastp |
| 87 | clover\|gb162\|BB 908328_T1 | clover | 1435 | 27 | 81 | 69.7580 645 | 100 | blastp |
| 88 | cotton\|gb164\|AF 009567_T1 | cotton | 1436 | 27 | 87 | 100 | 100 | blastp |
| 89 | cowpea\|gb166\|F F397761_T1 | cowpea | 1437 | 27 | 84 | 100 | 100 | blastp |
| 90 | cowpea\|gb166\|F C457059_T1 | cowpea | 1438 | 27 | 83 | 98.7903 226 | 98.7903 226 | blastp |
| 91 | dandelion\|gb161\| DY818755_T1 | dandelion | 1439 | 27 | 85 | 100 | 100 | blastp |
| 92 | ginger\|gb164\|DY 358186_T1 | ginger | 1440 | 27 | 80 | 98.3870 968 | 99.1836 735 | blastp |
| 93 | ginger\|gb164\|DY 351866_T1 | ginger | 1441 | 27 | 80 | 98.3870 968 | 99.1836 735 | blastp |
| 94 | iceplant\|gb164\|A F133532_T1 | iceplant | 1442 | 27 | 81 | 100 | 100 | blastp |
| 95 | ipomoea\|gb157.2 \|BJ576630_T1 | ipomoea | 1443 | 27 | 87 | 100 | 100 | blastp |
| 96 | lettuce\|gb157.2\| DW074363_T1 | lettuce | 1444 | 27 | 87 | 100 | 100 | blastp |
| 97 | lettuce\|gb157.2\| DW074363 T2 | lettuce | 1445 | 27 | 85 | 50.8064 516 | 90.6474 82 | blastp |
| 98 | lettuce\|gb157.2\| DW145132_T1 | lettuce | 1446 | 27 | 87 | 100 | 100 | blastp |
| 99 | lettuce\|gb157.2\| DW043760_T1 | lettuce | 1447 | 27 | 87 | 100 | 100 | blastp |
| 100 | lettuce\|gb157.2\| DW104999_T1 | lettuce | 1448 | 27 | 87 | 100 | 100 | blastp |
| 101 | lotus\|gb157.2\|BI 420407_T1 | lotus | 1449 | 27 | 84 | 100 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 102 | lotus\|gb157.2\|BF 177457_T1 | lotus | 1450 | 27 | 86 | 98.7903226 | 98.7951807 | blastp |
| 103 | medicago\|gb157. 2\|AI974300_T1 | medicago | 1451 | 27 | 83 | 89.1129032 | 94.8497854 | blastp |
| 104 | medicago\|gb157. 2\|AA660400_T1 | medicago | 1452 | 27 | 84 | 100 | 100 | blastp |
| 105 | nicotiana_bentha miana\|gb162\|CN 741988_T1 | nicotiana_benthamiana | 1453 | 27 | 86 | 98.7903226 | 98.7903226 | blastp |
| 106 | nicotiana_bentha miana\|gb162\|CN 655366_T1 | nicotiana_benthamiana | 1454 | 27 | 92 | 100 | 100 | blastp |
| 107 | nicotiana_bentha miana\|gb162\|CN 741998_T1 | nicotiana_benthamiana | 1455 | 27 | 89 | 98.7903226 | 98.7903226 | blastp |
| 108 | nicotiana_bentha miana\|gb162\|CN 742343_T1 | nicotiana_bentha miana | 1456 | 27 | 93 | 100 | 100 | blastp |
| 109 | peach\|gb157.2\|B U045214_T1 | peach | 1457 | 27 | 82 | 100 | 100 | blastp |
| 110 | pepper\|gb157.2\| CO776446_T1 | pepper | 1458 | 27 | 84 | 61.6935484 | 100 | blastp |
| 111 | pepper\|gb157.2\| CA518313_T1 | pepper | 1459 | 27 | 86 | 63.3064516 | 100 | blastp |
| 112 | periwinkle\|gb16 4\|EG555051_T1 | periwinkle | 1460 | 27 | 88 | 99.1935484 | 99.1935484 | blastp |
| 113 | petunia\|gb157.2\| CV296219_T1 | petunia | 1461 | 27 | 84 | 63.7096774 | 85.2517986 | tblastn |
| 114 | poplar\|gb157.2\|B 1129443_T1 | poplar | 1462 | 27 | 86 | 100 | 100 | blastp |
| 115 | poplar\|gb157.2\|A 1166943 T2 | poplar | 1463 | 27 | 83 | 61.6935484 | 100 | blastp |
| 116 | poplar\|gb157.2\|A 1166943_T1 | poplar | 1464 | 27 | 85 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 117 | poplar\|gb157.2\|B 1127662_T1 | poplar | 1465 | 27 | 89 | 79.0322 581 | 100 | blastp |
| 118 | potato\|gb157.2\|B Q513382_T1 | potato | 1466 | 27 | 97 | 100 | 100 | blastp |
| 119 | potato\|gb157.2\|B Q513382_T2 | potato | 1467 | 27 | 97 | 50.8064 516 | 96.1832 061 | blastp |
| 120 | radish\|gb164\|EV 538411_T1 | radish | 1468 | 27 | 82 | 92.3387 097 | 100 | blastp |
| 121 | radish\|gb164\|AB 010416_T1 | radish | 1469 | 27 | 81 | 100 | 100 | blastp |
| 122 | radish\|gb164\|EW 725945_T1 | radish | 1470 | 27 | 82 | 100 | 100 | blastp |
| 123 | radish\|gb164\|EV 527946_T1 | radish | 1471 | 27 | 81 | 100 | 100 | blastp |
| 124 | rose\|gb157.2\|BQ 104096_T1 | rose | 1472 | 27 | 85 | 85.0806 452 | 95.0450 45 | blastp |
| 125 | safflower\|gb162\| EL374001_T1 | safflower | 1473 | 27 | 87 | 100 | 100 | blastp |
| 126 | safflower\|gb162\| EL406178_T1 | safflower | 1474 | 27 | 83 | 100 | 100 | blastp |
| 127 | sesame\|gb157.2\| BU668161_T1 | sesame | 1475 | 27 | 81 | 61.2903 226 | 86.3636 364 | tblastn |
| 128 | soybean\|gb166\|A W349399_T1 | soybean | 1476 | 27 | 84 | 98.7903 226 | 98.7903 226 | blastp |
| 129 | soybean\|gb166\|C D416937_T1 | soybean | 1477 | 27 | 85 | 75.4032 258 | 100 | blastp |
| 130 | soybean\|gb166\|C A786095_T1 | soybean | 1478 | 27 | 84 | 98.7903 226 | 98.7903 226 | blastp |
| 131 | soybean\|gb166\|A W350817_T1 | soybean | 1479 | 27 | 81 | 100 | 100 | blastp |
| 132 | spruce\|gb162\|CO 216479_T1 | spruce | 1480 | 27 | 80 | 98.7903 226 | 98.4 | blastp |
| 133 | strawberry\|gb16 4\|DV438565_T1 | strawberry | 1481 | 27 | 82 | 100 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 134 | sunflower\|gb162\| X95952_T1 | sunflower | 1482 | 27 | 82 | 100 | 100 | blastp |
| 135 | sunflower\|gb162\| CD847513_T1 | sunflower | 1483 | 27 | 83 | 100 | 100 | blastp |
| 136 | sunflower\|gb162\| CD845750_T1 | sunflower | 1484 | 27 | 84 | 100 | 100 | blastp |
| 137 | sunflower\|gb162\| CD848081_T1 | sunflower | 1485 | 27 | 86 | 100 | 100 | blastp |
| 138 | sunflower\|gb162\| CD849577_T1 | sunflower | 1486 | 27 | 83 | 100 | 100 | blastp |
| 139 | tobacco\|gb162\|C V016921_T1 | tobacco | 1487 | 27 | 88 | 100 | 100 | blastp |
| 140 | tobacco\|gb162\|C V018684_T1 | tobacco | 1488 | 27 | 93 | 100 | 100 | blastp |
| 141 | tobacco\|gb162\|C V019641_T1 | tobacco | 1489 | 27 | 91 | 100 | 100 | blastp |
| 142 | tomato\|gb164\|A W626247_T1 | tomato | No predicted protein | 27 | 83 | 50 | 88.3610451 | tblastn |
| 143 | triphysaria\|gb16 4\|EX999390_T1 | triphysaria | 1490 | 27 | 81 | 100 | 100 | blastp |
| 144 | triphysaria\|gb16 4\|BM356478_T1 | triphysaria | 1491 | 27 | 80 | 100 | 100 | blastp |
| 145 | triphysaria\|gb16 4\|BM356478_T2 | triphysaria | 1492 | 27 | 81 | 81.0483871 | 98.0487805 | blastp |
| 146 | aquilegia\|gb157. 3\|DR922172_T1 | aquilegia | 1493 | 28 | 86 | 100 | 100 | blastp |
| 147 | arabidopsis\|gb16 5\|AT5G47450_T 1 | arabidopsis | 1494 | 28 | 82 | 98.8 | 98.8 | blastp |
| 148 | arabidopsis\|gb16 5\|AT4G17340_T 1 | arabidopsis | 1495 | 28 | 85 | 99.6 | 99.6 | blastp |
| 149 | artemisia\|gb164\| EY080612_T1 | artemisia | 1496 | 28 | 83 | 69.2 | 100 | blastp |
| 150 | b_rapa\|gb162\|E X104899_T1 | b_rapa | 1497 | 28 | 84 | 95.2 | 99.1666667 | blastp |
| 151 | canola\|gb161\|EE 430505_T1 | canola | 1498 | 28 | 85 | 95.2 | 94.8207171 | blastp |
| 152 | canola\|gb161\|D Y017904_T1 | canola | 1499 | 28 | 82 | 98.8 | 98.8 | blastp |
| 153 | canola\|gb161\|EL 590702_T1 | canola | 1500 | 28 | 84 | 99.6 | 99.6 | blastp |
| 154 | canola\|gb161\|CD 818320_T1 | canola | 1501 | 28 | 85 | 99.6 | 99.6 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 155 | cassava\|gb164\|D B923860_T1 | cassava | 1502 | 28 | 81 | 100 | 100 | blastp |
| 156 | centaurea\|gb161\| EL932179_T1 | centaurea | 1503 | 28 | 84 | 98.8 | 99.1935 484 | blastp |
| 157 | centaurea\|gb161\| EH718862_T1 | centaurea | 1504 | 28 | 82 | 87.6 | 88.9795 918 | blastp |
| 158 | cichorium\|gb161\| EH689841_T1 | cichorium | 1505 | 28 | 86 | 88.8 | 64.7230 321 | tblastn |
| 159 | citrus\|gb157.2\|C 0913277_T1 | citrus | 1506 | 28 | 84 | 100 | 100 | blastp |
| 160 | citrus\|gb157.2\|C 0912449_T1 | citrus | 1507 | 28 | 82 | 95.6 | 75.2360 965 | tblastn |
| 161 | cotton\|gb164\|DV 437956_T1 | cotton | 1508 | 28 | 88 | 50.8 | 100 | blastp |
| 162 | cotton\|gb164\|CD 486503_T1 | cotton | 1509 | 28 | 86 | 100 | 100 | blastp |
| 163 | dandelion\|gb161\| DY827614_T1 | dandelion | 1510 | 28 | 84 | 100 | 100 | blastp |
| 164 | dandelion\|gb161\| DY822865_T1 | dandelion | 1511 | 28 | 86 | 100 | 100 | blastp |
| 165 | dandelion\|gb161\| DY819043_T1 | dandelion | 1512 | 28 | 86 | 100 | 100 | blastp |
| 166 | iceplant\|gb164\|A F133533_T1 | iceplant | 1513 | 28 | 82 | 82 | 99.5145 631 | blastp |
| 167 | lettuce\|gb157.2\| DW057721_T1 | lettuce | 1514 | 28 | 85 | 100 | 100 | blastp |
| 168 | lettuce\|gb157.2\| DW045203_T1 | lettuce | 1515 | 28 | 85 | 100 | 100 | blastp |
| 169 | lettuce\|gb157.2\| DW046133_T1 | lettuce | 1516 | 28 | 84 | 100 | 100 | blastp |
| 170 | lettuce\|gb157.2\| DW080742_T1 | lettuce | 1517 | 28 | 85 | 85.6 | 100 | blastp |
| 171 | lettuce\|gb157.2\| DW075611_T1 | lettuce | 1518 | 28 | 86 | 100 | 100 | blastp |
| 172 | lettuce\|gb157.2\| DW123899_T1 | lettuce | 1519 | 28 | 86 | 100 | 100 | blastp |
| 173 | lettuce\|gb157.2\| DW103468_T1 | lettuce | 1520 | 28 | 84 | 100 | 100 | blastp |
| 174 | lettuce\|gb157.2\| DW161237_T1 | lettuce | 1521 | 28 | 85 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 175 | lettuce\|gb157.2\| DW079798_T1 | lettuce | 1522 | 28 | 85 | 100 | 100 | blastp |
| 176 | lettuce\|gb157.2\| DW079554_T1 | lettuce | 1523 | 28 | 85 | 100 | 100 | blastp |
| 177 | lettuce\|gb157.2\| DW147378_T1 | lettuce | 1524 | 28 | 85 | 100 | 100 | blastp |
| 178 | lettuce\|gb157.2\| DW052373_T1 | lettuce | 1525 | 28 | 83 | 100 | 100 | blastp |
| 179 | lettuce\|gb157.2\| DW105592_T1 | lettuce | 1526 | 28 | 86 | 100 | 100 | blastp |
| 180 | lettuce\|gb157.2\| DW075384_T1 | lettuce | 1527 | 28 | 85 | 100 | 100 | blastp |
| 181 | lettuce\|gb157.2\| DW155153_T1 | lettuce | 1528 | 28 | 86 | 100 | 100 | blastp |
| 182 | lettuce\|gb157.2\| CV699993_T1 | lettuce | 1529 | 28 | 85 | 100 | 100 | blastp |
| 183 | lettuce\|gb157.2\| DW078166_T1 | lettuce | 1530 | 28 | 84 | 100 | 100 | blastp |
| 184 | lotus\|gb157.2\|A V409092_T1 | lotus | 1531 | 28 | 80 | 53.2 | 100 | blastp |
| 185 | medicago\|gb157. 2\|AI974377_T1 | medicago | 1532 | 28 | 81 | 98.8 | 99.5951 417 | blastp |
| 186 | melon\|gb165\|A M725511_T1 | melon | 1533 | 28 | 84 | 100 | 100 | blastp |
| 187 | nicotiana_bentha miana\|gb162\|EH 370474_T1 | nicotiana_benthamiana | 1534 | 28 | 90 | 70.4 | 100 | blastp |
| 188 | onion\|gb162\|BE 205571_T1 | onion | 1535 | 28 | 83 | 99.6 | 99.5967 742 | blastp |
| 189 | onion\|gb162\|AA 601764_T1 | onion | 1536 | 28 | 86 | 95.2 | 96.3414 634 | blastp |
| 190 | papaya\|gb165\|E X255759_T1 | papaya | 1537 | 28 | 82 | 99.6 | 99.5983 936 | blastp |
| 191 | peanut\|gb161\|EH 043676_T1 | peanut | 1538 | 28 | 82 | 99.6 | 99.5967 742 | blastp |
| 192 | pepper\|gb157.2\| CK901741_T1 | pepper | 1539 | 28 | 94 | 86.4 | 100 | blastp |
| 193 | periwinkle\|gb16 4\|FD423620_T1 | periwinkle | 1540 | 28 | 86 | 64.4 | 96.4071 856 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 194 | poplar\|gb157.2\|B U886993_T1 | poplar | 1541 | 28 | 84 | 100 | 100 | blastp |
| 195 | poplar\|gb157.2\|C A826065_T1 | poplar | 1542 | 28 | 85 | 86.8 | 100 | blastp |
| 196 | potato\|gb157.2\|B G591546 T2 | potato | 1543 | 28 | 81 | 100 | 100 | blastp |
| 197 | radish\|gb164\|EV 531940_T1 | radish | 1544 | 28 | 83 | 94.8 | 94.8 | blastp |
| 198 | radish\|gb164\|EV 527785_T1 | radish | 1545 | 28 | 84 | 92.8 | 95.473251 | blastp |
| 199 | radish\|gb164\|EV 550763_T1 | radish | 1546 | 28 | 84 | 95.2 | 94.8207171 | blastp |
| 200 | radish\|gb164\|EX 902593_T1 | radish | 1547 | 28 | 85 | 95.2 | 99.58159 | blastp |
| 201 | radish\|gb164\|EV 535199_T1 | radish | 1548 | 28 | 84 | 96 | 98.7654321 | blastp |
| 202 | radish\|gb164\|EV 525705_T1 | radish | 1549 | 28 | 85 | 96 | 98.7654321 | blastp |
| 203 | safflower\|gb162\| EL399548_T1 | safflower | 1550 | 28 | 86 | 86.8 | 100 | blastp |
| 204 | safflower\|gb162\| EL376421_T1 | safflower | 1551 | 28 | 87 | 100 | 100 | blastp |
| 205 | spurge\|gb161\|D V127241_T1 | spurge | 1552 | 28 | 85 | 88.4 | 99.103139 | blastp |
| 206 | strawberry\|gb16 4\|GFXDQ178022X1_T1 | strawberry | 1553 | 28 | 82 | 100 | 100 | blastp |
| 207 | sunflower\|gb162\| X95953_T1 | sunflower | 1554 | 28 | 86 | 100 | 100 | blastp |
| 208 | sunflower\|gb162\| DY911049_T1 | sunflower | 1555 | 28 | 85 | 100 | 100 | blastp |
| 209 | sunflower\|gb162\| DY921796_T1 | sunflower | 1556 | 28 | 81 | 88.8 | 98.2300885 | blastp |
| 210 | sunflower\|gb162\| DY918762_T1 | sunflower | 1557 | 28 | 85 | 100 | 100 | blastp |
| 211 | sunflower\|gb162\| DY906198_T1 | sunflower | 1558 | 28 | 81 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 212 | sunflower\|gb162\| DY932268_T1 | sunflower | 1559 | 28 | 81 | 100 | 100 | blastp |
| 213 | tobacco\|gb162\|G FXS45406X1_T 1 | tobacco | 1560 | 28 | 93 | 100 | 100 | blastp |
| 214 | tobacco\|gb162\|E B445911_T1 | tobacco | 1561 | 28 | 89 | 100 | 100 | blastp |
| 215 | apricot\|gb157.2\| CB818493_T1 | apricot | 1562 | 29 | 81 | 54.5454 545 | 95.8333 333 | blastp |
| 216 | arabidopsis\|gb16 5\|AT4G01470_T 1 | arabidopsis | 1563 | 29 | 80 | 99.2094 862 | 99.6031 746 | blastp |
| 217 | avocado\|gb164\|C K760396_T1 | avocado | 1564 | 29 | 81 | 54.5454 545 | 93.8775 51 | blastp |
| 218 | b_rapa\|gb162\|E X017183_T1 | b_rapa | 1565 | 29 | 83 | 69.5652 174 | 94.1176 471 | blastp |
| 219 | barley\|gb157.3\|B E413237_T1 | barley | 1566 | 29 | 81 | 99.2094 862 | 99.6031 746 | blastp |
| 220 | cassava\|gb164\|C K644827_T1 | cassava | 1567 | 29 | 90 | 57.3122 53 | 99.3150 685 | blastp |
| 221 | cassava\|gb164\|B M259770_T1 | cassava | 1568 | 29 | 82 | 99.2094 862 | 99.6031 746 | blastp |
| 222 | cassava\|gb164\|C K645124_T1 | cassava | 1569 | 29 | 80 | 99.2094 862 | 99.6031 746 | blastp |
| 223 | castorbean\|gb16 0\|EG666198_T1 | castorbean | 1570 | 29 | 84 | 99.2094 862 | 99.6031 746 | blastp |
| 224 | castorbean\|gb16 0\|AJ605571_T1 | castorbean | 1571 | 29 | 82 | 99.2094 862 | 99.6015 936 | blastp |
| 225 | castorbean\|gb16 0\|AJ605570_T1 | castorbean | 1572 | 29 | 83 | 99.2094 862 | 99.6031 746 | blastp |
| 226 | centaurea\|gb161\| EL931525_T1 | centaurea | 1573 | 29 | 88 | 74.7035 573 | 97.9274 611 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 227 | cichorium\|gb161 \|EH707617_T1 | cichorium | 1574 | 29 | 87 | 65.6126482 | 99.4011976 | blastp |
| 228 | citrus\|gb157.2\|C F834233_T1 | citrus | 1575 | 29 | 80 | 94.4664032 | 94.4444444 | blastp |
| 229 | citrus\|gb157.2\|B Q624227_T1 | citrus | 1576 | 29 | 87 | 99.2094862 | 99.6031746 | blastp |
| 230 | citrus\|gb157.2\|B Q623056_T1 | citrus | 1577 | 29 | 87 | 97.2332016 | 98.4 | blastp |
| 231 | citrus\|gb157.2\|B Q624617_T1 | citrus | 1578 | 29 | 87 | 99.2094862 | 99.6031746 | blastp |
| 232 | cotton\|gb164\|CD 486523_T1 | cotton | 1579 | 29 | 81 | 99.2094862 | 99.6031746 | blastp |
| 233 | cotton\|gb164\|AI 729919_T1 | cotton | 1580 | 29 | 82 | 99.2094862 | 99.6031746 | blastp |
| 234 | cotton\|gb164\|BG 442315_T1 | cotton | 1581 | 29 | 86 | 99.2094862 | 99.6031746 | blastp |
| 235 | cotton\|gb164\|EX 167179_T1 | cotton | 1582 | 29 | 84 | 56.916996 | 100 | blastp |
| 236 | cotton\|gb164\|AI 726375_T1 | cotton | 1583 | 29 | 82 | 99.2094862 | 99.6031746 | blastp |
| 237 | cowpea\|gb166\|F F384697_T1 | cowpea | 1584 | 29 | 84 | 99.2094862 | 99.6031746 | blastp |
| 238 | dandelion\|gb161\| DY825779_T1 | dandelio n | 1585 | 29 | 88 | 99.2094862 | 99.6031746 | blastp |
| 239 | fescue\|gb161\|CK 802772_T1 | fescue | 1586 | 29 | 80 | 99.2094862 | 99.6031746 | blastp |
| 240 | grape\|gb160\|BQ 796848_T1 | grape | 1587 | 29 | 84 | 99.2094862 | 99.6015936 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 241 | grape\|gb160\|CF6 05030_T1 | grape | 1588 | 29 | 82 | 99.2094 862 | 99.6031 746 | blastp |
| 242 | ipomoea\|gb157.2 \|EE883704_T1 | ipomoea | 1589 | 29 | 85 | 53.7549 407 | 100 | blastp |
| 243 | ipomoea\|gb157.2 \|BJ554617_T1 | ipomoea | 1590 | 29 | 85 | 99.2094 862 | 99.6031 746 | blastp |
| 244 | lettuce\|gb157.2\| DW074608_T1 | lettuce | 1591 | 29 | 87 | 99.2094 862 | 99.6031 746 | blastp |
| 245 | lettuce\|gb157.2\| DY977540_T1 | lettuce | 1592 | 29 | 87 | 99.2094 862 | 99.6031 746 | blastp |
| 246 | lotus\|gb157.2\|B W615882_T1 | lotus | 1593 | 29 | 80 | 50.1976 285 | 100 | blastp |
| 247 | maize\|gb164\|DQ 245749_T1 | maize | 1594 | 29 | 81 | 99.2094 862 | 99.6031 746 | blastp |
| 248 | medicago\|gb157. 2\|BI266516_T1 | medicag o | 1595 | 29 | 82 | 99.2094 862 | 99.6031 746 | blastp |
| 249 | nicotiana_bentha miana\|gb162\|CN 743053_T1 | nicotiana _bentha miana | 1596 | 29 | 94 | 83.0039 526 | 99.5260 664 | blastp |
| 250 | papaya\|gb165\|E X256526_T1 | papaya | 1597 | 29 | 80 | 99.2094 862 | 99.6031 746 | blastp |
| 251 | papaya\|gb165\|E X255270_T1 | papaya | 1598 | 29 | 86 | 99.2094 862 | 99.6031 746 | blastp |
| 252 | peach\|gb157.2\|A F367456_T1 | peach | 1599 | 29 | 84 | 53.7549 407 | 100 | blastp |
| 253 | pepper\|gb157.2\| CK902019_T1 | pepper | 1600 | 29 | 81 | 73.1225 296 | 98.9304 813 | blastp |
| 254 | poplar\|gb157.2\|A 1163470_T1 | poplar | 1601 | 29 | 81 | 99.2094 862 | 99.6031 746 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 255 | poplar\|gb157.2\|A 1166549_T1 | poplar | 1602 | 29 | 82 | 99.2094 862 | 99.6031 746 | blastp |
| 256 | poplar\|gb157.2\|B U887722_T1 | poplar | 1603 | 29 | 81 | 99.2094 862 | 99.6031 746 | blastp |
| 257 | poplar\|gb157.2\|B U875073_T1 | poplar | 1604 | 29 | 83 | 99.2094 862 | 99.6031 746 | blastp |
| 258 | poplar\|gb157.2\|C A823737_T1 | poplar | 1605 | 29 | 88 | 53.3596 838 | 99.2647 059 | blastp |
| 259 | poplar\|gb157.2\|A 1166136_T1 | poplar | 1606 | 29 | 80 | 99.2094 862 | 99.6031 746 | blastp |
| 260 | radish\|gb164\|EV 544876_T1 | radish | 1607 | 29 | 80 | 99.2094 862 | 99.6031 746 | blastp |
| 261 | rice\|gb157.2\|AA 752956_T1 | rice | 1608 | 29 | 80 | 99.2094 862 | 99.6031 746 | blastp |
| 262 | soybean\|gb166\|C X703984_T1 | soybean | 1609 | 29 | 80 | 99.2094 862 | 99.6031 746 | blastp |
| 263 | soybean\|gb166\|S OYNODB_T1 | soybean | 1610 | 29 | 86 | 99.2094 862 | 99.6031 746 | blastp |
| 264 | spurge\|gb161\|D V146067_T1 | spurge | 1611 | 29 | 84 | 87.3517 787 | 100 | blastp |
| 265 | spurge\|gb161\|A W990927_T1 | spurge | 1612 | 29 | 80 | 99.2094 862 | 99.6031 746 | blastp |
| 266 | sunflower\|gb162\| DY919534_T1 | sunflower | 1613 | 29 | 86 | 99.2094 862 | 99.6031 746 | blastp |
| 267 | tobacco\|gb162\|E B443312_T1 | tobacco | 1614 | 29 | 92 | 99.2094 862 | 99.6031 746 | blastp |
| 268 | tobacco\|gb162\|C V019217_T1 | tobacco | 1615 | 29 | 81 | 98.0237 154 | 99.5967 742 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 269 | tobacco\|gb162\|E B443618_T1 | tobacco | 1616 | 29 | 92 | 99.2094 862 | 99.6031 746 | blastp |
| 270 | tobacco\|gb162\|C V018899_T1 | tobacco | 1617 | 29 | 81 | 98.0237 154 | 99.5967 742 | blastp |
| 271 | wheat\|gb164\|BE 418306_T1 | wheat | 1618 | 29 | 80 | 99.2094 862 | 99.6031 746 | blastp |
| 272 | wheat\|gb164\|BE 404792_T1 | wheat | 1619 | 29 | 82 | 52.9644 269 | 99.2592 593 | blastp |
| 273 | wheat\|gb164\|BE 216922_T1 | wheat | 1620 | 29 | 81 | 99.2094 862 | 99.6031 746 | blastp |
| 274 | artemisia\|gb164\| EY083433_T1 | artemisia | 1621 | 30 | 80 | 79.6 | 100 | blastp |
| 275 | banana\|gb160\|ES 432704_T1 | banana | 1622 | 30 | 81 | 88.8 | 93.6708 861 | blastp |
| 276 | banana\|gb160\|D N238541_T1 | banana | 1623 | 30 | 80 | 100 | 100 | blastp |
| 277 | barley\|gb157.3\|B E412510_T1 | barley | 1624 | 30 | 80 | 100 | 100 | blastp |
| 278 | cotton\|gb164\|AI 726168_T1 | cotton | 1625 | 30 | 80 | 98.8 | 99.5983 936 | blastp |
| 279 | cotton\|gb164\|AI 731742_T1 | cotton | 1626 | 30 | 80 | 100 | 100 | blastp |
| 280 | cotton\|gb164\|AI 055329_T1 | cotton | 1627 | 30 | 80 | 100 | 100 | blastp |
| 281 | grape\|gb160\|BQ 794219_T1 | grape | 1628 | 30 | 81 | 100 | 100 | blastp |
| 282 | ipomoea\|gb157.2 \|BJ554855_T1 | ipomoea | 1629 | 30 | 81 | 98 | 100 | blastp |
| 283 | ipomoea\|gb157.2 \|BM878761_T1 | ipomoea | 1630 | 30 | 84 | 100 | 100 | blastp |
| 284 | lettuce\|gb157.2\| DW045084_T1 | lettuce | 1631 | 30 | 80 | 86.8 | 98.1981 982 | blastp |
| 285 | lettuce\|gb157.2\| DW114621_T1 | lettuce | 1632 | 30 | 80 | 88 | 99.1031 39 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 286 | lettuce\|gb157.2\| DW078778_T1 | lettuce | 1633 | 30 | 81 | 50.8 | 100 | blastp |
| 287 | maize\|gb164\|CO 528320_T1 | maize | 1634 | 30 | 82 | 69.6 | 100 | blastp |
| 288 | maize\|gb164\|A W257922_T1 | maize | 1635 | 30 | 80 | 52.4 | 100 | blastp |
| 289 | maize\|gb164\|BI6 75058_T1 | maize | 1636 | 30 | 80 | 54 | 99.2592 593 | blastp |
| 290 | maize\|gb164\|A W352518_T1 | maize | 1637 | 30 | 80 | 51.2 | 100 | blastp |
| 291 | maize\|gb164\|AF 037061_T1 | maize | 1638 | 30 | 81 | 100 | 100 | blastp |
| 292 | nicotiana_bentha miana\|gb162\|CN 655062_T1 | nicotiana_benthamiana | 1639 | 30 | 90 | 100 | 100 | blastp |
| 293 | nicotiana_bentha miana\|gb162\|CN 741621_T1 | nicotiana_benthamiana | 1640 | 30 | 90 | 100 | 100 | blastp |
| 294 | oil_palm\|gb166\| CN599861_T1 | oil_palm | 1641 | 30 | 80 | 100 | 100 | blastp |
| 295 | papaya\|gb165\|E X246150_T1 | papaya | 1642 | 30 | 82 | 100 | 100 | blastp |
| 296 | pepper\|gb157.2\| BM060520_T1 | pepper | 1643 | 30 | 91 | 99.2 | 98.8 | blastp |
| 297 | periwinkle\|gb16 4\|EG554262_T1 | periwinkle | 1644 | 30 | 82 | 100 | 100 | blastp |
| 298 | petunia\|gb157.2\| AF452015_T1 | petunia | 1645 | 30 | 89 | 100 | 100 | blastp |
| 299 | potato\|gb157.2\|C K853059_T1 | potato | 1646 | 30 | 96 | 92 | 91.3043 478 | blastp |
| 300 | potato\|gb157.2\|C K852742_T1 | potato | 1647 | 30 | 82 | 60.4 | 100 | blastp |
| 301 | potato\|gb157.2\|B M407759_T1 | potato | 1648 | 30 | 99 | 81.2 | 97.5961 538 | blastp |
| 302 | potato\|gb157.2\|C K718033_T1 | potato | 1649 | 30 | 98 | 65.2 | 92.0903 955 | blastp |
| 303 | potato\|gb157.2\|C K717899_T1 | potato | 1650 | 30 | 100 | 62 | 95.0920 245 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 304 | potato\|gb157.2\|C V472240_T1 | potato | 1651 | 30 | 97 | 79.6 | 95.2153 11 | blastp |
| 305 | potato\|gb157.2\|B G098199 T1 | potato | 1652 | 30 | 95 | 93.2 | 99.5726 496 | blastp |
| 306 | rice\|gb157.2\|U37 925 T1 | rice | 1653 | 30 | 81 | 100 | 100 | blastp |
| 307 | rye\|gb164\|BE49 4266 T1 | rye | 1654 | 30 | 80 | 100 | 100 | blastp |
| 308 | sorghum\|gb161. xeno\|AF037061_T1 | sorghum | 1655 | 30 | 80 | 100 | 100 | blastp |
| 309 | sugarcane\|gb157 .2\|BQ535365_T 1 | sugarcane | 1656 | 30 | 80 | 80.4 | 100 | blastp |
| 310 | switchgrass\|gb16 5\|DN141449_T1 | switchgrass | 1657 | 30 | 81 | 100 | 100 | blastp |
| 311 | switchgrass\|gb16 5\|DN142089_T1 | switchgrass | 1658 | 30 | 81 | 100 | 100 | blastp |
| 312 | tobacco\|gb162\|C N824866 T1 | tobacco | 1659 | 30 | 90 | 100 | 100 | blastp |
| 313 | tobacco\|gb162\|C V017118 T1 | tobacco | 1660 | 30 | 90 | 100 | 100 | blastp |
| 314 | wheat\|gb164\|TA U86762 T1 | wheat | 1661 | 30 | 80 | 100 | 100 | blastp |
| 315 | wheat\|gb164\|BE 499589 T1 | wheat | 1662 | 30 | 80 | 72 | 100 | blastp |
| 316 | lettuce\|gb157.2\| DW087170 T1 | lettuce | 1663 | 31 | 80 | 100 | 100 | blastp |
| 317 | tobacco\|gb162\|E H616288 T1 | tobacco | 1664 | 32 | 87 | 50.7692 308 | 85.1612 903 | blastp |
| 318 | apple\|gb157.3\|C 0068608 T1 | apple | 1665 | 33 | 86 | 98.2638 889 | 98.9510 49 | blastp |
| 319 | apple\|gb157.3\|A B100869 T1 | apple | 1666 | 33 | 83 | 98.2638 889 | 99.3079 585 | blastp |
| 320 | apple\|gb157.3\|A B100870 T1 | apple | 1667 | 33 | 83 | 98.2638 889 | 99.3079 585 | blastp |
| 321 | apple\|gb157.3\|C N860225_T1 | apple | 1668 | 33 | 86 | 54.8611 111 | 90.2857 143 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 322 | apple\|gb157.3\|C K900645 T1 | apple | 1669 | 33 | 85 | 98.2638 889 | 98.9510 49 | blastp |
| 323 | apricot\|gb157.2\| CB822297 T1 | apricot | 1670 | 33 | 89 | 51.0416 667 | 98.6577 181 | blastp |
| 324 | apricot\|gb157.2\| CB819647 T1 | apricot | 1671 | 33 | 83 | 98.2638 889 | 98.9655 172 | blastp |
| 325 | aquilegia\|gb157. 3\|DR917005_T1 | aquilegia | 1672 | 33 | 86 | 98.2638 889 | 98.9547 038 | blastp |
| 326 | arabidopsis\|gb16 5\|AT4G00430_T 2 | arabidopsis | 1673 | 33 | 84 | 73.6111 111 | 97.2602 74 | blastp |
| 327 | arabidopsis\|gb16 5\|AT2G45960_T 3 | arabidopsis | 1674 | 33 | 86 | 88.1944 444 | 84.3853 821 | blastp |
| 328 | arabidopsis\|gb16 5\|AT4G23400_T 1 | arabidopsis | 1675 | 33 | 87 | 98.2638 889 | 98.9547 038 | blastp |
| 329 | arabidopsis\|gb16 5\|AT2G45960_T 1 | arabidopsis | 1676 | 33 | 86 | 98.2638 889 | 98.9510 49 | blastp |
| 330 | arabidopsis\|gb16 5\|AT4G00430_T 1 | arabidopsis | 1677 | 33 | 87 | 98.2638 889 | 98.9547 038 | blastp |
| 331 | arabidopsis\|gb16 5\|AT1G01620_T 1 | arabidopsis | 1678 | 33 | 88 | 98.2638 889 | 98.9510 49 | blastp |
| 332 | arabidopsis\|gb16 5\|AT3G61430_T 1 | arabidopsis | 1679 | 33 | 85 | 98.2638 889 | 98.9510 49 | blastp |
| 333 | arabidopsis\|gb16 5\|AT2G45960_T 4 | arabidopsis | 1680 | 33 | 86 | 88.1944 444 | 92.7007 299 | blastp |
| 334 | artemisia\|gb164 EY046087 T1 | artemisia | 1681 | 33 | 86 | 98.2638 889 | 98.9547 038 | blastp |
| 335 | artemisia\|gb164 EY046310 T1 | artemisia | 1682 | 33 | 87 | 73.6111 111 | 99.0697 674 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 336 | artemisia\|gb164 EY032836 T1 | artemisia | 1683 | 33 | 84 | 97.5694 444 | 98.2758 621 | blastp |
| 337 | artemisia\|gb164 EY031810 T1 | artemisia | 1684 | 33 | 84 | 89.2361 111 | 99.2307 692 | blastp |
| 338 | avocado\|gb164\|C K751385 T1 | avocado | 1685 | 33 | 86 | 98.2638 889 | 98.9547 038 | blastp |
| 339 | avocado\|gb164\|C K745633 T1 | avocado | 1686 | 33 | 91 | 73.6111 111 | 99.0654 206 | blastp |
| 340 | b_juncea\|gb164\| EVGN00081008 450640 T1 | b_juncea | 1687 | 33 | 87 | 98.2638 889 | 98.9510 49 | blastp |
| 341 | b_juncea\|gb164 EVGN00515811 862066 T1 | b_juncea | 1688 | 33 | 90 | 60.4166 667 | 96.1325 967 | blastp |
| 342 | b_juncea\|gb164 EVGN00230716 760965 T1 | b_juncea | 1689 | 33 | 89 | 73.6111 111 | 99.0654 206 | blastp |
| 343 | b_juncea\|gb164\| EVGN01776308 261252 T1 | b_juncea | 1690 | 33 | 84 | 66.3194 444 | 96.4646 465 | blastp |
| 344 | b_juncea\|gb164\| EVGN00910030 360678 T1 | b_juncea | 1691 | 33 | 91 | 65.2777 778 | 98.9473 684 | blastp |
| 345 | b_juncea\|gb164\| EVGN03812526 911787_T1 | b_juncea | 1692 | 33 | 88 | 51.0416 667 | 98.6577 181 | blastp |
| 346 | b_juncea\|gb164\| EVGN00227203 510305_T1 | b_juncea | 1693 | 33 | 91 | 65.2777 778 | 98.9473 684 | blastp |
| 347 | b_juncea\|gb164 EVGN00462518 410866_T1 | b_juncea | 1694 | 33 | 87 | 98.2638 889 | 98.9510 49 | blastp |
| 348 | b_juncea\|gb164\| EVGN00248411 120906_T1 | b_juncea | 1695 | 33 | 89 | 73.2638 889 | 99.0610 329 | blastp |
| 349 | b_juncea\|gb164 EF471211_T1 | b_juncea | 1696 | 33 | 86 | 98.2638 889 | 98.2638 889 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 350 | b_juncea\|gb164\| EVGN00440012 650683_T1 | b_juncea | 1697 | 33 | 86 | 98.2638 889 | 98.9510 49 | blastp |
| 351 | b_juncea\|gb164\| EVGN00452211 183349_T1 | b_juncea | 1698 | 33 | 86 | 98.2638 889 | 98.9510 49 | blastp |
| 352 | b_juncea\|gb164 EVGN03595331 210044_T1 | b_juncea | 1699 | 33 | 89 | 57.6388 889 | 93.2584 27 | blastp |
| 353 | b_juncea\|gb164\| EVGN00088009 631302_T1 | b_juncea | 1700 | 33 | 87 | 98.2638 889 | 98.9510 49 | blastp |
| 354 | b_juncea\|gb164\| EVGN00512912 541009_T1 | b_juncea | 1701 | 33 | 85 | 51.7361 111 | 93.9075 63 | tblastn |
| 355 | b_juncea\|gb164\| EVGN00756014 550623_T1 | b_juncea | 1702 | 33 | 84 | 69.0972 222 | 90.3177 005 | tblastn |
| 356 | b_oleracea\|gb16 1\|AF299051_T1 | b_oleracea | 1703 | 33 | 86 | 98.2638 889 | 98.9510 49 | blastp |
| 357 | b_oleracea\|gb16 1\|AM391520_T1 | b_oleracea | 1704 | 33 | 87 | 75.3472 222 | 99.5412 844 | blastp |
| 358 | b_oleracea\|gb16 1\|AM058918_T1 | b_oleracea | 1705 | 33 | 87 | 98.2638 889 | 98.9510 49 | blastp |
| 359 | b_oleracea\|gb16 1\|AF299050_T1 | b_oleracea | 1706 | 33 | 86 | 98.2638 889 | 98.9510 49 | blastp |
| 360 | b_oleracea\|gb16 1\|DY029936_T1 | b_oleracea | 1707 | 33 | 86 | 98.2638 889 | 98.9510 49 | blastp |
| 361 | b_oleracea\|gb16 1\|EH422530_T1 | b_oleracea | 1708 | 33 | 87 | 78.4722 222 | 100 | blastp |
| 362 | b_rapa\|gb162\|C V546930_T1 | b_rapa | 1709 | 33 | 84 | 71.5277 778 | 99.5169 082 | blastp |
| 363 | b_rapa\|gb162\|B G544387_T1 | b_rapa | 1710 | 33 | 86 | 95.4861 111 | 99.2779 783 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 364 | b_rapa\|gb162\|C A992432_T1 | b_rapa | 1711 | 33 | 86 | 98.2638889 | 98.951049 | blastp |
| 365 | b_rapa\|gb162\|C V546129_T2 | b_rapa | 1712 | 33 | 83 | 54.8611111 | 99.3710692 | blastp |
| 366 | b_rapa\|gb162\|EE 526280_T1 | b_rapa | 1713 | 33 | 87 | 98.2638889 | 98.951049 | blastp |
| 367 | b_rapa\|gb162\|L3 3552_T1 | b_rapa | 1714 | 33 | 86 | 98.2638889 | 98.951049 | blastp |
| 368 | b_rapa\|gb162\|B G543719_T1 | b_rapa | 1715 | 33 | 84 | 77.0833333 | 99.5515695 | blastp |
| 369 | b_rapa\|gb162\|AF 004293_T1 | b_rapa | 1716 | 33 | 87 | 98.2638889 | 98.951049 | blastp |
| 370 | b_rapa\|gb162\|B G544086_T1 | b_rapa | 1717 | 33 | 87 | 98.2638889 | 98.951049 | blastp |
| 371 | b_rapa\|gb162\|C X267412_T1 | b_rapa | 1718 | 33 | 86 | 98.2638889 | 99.2982456 | blastp |
| 372 | b_rapa\|gb162C V546129_T1 | b_rapa | 1719 | 33 | 86 | 98.2638889 | 98.2638889 | blastp |
| 373 | b_rapa\|gb162\|C V545634_T1 | b_rapa | 1720 | 33 | 83 | 56.5972222 | 90.5555556 | blastp |
| 374 | banana\|gb160\|D N238827_T1 | banana | 1721 | 33 | 84 | 60.7638889 | 99.4285714 | blastp |
| 375 | banana\|gb160\|ES 431094_T1 | banana | 1722 | 33 | 89 | 63.8888889 | 98.9247312 | blastp |
| 376 | barley\|gb157.3\|B E412959_T2 | barley | 1723 | 33 | 83 | 98.2638889 | 98.9726027 | blastp |
| 377 | barley\|gbl57.3\|A L507831_T1 | barley | 1724 | 33 | 85 | 99.3055556 | 99.6551724 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 378 | barley\|gb157.3\|B E412959_T1 | barley | 1725 | 33 | 83 | 98.2638 889 | 98.9726 027 | blastp |
| 379 | barley\|gb157.3\|B E412959_T5 | barley | 1726 | 33 | 82 | 98.2638 889 | 98.9830 508 | blastp |
| 380 | barley\|gb157.3\|B E412959_T4 | barley | 1727 | 33 | 82 | 98.2638 889 | 98.9830 508 | blastp |
| 381 | barley\|gb157.3\|A L502020_T1 | barley | 1728 | 33 | 82 | 98.2638 889 | 98.9726 027 | blastp |
| 382 | barley\|gb157.3\|B E412972_T1 | barley | 1729 | 33 | 85 | 98.2638 889 | 98.9583 333 | blastp |
| 383 | basilicum\|gb157. 3\|DY340092_T1 | basilicum | 1730 | 33 | 88 | 61.8055 556 | 99.4413 408 | blastp |
| 384 | basilicum\|gb157. 3\|DY332264_T1 | basilicum | 1731 | 33 | 87 | 73.9583 333 | 99.5327 103 | blastp |
| 385 | bean\|gb164\|CB5 43592_T1 | bean | 1732 | 33 | 88 | 98.2638 889 | 98.9547 038 | blastp |
| 386 | bean\|gb164\|PVU 97023_T1 | bean | 1733 | 33 | 84 | 98.2638 889 | 99.3079 585 | blastp |
| 387 | bean\|gb164\|CB5 42193_T1 | bean | 1734 | 33 | 84 | 98.6111 111 | 99.6539 792 | blastp |
| 388 | beet\|gb162\|BVU 60149_T1 | beet | 1735 | 33 | 84 | 98.2638 889 | 98.9510 49 | blastp |
| 389 | brachypodium\|g b161.xeno\|BE44 3278_T1 | brachypodium | 1736 | 33 | 83 | 82.9861 111 | 95.6521 739 | blastp |
| 390 | brachypodium\|g b161.xeno\|BE21 6990_T1 | brachypodium | 1737 | 33 | 85 | 98.2638 889 | 98.9583 333 | blastp |
| 391 | brachypodium\|g b161.xeno\|BE40 3307_T1 | brachypodium | 1738 | 33 | 82 | 86.8055 556 | 72.7011 494 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 392 | canola\|gb161\|CN 731957_T1 | canola | 1739 | 33 | 86 | 98.2638 889 | 98.9510 49 | blastp |
| 393 | canola\|gb161\|CX 194503_T1 | canola | 1740 | 33 | 86 | 98.2638 889 | 98.9510 49 | blastp |
| 394 | canola\|gb161\|CD 814405_T1 | canola | 1741 | 33 | 87 | 98.2638 889 | 98.9510 49 | blastp |
| 395 | canola\|gb161\|EG 020906_T1 | canola | 1742 | 33 | 86 | 98.2638 889 | 98.9510 49 | blastp |
| 396 | canola\|gb161\|H7 4720_T1 | canola | 1743 | 33 | 86 | 98.2638 889 | 98.9510 49 | blastp |
| 397 | canola\|gb161\|CN 831315_T1 | canola | 1744 | 33 | 86 | 84.0277 778 | 93.4362 934 | blastp |
| 398 | canola\|gb161\|CD 817408_T1 | canola | 1745 | 33 | 87 | 98.2638 889 | 98.9510 49 | blastp |
| 399 | canola\|gb161\|EE 502121_T1 | canola | 1746 | 33 | 89 | 52.7777 778 | 98.7096 774 | blastp |
| 400 | canola\|gb161\|CX 187544_T1 | canola | 1747 | 33 | 87 | 98.2638 889 | 98.9510 49 | blastp |
| 401 | canola\|gb161\|CD 822064_T1 | canola | 1748 | 33 | 86 | 98.2638 889 | 98.9510 49 | blastp |
| 402 | canola\|gb161\|CD 824965_T1 | canola | 1749 | 33 | 81 | 92.0138 889 | 93.0313 589 | blastp |
| 403 | canola\|gb161\|EE 485551_T1 | canola | 1750 | 33 | 87 | 98.2638 889 | 98.9510 49 | blastp |
| 404 | canola\|gb161\|CB 686274_T1 | canola | 1751 | 33 | 86 | 98.2638 889 | 98.9510 49 | blastp |
| 405 | canola\|gb161\|CD 814573_T1 | canola | 1752 | 33 | 83 | 76.3888 889 | 94.0425 532 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 406 | canola\|gb161\|CX 193398_T1 | canola | 1753 | 33 | 86 | 98.2638 889 | 98.2638 889 | blastp |
| 407 | canola\|gb161\|CD 818853_T1 | canola | 1754 | 33 | 86 | 98.2638 889 | 98.9510 49 | blastp |
| 408 | canola\|gb161\|D Y005979_T1 | canola | 1755 | 33 | 85 | 78.4722 222 | 99.5594 714 | blastp |
| 409 | canola\|gb161\|EE 464964_T1 | canola | 1756 | 33 | 85 | 81.5972 222 | 99.5762 712 | blastp |
| 410 | cassava\|gb164\|B M260264_T1 | cassava | 1757 | 33 | 85 | 97.9166 667 | 99.3031 359 | blastp |
| 411 | cassava\|gb164\|C K901165_T1 | cassava | 1758 | 33 | 87 | 73.6111 111 | 99.0697 674 | blastp |
| 412 | cassava\|gb164\|C K642415_T1 | cassava | 1759 | 33 | 87 | 98.2638 889 | 98.9547 038 | blastp |
| 413 | cassava\|gb164\|D V455398_T1 | cassava | 1760 | 33 | 85 | 97.9166 667 | 99.3031 359 | blastp |
| 414 | castorbean\|gb16 0\|T14819_T1 | castorbean | 1761 | 33 | 89 | 98.2638 889 | 98.9547 038 | blastp |
| 415 | castorbean\|gb16 0\|EG691229_T1 | castorbean | 1762 | 33 | 86 | 98.2638 889 | 98.9510 49 | blastp |
| 416 | castorbean\|gb16 0\|AJ605566_T1 | castorbean | 1763 | 33 | 87 | 97.9166 667 | 99.3031 359 | blastp |
| 417 | castorbean\|gb16 0\|MDL29969M0 00266_T1 | castorbean | 1764 | 33 | 84 | 98.2638 889 | 99.3055 556 | blastp |
| 418 | castorbean\|gb16 0\|AJ605574_T1 | castorbean | 1765 | 33 | 87 | 97.9166 667 | 98.9583 333 | blastp |
| 419 | centaurea\|gb161\| EH732068_T1 | centaurea | 1766 | 33 | 82 | 97.5694 444 | 98.6062 718 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 420 | cichorium\|gb161\|EH673032_T1 | cichorium | 1767 | 33 | 87 | 98.2638889 | 98.9547038 | blastp |
| 421 | cichorium\|gb161\|EH706808_T1 | cichorium | 1768 | 33 | 90 | 51.7361111 | 98.6842105 | blastp |
| 422 | cichorium\|gb161\|EH701938_T1 | cichorium | 1769 | 33 | 86 | 64.9305556 | 95.4314721 | blastp |
| 423 | citrus\|gb157.2\|C 0912471_T1 | citrus | 1770 | 33 | 82 | 69.4444444 | 99.5098039 | blastp |
| 424 | citrus\|gb157.2\|B Q624312_T1 | citrus | 1771 | 33 | 88 | 98.2638889 | 98.9547038 | blastp |
| 425 | citrus\|gb157.2\|C N182376_T1 | citrus | 1772 | 33 | 84 | 92.7083333 | 94.0559441 | blastp |
| 426 | citrus\|gb157.2\|C B291370_T1 | citrus | 1773 | 33 | 89 | 98.2638889 | 98.9547038 | blastp |
| 427 | citrus\|gb157.2\|C F833327_T1 | citrus | 1774 | 33 | 85 | 98.2638889 | 99.3031359 | blastp |
| 428 | citrus\|gb1572\|B Q624860_T1 | citrus | 1775 | 33 | 85 | 97.9166667 | 99.6503497 | blastp |
| 429 | citrus\|gb157.2\|C F508404_T1 | citrus | 1776 | 33 | 81 | 97.9166667 | 99.6503497 | blastp |
| 430 | citrus\|gbl57.2\|C B293694_T1 | citrus | 1777 | 33 | 84 | 98.2638889 | 99.3031359 | blastp |
| 431 | citrus\|gb157.2\|B Q622975_T1 | citrus | 1778 | 33 | 85 | 97.9166667 | 99.6503497 | blastp |
| 432 | citrus\|gb157.2\|B E213453_T1 | citrus | 1779 | 33 | 86 | 73.6111111 | 99.5305164 | blastp |
| 433 | citrus\|gb157.2\|C F828110_T1 | citrus | 1780 | 33 | 85 | 98.6111111 | 99.6527778 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 434 | citrus|gb157.2|B Q623397_T1 | citrus | 1781 | 33 | 86 | 93.4027778 | 99.6336996 | blastp |
| 435 | clover|gb162|BB 903117_T1 | clover | 1782 | 33 | 85 | 98.6111111 | 99.6539792 | blastp |
| 436 | coffea|gb157.2|B Q449035_T1 | coffea | 1783 | 33 | 88 | 98.9583333 | 99.3055556 | blastp |
| 437 | coffea|gb157.2|D V663743_T1 | coffea | 1784 | 33 | 85 | 98.2638889 | 98.9473684 | blastp |
| 438 | cotton|gb164|CD 486529_T1 | cotton | 1785 | 33 | 83 | 98.2638889 | 99.3055556 | blastp |
| 439 | cotton|gb164|BE 052445 T1 | cotton | 1786 | 33 | 87 | 98.2638889 | 98.9547038 | blastp |
| 440 | cotton|gb164|AI 726690_T1 | cotton | 1787 | 33 | 86 | 98.2638889 | 98.9547038 | blastp |
| 441 | cotton|gb164|DN 803576_T1 | cotton | 1788 | 33 | 83 | 87.8472222 | 98.828125 | blastp |
| 442 | cotton|gb164|B M358242_T1 | cotton | 1789 | 33 | 81 | 98.2638889 | 99.2882562 | blastp |
| 443 | cotton|gb164|CO 085369_T1 | cotton | 1790 | 33 | 81 | 52.7777778 | 99.3506494 | blastp |
| 444 | cotton|gb164|CO 098674_T1 | cotton | 1791 | 33 | 84 | 98.2638889 | 99.3055556 | blastp |
| 445 | cotton|gb164|CO 070796_T1 | cotton | 1792 | 33 | 84 | 98.2638889 | 99.3031359 | blastp |
| 446 | cotton|gb164|AI 729945_T1 | cotton | 1793 | 33 | 85 | 98.2638889 | 99.3079585 | blastp |
| 447 | cotton|gb164|D W496760_T1 | cotton | 1794 | 33 | 86 | 80.5555556 | 98.3122363 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 448 | cowpea\|gb166\|F F384916_T1 | cowpea | 1795 | 33 | 82 | 98.2638889 | 99.3031359 | blastp |
| 449 | cowpea\|gb166\|F F555791_T1 | cowpea | 1796 | 33 | 82 | 98.6111111 | 99.6539792 | blastp |
| 450 | cowpea\|gb166\|F C457489_T1 | cowpea | 1797 | 33 | 87 | 98.2638889 | 98.9547038 | blastp |
| 451 | cowpea\|gb166\|A B037241_T1 | cowpea | 1798 | 33 | 84 | 98.2638889 | 99.3079585 | blastp |
| 452 | cryptomeria\|gb166\| DC429824_T 1 | cryptomeria | 1799 | 33 | 84 | 71.5277778 | 99.5215311 | blastp |
| 453 | cryptomeria\|gb166\| AU036730_T 1 | cryptomeria | 1800 | 33 | 85 | 98.6111111 | 99.6515679 | blastp |
| 454 | dandelion\|gb161\| DY814032_T1 | dandelion | 1801 | 33 | 84 | 82.2916667 | 93.7007874 | blastp |
| 455 | dandelion\|gb161\| DY802714_T1 | dandelion | 1802 | 33 | 82 | 98.2638889 | 99.3031359 | blastp |
| 456 | dandelion\|gb161\| DY822683_T1 | dandelion | 1803 | 33 | 84 | 95.8333333 | 99.6415771 | blastp |
| 457 | dandelion\|gb161\| DY806788_T1 | dandelion | 1804 | 33 | 87 | 98.2638889 | 98.9583333 | blastp |
| 458 | dandelion\|gb161\| DY808781_T1 | dandelion | 1805 | 33 | 84 | 84.0277778 | 99.5918367 | blastp |
| 459 | dandelion\|gb161\| DY810613_T1 | dandelion | 1806 | 33 | 81 | 76.3888889 | 94.8497854 | blastp |
| 460 | fescue\|gb161\|CK 803261_T1 | fescue | 1807 | 33 | 85 | 97.9166667 | 98.6111111 | blastp |
| 461 | fescue\|gb161\|DT 682664_T1 | fescue | 1808 | 33 | 84 | 67.3611111 | 99.4923858 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 462 | fescue\|gb161\|DT 677062_T1 | fescue | 1809 | 33 | 83 | 98.2638889 | 98.9655172 | blastp |
| 463 | fescue\|gb161\|DT 679061_T1 | fescue | 1810 | 33 | 86 | 98.2638889 | 98.9619377 | blastp |
| 464 | flax\|gb157.3\|CV 478314_T1 | flax | 1811 | 33 | 84 | 71.875 | 99.5260664 | blastp |
| 465 | ginger\|gb164\|DY 345344_T1 | ginger | 1812 | 33 | 88 | 98.2638889 | 98.951049 | blastp |
| 466 | ginger\|gb164\|DY 358322_T1 | ginger | 1813 | 33 | 85 | 98.2638889 | 98.9473684 | blastp |
| 467 | ginger\|gb164\|DY 360757_T1 | ginger | 1814 | 33 | 84 | 98.6111111 | 99.6478873 | blastp |
| 468 | ginger\|gb164\|DY 345596_T1 | ginger | 1815 | 33 | 86 | 98.2638889 | 98.9473684 | blastp |
| 469 | grape\|gb164\|AF1 88844_T1 | grape | 1816 | 33 | 87 | 98.2638889 | 98.9547038 | blastp |
| 470 | grape\|gb160\|AF1 88843_T1 | grape | 1817 | 33 | 85 | 98.2638889 | 98.951049 | blastp |
| 471 | grape\|gb160\|AF1 88843_T3 | grape | 1818 | 33 | 85 | 98.2638889 | 98.951049 | blastp |
| 472 | grape\|gb160\|CB 971128_T1 | grape | 1819 | 33 | 87 | 98.2638889 | 99.3006993 | blastp |
| 473 | grape\|gb160\|AF1 88843_T4 | grape | 1820 | 33 | 84 | 72.2222222 | 86.3070539 | blastp |
| 474 | iceplant\|gb164\| MCU26537_T1 | iceplant | 1821 | 33 | 85 | 98.2638889 | 98.9473684 | blastp |
| 475 | iceplant\|gb164\|C IPMIPA_T1 | iceplant | 1822 | 33 | 85 | 98.2638889 | 99.2957746 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 476 | iceplant\|gb164\|C IPMIPB_T1 | iceplant | 1823 | 33 | 87 | 98.2638889 | 98.9473684 | blastp |
| 477 | ipomoea\|gb157.2 \|BM878883_T1 | ipomoea | 1824 | 33 | 87 | 98.9583333 | 99.3031359 | blastp |
| 478 | ipomoea\|gb157.2 \|BJ553988_T1 | ipomoea | 1825 | 33 | 85 | 98.6111111 | 99.6491228 | blastp |
| 479 | ipomoea\|gb157.2 \|BJ553369_T1 | ipomoea | 1826 | 33 | 84 | 98.6111111 | 99.6478873 | blastp |
| 480 | ipomoea\|gb157.2 \|BJ553198_T1 | ipomoea | 1827 | 33 | 89 | 98.9583333 | 99.3031359 | blastp |
| 481 | lettuce\|gb157.2\| DW079915_T1 | lettuce | 1828 | 33 | 86 | 98.2638889 | 97.9310345 | blastp |
| 482 | lettuce\|gb157.2\| DW043941_T1 | lettuce | 1829 | 33 | 87 | 98.2638889 | 98.9547038 | blastp |
| 483 | lettuce\|gb157.2\| DW047538_T1 | lettuce | 1830 | 33 | 87 | 96.875 | 98.245614 | blastp |
| 484 | lettuce\|gb157.2\| DW104582_T1 | lettuce | 1831 | 33 | 84 | 98.2638889 | 99.3031359 | blastp |
| 485 | lettuce\|gb157.2\| DW044606_T1 | lettuce | 1832 | 33 | 84 | 98.2638889 | 99.3031359 | blastp |
| 486 | lettuce\|gb157.2\| DW148209_T1 | lettuce | 1833 | 33 | 85 | 89.2361111 | 94.1605839 | blastp |
| 487 | lettuce\|gb157.2\| DW148478_T1 | lettuce | 1834 | 33 | 83 | 98.6111111 | 95.3333333 | blastp |
| 488 | lettuce\|gb157.2\| DW108503_T1 | lettuce | 1835 | 33 | 86 | 98.2638889 | 98.9547038 | blastp |
| 489 | lettuce\|gb157.2\| DW046100_T1 | lettuce | 1836 | 33 | 86 | 96.875 | 99.6441281 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 490 | lettuce\|gb157.2\| DW075079_T1 | lettuce | 1837 | 33 | 85 | 98.2638 889 | 99.3031 359 | blastp |
| 491 | lettuce\|gb157.2\| DW076402_T1 | lettuce | 1838 | 33 | 87 | 98.2638 889 | 98.9547 038 | blastp |
| 492 | lettuce\|gb157.2\| DW084041_T1 | lettuce | 1839 | 33 | 86 | 89.5833 333 | 92.8315 412 | blastp |
| 493 | lettuce\|gb157.2\| DW145601_T1 | lettuce | 1840 | 33 | 87 | 98.2638 889 | 98.9547 038 | blastp |
| 494 | lettuce\|gb157.2\| CV699980_T1 | lettuce | 1841 | 33 | 86 | 98.2638 889 | 98.9547 038 | blastp |
| 495 | lettuce\|gb157.2\| DW064849_T1 | lettuce | 1842 | 33 | 87 | 98.2638 889 | 98.9547 038 | blastp |
| 496 | lettuce\|gb157.2\| DW147179_T1 | lettuce | 1843 | 33 | 83 | 98.6111 111 | 89.0965 732 | blastp |
| 497 | lettuce\|gb157.2\| DW045991_T1 | lettuce | 1844 | 33 | 83 | 98.6111 111 | 97.2789 116 | blastp |
| 498 | lotus\|gb157.2\|AF 145707_T1 | lotus | 1845 | 33 | 84 | 98.2638 889 | 99.3079 585 | blastp |
| 499 | lotus\|gb157.2\|AI 967594_T1 | lotus | 1846 | 33 | 86 | 96.875 | 98.2456 14 | blastp |
| 500 | lotus\|gb157.2\|AF 145708_T1 | lotus | 1847 | 33 | 87 | 66.6666 667 | 100 | blastp |
| 501 | maize\|gb164\|EC 881658_T1 | maize | 1848 | 33 | 86 | 54.5138 889 | 98.7421 384 | blastp |
| 502 | maize\|gb164\|AI3 72377_T1 | maize | 1849 | 33 | 85 | 98.2638 889 | 98.9583 333 | blastp |
| 503 | maize\|gb164\|AF 145706_T1 | maize | 1850 | 33 | 83 | 60.7638 889 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 504 | maize\|gb164\|AI8 55222_T1 | maize | 1851 | 33 | 84 | 98.2638 889 | 98.9726 027 | blastp |
| 505 | maize\|gb164\|AI6 19392_T1 | maize | 1852 | 33 | 86 | 98.2638 889 | 98.9619 377 | blastp |
| 506 | maize\|gb164\|AI8 61086 T1 | maize | 1853 | 33 | 84 | 98.2638 889 | 98.9583 333 | blastp |
| 507 | medicago\|gb157. 2\|AW684000_T 1 | medicago | 1854 | 33 | 84 | 98.2638 889 | 99.3079 585 | blastp |
| 508 | medicago\|gb157. 2\|AI974398_T1 | medicago | 1855 | 33 | 83 | 98.2638 889 | 99.3079 585 | blastp |
| 509 | medicago\|gb157. 2\|AL366983_T1 | medicago | 1856 | 33 | 88 | 97.5694 444 | 98.6062 718 | blastp |
| 510 | medicago\|gb157. 2\|AI737528_T1 | medicago | 1857 | 33 | 84 | 98.6111 111 | 99.3103 448 | blastp |
| 511 | medicago\|gb157. 2\|BQ151876_T1 | medicago | 1858 | 33 | 84 | 82.2916 667 | 87.2262 774 | blastp |
| 512 | melon\|gb165\|DV 632745_T1 | melon | 1859 | 33 | 84 | 98.2638 889 | 99.3150 685 | blastp |
| 513 | melon\|gb164\|CF 674915_T1 | melon | 1860 | 33 | 83 | 98.2638 889 | 99.3150 685 | blastp |
| 514 | melon\|gb165\|DV 632772_T1 | melon | 1861 | 33 | 85 | 98.2638 889 | 98.9510 49 | blastp |
| 515 | millet\|gb161\|CD 724341_T1 | millet | 1862 | 33 | 83 | 57.2916 667 | 92.1787 709 | blastp |
| 516 | nicotiana_bentha miana\|gb164\|ES 885295_T1 | nicotiana_benthamiana | 1863 | 33 | 84 | 66.6666 667 | 97.9487 179 | blastp |
| 517 | oil_palm\|gb166\| CN600863_T1 | oil_palm | 1864 | 33 | 85 | 98.2638 889 | 98.9547 038 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 518 | oil_palm\|gb166\| CN600797_T1 | oil_palm | 1865 | 33 | 86 | 98.2638 889 | 98.9547 038 | blastp |
| 519 | oniony\|gb162\|AF 255796_T1 | onion | 1866 | 33 | 86 | 98.2638 889 | 98.9583 333 | blastp |
| 520 | papaya\|gb165\|E X228092_T1 | papaya | 1867 | 33 | 84 | 72.2222 222 | 93.2735 426 | blastp |
| 521 | papaya\|gb165\|AJ 000031_T1 | papaya | 1868 | 33 | 85 | 98.2638 889 | 99.3079 585 | blastp |
| 522 | papaya\|gb165\|E X257869_T1 | papaya | 1869 | 33 | 83 | 98.2638 889 | 99.3031 359 | blastp |
| 523 | papaya\|gb165\|A M903842_T1 | papaya | 1870 | 33 | 90 | 98.2638 889 | 98.9510 49 | blastp |
| 524 | peach\|gb157.2\|B U039203_T1 | peach | 1871 | 33 | 84 | 98.2638 889 | 98.9655 172 | blastp |
| 525 | peach\|gb157.2\|B U040913_T1 | peach | 1872 | 33 | 90 | 51.3888 889 | 98.6666 667 | blastp |
| 526 | peanut\|gb161\|C D038184_T1 | peanut | 1873 | 33 | 83 | 98.6111 111 | 99.6539 792 | blastp |
| 527 | peanut\|gb161\|ES 490696_T1 | peanut | 1874 | 33 | 82 | 73.9583 333 | 99.5348 837 | blastp |
| 528 | peanut\|gb164\|C D038104_T1 | peanut | 1875 | 33 | 83 | 98.2638 889 | 99.3079 585 | blastp |
| 529 | pepper\|gb157.2\| CA523071_T1 | pepper | 1876 | 33 | 97 | 96.875 | 100 | blastp |
| 530 | pepper\|gb157.2\| BM063708_T1 | pepper | 1877 | 33 | 95 | 99.3055 556 | 100 | blastp |
| 531 | periwinkle\|gb16 4\|EG554502_T1 | periwinkle | 1878 | 33 | 88 | 98.9583 333 | 99.3031 359 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 532 | periwinkle\|gb16 4\|EG554518_T1 | periwinkle | 1879 | 33 | 87 | 98.9583 333 | 99.3031 359 | blastp |
| 533 | periwinkle\|gb16 4\|EG556773_T1 | periwinkle | 1880 | 33 | 83 | 84.0277 778 | 96.8 | blastp |
| 534 | petunia\|gb157.2\| AF452010_T1 | petunia | 1881 | 33 | 93 | 99.3055 556 | 100 | blastp |
| 535 | petunia\|gb157.2\| CV292775_T1 | petunia | 1882 | 33 | 92 | 61.8055 556 | 100 | blastp |
| 536 | petunia\|gb157.2\| AF452011_T1 | petunia | 1883 | 33 | 86 | 98.2638 889 | 99.3006 993 | blastp |
| 537 | pine\|gb157.2\|AL 751335_T1 | pine | 1884 | 33 | 83 | 98.2638 889 | 98.9583 333 | blastp |
| 538 | pine\|gb157.2\|AA 556193_T1 | pine | 1885 | 33 | 85 | 98.2638 889 | 98.9583 333 | blastp |
| 539 | pine\|gb157.2\|AL 750485 T1 | pine | 1886 | 33 | 85 | 98.2638 889 | 98.9583 333 | blastp |
| 540 | pineapple\|gb157. 2\|DT335964_T1 | pineapple | 1887 | 33 | 83 | 98.2638 889 | 97.9452 055 | blastp |
| 541 | pineapple\|gb157. 2\|DT338557_T1 | pineapple | 1888 | 33 | 86 | 98.2638 889 | 98.9583 333 | blastp |
| 542 | poplar\|gb157.2\|B U817536_T1 | poplar | 1889 | 33 | 83 | 98.2638 889 | 99.3031 359 | blastp |
| 543 | poplar\|gb157.2\|A 1162483_T1 | poplar | 1890 | 33 | 88 | 98.2638 889 | 98.9583 333 | blastp |
| 544 | poplar\|gb157.2\|B 1122420 T1 | poplar | 1891 | 33 | 87 | 97.9166 667 | 99.3031 359 | blastp |
| 545 | poplar\|gb157.2\|A 1165418_T1 | poplar | 1892 | 33 | 85 | 97.9166 667 | 99.3031 359 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 546 | poplar\|gb157.2\|B U817536_T3 | poplar | 1893 | 33 | 82 | 88.1944444 | 92.0863309 | blastp |
| 547 | poplar\|gb157.2\|B U881784_T1 | poplar | 1894 | 33 | 83 | 98.2638889 | 99.3031359 | blastp |
| 548 | potato\|gb157.2\|B E923816_T1 | potato | 1895 | 33 | 85 | 98.2638889 | 99.3006993 | blastp |
| 549 | potato\|gb157.2\|C K260061_T1 | potato | 1896 | 33 | 91 | 62.5 | 98.9010989 | blastp |
| 550 | potato\|gb157.2\|B E924585_T1 | potato | 1897 | 33 | 86 | 98.2638889 | 98.9473684 | blastp |
| 551 | potato\|gb157.2\|B F153976_T1 | potato | 1898 | 33 | 86 | 61.1111111 | 100 | blastp |
| 552 | potato\|gb157.2\|A J487323_T1 | potato | 1899 | 33 | 97 | 100 | 100 | blastp |
| 553 | potato\|gb157.2\|B F154021_T1 | potato | 1900 | 33 | 92 | 99.3055556 | 100 | blastp |
| 554 | potato\|gb157.2\|B G599633_T1 | potato | 1901 | 33 | 95 | 98.9583333 | 99.3031359 | blastp |
| 555 | potato\|gb157.2\|B E922307_T1 | potato | 1902 | 33 | 85 | 98.2638889 | 99.3006993 | blastp |
| 556 | radish\|gb164\|EV 536875_T1 | radish | 1903 | 33 | 86 | 98.2638889 | 98.951049 | blastp |
| 557 | radish\|gb164\|EW 726189_T1 | radish | 1904 | 33 | 83 | 60.0694444 | 96.1111111 | blastp |
| 558 | radish\|gb164\|EX 756217_T1 | radish | 1905 | 33 | 86 | 98.2638889 | 98.951049 | blastp |
| 559 | radish\|gb164\|AB 030696 T1 | radish | 1906 | 33 | 86 | 98.2638889 | 98.951049 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 560 | radish\|gb164\|AB 030695_T1 | radish | 1907 | 33 | 87 | 98.2638889 | 98.951049 | blastp |
| 561 | radish\|gb164\|AB 012044_T1 | radish | 1908 | 33 | 85 | 98.2638889 | 98.951049 | blastp |
| 562 | radish\|gb164\|EV 567230_T1 | radish | 1909 | 33 | 87 | 98.2638889 | 98.9547038 | blastp |
| 563 | radish\|gb164\|EY 936735_T1 | radish | 1910 | 33 | 86 | 98.2638889 | 98.951049 | blastp |
| 564 | rice\|gb157.2\|U37 951_T1 | rice | 1911 | 33 | 86 | 98.2638889 | 98.9619377 | blastp |
| 565 | rice\|gb157.2\|U40 140_T1 | rice | 1912 | 33 | 86 | 98.2638889 | 98.9583333 | blastp |
| 566 | rice\|gb157.2\|BE0 39992_T1 | rice | 1913 | 33 | 82 | 98.2638889 | 98.9583333 | blastp |
| 567 | rice\|gb157.2\|U37 951_T2 | rice | 1914 | 33 | 86 | 73.6111111 | 99.0654206 | blastp |
| 568 | rose\|gb157.2\|BQ 104887_T1 | rose | 1915 | 33 | 83 | 97.5694444 | 98.6206897 | blastp |
| 569 | rose\|gb157.2\|BQ 103877_T1 | rose | 1916 | 33 | 85 | 98.2638889 | 98.9547038 | blastp |
| 570 | rose\|gb157.2\|EC 586734_T1 | rose | 1917 | 33 | 80 | 62.1527778 | 99.4444444 | blastp |
| 571 | rye\|gb164\|BE58 6240_T1 | rye | 1918 | 33 | 83 | 98.2638889 | 98.9726027 | blastp |
| 572 | safflower\|gb162\| EL407054_T1 | sallower | 1919 | 33 | 86 | 89.5833333 | 94.5454545 | blastp |
| 573 | safflower\|gb162\| EL400504_T1 | sallower | 1920 | 33 | 85 | 98.2638889 | 92.5081433 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 574 | safflower\|gb162\| EL400004_T1 | safflower | 1921 | 33 | 83 | 84.375 | 99.5934 959 | blastp |
| 575 | sesame\|gb157.2\| BU668587_T1 | sesame | 1922 | 33 | 91 | 57.2916 667 | 100 | blastp |
| 576 | sesame\|gb157.2\| BU669929 T1 | sesame | 1923 | 33 | 87 | 55.2083 333 | 99.375 | blastp |
| 577 | sorghum\|gb161. xeno\|AI372377_ T1 | sorghum | 1924 | 33 | 85 | 98.2638 889 | 98.9583 333 | blastp |
| 578 | sorghum\|gb161. xeno\|AI861086_ T1 | sorghum | 1925 | 33 | 82 | 98.2638 889 | 98.9655 172 | blastp |
| 579 | sorghum\|gb161. xeno\|SBU87981 T1 | sorghum | 1926 | 33 | 86 | 98.2638 889 | 98.9619 377 | blastp |
| 580 | soybean\|gb166\|C D401115_T1 | soybean | 1927 | 33 | 82 | 98.2638 889 | 99.3031 359 | blastp |
| 581 | soybean\|gb166\|A W348556_T1 | soybean | 1928 | 33 | 84 | 98.6111 111 | 99.6539 792 | blastp |
| 582 | soybean\|gb166\|B E352670_T1 | soybean | 1929 | 33 | 86 | 98.2638 889 | 98.9547 038 | blastp |
| 583 | soybean\|gb166\|B 1967765_T1 | soybean | 1930 | 33 | 86 | 98.2638 889 | 98.9619 377 | blastp |
| 584 | soybean\|gb166\|B E661219_T1 | soybean | 1931 | 33 | 82 | 98.2638 889 | 99.3079 585 | blastp |
| 585 | soybean\|gb166\|B E352747_T5 | soybean | 1932 | 33 | 81 | 88.1944 444 | 98.4732 824 | blastp |
| 586 | soybean\|gb166\|C D416359_T1 | soybean | 1933 | 33 | 85 | 97.5694 444 | 98.6013 986 | blastp |
| 587 | soybean\|gb166\|A W350352_T1 | soybean | 1934 | 33 | 84 | 97.5694 444 | 98.6013 986 | blastp |

EP 2 240 510 B1

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 588 | soybean\|gb166\|B E352747_T1 | soybean | 1935 | 33 | 82 | 98.2638889 | 99.3079585 | blastp |
| 589 | soybean\|gb166\|B E820629_T1 | soybean | 1936 | 33 | 85 | 98.2638889 | 99.2957746 | blastp |
| 590 | spikemoss\|gb165\| \|DN838148_T4 | spikemoss | 1937 | 33 | 82 | 51.0416667 | 99.3243243 | blastp |
| 591 | spruce\|gb162\|CO 224550 T1 | spruce | 1938 | 33 | 80 | 96.875 | 98.9473684 | blastp |
| 592 | spruce\|gb162\|CO 216100 T1 | spruce | 1939 | 33 | 86 | 98.2638889 | 98.9726027 | blastp |
| 593 | spruce\|gb162\|CO 216028 T1 | spruce | 1940 | 33 | 85 | 98.2638889 | 98.9583333 | blastp |
| 594 | spurge\|gb161\|B G354070 T1 | spurge | 1941 | 33 | 87 | 97.9166667 | 98.2638889 | blastp |
| 595 | strawberry\|gb16 4\|CO378647_T1 | strawberry | 1942 | 33 | 82 | 98.2638889 | 99.3103448 | blastp |
| 596 | strawberry\|gb16 4\|CX661400_T1 | strawberry | 1943 | 33 | 84 | 98.2638889 | 98.9547038 | blastp |
| 597 | strawberry\|gb16 4\|DV438296_T1 | strawberry | 1944 | 33 | 83 | 98.2638889 | 98.951049 | blastp |
| 598 | sugarcane\|gb157 .2\|CA264801_T 1 | sugarcane | 1945 | 33 | 80 | 60.0694444 | 88.8888889 | blastp |
| 599 | sugarcane\|gb157 .2\|CA086058_T 1 | sugarcane | 1946 | 33 | 85 | 98.2638889 | 98.9583333 | blastp |
| 600 | sugarcane\|gb157 .2\|CA071197_T 1 | sugarcane | 1947 | 33 | 84 | 97.2222222 | 98.2638889 | blastp |
| 601 | sugarcane\|gb157 .2\|BQ530399_T 1 | sugarcane | 1948 | 33 | 84 | 91.6666667 | 99.2537313 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 602 | sugarcane\|gb157 .2\|CA085969_T 1 | sugarcane | 1949 | 33 | 82 | 74.3055 556 | 99.5391 705 | blastp |
| 603 | sugarcane\|gb157 .2\|CA074778_T 1 | sugarcane | 1950 | 33 | 84 | 71.1805 556 | 93.6936 937 | blastp |
| 604 | sugarcane\|gb157 .2\|CA130651_T 1 | sugarcane | 1951 | 33 | 81 | 62.1527 778 | 99.4505 495 | blastp |
| 605 | sugarcane\|gb157 .2\|AA525652_T 1 | sugarcane | 1952 | 33 | 88 | 53.4722 222 | 98.7179 487 | blastp |
| 606 | sugarcane\|gb157 .2\|BQ536359_T 1 | sugarcane | 1953 | 33 | 86 | 98.2638 889 | 98.9619 377 | blastp |
| 607 | sunflower\|gb162\| DY909123_T1 | sunflower | 1954 | 33 | 86 | 98.2638 889 | 98.9547 038 | blastp |
| 608 | sunflower\|gb162\| CD846367_T1 | sunflower | 1955 | 33 | 85 | 98.2638 889 | 98.9547 038 | blastp |
| 609 | sunflower\|gb162\| CD846084_T1 | sunflower | 1956 | 33 | 86 | 98.2638 889 | 98.9547 038 | blastp |
| 610 | sunflower\|gb162\| DY915760_T1 | sunflower | 1957 | 33 | 84 | 72.2222 222 | 100 | blastp |
| 611 | sunflower\|gb162\| CF080940 T1 | sunflower | 1958 | 33 | 83 | 73.6111 111 | 99.5327 103 | blastp |
| 612 | sunflower\|gb162\| CF087907_T1 | sunflower | 1959 | 33 | 83 | 96.875 | 97.5694 444 | blastp |
| 613 | sunflower\|gb162\| CX946986_T1 | sunflower | 1960 | 33 | 84 | 98.2638 889 | 98.6111 111 | blastp |
| 614 | sunflower\|gb162\| DY918780_T1 | sunflower | 1961 | 33 | 87 | 73.6111 111 | 99.0697 674 | blastp |
| 615 | switchgrass\|gb16 5\|FE619753_T1 | switchgrass | 1962 | 33 | 86 | 98.2638 889 | 98.9583 333 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 616 | switchgrass\|gb16 5\|DN142591_T1 | switchgrass | 1963 | 33 | 85 | 98.2638 889 | 98.9583 333 | blastp |
| 617 | switchgrass\|gb16 5\|DN141716_T1 | switchgrass | 1964 | 33 | 85 | 98.2638 889 | 98.9619 377 | blastp |
| 618 | switchgrass\|gb16 5\|DN141343_T1 | switchgrass | 1965 | 33 | 86 | 98.2638 889 | 98.9583 333 | blastp |
| 619 | switchgrass\|gb16 5\|DN142037_T1 | switchgrass | 1966 | 33 | 85 | 98.2638 889 | 98.9619 377 | blastp |
| 620 | thellungiella\|gb1 57.2\|DN774595_ T1 | thellungiella | 1967 | 33 | 85 | 98.2638 889 | 98.9510 49 | blastp |
| 621 | thellungiella\|gb1 57.2\|BM986095 T1 | thellungiella | 1968 | 33 | 86 | 98.2638 889 | 98.9510 49 | blastp |
| 622 | thellungiella\|gb1 57.2\|BI698563_ T1 | thellungiella | 1969 | 33 | 85 | 72.5694 444 | 99.5238 095 | blastp |
| 623 | tobacco\|gb162\|E B426225 T1 | tobacco | 1970 | 33 | 87 | 98.2638 889 | 98.2578 397 | blastp |
| 624 | tobacco\|gb162\|C K720591 T1 | tobacco | 1971 | 33 | 95 | 99.3055 556 | 99.6515 679 | blastp |
| 625 | tobacco\|gb162\|C K720595 T1 | tobacco | 1972 | 33 | 96 | 73.6111 111 | 99.0654 206 | blastp |
| 626 | tobacco\|gb162\|E B427872 T1 | tobacco | 1973 | 33 | 88 | 98.2638 889 | 99.2982 456 | blastp |
| 627 | tobacco\|gb162\|C K720593 T1 | tobacco | 1974 | 33 | 87 | 97.9166 667 | 98.9473 684 | blastp |
| 628 | tobacco\|gb162\|A F024511 T1 | tobacco | 1975 | 33 | 95 | 99.3055 556 | 99.6515 679 | blastp |
| 629 | tobacco\|gb162\|C K720596 T1 | tobacco | 1976 | 33 | 96 | 98.9583 333 | 99.3031 359 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 630 | tobacco\|gb162\|N TU62280 T1 | tobacco | 1977 | 33 | 96 | 98.9583333 | 99.3031359 | blastp |
| 631 | tomato\|gb164\|A W622243 T1 | tomato | 1978 | 33 | 94 | 98.9583333 | 99.3031359 | blastp |
| 632 | tomato\|gb164\|B G123213 T1 | tomato | 1979 | 33 | 94 | 99.3055556 | 100 | blastp |
| 633 | tomato\|gb164\|AI 637363 T1 | tomato | 1980 | 33 | 87 | 98.2638889 | 98.9473684 | blastp |
| 634 | tomato\|gb164\|B G123955 T1 | tomato | 1981 | 33 | 85 | 98.2638889 | 99.3006993 | blastp |
| 635 | tomato\|gb164\|BP 876517 T1 | tomato | 1982 | 33 | 80 | 55.9027778 | 86.8705036 | tblastn |
| 636 | triphysaria\|gb16 4\|BM357654_T1 | triphysaria | 1983 | 33 | 86 | 73.6111111 | 99.5305164 | blastp |
| 637 | triphysaria\|gb16 4\|EY141207_T1 | triphysaria | 1984 | 33 | 90 | 65.625 | 99.4736842 | blastp |
| 638 | triphysaria\|gb16 4\|DR174621_T1 | triphysaria | 1985 | 33 | 87 | 69.0972222 | 100 | blastp |
| 639 | triphysaria\|gb16 4\|BM356761_T1 | triphysaria | 1986 | 33 | 86 | 88.5416667 | 99.6108949 | blastp |
| 640 | triphysaria\|gb16 4\|DR169763_T1 | triphysaria | 1987 | 33 | 87 | 98.2638889 | 99.6466431 | blastp |
| 641 | triphysaria\|gb16 4\|BM356902_T1 | triphysaria | 1988 | 33 | 86 | 96.5277778 | 99.6428571 | blastp |
| 642 | triphysaria\|gb16 4\|DR174271_T1 | triphysaria | 1989 | 33 | 86 | 92.7083333 | 97.4545455 | blastp |
| 643 | triphysaria\|gb16 4\|DR171777_T1 | triphysaria | 1990 | 33 | 88 | 100 | 99.6539792 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 644 | wheat\|gb164\|BE 406715 T1 | wheat | 1991 | 33 | 83 | 98.2638 889 | 98.9726 027 | blastp |
| 645 | wheat\|gb164\|BQ 838456_T1 | wheat | 1992 | 33 | 83 | 98.2638 889 | 98.9726 027 | blastp |
| 646 | wheat\|gb164\|BE 426386_T1 | wheat | 1993 | 33 | 82 | 98.2638 889 | 98.9726 027 | blastp |
| 647 | wheat\|gb164\|BE 403388_T1 | wheat | 1994 | 33 | 88 | 51.0416 667 | 98.6577 181 | blastp |
| 648 | wheat\|gb164\|BE 403307_T1 | wheat | 1995 | 33 | 83 | 98.2638 889 | 98.9726 027 | blastp |
| 649 | wheat\|gb164\|BE 498268_T1 | wheat | 1996 | 33 | 81 | 60.4166 667 | 96.7213 115 | blastp |
| 650 | wheat\|gb164\|AL 828763_T1 | wheat | 1997 | 33 | 84 | 84.7222 222 | 96.4705 882 | blastp |
| 651 | wheat\|gb164\|AF 139816_T1 | wheat | 1998 | 33 | 83 | 98.2638 889 | 98.9726 027 | blastp |
| 652 | wheat\|gb164\|BE 216990_T1 | wheat | 1999 | 33 | 86 | 98.2638 889 | 98.9583 333 | blastp |
| 653 | wheat\|gb164\|BE 403886_T1 | wheat | 2000 | 33 | 85 | 99.3055 556 | 99.6551 724 | blastp |
| 654 | wheat\|gb164\|BE 430165_T1 | wheat | 2001 | 33 | 85 | 99.3055 556 | 99.6551 724 | blastp |
| 655 | wheat\|gb164\|CA 484202_T1 | wheat | 2002 | 33 | 87 | 56.9444 444 | 97.6190 476 | blastp |
| 656 | wheat\|gb164\|BE 404199_T1 | wheat | 2003 | 33 | 86 | 98.2638 889 | 98.9583 333 | blastp |
| 657 | wheat\|gb164\|BE 406086_T1 | wheat | 2004 | 33 | 83 | 98.2638 889 | 98.9726 027 | blastp |

EP 2 240 510 B1

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 658 | wheat\|gb164\|BF 293776_T1 | wheat | 2005 | 33 | 83 | 98.2638889 | 98.9655172 | blastp |
| 659 | wheat\|gb164\|CK 193386_T1 | wheat | 2006 | 33 | 81 | 97.2222222 | 96.6216216 | blastp |
| 660 | castorbean\|gb16 0\|AJ605572_T1 | castorbean | 2007 | 34 | 80 | 99.1902834 | 98.7854251 | blastp |
| 661 | citrus\|gb157.2\|C K740163_T1 | citrus | 2008 | 34 | 80 | 99.1902834 | 98.7854251 | blastp |
| 662 | coffea\|gb164\|gb157.2\|D V664793_T1 | coffea | 2009 | 34 | 80 | 100 | 100 | blastp |
| 663 | lettuce\|gb157.2\| DW074942_T1 | lettuce | 2010 | 34 | 80 | 99.1902834 | 99.5934959 | blastp |
| 664 | pepper\|gb157.2\| BM063938_T1 | pepper | 2011 | 34 | 95 | 76.1133603 | 100 | blastp |
| 665 | periwinkle\|gb16 4\|EG558295_T1 | periwinkle | 2012 | 34 | 81 | 99.1902834 | 98.7903226 | blastp |
| 666 | potato\|gb157.2\|B M112462_T1 | potato | 2013 | 34 | 98 | 94.7368421 | 99.5744681 | blastp |
| 667 | tobacco\|gb162\|A J237751_T1 | tobacco | 2014 | 34 | 95 | 100 | 100 | blastp |
| 668 | tobacco\|gb162\|E B425012_T1 | tobacco | 2015 | 34 | 93 | 100 | 100 | blastp |
| 669 | nicotiana_bentha miana\|gb162\|CK 284579_T1 | nicotiana_benthamiana | 2016 | 35 | 86 | 74.6177 37 | 97.5903 614 | blastp |
| 670 | potato\|gb157.2\|B G594926_T1 | potato | 2017 | 35 | 96 | 100 | 96.4497 041 | blastp |
| 671 | tomato\|gb164\|D B679435_T1 | tomato | 2018 | 35 | 90 | 76.1467 89 | 100 | blastp |
| 672 | tobacco\|gb162\|C K720588_T1 | tobacco | 2019 | 36 | 81 | 53.5580 524 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 673 | apple\|gb157.3\|C N494715_T1 | apple | 2020 | 37 | 84 | 85.7142857 | 94.7368421 | blastp |
| 674 | apple\|gb157.3\|C 0066689_T1 | apple | 2021 | 37 | 81 | 94.2857143 | 76.1538462 | blastp |
| 675 | castorbean\|gb16 0\|MDL30026M0 01488_T1 | castorbean | 2022 | 37 | 90 | 95.2380952 | 36.900369 | blastp |
| 676 | citrus\|gb157.2\|C X300349_T1 | citrus | 2023 | 37 | 83 | 99.047619 | 72.7272727 | blastp |
| 677 | coffea\|gb157.2\|D V663640_T1 | coffea | 2024 | 37 | 80 | 93.3333333 | 34.5070423 | blastp |
| 678 | cowpea\|gb166\|F F395821-T1 | cowpea | 2025 | 37 | 82 | 93.3333333 | 95.1456311 | blastp |
| 679 | cowpea\|gb166\|F F395821 T2 | cowpea | 2026 | 37 | 82 | 94.2857143 | 13.2450331 | tblastn |
| 680 | ipomoea\|gb257.2 \|CJ769054_T1 | ipomoea | 2027 | 37 | 87 | 100 | 59.3220339 | blastp |
| 681 | lettuce\|gb157.2\| CV699989_T1 | lettuce | 2028 | 37 | 83 | 98.0952381 | 36.5248227 | blastp |
| 682 | medicago\|gb157. 2\|AW208262_T 1 | medicago | 2029 | 37 | 80 | 95.2380952 | 37.1747212 | blastp |
| 683 | melon\|gb165\|A M727408_T1 | melon | 2030 | 37 | 82 | 97.1428571 | 36.9565217 | blastp |
| 684 | poplar\|gb157.2\|C N517706_T1 | poplar | 2031 | 37 | 84 | 96.1904762 | 36.5942029 | blastp |
| 685 | poplar\|gb157.2\|B U813630_T1 | poplar | 2032 | 37 | 84 | 99.047619 | 37.6811594 | blastp |
| 686 | potato\|gb157.2\|D N587628_T1 | potato | 2033 | 37 | 90 | 96.1904762 | 93.5185185 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 687 | rice\|gb157.2\|BI8 05522_T1 | rice | 2034 | 37 | 80 | 97.1428 571 | 35.9154 93 | blastp |
| 688 | soybean\|gb166\|F K397604_T1 | soybean | 2035 | 37 | 82 | 54.2857 143 | 98.2758 621 | blastp |
| 689 | sunflower\|gb162\| DY951259_T1 | sunflower | 2036 | 37 | 82 | 98.0952 381 | 39.0151 515 | blastp |
| 690 | sunflower\|gb162\| DY942645_T1 | sunflower | 2037 | 37 | 83 | 98.0952 381 | 37.3188 406 | blastp |
| 691 | triphysaria\|gb16 4\|EY130232_T1 | triphysaria | 2038 | 37 | 85 | 99.0476 19 | 37.6811 594 | blastp |
| 692 | arabidopsis\|gb16 5\|AT4G10380_T 1 | arabidopsis | 2039 | 38 | 80 | 97.6271 186 | 96.0526 316 | blastp |
| 693 | artemisia\|gb164\| EY089420_T1 | artemisia | 2040 | 38 | 87 | 55.5932 203 | 100 | blastp |
| 694 | artemisia\|gb164\| EY113317_T1 | artemisia | 2041 | 38 | 89 | 51.8644 068 | 100 | blastp |
| 695 | b_oleracea\|gb16 1\|AM391026_T1 | b_oleracea | 2042 | 38 | 81 | 93.8983 051 | 95.8620 69 | blastp |
| 696 | b_rapa\|gb162\|C V545128_T1 | b_rapa | 2043 | 38 | 81 | 97.6271 186 | 96.0132 89 | blastp |
| 697 | canola\|gb161\|ES 903871_T1 | canola | 2044 | 38 | 85 | 52.2033 898 | 100 | blastp |
| 698 | cassava\|gb164\|C K641734_T1 | cassava | 2045 | 38 | 89 | 93.8983 051 | 98.9247 312 | blastp |
| 699 | castorbean\|gb16 0\|EG668085_T1 | castorbean | 2046 | 38 | 85 | 100 | 100 | blastp |
| 700 | castorbean\|gb16 0\|IG668085_T2 | castorbean | 2047 | 38 | 90 | 62.0338 983 | 100 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 701 | centaurea\|gb161\| EH739099_T1 | centaurea | 2048 | 38 | 83 | 69.1525 424 | 98.0487 805 | blastp |
| 702 | centaurea\|gb161\| EH710762_T1 | centaurea | 2049 | 38 | 89 | 80.6779 661 | 95.9677 419 | blastp |
| 703 | citrus\|gb157.2\|C X299695_T1 | citrus | 2050 | 38 | 98 | 62.0338 983 | 100 | blastp |
| 704 | citrus\|gb157.2\|C O912981_T1 | citrus | 2051 | 38 | 80 | 100 | 100 | blastp |
| 705 | citrus\|gb157.2\|C 0912981 T2 | citrus | 2052 | 38 | 81 | 78.3050 847 | 95.5465 587 | blastp |
| 706 | cotton\|gb164\|CO 071578_T1 | cotton | 2053 | 38 | 90 | 77.9661 017 | 95.4356 846 | blastp |
| 707 | cotton\|gb164\|BE 052767_T1 | cotton | 2054 | 38 | 88 | 86.4406 78 | 100 | blastp |
| 708 | grape\|gb160\|CB 350030_T1 | grape | 2055 | 38 | 86 | 100 | 100 | blastp |
| 709 | lettuce\|gb157.2\| DW123895_T1 | lettuce | 2056 | 38 | 86 | 97.6271 186 | 96.6555 184 | blastp |
| 710 | melon\|gb165\|A M726471_T1 | melon | 2057 | 38 | 80 | 78.6440 678 | 92.8 | blastp |
| 711 | nicotiana_bentha miana\|gb162\|CK 281387_T1 | nicotiana_benthamiana | 2058 | 38 | 94 | 100 | 100 | blastp |
| 712 | onion\|gb162\|CF4 36356_T1 | onion | 2059 | 38 | 83 | 86.1016 949 | 98.0988 593 | blastp |
| 713 | poplar\|gb157.2\|B U895174_T1 | poplar | 2060 | 38 | 84 | 97.6271 186 | 96.3333 333 | blastp |
| 714 | poplar\|gb157.2\|B I126692_T1 | poplar | 2061 | 38 | 85 | 100 | 100 | blastp |
| 715 | radish\|gb164\|EX 772276_T1 | radish | 2062 | 38 | 87 | 62.0338 983 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 716 | safflower\|gb162\| EL376221_T1 | safflower | 2063 | 38 | 87 | 55.5932 203 | 94.2528 736 | blastp |
| 717 | soybean\|gb166\|A W351195_T1 | soybean | 2064 | 38 | 83 | 57.2881 356 | 100 | blastp |
| 718 | spurge\|gb161\|D V120704_T1 | spurge | 2065 | 38 | 81 | 66.1016 949 | 100 | blastp |
| 719 | strawberry\|gb16 4\|EX663538_T1 | strawberry | 2066 | 38 | 87 | 73.5593 22 | 100 | blastp |
| 720 | sunflower\|gb162\| BQ915292_T1 | sunflower | 2067 | 38 | 83 | 69.8305 085 | 95.0636 943 | tblastn |
| 721 | tobacco\|gb162\|E B426773_T1 | tobacco | 2068 | 38 | 94 | 100 | 100 | blastp |
| 722 | triphysaria\|gb16 4\|EY008469_T1 | triphysaria | 2069 | 38 | 90 | 67.7966 102 | 100 | blastp |
| 723 | pepper\|gb157.2\| BM066463_T1 | pepper | 2070 | 39 | 91 | 60 | 100 | blastp |
| 724 | tobacco\|gb162\|E B445778_T1 | tobacco | 2071 | 39 | 88 | 85.8333 333 | 100 | blastp |
| 725 | pepper\|gb157.2\| CA515996_T1 | pepper | 2072 | 40 | 82 | 64.4628 099 | 100 | blastp |
| 726 | potato\|gb157.2\|B E341068_T1 | potato | 2073 | 40 | 92 | 100 | 100 | blastp |
| 727 | potato\|gb157.2\|B G887984_T1 | potato | 2074 | 41 | 100 | 79.4238 683 | 91.4691 943 | blastp |
| 728 | apple\|gb157.3\|C N898142_T1 | apple | 2075 | 42 | 86 | 70.9677 419 | 98.0582 524 | blastp |
| 729 | apple\|gb157.3\|C N492544_T1 | apple | 2076 | 42 | 82 | 99.6415 771 | 98.9399 293 | blastp |
| 730 | apple\|gb157.3\|C N495819_T1 | apple | 2077 | 42 | 84 | 99.6415 771 | 98.9547 038 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 731 | apple\|gb157.3\|C N869175_T1 | apple | 2078 | 42 | 86 | 100 | 100 | blastp |
| 732 | apple\|gb157.3\|C N488973_T1 | apple | 2079 | 42 | 87 | 100 | 100 | blastp |
| 733 | apricot\|gb157.2\| CB820380_T1 | apricot | 2080 | 42 | 88 | 69.8924 731 | 98.4848 485 | blastp |
| 734 | aquilegia\|gb157. 3\|DR921860_T1 | aquilegia | 2081 | 42 | 85 | 94.9820 789 | 100 | blastp |
| 735 | arabidopsis\|gb16 5\|AT2G37170_T 1 | arabidopsis | 2082 | 42 | 80 | 100 | 99.2982 456 | blastp |
| 736 | arabidopsis\|gb16 5\|AT3G54820_T 1 | arabidopsis | 2083 | 42 | 81 | 95.6989 247 | 94.7552 448 | blastp |
| 737 | arabidopsis\|gb16 5\|AT3G53420_T 1 | arabidopsis | 2084 | 42 | 81 | 100 | 99.3031 359 | blastp |
| 738 | arabidopsis\|gb16 5\|AT5G60660_T 1 | arabidopsis | 2085 | 42 | 80 | 99.2831 541 | 97.2508 591 | blastp |
| 739 | artemisia\|gb164\| EY056827_T1 | artemisia | 2086 | 42 | 89 | 79.5698 925 | 100 | blastp |
| 740 | artemisia\|gb164\| EY033689_T1 | artemisia | 2087 | 42 | 83 | 100 | 100 | blastp |
| 741 | artemisia\|gb164\| EY032199_T1 | artemisia | 2088 | 42 | 81 | 100 | 100 | blastp |
| 742 | artemisia\|gb164\| EY042731_T1 | artemisia | 2089 | 42 | 88 | 100 | 100 | blastp |
| 743 | artemisia\|gb164\| EX980079_T1 | artemisia | 2090 | 42 | 82 | 99.6415 771 | 98.9473 684 | blastp |
| 744 | avocado\|gb164\|C K754546_T1 | avocado | 2091 | 42 | 86 | 51.6129 032 | 97.2972 973 | blastp |
| 745 | b_juncea\|gb164\| EVGN00454408 761136_T1 | b_juncea | 2092 | 42 | 81 | 100 | 99.2982 456 | blastp |
| 746 | b_juncea\|gb164\| EVGN00454408 761136 T2 | b_juncea | 2093 | 42 | 81 | 98.9247 312 | 97.9020 979 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 747 | b_juncea\|gb164\| EVGN00748222 952488_T2 | b_juncea | 2094 | 42 | 80 | 100 | 88.7850 467 | blastp |
| 748 | b_juncea\|gb164\| EVGN00204411 253360_T1 | b_juncea | 2095 | 42 | 84 | 82.0788 53 | 97.8991 597 | blastp |
| 749 | b_juncea\|gb164\| EVGN00054208 600715_T1 | b_juncea | 2096 | 42 | 80 | 100 | 99.2982 456 | blastp |
| 750 | b_juncea\|gb164\| EVGN00049614 332152_T1 | b_juncea | 2097 | 42 | 83 | 64.5161 29 | 96.2566 845 | blastp |
| 751 | b_juncea\|gb164\| EVGN01023711 071914_T1 | b_juncea | 2098 | 42 | 85 | 58.4229 391 | 100 | blastp |
| 752 | b_juncea\|gb164\| EVGN00247216 171316_T1 | b_juncea | 2099 | 42 | 81 | 100 | 99.3031 359 | blastp |
| 753 | b_juncea\|gb164\| EVGN00778009 020884_T1 | b_juncea | 2100 | 42 | 88 | 64.1577 061 | 100 | blastp |
| 754 | b_juncea\|gb164\| EVGN02648808 940517_T1 | b_juncea | 2101 | 42 | 85 | 53.4050 179 | 98.6754 967 | blastp |
| 755 | b_juncea\|gb164\| EVGN00316414 413452_T1 | b_juncea | 2102 | 42 | 82 | 82.7956 989 | 100 | blastp |
| 756 | b_juncea\|gb164\| DT317706_T1 | b_juncea | 2103 | 42 | 81 | 100 | 99.3031 359 | blastp |
| 757 | b_juncea\|gb164\| EVGN00748222 952488_T1 | b_juncea | 2104 | 42 | 81 | 100 | 99.3031 359 | blastp |
| 758 | b_oleracea\|gb16 1\|AM386520_T1 | b_oleracea | 2105 | 42 | 80 | 100 | 100 | blastp |
| 759 | b_oleracea\|gb16 1\|AM058395_T1 | b_oleracea | 2106 | 42 | 83 | 57.3476 703 | 91.9540 23 | blastp |
| 760 | b_oleracea\|gb16 1\|AM385504_T1 | b_oleracea | 2107 | 42 | 81 | 100 | 99.2982 456 | blastp |
| 761 | b_rapa\|gb162\|B G544498_T1 | b_rapa | 2108 | 42 | 81 | 100 | 100 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 762 | b_rapa\|gb162\|B Q791962_T2 | b_rapa | 2109 | 42 | 81 | 100 | 99.2982 456 | blastp |
| 763 | b_rapa\|gb162\|B Q791962_T1 | b_rapa | 2110 | 42 | 81 | 100 | 99.2982 456 | blastp |
| 764 | b_rapa\|gb162\|E X065729_T1 | b_rapa | 2111 | 42 | 81 | 92.1146 953 | 100 | blastp |
| 765 | b_rapa\|gb162\|C A992278_T1 | b_rapa | 2112 | 42 | 83 | 89.9641 577 | 98.8372 093 | blastp |
| 766 | b_rapa\|gb162\|C 0749284_T1 | b_rapa | 2113 | 42 | 81 | 100 | 99.2982 456 | blastp |
| 767 | barley\|gb157.3\|B E412486_T1 | barley | 2114 | 42 | 81 | 100 | 100 | blastp |
| 768 | bean\|gb164\|CB5 42746_T1 | bean | 2115 | 42 | 80 | 100 | 100 | blastp |
| 769 | bean\|gb164\|CB2 80567_T1 | bean | 2116 | 42 | 86 | 99.6415 771 | 98.9473 684 | blastp |
| 770 | bean\|gb164\|BQ4 81649_T1 | bean | 2117 | 42 | 82 | 100 | 100 | blastp |
| 771 | bean\|gb164\|CV5 32291_T1 | bean | 2118 | 42 | 86 | 99.6415 771 | 98.9547 038 | blastp |
| 772 | brachypodium\|g b161.xeno\|BE41 6137_T1 | brachypodium | 2119 | 42 | 80 | 94.9820 789 | 100 | blastp |
| 773 | brachypodium\|g b161.xeno\|AF13 9814_T1 | brachypodium | 2120 | 42 | 80 | 100 | 100 | blastp |
| 774 | canola\|gb161\|CN 729066_T1 | canola | 2121 | 42 | 81 | 100 | 99.3031 359 | blastp |
| 775 | canola\|gb161\|D Q068169_T1 | canola | 2122 | 42 | 81 | 100 | 99.2982 456 | blastp |
| 776 | canola\|gb161\|AF 118382_T1 | canola | 2123 | 42 | 81 | 100 | 99.3031 359 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 777 | canola\|gb161\|AF 118383_T1 | canola | 2124 | 42 | 81 | 100 | 100 | blastp |
| 778 | canola\|gb161\|CD 819509_T1 | canola | 2125 | 42 | 80 | 100 | 99.2982 456 | blastp |
| 779 | canola\|gb161\|EE 419467_T1 | canola | 2126 | 42 | 81 | 100 | 100 | blastp |
| 780 | canola\|gb161\|EE 459735_T1 | canola | 2127 | 42 | 81 | 100 | 99.2982 456 | blastp |
| 781 | canola\|gb161\|CX 192356_T1 | canola | 2128 | 42 | 80 | 100 | 100 | blastp |
| 782 | canola\|gb161\|CN 827413_T1 | canola | 2129 | 42 | 81 | 100 | 99.2982 456 | blastp |
| 783 | canola\|gb161\|EE 432011_T1 | canola | 2130 | 42 | 81 | 100 | 99.2982 456 | blastp |
| 784 | cassava\|gb164\|D B922106_T1 | cassava | 2131 | 42 | 81 | 93.9068 1 | 92.5266 904 | blastp |
| 785 | cassava\|gb164\|C K640888_T1 | cassava | 2132 | 42 | 83 | 100 | 100 | blastp |
| 786 | cassava\|gb164\|C K642866_T1 | cassava | 2133 | 42 | 84 | 99.6415 771 | 98.9510 49 | blastp |
| 787 | cassava\|gb164\|C K642551_T1 | cassava | 2134 | 42 | 86 | 100 | 99.3055 556 | blastp |
| 788 | castorbean\|gb16 0\|AJ605565_T1 | castorbean | 2135 | 42 | 86 | 100 | 99.3055 556 | blastp |
| 789 | castorbean\|gb16 0\|AJ605568_T1 | castorbean | 2136 | 42 | 87 | 99.6415 771 | 98.9399 293 | blastp |
| 790 | castorbean\|gb16 0\|EE259660_T1 | castorbean | 2137 | 42 | 85 | 100 | 100 | blastp |
| 791 | centaurea\|gb161\| EL933765_T1 | centaurea | 2138 | 42 | 84 | 87.0967 742 | 92.8571 429 | blastp |
| 792 | centaurea\|gb161\| EL931761_T1 | centaurea | 2139 | 42 | 82 | 75.9856 631 | 89.2561 983 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 793 | cichorium\|gb161\|DT212328_T1 | cichorium | 2140 | 42 | 86 | 100 | 100 | blastp |
| 794 | citrus\|gb157.2\|B Q625054_T1 | citrus | 2141 | 42 | 83 | 100 | 100 | blastp |
| 795 | citrus\|gb157.2\|C F509045_T1 | citrus | 2142 | 42 | 86 | 100 | 99.3031359 | blastp |
| 796 | citrus\|gb157.2\|C B291797_T1 | citrus | 2143 | 42 | 80 | 100 | 84.0707965 | blastp |
| 797 | citrus\|gb157.2\|B Q623325_T1 | citrus | 2144 | 42 | 86 | 100 | 99.3031359 | blastp |
| 798 | citrus\|gb157.2\|B Q623742_T1 | citrus | 2145 | 42 | 88 | 94.9820789 | 99.2619926 | blastp |
| 799 | citrus\|gb157.2\|C F417769_T1 | citrus | 2146 | 42 | 83 | 100 | 99.3031359 | blastp |
| 800 | citrus\|gb157.2\|B Q623128_T1 | citrus | 2147 | 42 | 88 | 68.4587814 | 98.9637306 | blastp |
| 801 | citrus\|gb157.2\|C B293000_T1 | citrus | 2148 | 42 | 82 | 93.1899642 | 98.8847584 | blastp |
| 802 | citrus\|gb157.2\|B Q622991_T1 | citrus | 2149 | 42 | 84 | 96.4157706 | 98.5559567 | blastp |
| 803 | citrus\|gb157.2\|C F503882_T1 | citrus | 2150 | 42 | 83 | 100 | 100 | blastp |
| 804 | cotton\|gb164\|BE 052942_T1 | cotton | 2151 | 42 | 84 | 99.2831541 | 98.5815603 | blastp |
| 805 | cotton\|gb164\|AF 064467_T1 | cotton | 2152 | 42 | 80 | 100 | 100 | blastp |
| 806 | cotton\|gb164\|BG 443494_T1 | cotton | 2153 | 42 | 85 | 100 | 99.2982456 | blastp |
| 807 | cotton\|gb164\|CO 086106_T1 | cotton | 2154 | 42 | 88 | 70.2508961 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 808 | cotton\|gb164\|BQ 406033_T1 | cotton | 2155 | 42 | 82 | 100 | 99.2982 456 | blastp |
| 809 | cotton\|gb164\|CO 109551_T1 | cotton | 2156 | 42 | 82 | 100 | 100 | blastp |
| 810 | cotton\|gb164\|DV 437970_T1 | cotton | 2157 | 42 | 80 | 64.1577 061 | 95.3608 247 | blastp |
| 811 | cotton\|gb164\|AI 725803_T1 | cotton | 2158 | 42 | 84 | 100 | 99.2982 456 | blastp |
| 812 | cotton\|gb164\|CD 486305_T1 | cotton | 2159 | 42 | 81 | 98.9247 312 | 94.0199 336 | blastp |
| 813 | cowpea\|gb166\|E S884222 T2 | cowpea | 2160 | 42 | 85 | 86.3799 283 | 93.5606 061 | blastp |
| 814 | cowpea\|gb166\|E S884222_T1 | cowpea | 2161 | 42 | 86 | 99.6415 771 | 98.9547 038 | blastp |
| 815 | cowpea\|gb166\|F F538675_T1 | cowpea | 2162 | 42 | 89 | 52.6881 72 | 98 | blastp |
| 816 | cowpea\|gb166\|F C458151_T1 | cowpea | 2163 | 42 | 80 | 100 | 100 | blastp |
| 817 | cowpea\|gb166\|F C458381_T1 | cowpea | 2164 | 42 | 85 | 99.6415 771 | 98.9473 684 | blastp |
| 818 | cryptomeria\|gb1 66\|AU036821_T 1 | cryptomeria | 2165 | 42 | 83 | 95.6989 247 | 93.6395 76 | blastp |
| 819 | cryptomeria\|gb1 66\|BW995927_T 1 | cryptomeria | 2166 | 42 | 81 | 53.0465 95 | 98.0132 45 | blastp |
| 820 | dandelion\|gb161\| DY827637_T1 | dandelion | 2167 | 42 | 87 | 93.9068 1 | 88.9632 107 | blastp |
| 821 | dandelion\|gb161\| DY814583_T1 | dandelion | 2168 | 42 | 85 | 93.5483 871 | 93.2862 191 | blastp |
| 822 | dandelion\|gb161\| DY828216_T1 | dandelion | 2169 | 42 | 85 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 823 | dandelion\|gb161\| DY818322_T1 | dandelion | 2170 | 42 | 80 | 85.3046 595 | 98.7654 321 | blastp |
| 824 | dandelion\|gb161\| DY805523_T1 | dandelion | 2171 | 42 | 82 | 98.9247 312 | 98.2456 14 | blastp |
| 825 | fescue\|gb161\|DT 675934_T1 | fescue | 2172 | 42 | 82 | 61.2903 226 | 93.9226 519 | blastp |
| 826 | ginger\|gb164\|DY 345807_T1 | ginger | 2173 | 42 | 81 | 100 | 100 | blastp |
| 827 | ginger\|gb164\|DY 373920_T1 | ginger | 2174 | 42 | 88 | 69.5340 502 | 100 | blastp |
| 828 | grape\|gb160\|BQ 792080_T1 | grape | 2175 | 42 | 81 | 100 | 99.3031 359 | blastp |
| 829 | grape\|gb160\|CB 973593_T1 | grape | 2176 | 42 | 84 | 100 | 100 | blastp |
| 830 | grape\|gb160\|BM 437196_T1 | grape | 2177 | 42 | 84 | 100 | 99.2957 746 | blastp |
| 831 | iceplant\|gb164\|C IPMIPC_T1 | iceplant | 2178 | 42 | 83 | 100 | 100 | blastp |
| 832 | iceplant\|gb164\|B E035661_T1 | iceplant | 2179 | 42 | 82 | 99.6415 771 | 98.6254 296 | blastp |
| 833 | ipomoea\|gb157.2 \|BM878800_T1 | ipomoea | 2180 | 42 | 80 | 99.6415 771 | 100 | blastp |
| 834 | ipomoea\|gb157.2 \|AU224434_T2 | ipomoea | 2181 | 42 | 90 | 86.7383 513 | 93.2330 827 | blastp |
| 835 | ipomoea\|gb157.2 \|BJ553793_T1 | ipomoea | 2182 | 42 | 82 | 100 | 99.2957 746 | blastp |
| 836 | ipomoea\|gb157.2 \|AU224434_T1 | ipomoea | 2183 | 42 | 89 | 100 | 100 | blastp |
| 837 | lettuce\|gb157.2\| DW115660_T1 | lettuce | 2184 | 42 | 85 | 99.2831 541 | 98.5964 912 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 838 | lettuce\|gb157.2\| DW113963_T1 | lettuce | 2185 | 42 | 80 | 97.8494 624 | 95.7894 737 | blastp |
| 839 | lettuce\|gb157.2\| DW110249_T1 | lettuce | 2186 | 42 | 84 | 99.6415 771 | 98.9399 293 | blastp |
| 840 | lettuce\|gb157.2\| DW051453_T1 | lettuce | 2187 | 42 | 86 | 100 | 100 | blastp |
| 841 | lettuce\|gb157.2\| DW076507_T1 | lettuce | 2188 | 42 | 84 | 100 | 100 | blastp |
| 842 | lettuce\|gb157.2\| DW127617_T1 | lettuce | 2189 | 42 | 84 | 100 | 100 | blastp |
| 843 | lettuce\|gb157.2\| AJ937963_T1 | lettuce | 2190 | 42 | 80 | 100 | 100 | blastp |
| 844 | lettuce\|gb157.2\| DW049421_T1 | lettuce | 2191 | 42 | 83 | 92.8315 412 | 98.5018 727 | blastp |
| 845 | lettuce\|gb157.2\| DW114305_T1 | lettuce | 2192 | 42 | 87 | 99.2831 541 | 98.9473 684 | blastp |
| 846 | lettuce\|gb157.2\| DW053722_T1 | lettuce | 2193 | 42 | 80 | 100 | 100 | blastp |
| 847 | lettuce\|gb157.2\| DW146178_T1 | lettuce | 2194 | 42 | 81 | 100 | 100 | blastp |
| 848 | lettuce\|gb157.2\| DW077710_T1 | lettuce | 2195 | 42 | 85 | 99.2831 541 | 98.5964 912 | blastp |
| 849 | lettuce\|gb157.2\| DW070566_T1 | lettuce | 2196 | 42 | 84 | 100 | 100 | blastp |
| 850 | lettuce\|gb157.2\| DW093078_T1 | lettuce | 2197 | 42 | 85 | 94.9820 789 | 100 | blastp |
| 851 | lettuce\|gb157.2\| DW074446_T1 | lettuce | 2198 | 42 | 84 | 97.4910 394 | 96.1672 474 | blastp |
| 852 | lettuce\|gb157.2\| DW153482_T1 | lettuce | 2199 | 42 | 83 | 99.6415 771 | 98.9399 293 | blastp |
| 853 | lettuce\|gb157.2\| DW080306_T1 | lettuce | 2200 | 42 | 86 | 100 | 100 | blastp |
| 854 | lettuce\|gb157.2\| DW043674_T1 | lettuce | 2201 | 42 | 84 | 99.6415 771 | 98.9399 293 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 855 | lettuce\|gb157.2\| DW095979_T1 | lettuce | 2202 | 42 | 83 | 97.1326165 | 98.5559567 | blastp |
| 856 | lettuce\|gb157.2\| DW077206_T1 | lettuce | 2203 | 42 | 84 | 100 | 100 | blastp |
| 857 | lettuce\|gb157.2\| DW047573_T1 | lettuce | 2204 | 42 | 86 | 98.9247312 | 99.6453901 | blastp |
| 858 | lettuce\|gb157.2\| DW096304_T1 | lettuce | 2205 | 42 | 87 | 100 | 100 | blastp |
| 859 | lettuce\|gb157.2\| DW075191_T1 | lettuce | 2206 | 42 | 81 | 100 | 100 | blastp |
| 860 | lotus\|gb157.2\|AI 967757_T1 | lotus | 2207 | 42 | 85 | 99.6415771 | 98.9547038 | blastp |
| 861 | lotus\|gb157.2\|AI 967387 T2 | lotus | 2208 | 42 | 80 | 99.6415771 | 99.6515679 | blastp |
| 862 | lotus\|gb157.2\|B G662315_T1 | lotus | 2209 | 42 | 82 | 99.6415771 | 98.9619377 | blastp |
| 863 | maize\|gb164\|BE 552783_T1 | maize | 2210 | 42 | 80 | 97.4910394 | 95.890411 | blastp |
| 864 | maize\|gb164\|AI6 22334_T1 | maize | 2211 | 42 | 83 | 97.4910394 | 95.862069 | blastp |
| 865 | maize\|gb164\|AI8 55280_T1 | maize | 2212 | 42 | 81 | 96.7741935 | 95.1557093 | blastp |
| 866 | medicago\|gb157. 2\|AW981259_T 1 | medicago | 2213 | 42 | 80 | 100 | 99.3031359 | blastp |
| 867 | medicago\|gb157. 2\|AA660788_T1 | medicago | 2214 | 42 | 83 | 99.6415771 | 98.9547038 | blastp |
| 868 | melon\|gb165\|DV 631824_T1 | melon | 2215 | 42 | 84 | 100 | 100 | blastp |
| 869 | melon\|gb165\|DV 633977_T1 | melon | 2216 | 42 | 83 | 64.516129 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 870 | melon\|gb165\|A M720039_T1 | melon | 2217 | 42 | 81 | 86.0215054 | 100 | blastp |
| 871 | nicotiana_bentha miana\|gb162\|CN 743200_T1 | nicotiana_benthamiana | 2218 | 42 | 80 | 100 | 100 | blastp |
| 872 | nicotiana_bentha miana\|gb162\|CK 294539_T1 | nicotiana_benthamiana | 2219 | 42 | 80 | 98.2078853 | 96.8641115 | blastp |
| 873 | onion\|gb162\|AF 255795_T1 | onion | 2220 | 42 | 82 | 97.1326165 | 95.532646 | blastp |
| 874 | onion\|gb162\|CF4 34704_T1 | onion | 2221 | 42 | 80 | 99.2831541 | 99.2957746 | blastp |
| 875 | papaya\|gb165\|E L784273_T1 | papaya | 2222 | 42 | 82 | 82.078853 | 97.5206612 | blastp |
| 876 | papaya\|gb165\|A M904340_T1 | papaya | 2223 | 42 | 84 | 100 | 99.2882562 | blastp |
| 877 | peach\|gb157.2\|A F367458_T1 | peach | 2224 | 42 | 84 | 87.0967742 | 100 | blastp |
| 878 | peach\|gb157.2\|B U040116_T1 | peach | 2225 | 42 | 83 | 99.6415771 | 98.9547038 | blastp |
| 879 | peach\|gb157.2\|A F367460_T1 | peach | 2226 | 42 | 86 | 87.0967742 | 100 | blastp |
| 880 | peanut\|gb161\|C D037924_T1 | peanut | 2227 | 42 | 86 | 99.6415771 | 98.9547038 | blastp |
| 881 | peanut\|gb161\|C D038296_T1 | peanut | 2228 | 42 | 85 | 99.6415771 | 98.9547038 | blastp |
| 882 | peanut\|gb161\|C D037924_T2 | peanut | 2229 | 42 | 86 | 99.6415771 | 98.9547038 | blastp |
| 883 | peanut\|gb161\|C D037884_T1 | peanut | 2230 | 42 | 88 | 51.2544803 | 97.9452055 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 884 | pepper\|gb157.2\| BM061612_T1 | pepper | 2231 | 42 | 96 | 100 | 100 | blastp |
| 885 | pepper\|gb157.2\| BM061611_T1 | pepper | 2232 | 42 | 97 | 100 | 100 | blastp |
| 886 | pepper\|gb157.2\| BM061005_T1 | pepper | 2233 | 42 | 81 | 92.4731 183 | 97.3977 695 | blastp |
| 887 | petunia\|gb157.2\| AF452014_T1 | petunia | 2234 | 42 | 83 | 97.4910 394 | 98.2332 155 | blastp |
| 888 | petunia\|gb157.2\| CV295523_T1 | petunia | 2235 | 42 | 95 | 53.0465 95 | 93.6708 861 | blastp |
| 889 | petuia\|gb157.2\| CV293001_T1 | petunia | 2236 | 42 | 83 | 55.5555 556 | 100 | blastp |
| 890 | pine\|gb157.2\|AI8 13221_T1 | pine | 2237 | 42 | 81 | 96.0573 477 | 94.3262 411 | blastp |
| 891 | pine\|gb157.2\|CA 844411_T1 | pine | 2238 | 42 | 81 | 56.6308 244 | 98.75 | blastp |
| 892 | poplar\|gb157.2\|B 1130501 T3 | poplar | 2239 | 42 | 85 | 100 | 99.2982 456 | blastp |
| 893 | poplar\|gb157.2\|A 1165755_T1 | poplar | 2240 | 42 | 86 | 99.6415 771 | 98.9473 684 | blastp |
| 894 | poplar\|gb157.2\|B U835712_T1 | poplar | 2241 | 42 | 85 | 99.6415 771 | 98.9473 684 | blastp |
| 895 | poplar\|gb157.2\|B 1130501_T1 | poplar | 2242 | 42 | 85 | 100 | 99.2982 456 | blastp |
| 896 | poplar\|gb157.2\|B 1130501 T4 | poplar | 2243 | 42 | 85 | 100 | 99.2982 456 | blastp |
| 897 | poplar\|gb157.2\|A I162288_T1 | poplar | 2244 | 42 | 86 | 99.2831 541 | 98.5964 912 | blastp |
| 898 | poplar\|gb257.2\|A J534524_T1 | poplar | 2245 | 42 | 84 | 100 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 899 | potato\|gb157.2\|B G096672_T1 | potato | 2246 | 42 | 96 | 100 | 100 | blastp |
| 900 | potato\|gb157.2\|B M109370_T1 | potato | 2247 | 42 | 80 | 98.2078853 | 96.8641115 | blastp |
| 901 | potato\|gb157.2\|B G589618_T1 | potato | 2248 | 42 | 80 | 98.2078853 | 96.8641115 | blastp |
| 902 | potato\|gb157.2\|C K261080_T1 | potato | 2249 | 42 | 83 | 70.2508961 | 100 | blastp |
| 903 | potato\|gb157.2\|B G098124_T1 | potato | 2250 | 42 | 97 | 99.2831541 | 100 | blastp |
| 904 | potato\|gb157.2\|B I406400_T1 | potato | 2251 | 42 | 80 | 98.2078853 | 96.8641115 | blastp |
| 905 | potato\|gb157.2\|B E920139_T1 | potato | 2252 | 42 | 90 | 100 | 100 | blastp |
| 906 | potato\|gb157.2\|B M112017_T1 | potato | 2253 | 42 | 80 | 98.2078853 | 96.8641115 | blastp |
| 907 | potato\|gb157.2\|B E921679_T1 | potato | 2254 | 42 | 96 | 100 | 100 | blastp |
| 908 | potato\|gb157.2\|B G600158_T1 | potato | 2255 | 42 | 80 | 98.2078853 | 96.8641115 | blastp |
| 909 | potato\|gb157.2\|B I406047_T1 | potato | 2256 | 42 | 80 | 100 | 100 | blastp |
| 910 | potato\|gb157.2\|C K719282_T1 | potato | 2257 | 42 | 80 | 98.2078853 | 96.8641115 | blastp |
| 911 | radish\|gb164\|EV 545956_T1 | radish | 2258 | 42 | 81 | 100 | 99.2982456 | blastp |
| 912 | radish\|gb164\|EX 904869_T1 | radish | 2259 | 42 | 80 | 100 | 100 | blastp |
| 913 | radish\|gb164\|EV 545247_T1 | radish | 2260 | 42 | 80 | 100 | 100 | blastp |
| 914 | radish\|gb164\|EX 756889_T1 | radish | 2261 | 42 | 80 | 100 | 100 | blastp |
| 915 | radish\|gb164\|EV 539533_T1 | radish | 2262 | 42 | 81 | 100 | 99.3031359 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 916 | radish\|gb164\|EV 546186\|T1 | radish | 2263 | 42 | 82 | 55.5555556 | 95.6790123 | blastp |
| 917 | radish\|gb164\|AB 012045_T1 | radish | 2264 | 42 | 81 | 100 | 99.3031359 | blastp |
| 918 | radish\|gb164\|EV 573001_T1 | radish | 2265 | 42 | 83 | 62.0071685 | 98.8571429 | blastp |
| 919 | radish\|gb164\|AB 030698_T1 | radish | 2266 | 42 | 81 | 100 | 100 | blastp |
| 920 | radish\|gb164\|EX 749049_T1 | radish | 2267 | 42 | 83 | 78.4946237 | 100 | blastp |
| 921 | radish\|gb164\|AB 030697_T1 | radish | 2268 | 42 | 80 | 100 | 100 | blastp |
| 922 | radish\|gb164\|EW 735060_T1 | radish | 2269 | 42 | 80 | 96.4157706 | 95.4545455 | blastp |
| 923 | radish\|gb164\|FD 936119_T1 | radish | 2270 | 42 | 83 | 50.5376344 | 100 | blastp |
| 924 | radish\|gb164\|EV 543747_T1 | radish | 2271 | 42 | 81 | 100 | 99.3031359 | blastp |
| 925 | rice\|gb157.2\|BE0 40651 T2 | rice | 2272 | 42 | 80 | 86.7383513 | 94.3820225 | blastp |
| 926 | rice\|gb157.2\|BE0 40651_T1 | rice | 2273 | 42 | 80 | 100 | 100 | blastp |
| 927 | rice\|gb157.2\|AA 754435 T5 | rice | 2274 | 42 | 80 | 85.6630824 | 87.7192982 | blastp |
| 928 | rice\|gb157.2\|AA 754435_T1 | rice | 2275 | 42 | 81 | 100 | 100 | blastp |
| 929 | rose\|gb157.2\|BI9 77420_T1 | rose | 2276 | 42 | 84 | 69.8924731 | 91.627907 | blastp |
| 930 | rose\|gb157.2\|BI9 77750_T1 | rose | 2277 | 42 | 84 | 77.7777778 | 100 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 931 | rose\|gb157.2\|BI9 78110_T1 | rose | 2278 | 42 | 83 | 64.5161 29 | 98.3783 784 | blastp |
| 932 | safflower\|gb162\| EL406648_T1 | safflower | 2279 | 42 | 81 | 98.5663 082 | 100 | blastp |
| 933 | safflower\|gb162\| EL411110_T1 | safflower | 2280 | 42 | 86 | 92.4731 183 | 97.7611 94 | blastp |
| 934 | safflower\|gb162\| EL401452_T1 | safflower | 2281 | 42 | 85 | 100 | 100 | blastp |
| 935 | safflower\|gb162\| EL402424_T1 | safflower | 2282 | 42 | 83 | 97.1326 165 | 100 | blastp |
| 936 | safflower\|gb162\| EL400227_T1 | safflower | 2283 | 42 | 85 | 86.7383 513 | 67.9193 401 | tblastn |
| 937 | sorghum\|gb161. xeno\|AI622334_ T1 | sorghum | 2284 | 42 | 83 | 97.4910 394 | 95.8620 69 | blastp |
| 938 | soybean\|gb166\|C D393286_T1 | soybean | 2285 | 42 | 80 | 99.6415 771 | 99.6515 679 | blastp |
| 939 | soybean\|gb166\|G MU27347_T1 | soybean | 2286 | 42 | 86 | 99.6415 771 | 98.9473 684 | blastp |
| 940 | soybean\|gb166\|A W349289_T1 | soybean | 2287 | 42 | 85 | 99.6415 771 | 98.9473 684 | blastp |
| 941 | soybean\|gb166\|B E823946_T1 | soybean | 2288 | 42 | 87 | 99.6415 771 | 98.9436 62 | blastp |
| 942 | soybean\|gb166\|B E352729 T2 | soybean | 2289 | 42 | 80 | 56.2724 014 | 100 | blastp |
| 943 | soybean\|gb166\|A W350475_T1 | soybean | 2290 | 42 | 85 | 99.6415 771 | 98.9547 038 | blastp |
| 944 | soybean\|gb166\|B I119558_T1 | soybean | 2291 | 42 | 80 | 100 | 100 | blastp |
| 945 | soybean\|gb166\|A W349392_T1 | soybean | 2292 | 42 | 80 | 100 | 100 | blastp |

EP 2 240 510 B1

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 946 | spruce\|gb162\|AF 051202_T1 | spruce | 2293 | 42 | 81 | 96.0573 477 | 94.3262 411 | blastp |
| 947 | spruce\|gb162\|CO 227554_T1 | spruce | 2294 | 42 | 80 | 96.0573 477 | 93.6619 718 | blastp |
| 948 | spruce\|gb162\|CO 220480_T1 | spruce | 2295 | 42 | 80 | 96.4157 706 | 94.6808 511 | blastp |
| 949 | spruce\|gb162\|CO 217151_T1 | spruce | 2296 | 42 | 80 | 96.0573 477 | 94.3262 411 | blastp |
| 950 | spurge\|gb161\|A W990929_T1 | spurge | 2297 | 42 | 80 | 59.8566 308 | 91.5343 915 | blastp |
| 951 | spurge\|gb161\|B G354126_T1 | spurge | 2298 | 42 | 83 | 100 | 100 | blastp |
| 952 | spurge\|gb161\|D V125170_T1 | spurge | 2299 | 42 | 87 | 83.5125 448 | 99.5780 591 | blastp |
| 953 | strawberry\|gb16 4\|DV438166_T1 | strawberry | 2300 | 42 | 85 | 99.6415 771 | 98.9473 684 | blastp |
| 954 | strawberry\|gb16 4\|EX665494_T1 | strawberry | 2301 | 42 | 86 | 100 | 99.2957 746 | blastp |
| 955 | strawberry\|gb16 4\|CO817390_T1 | strawberry | 2302 | 42 | 85 | 100 | 100 | blastp |
| 956 | sugarcane\|gb157 .21BQ536871_T 2 | sugarcane | 2303 | 42 | 85 | 70.2508 961 | 100 | blastp |
| 957 | sugarcane\|gb157 .2\|BU103568_T 1 | sugarcane | 2304 | 42 | 83 | 97.4910 394 | 92.6666 667 | blastp |
| 958 | sugarcane\|gb157 .2\|BQ536871_T 1 | sugarcane | 2305 | 42 | 81 | 97.4910 394 | 95.8620 69 | blastp |
| 959 | sugarcane\|gb157 .2\|BQ535332_T 1 | sugarcane | 2306 | 42 | 84 | 97.4910 394 | 95.8620 69 | blastp |
| 960 | sunflower\|gb162\| CD849689_T1 | sunflower | 2307 | 42 | 83 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 961 | sunflower\|gb162\| CD849494_T1 | sunflower | 2308 | 42 | 83 | 94.9820 789 | 95.0704 225 | blastp |
| 962 | sunflower\|gb162\| CF091932_T1 | sunflower | 2309 | 42 | 81 | 83.5125 448 | 98.75 | blastp |
| 963 | sunflower\|gb162\| DY915644_T1 | sunflower | 2310 | 42 | 81 | 100 | 100 | blastp |
| 964 | sunflower\|gb162\| EL462160_T1 | sunflower | 2311 | 42 | 81 | 78.4946 237 | 88.8446 215 | blastp |
| 965 | sunflower\|gb162\| DY918039_T1 | sunflower | 2312 | 42 | 87 | 95.6989 247 | 100 | blastp |
| 966 | sunflower\|gb162\| DY951506_T1 | sunflower | 2313 | 42 | 87 | 89.9641 577 | 95.5223 881 | blastp |
| 967 | sunflower\|gb162\| DY933519_T1 | sunflower | 2314 | 42 | 84 | 99.6415 771 | 98.9473 684 | blastp |
| 968 | sunflower\|gb162\| DY917102_T1 | sunflower | 2315 | 42 | 82 | 100 | 100 | blastp |
| 969 | sunflower\|gb162\| DY932916_T1 | sunflower | 2316 | 42 | 86 | 58.0645 161 | 100 | blastp |
| 970 | sunflower\|gb162\| CD857425_T1 | sunflower | 2317 | 42 | 84 | 100 | 100 | blastp |
| 971 | sunflower\|gb162\| CD846314_T1 | sunflower | 2318 | 42 | 85 | 53.4050 179 | 97.4683 544 | blastp |
| 972 | sunflower\|gb162\| CX944368_T1 | sunflower | 2319 | 42 | 88 | 68.1003 584 | 89.6226 415 | blastp |
| 973 | sunflower\|gb162\| DY908592\|T1 | sunflower | 2320 | 42 | 80 | 100 | 100 | blastp |
| 974 | switchgrass\|gb16 5\|DN141399_T1 | switchgrass | 2321 | 42 | 81 | 96.7741 935 | 98.9208 633 | blastp |
| 975 | switchgrass\|gb16 5\|FE621421_T1 | switchgrass | 2322 | 42 | 86 | 51.9713 262 | 99.3150 685 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 976 | switchgrass\|gb16 5\|DN145656_T1 | switchgrass | 2323 | 42 | 83 | 97.4910 394 | 95.8620 69 | blastp |
| 977 | switchgrass\|gb16 5\|FE637674_T1 | switchgrass | 2324 | 42 | 81 | 82.4372 76 | 100 | blastp |
| 978 | switchgrass\|gb16 5\|DN141334_T1 | switchgrass | 2325 | 42 | 83 | 77.0609 319 | 94.8497 854 | blastp |
| 979 | switchgrass\|gb16 5\|FE621578_T1 | switchgrass | 2326 | 42 | 83 | 85.6630 824 | 100 | blastp |
| 980 | thellungiella\|gb1 57.2\|DN775526_T1 | thellungiella | 2327 | 42 | 81 | 72.4014 337 | 100 | blastp |
| 981 | tobacco\|gb162\|C K720599_T1 | tobacco | 2328 | 42 | 95 | 100 | 100 | blastp |
| 982 | tobacco\|gb162\|C K720599 T2 | tobacco | 2329 | 42 | 94 | 100 | 100 | blastp |
| 983 | tobacco\|gb162\|E B445427_T1 | tobacco | 2330 | 42 | 81 | 100 | 100 | blastp |
| 984 | tobacco\|gb162\|E B443112_T1 | tobacco | 2331 | 42 | 89 | 100 | 100 | blastp |
| 985 | tobacco\|gb162\|A F154641_T1 | tobacco | 2332 | 42 | 80 | 100 | 100 | blastp |
| 986 | tobacco\|gb162\|E B425288_T1 | tobacco | 2333 | 42 | 94 | 98.2078 853 | 100 | blastp |
| 987 | tomato\|gb164\|B G123951 T2 | tomato | 2334 | 42 | 81 | 92.8315 412 | 97.4074 074 | blastp |
| 988 | tomato\|gb164\|B G123951_T1 | tomato | 2335 | 42 | 80 | 98.2078 853 | 96.8641 115 | blastp |
| 989 | tomato\|gb164\|B G713781_T1 | tomato | 2336 | 42 | 92 | 53.4050 179 | 98.0519 481 | blastp |
| 990 | tomato\|gb164\|A W219533_T1 | tomato | 2337 | 42 | 81 | 97.1326 165 | 98.2142 857 | blastp |
| 991 | triphysaria\|gb16 4\|DR170852_T1 | triphysaria | 2338 | 42 | 88 | 99.2831 541 | 99.2857 143 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 992 | triphysaria\|gb16 4\|BM356582_T1 | triphysaria | 2339 | 42 | 88 | 64.8745 52 | 100 | blastp |
| 993 | triphysaria\|gb16 4\|BE574767_T1 | triphysaria | 2340 | 42 | 80 | 100 | 100 | blastp |
| 994 | wheat\|gb164\|BE 444481_T1 | wheat | 2341 | 42 | 82 | 55.5555 556 | 100 | blastp |
| 995 | wheat\|gb164\|BE 398316_T1 | wheat | 2342 | 42 | 81 | 100 | 100 | blastp |
| 996 | wheat\|gb164\|BE 404002_T1 | wheat | 2343 | 42 | 82 | 51.6129 032 | 99.3055 556 | blastp |
| 997 | apple\|gb157.3\|C N892655_T1 | apple | 2344 | 43 | 84 | 100 | 100 | blastp |
| 998 | apple\|gb157.3\|A U223658_T1 | apple | 2345 | 43 | 85 | 100 | 100 | blastp |
| 999 | aquilegia\|gb157. 3\|DR914359_T1 | aquilegia | 2346 | 43 | 84 | 100 | 100 | blastp |
| 1000 | arabidopsis\|gb16 5\|AT2G16850_T 1 | arabidopsis | 2347 | 43 | 84 | 100 | 100 | blastp |
| 1001 | arabidopsis\|gb16 5\|AT4G35100_T 1 | arabidopsis | 2348 | 43 | 86 | 100 | 100 | blastp |
| 1002 | avocado\|gb164\|C K753629 T1 | avocado | 2349 | 43 | 85 | 66.4310 954 | 92.6108 374 | blastp |
| 1003 | avocado\|gb164\|C K752541_T1 | avocado | 2350 | 43 | 84 | 62.1908 127 | 100 | blastp |
| 1004 | b_juncea\|gb164\| EVGN01060535 361904_T1 | b_juncea | 2351 | 43 | 86 | 67.1378 092 | 100 | blastp |
| 1005 | b_juncea\|gb164\| EVGN00138211 060167_T1 | b_juncea | 2352 | 43 | 86 | 85.1590 106 | 100 | blastp |
| 1006 | b_juncea\|gb164\| EVGN01177009 332048_T1 | b_juncea | 2353 | 43 | 86 | 66.0777 385 | 100 | blastp |
| 1007 | b_juncea\|gb164\| EVGN00071418 640425_T1 | b_juncea | 2354 | 43 | 86 | 90.4593 64 | 100 | blastp |
| 1008 | b_juncea\|gb164\| EVGN01391814 101746 _T1 | b_juncea | 2355 | 43 | 92 | 53.0035 336 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1009 | b_juncea\|gb164\| EVGN00206114 600060_T1 | b_juncea | 2356 | 43 | 85 | 100 | 100 | blastp |
| 1010 | b_oleracea\|gb16 1\|AF314656_T1 | b_oleracea | 2357 | 43 | 85 | 100 | 100 | blastp |
| 1011 | b_oleracea\|gb16 1\|DY029187_T1 | b_oleracea | 2358 | 43 | 86 | 100 | 100 | blastp |
| 1012 | b_oleracea\|gb16 1\|DY014978_T1 | b_oleracea | 2359 | 43 | 87 | 54.7703 18 | 100 | blastp |
| 1013 | b_rapa\|gb162\|B Q791230_T1 | b_rapa | 2360 | 43 | 86 | 77.0318 021 | 97.7375 566 | blastp |
| 1014 | b_rapa\|gb162\|E X033370_T1 | b_rapa | 2361 | 43 | 87 | 100 | 100 | blastp |
| 1015 | b_rapa\|gb162\|C V432651_T1 | b_rapa | 2362 | 43 | 86 | 100 | 100 | blastp |
| 1016 | b_rapa\|gb162\|B G543906_T1 | b_rapa | 2363 | 43 | 85 | 100 | 100 | blastp |
| 1017 | bean\|gb164\|CB5 39790_T1 | bean | 2364 | 43 | 83 | 100 | 100 | blastp |
| 1018 | beet\|gb162\|BVU 60148_T1 | beet | 2365 | 43 | 80 | 100 | 100 | blastp |
| 1019 | canola\|gb161\|D Y007064_T1 | canola | 2366 | 43 | 86 | 100 | 100 | blastp |
| 1020 | canola\|gb161\|CD 815284_T1 | canola | 2367 | 43 | 85 | 100 | 100 | blastp |
| 1021 | canola\|gb161\|CD 817684_T1 | canola | 2368 | 43 | 85 | 100 | 100 | blastp |
| 1022 | canola\|gb161\|CD 821191_T1 | canola | 2369 | 43 | 86 | 100 | 100 | blastp |
| 1023 | canola\|gb161\|CD 820375 _T1 | canola | 2370 | 43 | 87 | 100 | 100 | blastp |
| 1024 | cassava\|gb164\|B M259748 _T1 | cassava | 2371 | 43 | 84 | 100 | 100 | blastp |
| 1025 | castorbean\|gb16 0\|AJ605569_T1 | castorbean | 2372 | 43 | 84 | 100 | 100 | blastp |
| 1026 | castorbean\|gb16 0\|AJ605569_T2 | castorbean | 2373 | 43 | 84 | 69.9646 643 | 95.1219 512 | blastp |
| 1027 | cichorium\|gb161 \|EH704748_T1 | cichorium | 2374 | 43 | 89 | 67.1378 092 | 100 | blastp |
| 1028 | citrus\|gb157.2\|B Q623127_T1 | citrus | 2375 | 43 | 85 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1029 | citrus\|gb157.2\|C X664964_T1 | citrus | 2376 | 43 | 84 | 100 | 100 | blastp |
| 1030 | clover\|gb162\|BB 911260_T1 | clover | 2377 | 43 | 82 | 54.0636 042 | 100 | blastp |
| 1031 | coffea\|gb157.2\|B Q448890_T1 | coffea | 2378 | 43 | 86 | 100 | 100 | blastp |
| 1032 | cotton\|gb164\|AI 726086_T1 | cotton | 2379 | 43 | 84 | 97.8798 587 | 88.0258 9 | blastp |
| 1033 | cotton\|gb164\|CO 098535_T1 | cotton | 2380 | 43 | 82 | 92.2261 484 | 95.5719 557 | blastp |
| 1034 | cotton\|gb164\|BE 051956_T1 | cotton | 2381 | 43 | 83 | 100 | 100 | blastp |
| 1035 | cotton\|gb164\|BQ 414250_T1 | cotton | 2382 | 43 | 85 | 59.0106 007 | 100 | blastp |
| 1036 | cotton\|gb164\|BF 268907_T1 | cotton | 2383 | 43 | 87 | 90.1060 071 | 100 | blastp |
| 1037 | cotton\|gb164\|CO 075847_T1 | cotton | 2384 | 43 | 84 | 62.5441 696 | 100 | blastp |
| 1038 | cotton\|gb164\|BQ 405584_T1 | cotton | 2385 | 43 | 82 | 95.4063 604 | 95.7142 857 | blastp |
| 1039 | cotton\|gb164\|AI 055551_T1 | cotton | 2386 | 43 | 85 | 100 | 100 | blastp |
| 1040 | cowpea\|gb166\|F C456786_T1 | cowpea | 2387 | 43 | 84 | 100 | 100 | blastp |
| 1041 | dandelion\|gb161\| DY803814_T1 | dandelion | 2388 | 43 | 85 | 100 | 100 | blastp |
| 1042 | ginger\|gb164\|DY 347270_T1 | ginger | 2389 | 43 | 80 | 100 | 100 | blastp |
| 1043 | ginger\|gb164\|DY 347296_T1 | ginger | 2390 | 43 | 84 | 94.6996 466 | 92.7335 64 | blastp |
| 1044 | ginger\|gb164\|DY 358056_T1 | ginger | 2391 | 43 | 81 | 100 | 100 | blastp |
| 1045 | ginger\|gb164\|DY 347277_T1 | ginger | 2392 | 43 | 81 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1046 | ginger\|gb164\|DY 360032_T1 | ginger | 2393 | 43 | 81 | 81.9787 986 | 93.9024 39 | blastp |
| 1047 | grape\|gb160\|BM 437910_T1 | grape | 2394 | 43 | 84 | 100 | 100 | blastp |
| 1048 | iceplant\|gb164\| MCU26538_T1 | iceplant | 2395 | 43 | 82 | 100 | 100 | blastp |
| 1049 | ipomoea\|gb157.2 \|BJ553491_T1 | ipomoea | 2396 | 43 | 83 | 100 | 100 | blastp |
| 1050 | lettuce\|gb157.2\| DW046509_T1 | lettuce | 2397 | 43 | 84 | 100 | 100 | blastp |
| 1051 | lettuce\|gb157.2\| DW106553_T1 | lettuce | 2398 | 43 | 84 | 97.8798 587 | 100 | blastp |
| 1052 | lettuce\|gb157.2\| DW146059_T1 | lettuce | 2399 | 43 | 84 | 100 | 100 | blastp |
| 1053 | lettuce\|gb157.2\| DW110223_T1 | lettuce | 2400 | 43 | 83 | 97.1731 449 | 100 | blastp |
| 1054 | lettuce\|gb157.2\| DW079482_T1 | lettuce | 2401 | 43 | 84 | 100 | 100 | blastp |
| 1055 | lettuce\|gb157.2\| DW094572_T1 | lettuce | 2402 | 43 | 83 | 97.5265 018 | 92.8327 645 | blastp |
| 1056 | lotus\|gb157.2\|A W163949_T1 | lotus | 2403 | 43 | 85 | 54.4169 611 | 93.9024 39 | blastp |
| 1057 | lotus\|gb157.2\|AI 967574_T1 | lotus | 2404 | 43 | 82 | 98.5865 724 | 97.5352 113 | blastp |
| 1058 | melon\|gb165\|DV 632217_T1 | melon | 2405 | 43 | 83 | 100 | 100 | blastp |
| 1059 | oil_palm\|gb166\| CN600073_T1 | oil_palm | 2406 | 43 | 80 | 100 | 100 | blastp |
| 1060 | papaya\|gb165\|E X227970_T1 | papaya | 2407 | 43 | 85 | 100 | 100 | blastp |
| 1061 | peach\|gb157.2\|A F367457_T1 | peach | 2408 | 43 | 85 | 85.1590 106 | 99.5833 333 | blastp |
| 1062 | pepper\|gb157.2\| BM066074_T1 | pepper | 2409 | 43 | 96 | 67.4911 661 | 98.9637 306 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1063 | pepper\|gb157.2\| CA518686_T1 | pepper | 2410 | 43 | 88 | 53.7102473 | 100 | blastp |
| 1064 | periwinkle\|gb16 4\|AM232518_T1 | periwinkle | 2411 | 43 | 90 | 100 | 100 | blastp |
| 1065 | petunia\|gb157.2\| AF452013_T1 | petunia | 2412 | 43 | 89 | 100 | 100 | blastp |
| 1066 | poplar\|gb157.2\|A I163573_T1 | poplar | 2413 | 43 | 84 | 100 | 100 | blastp |
| 1067 | poplar\|gb157.2\|A 1162424_T1 | poplar | 2414 | 43 | 83 | 100 | 100 | blastp |
| 1068 | potato\|gb157.2\|B F053675_T1 | potato | 2415 | 43 | 89 | 83.745583 | 100 | blastp |
| 1069 | potato\|gb157.2\|B F459952_T1 | potato | 2416 | 43 | 97 | 100 | 100 | blastp |
| 1070 | radish\|gb164\|EV 526465_T1 | radish | 2417 | 43 | 86 | 100 | 100 | blastp |
| 1071 | radish\|gb164\|EV 539317_T1 | radish | 2418 | 43 | 85 | 100 | 100 | blastp |
| 1072 | radish\|gb164\|EV 525026_T1 | radish | 2419 | 43 | 85 | 100 | 100 | blastp |
| 1073 | rose\|gb157.2\|BQ 103996_T1 | rose | 2420 | 43 | 82 | 100 | 100 | blastp |
| 1074 | safflower\|gb162\| EL372747_T1 | safflower | 2421 | 43 | 83 | 100 | 100 | blastp |
| 1075 | sesame\|gb157.2\| BU669158_T1 | sesame | 2422 | 43 | 88 | 50.8833922 | 100 | blastp |
| 1076 | sesame\|gb157.2\| BU668646_T1 | sesame | 2423 | 43 | 87 | 51.9434629 | 100 | blastp |
| 1077 | soybean\|gb166\|B E823128_T1 | soybean | 2424 | 43 | 82 | 100 | 100 | blastp |
| 1078 | soybean\|gb166\|B E352716_T1 | soybean | 2425 | 43 | 82 | 100 | 100 | blastp |
| 1079 | spruce\|gb162\|CO 217407_T1 | spruce | 2426 | 43 | 80 | 93.2862191 | 93.5714286 | blastp |
| 1080 | spurge\|gb161\|A W821924_T1 | spurge | 2427 | 43 | 84 | 100 | 97.2125436 | blastp |
| 1081 | strawberry\|gb16 4\|CX661107_T1 | strawberry | 2428 | 43 | 82 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1082 | sunflower\|gb162\| CD853582_T1 | sunflower | 2429 | 43 | 85 | 100 | 100 | blastp |
| 1083 | sunflower\|gb162\| DY939653_T1 | sunflower | 2430 | 43 | 80 | 97.1731 449 | 97.8339 35 | blastp |
| 1084 | sunflower\|gb162\| CD849663_T1 | sunflower | 2431 | 43 | 81 | 100 | 100 | blastp |
| 1085 | thellungiella\|gb1 57.2\|DN777165_ T1 | thellungiella | 2432 | 43 | 86 | 72.0848 057 | 90.5829 596 | blastp |
| 1086 | tobacco\|gb162\|C K720587_T1 | tobacco | 2433 | 43 | 90 | 99.6466 431 | 100 | blastp |
| 1087 | tobacco\|gb162\|C K720585_T1 | tobacco | 2434 | 43 | 90 | 100 | 100 | blastp |
| 1088 | tobacco\|gb162\|C V016422_T1 | tobacco | 2435 | 43 | 96 | 59.7173 145 | 100 | blastp |
| 1089 | tobacco\|gb162\|C K720589_T1 | tobacco | 2436 | 43 | 94 | 100 | 100 | blastp |
| 1090 | tomato\|gb164\|B G124140_T1 | tomato | 2437 | 43 | 88 | 100 | 100 | blastp |
| 1091 | triphysaria\|gb16 4\|EY018490_T1 | triphysaria | 2438 | 43 | 83 | 100 | 100 | blastp |
| 1092 | triphysaria\|gb16 4\|EY007858_T1 | triphysaria | 2439 | 43 | 84 | 100 | 100 | blastp |
| 1093 | apple\|gb157.3\|C N494428_T1 | apple | 2440 | 44 | 80 | 79.1390 728 | 93.0232 558 | blastp |
| 1094 | castorbean\|gb16 0\|EG696741_T1 | castorbean | 2441 | 44 | 82 | 99.0066 225 | 97.7272 727 | blastp |
| 1095 | citrus\|gb157.2\|C X074332_T1 | citrus | 2442 | 44 | 82 | 99.0066 225 | 97.6973 684 | blastp |
| 1096 | citrus\|gb157.2\|C X674035_T1 | citrus | 2443 | 44 | 80 | 86.7549 669 | 85.8085 809 | blastp |
| 1097 | cotton\|gb164\|D W234737_T1 | cotton | 2444 | 44 | 80 | 99.6688 742 | 98.3333 333 | blastp |
| 1098 | lettuce\|gb157.2\| DW066284_T1 | lettuce | 2445 | 44 | 80 | 95.0331 126 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1099 | petunia\|gb157.2\| CV298254_T1 | petunia | 2446 | 44 | 84 | 65.8940 397 | 100 | blastp |
| 1100 | potato\|gb157.2\|C V500020_T1 | potato | 2447 | 44 | 94 | 72.1854 305 | 99.5433 79 | blastp |
| 1101 | tobacco\|gb162\|E B426672_T1 | tobacco | 2448 | 44 | 89 | 99.0066 225 | 97.6897 69 | blastp |
| 1102 | brachypodium\|g b161.xeno\|BE41 5047_T1 | brachypodium | 2449 | 46 | 92 | 100 | 100 | blastp |
| 1103 | maize\|gb164\|CF 244342 _T1 | maize | 2450 | 46 | 90 | 90.7630 522 | 100 | blastp |
| 1104 | maize\|gb164\|AF 057183_T1 | maize | 2451 | 46 | 89 | 100 | 100 | blastp |
| 1105 | sorghum\|gb161. xeno\|AF057183_ T1 | sorghum | 2452 | 46 | 88 | 100 | 100 | blastp |
| 1106 | sugarcane\|gb157 .2\|CA132045_T 1 | sugarcane | 2453 | 46 | 89 | 100 | 100 | blastp |
| 1107 | switchgrass\|gb16 5\|FE617713_T1 | switchgrass | 2454 | 46 | 90 | 100 | 100 | blastp |
| 1108 | wheat\|gb164\|BE 430088_T1 | wheat | 2455 | 46 | 95 | 100 | 100 | blastp |
| 1109 | apple\|gb157.3\|D T001281_T1 | apple | 2456 | 47 | 81 | 87.9518 072 | 100 | blastp |
| 1110 | brachypodium\|g b161.xeno\|BE40 2447_T1 | brachypodium | 2457 | 47 | 95 | 100 | 100 | blastp |
| 1111 | ginger\|gb164\|DY 364894_T1 | ginger | 2458 | 47 | 88 | 64.2570 281 | 98.7654 321 | blastp |
| 1112 | maize\|gb164\|AI8 55402_T1 | maize | 2459 | 47 | 89 | 100 | 100 | blastp |
| 1113 | maize\|gb164\|A W017703_T1 | maize | 2460 | 47 | 85 | 100 | 100 | blastp |
| 1114 | onion\|gb162\|AC U58207_T1 | onion | 2461 | 47 | 83 | 99.5983 936 | 99.5967 742 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1115 | onion\|gb162\|CF4 37464_T1 | onion | 2462 | 47 | 85 | 95.9839 357 | 98.75 | blastp |
| 1116 | onion\|gb162\|CF4 35351_T1 | onion | 2463 | 47 | 84 | 100 | 100 | blastp |
| 1117 | rice\|gb157.2\|AU 031632_T1 | rice | 2464 | 47 | 91 | 100 | 100 | blastp |
| 1118 | rice\|gb157.2\|CA 756239_T1 | rice | 2465 | 47 | 87 | 97.5903 614 | 31.6406 25 | blastp |
| 1119 | sorghum\|gb161. xeno\|AI855402_ T1 | sorghum | 2466 | 47 | 89 | 100 | 100 | blastp |
| 1120 | sugarcane\|gb157 .2\|CA132045_T 2 | sugarcane | 2467 | 47 | 90 | 99.5983 936 | 99.5967 742 | blastp |
| 1121 | switchgrass\|gb16 5\|FE624217_T1 | switchgrass | 2468 | 47 | 90 | 100 | 100 | blastp |
| 1122 | wheat\|gb164\|BE 404100_T1 | wheat | 2469 | 47 | 97 | 100 | 100 | blastp |
| 1123 | fescue\|gb161\|DT 700572_T1 | fescue | 2470 | 48 | 94 | 100 | 100 | blastp |
| 1124 | ginger\|gb164\|DY 361836_T1 | ginger | 2471 | 48 | 80 | 95.1612 903 | 96.7346 939 | blastp |
| 1125 | 952 T1 | rice | 2472 | 48 | 90 | 100 | 100 | blastp |
| 1126 | rice\|gb157.2\|U37 952 T3 | rice | 2473 | 48 | 89 | 51.6129 032 | 89.5104 895 | blastp |
| 1127 | rye\|gb164\|BE49 5605_T1 | rye | 2474 | 48 | 98 | 80.6451 613 | 100 | blastp |
| 1128 | sorghum\|gb161. xeno\|AI724211_ T1 | sorghum | 2475 | 48 | 90 | 100 | 100 | blastp |
| 1129 | sugarcane\|gb157 .2\|CA110414_T 1 | sugarcane | 2476 | 48 | 82 | 92.3387 097 | 100 | blastp |
| 1130 | sugarcane\|gb157 .2\|CA065356_T 1 | sugarcane | 2477 | 48 | 83 | 90.3225 806 | 96.5517 241 | blastp |
| 1131 | sugarcane\|gb157 .2\|CA143208_T 1 | sugarcane | 2478 | 48 | 83 | 71.3709 677 | 95.6756 757 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1132 | sugarcane\|gb157 .2\|CA101765_T 1 | sugarcane | 2479 | 48 | 91 | 100 | 100 | blastp |
| 1133 | sugarcane\|gb157 .2\|CA133231_T 1 | sugarcane | 2480 | 48 | 90 | 83.4677 419 | 95.8333 333 | blastp |
| 1134 | switchgrass\|gb16 5\|FE605472_T1 | switchgrass | 2481 | 48 | 91 | 100 | 100 | blastp |
| 1135 | wheat\|gb164\|BE 489764_T1 | wheat | 2482 | 48 | 95 | 100 | 100 | blastp |
| 1136 | wheat\|gb164\|TA U86763_T1 | wheat | 2483 | 48 | 95 | 100 | 100 | blastp |
| 1137 | wheat\|gb164\|BE 415001_T1 | wheat | 2484 | 48 | 95 | 100 | 100 | blastp |
| 1138 | b_juncea\|gb164\| EVGN00504508 791211_T1 | b_juncea | 2485 | 50 | 80 | 85.0931 677 | 100 | blastp |
| 1139 | b_juncea\|gb164\| EVGN01684214 261870_T1 | b_juncea | 2486 | 50 | 84 | 50.9316 77 | 91.9540 23 | blastp |
| 1140 | banana\|gb160\|D N239388_T1 | banana | 2487 | 50 | 84 | 80.1242 236 | 91.9708 029 | blastp |
| 1141 | barley\|gb157.3\|B F253694_T1 | barley | 2488 | 50 | 91 | 58.3850 932 | 98.9690 722 | blastp |
| 1142 | barley\|gb157.3\|B E412959 T3 | barley | 2489 | 50 | 95 | 100 | 62.6923 077 | blastp |
| 1143 | bean\|gb164\|FD7 93482_T1 | bean | 2490 | 50 | 82 | 100 | 91.9075 145 | blastp |
| 1144 | canola\|gb161\|CX 193398 T3 | canola | 2491 | 50 | 83 | 99.3788 82 | 66.9527 897 | blastp |
| 1145 | canola\|gb161\|EV 123336_T1 | canola | 2492 | 50 | 81 | 52.7950 311 | 91.2087 912 | blastp |
| 1146 | centaurea\|gb161\| EL931277_T1 | centaurea | 2493 | 50 | 81 | 98.1366 46 | 62.2489 96 | blastp |
| 1147 | cichorium\|gb161 DT213939 T1 | cichorium | 2494 | 50 | 83 | 64.5962 733 | 95.3271 028 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1148 | fescue\|gb161\|DT 703843_T1 | fescue | 2495 | 50 | 99 | 100 | 94.1520 468 | blastp |
| 1149 | lotus\|gb157.2\|BI 418499_T1 | lotus | 2496 | 50 | 86 | 98.1366 46 | 88.7005 65 | blastp |
| 1150 | onion\|gb162BQ 579939_T1 | onion | 2497 | 50 | 86 | 100 | 57.1942 446 | blastp |
| 1151 | peach\|gb157.2\|B U040795_T1 | peach | 2498 | 50 | 82 | 98.1366 46 | 56.2043 796 | blastp |
| 1152 | peach\|gb157.2\|D W351857_T1 | peach | 2499 | 50 | 83 | 98.1366 46 | 91.8128 655 | blastp |
| 1153 | rye\|gb164\|BF429 463_T1 | rye | 2500 | 50 | 88 | 93.1677 019 | 100 | blastp |
| 1154 | soybean\|gb166\|B E352747 T4 | soybean | 2501 | 50 | 81 | 98.1366 46 | 70.7207 207 | blastp |
| 1155 | sugarcane\|gb157 .2\|CA194640_T 1 | sugarcane | 2502 | 50 | 81 | 98.1366 46 | 56.6787 004 | blastp |
| 1156 | sugarcane\|gb157 .2\|CA103332_T 1 | sugarcane | 2503 | 50 | 84 | 96.2732 919 | 87.0056 497 | blastp |
| 1157 | sugarcane\|gb157 .2\|CA167616_T 1 | sugarcane | 2504 | 50 | 82 | 93.7888 199 | 74.2574 257 | blastp |
| 1158 | sugarcane\|gb157 .2\|CA103740_T 1 | sugarcane | 2505 | 50 | 90 | 100 | 68.5344 828 | blastp |
| 1159 | sugarcane\|gb157 .2\|CA184547_T 1 | sugarcane | 2506 | 50 | 82 | 97.5155 28 | 77.4834 437 | tblastn |
| 1160 | sunflower\|gb162\| CF089373_T1 | sunflower | 2507 | 50 | 87 | 98.1366 46 | 87.5 | blastp |
| 1161 | wheat\|gb164\|CA 484201_T1 | wheat | 2508 | 50 | 89 | 96.8944 099 | 65.9574 468 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1162 | apricot\|gb157.2\| CV049856_T1 | apricot | 2509 | 51 | 86 | 100 | 65.9574 468 | blastp |
| 1163 | arabidopsis\|gb16 5\|AT2G37180_T 1 | arabidopsis | 2510 | 51 | 89 | 100 | 32.6315 789 | blastp |
| 1164 | arabidopsis\|gb16 5\|AT2G39010_T 1 | arabidopsis | 2511 | 51 | 92 | 100 | 32.1799 308 | blastp |
| 1165 | b_juncea\|gb164\| EVGN00130608 921231_T1 | b_juncea | 2512 | 51 | 87 | 100 | 86.9158 879 | blastp |
| 1166 | b_juncea\|gb164\| EVGN00605203 140273_T1 | b_juncea | 2513 | 51 | 86 | 90.3225 806 | 86.5979 381 | blastp |
| 1167 | b_juncea\|gb164\| EVGN21262514 941904_T1 | b_juncea | 2514 | 51 | 86 | 56.9892 473 | 91.3793 103 | blastp |
| 1168 | b_juncea\|gb164\| EVGN00733314 152324_T1 | b_juncea | 2515 | 51 | 86 | 100 | 85.3211 009 | blastp |
| 1169 | b_juncea\|gb164\| EVGN00041211 340240 T1 | b_juncea | 2516 | 51 | 86 | 89.2473 118 | 49.4047 619 | blastp |
| 1170 | b_juncea\|gb164\| DT317704_T1 | b_juncea | 2517 | 51 | 91 | 100 | 80.1724 138 | blastp |
| 1171 | b_juncea\|gb164\| EVGN00208014 701957_T1 | b_juncea | 2518 | 51 | 85 | 89.2473 118 | 79.8076 923 | blastp |
| 1172 | b_juncea\|gb164\| EVGN00550314 491066_T1 | b_juncea | 2519 | 51 | 90 | 100 | 36.3281 25 | blastp |
| 1173 | b_juncea\|gb164\| EVGN01267508 262672_T1 | b_juncea | 2520 | 51 | 90 | 90.3225 806 | 95.4545 455 | blastp |
| 1174 | b_juncea\|gb164\| EVGN01803715 320789_T1 | b_juncea | 2521 | 51 | 89 | 100 | 51.3812 155 | blastp |
| 1175 | b_juncea\|gb164\| EVGN00397011 681539_T1 | b_juncea | 2522 | 51 | 91 | 100 | 34.4019 729 | tblastn |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1176 | b_rapa\|gb162\|D N965016_T1 | b_rapa | 2523 | 51 | 88 | 100 | 69.4029 851 | blastp |
| 1177 | b_rapa\|gb162\|E X025548_T1 | b_rapa | 2524 | 51 | 87 | 100 | 32.5174 825 | blastp |
| 1178 | b_rapa\|gb162\|B G543764_T1 | b_rapa | 2525 | 51 | 92 | 100 | 32.2916 667 | blastp |
| 1179 | banana\|gb160\|D N238638_T1 | banana | 2526 | 51 | 89 | 100 | 27.8721 279 | tblastn |
| 1180 | banana\|gb160\|D N238638 T2 | banana | 2527 | 51 | 89 | 100 | 30.5921 053 | tblastn |
| 1181 | barley\|gb157.3\|B E421292_T1 | barley | 2528 | 51 | 87 | 100 | 32.7464 789 | blastp |
| 1182 | barley\|gb157.3\|B J446923_T1 | barley | 2529 | 51 | 83 | 100 | 64.5833 333 | blastp |
| 1183 | beet\|gb162\|BQ4 88455_T1 | beet | 2530 | 51 | 81 | 100 | 26.6862 17 | blastp |
| 1184 | beet\|gb162\|BVU 60147_T1 | beet | 2531 | 51 | 84 | 100 | 32.2916 667 | blastp |
| 1185 | canola\|gb161\|CX 189721_T1 | canola | 2532 | 51 | 87 | 100 | 32.5174 825 | blastp |
| 1186 | canola\|gb161\|CB 686155_T1 | canola | 2533 | 51 | 92 | 100 | 32.2916 667 | blastp |
| 1187 | canola\|gb161\|D Y007249_T1 | canola | 2534 | 51 | 87 | 100 | 32.5174 825 | blastp |
| 1188 | canola\|gb161\|ES 986486_T1 | canola | 2535 | 51 | 86 | 96.7741 935 | 87.3786 408 | blastp |
| 1189 | canola\|gb161\|EV 203446_T1 | canola | 2536 | 51 | 82 | 100 | 38.0108 992 | tblastn |

EP 2 240 510 B1

94

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1190 | cassava\|gb164\|D N740353_T1 | cassava | 2537 | 51 | 93 | 100 | 62.8378378 | blastp |
| 1191 | cassava\|gb164D V449516_T1 | cassava | 2538 | 51 | 94 | 100 | 68.8888889 | blastp |
| 1192 | cassava\|gb164\|B M259717_T1 | cassava | 2539 | 51 | 81 | 100 | 32.8621908 | blastp |
| 1193 | castorbean\|gb16 0\|EE257493_T2 | castorbean | 2540 | 51 | 81 | 100 | 46.5 | blastp |
| 1194 | castorbean\|gb16 0\|EE257493_T1 | castorbean | 2541 | 51 | 81 | 100 | 34.4444444 | blastp |
| 1195 | cichorium\|gb161 \|EH706421_T1 | cichorium | 2542 | 51 | 94 | 100 | 80.8695652 | blastp |
| 1196 | cichorium\|gb161 \|EH708948_T1 | cichorium | 2543 | 51 | 90 | 100 | 32.5554259 | tblastn |
| 1197 | cichorium\|gb161 \|EH692078_T1 | cichorium | 2544 | 51 | 90 | 100 | 24.668435 | tblastn |
| 1198 | citrus\|gb157.2\|C B291468_T1 | citrus | 2545 | 51 | 82 | 100 | 86.1111111 | blastp |
| 1199 | citrus\|gb157.2\|B Q624699_T1 | citrus | 2546 | 51 | 90 | 100 | 27.56917 | tblastn |
| 1200 | clover\|gb162\|BB 903718_T1 | clover | 2547 | 51 | 91 | 100 | 32.6315789 | blastp |
| 1201 | clover\|gb162\|BB 930902_T1 | clover | 2548 | 51 | 88 | 100 | 32.4041812 | blastp |
| 1202 | clover\|gb162\|BB 911526_T1 | clover | 2549 | 51 | 91 | 92.4731183 | 80.3738318 | blastp |
| 1203 | clover\|gb162\|BB 913405_T1 | clover | 2550 | 51 | 90 | 96.7741935 | 81.0810811 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1204 | cotton\|gb164\|ES 804497_T1 | cotton | 2551 | 51 | 86 | 100 | 58.490566 | blastp |
| 1205 | cotton\|gb164\|EX 172153_T1 | cotton | 2552 | 51 | 89 | 90.3225806 | 41.5156507 | tblastn |
| 1206 | cowpea\|gb166\|F F384339_T1 | cowpea | 2553 | 51 | 89 | 90.3225806 | 80 | blastp |
| 1207 | cowpea\|gb166\|F F396241_T1 | cowpea | 2554 | 51 | 82 | 97.8494624 | 88.3495146 | blastp |
| 1208 | cowpea\|gb166\|E S884224_T1 | cowpea | 2555 | 51 | 88 | 100 | 32.4041812 | blastp |
| 1209 | cowpea\|gb166\|F G857474_T1 | cowpea | 2556 | 51 | 89 | 100 | 68.8888889 | blastp |
| 1210 | cryptomeria\|gb1 66\|BY902595_T 1 | cryptomeria | 2557 | 51 | 81 | 100 | 78.8135593 | blastp |
| 1211 | cryptomeria\|gb1 66\|BJ937695_T1 | cryptomeria | 2558 | 51 | 83 | 100 | 31.7406143 | blastp |
| 1212 | cryptomeria\|gb1 66\|BW993227_T 1 | cryptomeria | 2559 | 51 | 82 | 88.172043 | 86.3157895 | blastp |
| 1213 | dandelion\|gb161\| DY802675_T1 | dandelion | 2560 | 51 | 87 | 91.3978495 | 75.2212389 | blastp |
| 1214 | fescue\|gb161\|DT 674412_T1 | fescue | 2561 | 51 | 82 | 84.9462366 | 85.8695652 | blastp |
| 1215 | fescue\|gb161\|DT 695652_T1 | fescue | 2562 | 51 | 84 | 100 | 76.8595041 | blastp |
| 1216 | fescue\|gb161\|DT 702501_T1 | fescue | 2563 | 51 | 83 | 95.6989247 | 96.7391304 | blastp |
| 1217 | fescue\|gb161\|DT 688112_T1 | fescue | 2564 | 51 | 83 | 100 | 85.3211009 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1218 | fescue\|gb161\|DT 688728_T1 | fescue | 2565 | 51 | 92 | 100 | 81.5789 474 | blastp |
| 1219 | ginger\|gb164\|DY 358169_T1 | ginger | 2566 | 51 | 87 | 100 | 32.9787 234 | blastp |
| 1220 | ginger\|gb164\|DY 366672_T1 | ginger | 2567 | 51 | 82 | 93.5483 871 | 87 | blastp |
| 1221 | ginger\|gb164\|DY 360033_T1 | ginger | 2568 | 51 | 91 | 100 | 27.7888 446 | tblastn |
| 1222 | grape\|gb160\|AF1 88843 T5 | grape | 2569 | 51 | 82 | 100 | 32.5174 825 | blastp |
| 1223 | ipomoea\|gb157.2 \|BJ556470_T1 | ipomoea | 2570 | 51 | 88 | 100 | 32.4041 812 | blastp |
| 1224 | lettuce\|gb157.2\| DW158018_T1 | lettuce | 2571 | 51 | 90 | 100 | 32.6315 789 | blastp |
| 1225 | lettuce\|gb157.2\| DW091407_T1 | lettuce | 2572 | 51 | 90 | 100 | 34.7014 925 | blastp |
| 1226 | lettuce\|gb157.2\| DW060777_T1 | lettuce | 2573 | 51 | 89 | 89.2473 118 | 32.6771 654 | blastp |
| 1227 | lotus\|gb157.2\|A V775277_T1 | lotus | 2574 | 51 | 83 | 100 | 88.5714 286 | blastp |
| 1228 | lotus\|gb157.2\|AI 967387_T1 | lotus | 2575 | 51 | 84 | 100 | 32.4041 812 | blastp |
| 1229 | lotus\|gb157.2\|A V774377_T1 | lotus | 2576 | 51 | 81 | 100 | 88.5714 286 | blastp |
| 1230 | lotus\|gb157.2\|BP 059122 T1 | lotus | 2577 | 51 | 80 | 73.1182 796 | 85 | blastp |
| 1231 | lotus\|gb157.2\|BI 419853_T1 | lotus | 2578 | 51 | 81 | 94.6236 559 | 88 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1232 | lotus\|gb157.2\|BP 049219_T1 | lotus | 2579 | 51 | 81 | 100 | 76.2295082 | blastp |
| 1233 | lotus\|gb157.2\|A V775053_T1 | lotus | 2580 | 51 | 82 | 84.9462366 | 86.8131868 | blastp |
| 1234 | lotus\|gb157.2\|A V775249_T1 | lotus | 2581 | 51 | 85 | 95.6989247 | 80.9090909 | blastp |
| 1235 | maize\|gb164\|AF 326496_T1 | maize | 2582 | 51 | 88 | 100 | 32.4041812 | blastp |
| 1236 | maize\|gb164\|AY 107589_T1 | maize | 2583 | 51 | 84 | 100 | 32.8621908 | blastp |
| 1237 | medicago\|gb157. 2\|AI974409_T1 | medicago | 2584 | 51 | 87 | 100 | 32.4041812 | blastp |
| 1238 | medicago\|gb157. 2\|AI97423_T1 | medicago | 2585 | 51 | 89 | 100 | 32.6315789 | blastp |
| 1239 | melon\|gb165\|EB 715587_T1 | melon | 2586 | 51 | 86 | 100 | 23.4848485 | tblastn |
| 1240 | oat\|gb164\|CN81 6056_T1 | oat | 2587 | 51 | 82 | 100 | 84.5454545 | blastp |
| 1241 | oil_palm\|gb166\| CN601069_T1 | oil_palm | 2588 | 51 | 89 | 100 | 32.9787234 | blastp |
| 1242 | oil_palm_gb166\| EL686181_T1 | oil_palm | 2589 | 51 | 84 | 100 | 32.9787234 | blastp |
| 1243 | peanut\|gb161\|C D037823_T1 | peanut | 2590 | 51 | 82 | 100 | 31.8493151 | blastp |
| 1244 | peanut\|gb161\|C D038014_T1 | peanut | 2591 | 51 | 88 | 100 | 32.1799308 | blastp |
| 1245 | periwinkle\|gb16 4\|AM232518_T2 | periwinkle | 2592 | 51 | 87 | 100 | 65.9574468 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1246 | physcomitrella\|g b157\|BI436955_T1 | physcomitrella | 2593 | 51 | 89 | 100 | 33.3333333 | blastp |
| 1247 | physcomitrella\|g b157\|BJ198543_T1 | physcomitrella | 2594 | 51 | 82 | 100 | 32.5174825 | blastp |
| 1248 | physcomitrella\|g b157\|AW476973 T3 | physcomitrella | 2595 | 51 | 90 | 100 | 33.2142857 | blastp |
| 1249 | physcomitrella\|g b157\|BJ962015_T1 | physcomitrella | 2596 | 51 | 82 | 100 | 32.5259516 | blastp |
| 1250 | pine\|gb57.2\|A W870138_T1 | pine | 2597 | 51 | 82 | 100 | 34.7014925 | blastp |
| 1251 | pine\|gb157.2\|AI8 13147_T1 | pine | 2598 | 51 | 87 | 100 | 33.0960854 | blastp |
| 1252 | pine\|gb157.2\|AA 739836_T1 | pine | 2599 | 51 | 87 | 100 | 33.0960854 | blastp |
| 1253 | pine\|gb157.2\|AL 750425_T1 | pine | 2600 | 51 | 88 | 100 | 33.0960854 | blastp |
| 1254 | pine\|gb157.2\|BG 038984_T1 | pine | 2601 | 51 | 80 | 100 | 32.8621908 | blastp |
| 1255 | pine\|gb157.2\|A W225939_T1 | pine | 2602 | 51 | 80 | 100 | 34.1911765 | blastp |
| 1256 | pine\|gb157.2\|BQ 696500_T1 | pine | 2603 | 51 | 81 | 100 | 32.8621908 | blastp |
| 1257 | pine\|gb157.2\|AL 751198_T1 | pine | 2604 | 51 | 87 | 100 | 33.3333333 | blastp |
| 1258 | pine\|gb157.2\|BQ 695693_T1 | pine | 2605 | 51 | 82 | 100 | 32.8621908 | blastp |
| 1259 | pine\|gb157.2\|BF 517326_T1 | pine | 2606 | 51 | 80 | 100 | 60.3896104 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1260 | pine\|gb157.2\|AA 739625_T1 | pine | 2607 | 51 | 81 | 100 | 34.7014 925 | blastp |
| 1261 | pine\|gb157.2\|BG 317873_T1 | pine | 2608 | 51 | 82 | 100 | 32.8621 908 | blastp |
| 1262 | pine\|gb157.2\|AI8 13147_T2 | pine | 2609 | 51 | 87 | 100 | 32.9787 234 | blastp |
| 1263 | pine\|gb157.2\|CF 388120_T1 | pine | 2610 | 51 | 87 | 100 | 33.3333 333 | blastp |
| 1264 | pine\|gb157.2\|BE 662590_T1 | pine | 2611 | 51 | 81 | 100 | 35.7692 308 | blastp |
| 1265 | pine\|gb157.2\|BG 318657_T1 | pine | 2612 | 51 | 82 | 100 | 32.8621 908 | blastp |
| 1266 | pine\|gb157.2\|AA 557104_T1 | pine | 2613 | 51 | 88 | 100 | 33.3333 333 | blastp |
| 1267 | pine\|gb157.2\|A W870138_T2 | pine | 2614 | 51 | 82 | 100 | 32.8621 908 | blastp |
| 1268 | pine\|gb157.2\|A W289749_T1 | pine | 2615 | 51 | 82 | 100 | 32.8621 908 | blastp |
| 1269 | pine\|gb157.2\|H7 5016_T1 | pine | 2616 | 51 | 88 | 100 | 32.7464 789 | blastp |
| 1270 | pine\|gb157.2\|AA 740005_T1 | pine | 2617 | 51 | 80 | 100 | 35.0943 396 | blastp |
| 1271 | pine\|gb157.2\|CA 305579_T1 | pine | 2618 | 51 | 91 | 100 | 75.6097 561 | blastp |
| 1272 | pine\|gb157.2\|BE 187350_T1 | pine | 2619 | 51 | 83 | 100 | 32.8621 908 | blastp |
| 1273 | pine\|gb157.2\|CF 473539_T1 | pine | 2620 | 51 | 84 | 100 | 32.9787 234 | blastp |

EP 2 240 510 B1

100

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1274 | pine\|gb157.2\|A W290370_T1 | pine | 2621 | 51 | 83 | 100 | 32.7464789 | blastp |
| 1275 | pine\|gb157.2\|A W290691_T1 | pine | 2622 | 51 | 82 | 75.2688172 | 88.6075949 | blastp |
| 1276 | pine\|gb157.2\|BG 318695_T1 | pine | 2623 | 51 | 82 | 100 | 34.7014925 | blastp |
| 1277 | pineapple\|gb157. 2\|DT339628_T1 | pineapple | 2624 | 51 | 91 | 98.9247312 | 78.6324786 | blastp |
| 1278 | poplar\|gb157.2\|A 1162483_T2 | poplar | 2625 | 51 | 86 | 100 | 24.3455497 | tblastn |
| 1279 | poplar\|gb157.2\|B U817536_T4 | poplar | 2626 | 51 | 87 | 100 | 22.6094003 | tblastn |
| 1280 | potato\|gb157.2\|B Q515617_T1 | potato | 2627 | 51 | 80 | 98.9247312 | 36.6533865 | blastp |
| 1281 | potato\|gb157.2\|B E920139_T2 | potato | 2628 | 51 | 90 | 100 | 41.5178571 | blastp |
| 1282 | radish\|gb164\|EV 526963_T1 | radish | 2629 | 51 | 87 | 100 | 32.5174825 | blastp |
| 1283 | radish\|gb164\|EV 567230_T2 | radish | 2630 | 51 | 84 | 100 | 46.039604 | blastp |
| 1284 | radish\|gb\|164\|EV 551004_T1 | radish | 2631 | 51 | 92 | 100 | 32.2916667 | blastp |
| 1285 | radish\|gb164\|EX 756464_T1 | radish | 2632 | 51 | 86 | 100 | 48.1865285 | blastp |
| 1286 | radish\|gb164\|EY 910551_T1 | radish | 2633 | 51 | 91 | 100 | 31.9587629 | blastp |
| 1287 | radish\|gb164\|EW 730466_T1 | radish | 2634 | 51 | 84 | 98.9247312 | 87.6190476 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1288 | radish\|gb164\|AF 051128_T1 | radish | 2635 | 51 | 81 | 87.0967 742 | 48.6 | tblastn |
| 1289 | radish\|gb164\|EX 756028_T1 | radish | No predicted Protein | 51 | 86 | 100 | 40.0286 944 | tblastn |
| 1290 | rice\|gb157.2\|BE2 29418_T1 | rice | 2636 | 51 | 90 | 100 | 32.9787 234 | blastp |
| 1291 | rice\|gb157.2\|BE2 29418 T3 | rice | 2637 | 51 | 90 | 100 | 38.5892 116 | blastp |
| 1292 | rice\|gb157.2\|AK 107700_T1 | rice | 2638 | 51 | 91 | 100 | 68.8888 889 | blastp |
| 1293 | ricegb\|157.2\|BE2 29418 T4 | rice | 2639 | 51 | 90 | 100 | 54.0697 674 | blastp |
| 1294 | rice\|gb157.2\|NM 001066078_T1 | rice | 2640 | 51 | 92 | 100 | 41.7040 359 | blastp |
| 1295 | rice\|gb157.2\|AW 155505_T1 | rice | 2641 | 51 | 83 | 100 | 32.0689 655 | blastp |
| 1296 | rice\|gb157.2\|AW 155505 T2 | rice | 2642 | 51 | 83 | 98.9247 312 | 35.7976 654 | blastp |
| 1297 | rice\|**g**b157.2\|AK 106746_T1 | rice | 2643 | 51 | 83 | 100 | 22.6461 039 | tblastn |
| 1298 | sorghum\|gb161. xeno\|AY107589 T1 | sorghum | 2644 | 51 | 86 | 100 | 33.3333 333 | blastp |
| 1299 | sorghum\|gb161. xeno\|AI947598_ T1 | sorghum | 2645 | 51 | 92 | 100 | 32.5174 825 | blastp |
| 1300 | sorghum\|gb161. xeno\|AI855413_ T1 | sorghum | 2646 | 51 | 80 | 100 | 31.4189 189 | blastp |
| 1301 | sorghum\|gb161. xeno\|AW565915 _T1 | sorghum | 2647 | 51 | 81 | 100 | 31.1418 685 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1302 | sorghum_gb161. xeno\|CF481617_T1 | sorghum | 2648 | 51 | 90 | 100 | 79.6610169 | blastp |
| 1303 | soybean\|gb166\|B U549322_T1 | soybean | 2649 | 51 | 89 | 100 | 32.5174825 | blastp |
| 1304 | soybean\|gb166\|B E352729_T1 | soybean | 2650 | 51 | 88 | 100 | 32.4041812 | blastp |
| 1305 | soybean\|gb166\|B 1974981_T1 | soybean | 2651 | 51 | 91 | 100 | 71.5384615 | blastp |
| 1306 | soybean\|gb166\|B E658685_T1 | soybean | 2652 | 51 | 89 | 100 | 32.6315789 | blastp |
| 1307 | soybean\|gb166\|B E352747_T3 | soybean | 2653 | 51 | 81 | 100 | 20.5904059 | tblastn |
| 1308 | spikemoss\|gb165 \|DN838269_T1 | spikemoss | 2654 | 51 | 88 | 100 | 32.0689655 | blastp |
| 1309 | spikemoss\|gb165 \|FE434019_T1 | spikemoss | 2655 | 51 | 88 | 100 | 32.0689655 | blastp |
| 1310 | spruce\|gb162\|CO 225164_T1 | spruce | 2656 | 51 | 89 | 100 | 32.8621908 | blastp |
| 1311 | spruce\|gb162\|CO 258147_T1 | spruce | 2657 | 51 | 81 | 100 | 33.8181818 | blastp |
| 1312 | spruce\|gb162\|CO 230791_T1 | spruce | 2658 | 51 | 88 | 100 | 46.0396004 | blastp |
| 1313 | spruce\|gb162\|DR 546674_T1 | spruce | 2659 | 51 | 82 | 100 | 32.5 | blastp |
| 1314 | spruce\|gb162\|DR 560237_T1 | spruce | 2660 | 51 | 81 | 100 | 34.1911765 | blastp |
| 1315 | spurge\|gb161\|D V116550_T1 | spurge | 2661 | 51 | 83 | 100 | 86.1111111 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1316 | sugarcane\|gb157 .2\|CA088464_T 1 | sugarcane | 2662 | 51 | 87 | 88.1720 43 | 81.1881 188 | blastp |
| 1317 | sugarcane\|gb157 .2\|CA086583_T 1 | sugarcane | 2663 | 51 | 92 | 100 | 62.4161 074 | blastp |
| 1318 | sugarcane\|gb157 .2\|CA234165_T 1 | sugarcane | 2664 | 51 | 86 | 62.3655 914 | 100 | blastp |
| 1319 | sugarcane\|gb157 .2\|CA107998_T 1 | sugarcane | 2665 | 51 | 89 | 100 | 32.5174 825 | blastp |
| 1320 | sugarcane\|gb157 .2\|CA204327_T 1 | sugarcane | 2666 | 51 | 95 | 100 | 34.1911 765 | tblastn |
| 1321 | sugarcane\|gb157 .2\|CA067786_T 1 | sugarcane | 2667 | 51 | 92 | 86.0215 054 | 18.6480 186 | tblastn |
| 1322 | sunflower\|gb162\| DY944685_T1 | sunflower | 2668 | 51 | 81 | 100 | 31.9587 629 | blastp |
| 1323 | sunflower\|gb162\| BQ968590_T1 | sunflower | 2669 | 51 | 87 | 52.6881 72 | 100 | blastp |
| 1324 | sunflower\|gb162\| CD848850_T1 | sunflower | 2670 | 51 | 89 | 98.9247 312 | 41.3173 653 | tblastn |
| 1325 | tobacco\|gb162\|E B445876_T1 | tobacco | 2671 | 51 | 90 | 100 | 32.4041 812 | blastp |
| 1326 | tobacco\|gb162\|E B445188_T1 | tobacco | 2672 | 51 | 88 | 100 | 32.4041 812 | blastp |
| 1327 | tobacco\|gb162\|C K720586_T1 | tobacco | 2673 | 51 | 81 | 100 | 34.7014 925 | blastp |
| 1328 | tomato\|gb164\|C 0750818_T1 | tomato | 2674 | 51 | 92 | 88.1720 43 | 82.8282 828 | blastp |
| 1329 | tomato\|gb164\|AI 772191_T1 | tomato | 2675 | 51 | 81 | 98.9247 312 | 33.6996 337 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1330 | tomato\|gb164\|A W219533 T2 | tomato | 2676 | 51 | 89 | 100 | 39.2405 063 | tblastn |
| 1331 | triphysaria\|gb16 4\|DR173305_T1 | triphysaria | 2677 | 51 | 88 | 95.6989 247 | 80.9090 909 | blastp |
| 1332 | triphysaria\|gb16 4\|EX984185_T1 | triphysaria | 2678 | 51 | 90 | 100 | 75.6097 561 | blastp |
| 1333 | triphysaria\|gb16 4\|DR174019_T1 | triphysaria | 2679 | 51 | 91 | 100 | 67.8832 117 | blastp |
| 1334 | wheat\|gb164\|CA 609068_T1 | wheat | 2680 | 51 | 94 | 100 | 67.3913 043 | blastp |
| 1335 | wheat\|gb164\|BE 430411_T1 | wheat | 2681 | 51 | 86 | 100 | 32.4041 812 | blastp |
| 1336 | wheat\|gb164\|CK 208980_T1 | wheat | 2682 | 51 | 80 | 100 | 30 | blastp |
| 1337 | wheat\|gb164\|CA 620158_T1 | wheat | 2683 | 51 | 96 | 95.6989 247 | 87.2549 02 | blastp |
| 1338 | wheat\|gb164\|BE 405395_T1 | wheat | 2684 | 51 | 88 | 100 | 32.7464 789 | blastp |
| 1339 | wheat\|gb164\|CA 647310_T1 | wheat | 2685 | 51 | 80 | 77.4193 548 | 93.5064 935 | blastp |
| 1340 | wheat\|gb164\|BE 492099_T1 | wheat | 2686 | 51 | 87 | 100 | 32.7464 789 | blastp |
| 1341 | wheat\|gb164\|BE 402029_T1 | wheat | 2687 | 51 | 87 | 100 | 32.7464 789 | blastp |
| 1342 | wheat\|gb164\|CA 618130_T1 | wheat | 2688 | 51 | 86 | 88.1720 43 | 73.2142 857 | blastp |
| 1343 | wheat\|gb164\|CJ6 05707_T1 | wheat | 2689 | 51 | 82 | 100 | 69.4029 851 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1344 | wheat\|gb164\|CA 614209_T1 | wheat | 2690 | 51 | 98 | 59.1397 849 | 98.2142 857 | blastp |
| 1345 | wheat\|gb164\|CA 701714_T1 | wheat | 2691 | 51 | 82 | 100 | 77.5 | blastp |
| 1346 | wheat\|gb164\|BE 403921_T1 | wheat | 2692 | 51 | 93 | 96.7741 935 | 82.5688 073 | blastp |
| 1347 | wheat\|gb164\|BE 217049_T1 | wheat | 2693 | 51 | 83 | 100 | 31.9587 629 | blastp |
| 1348 | wheat\|gb164\|CA 602649_T1 | wheat | 2694 | 51 | 84 | 100 | 30.0322 928 | tblastn |
| 1349 | wheat\|gb164\|CA 486220_T1 | wheat | 2695 | 51 | 82 | 96.7741 935 | 33.3759 591 | tblastn |
| 1350 | b_juncea\|gb164\| EVGN00044413 933329_T1 | b_juncea | 2696 | 52 | 85 | 64.3835 616 | 99.2957 746 | blastp |
| 1351 | b_juncea\|gb164\| EVGN00137910 990746_T1 | b_juncea | 2697 | 52 | 90 | 53.4246 575 | 99.1525 424 | blastp |
| 1352 | barley\|gb157.3\|B E413268_T1 | barley | 2698 | 52 | 81 | 96.8036 53 | 73.4482 759 | blastp |
| 1353 | barley\|gb157.3\|A J433979_T1 | barley | 2699 | 52 | 81 | 96.8036 53 | 73.4482 759 | blastp |
| 1354 | barley\|gb157.3\|B E412979_T1 | barley | 2700 | 52 | 82 | 96.8036 53 | 74.1258 741 | blastp |
| 1355 | brachypodium\|g b161.xeno\|AF13 9815_T1 | brachypodium | 2701 | 52 | 83 | 96.8036 53 | 73.6111 111 | blastp |
| 1356 | citrus\|gb157.2\|C X052950_T1 | citrus | 2702 | 52 | 84 | 50.2283 105 | 100 | blastp |
| 1357 | clover\|gb162\|BB 913131_T1 | clover | 2703 | 52 | 93 | 52.5114 155 | 99.1379 31 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1358 | clover\|gb162\|BB 918704_T1 | clover | 2704 | 52 | 82 | 63.9269 406 | 99.2957 746 | blastp |
| 1359 | cotton\|gb164\|CO 103246_T1 | cotton | 2705 | 52 | 91 | 56.6210 046 | 93.9393 939 | blastp |
| 1360 | cowpea\|gb166\|F G883860_T1 | cowpea | 2706 | 52 | 83 | 51.1415 525 | 99.1150 442 | blastp |
| 1361 | fescue\|gb161\|DT 702489_T1 | fescue | 2707 | 52 | 96 | 57.0776 256 | 93.2835 821 | blastp |
| 1362 | fescue\|gb161\|DT 702846_T1 | fescue | 2708 | 52 | 96 | 57.0776 256 | 96.8992 248 | blastp |
| 1363 | ipomoea\|gb157.2 BU691146_T1 | ipomoea | 2709 | 52 | 81 | 87.2146 119 | 74.7035 573 | blastp |
| 1364 | maize\|gb164\|AI9 39909_T1 | maize | 2710 | 52 | 80 | 96.8036 53 | 73.6111 111 | blastp |
| 1365 | maize\|gb164\|AF 130975_T1 | maize | 2711 | 52 | 83 | 96.8036 53 | 74.3859 649 | blastp |
| 1366 | maize\|gb164\|AI9 47831_T1 | maize | 2712 | 52 | 83 | 96.8036 53 | 73.6111 111 | blastp |
| 1367 | oil_palm\|gb166\| EL692065_T1 | oil_palm | 2713 | 52 | 80 | 96.8036 53 | 75.1773 05 | blastp |
| 1368 | physcomitrella\|g b157\|AW476973 _T1 | physcomitrella | 2714 | 52 | 80 | 93.6073 059 | 73.4767 025 | blastp |
| 1369 | physcomitrella\|g b157\|BI894596_ T1 | physcomitrella | 2715 | 52 | 80 | 96.3470 32 | 73.0103 806 | blastp |
| 1370 | physcomitrella\|g b157\|AW476973 T2 | physcomitrella | 2716 | 52 | 80 | 93.6073 059 | 73.4767 025 | blastp |
| 1371 | pine\|gb157.2\|CF 387570_T1 | pine | 2717 | 52 | 88 | 51.1415 525 | 99.1150 442 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1372 | radish\|gb164\|EY 904434 T2 | radish | 2718 | 52 | 88 | 54.3378 995 | 99.1666 667 | blastp |
| 1373 | radish\|gb164\|FD 960377_T1 | radish | 2719 | 52 | 84 | 63.0136 986 | 99.2805 755 | blastp |
| 1374 | rice\|gb157.2\|AU 093957_T1 | rice | 2720 | 52 | 81 | 96.8036 53 | 74.1258 741 | blastp |
| 1375 | rice\|gb157.2\|BE0 39992 T2 | rice | 2721 | 52 | 92 | 57.0776 256 | 97.6562 5 | blastp |
| 1376 | rice\|gb157.2\|BE5 30955_T1 | rice | 2722 | 52 | 80 | 96.8036 53 | 73.1034 483 | blastp |
| 1377 | rye\|gb164\|BE58 6469_T1 | rye | 2723 | 52 | 82 | 61.1872 146 | 97.8102 19 | blastp |
| 1378 | sorghum\|gb161. xeno\|AW922622 _T1 | sorghum | 2724 | 52 | 83 | 96.8036 53 | 72.6027 397 | blastp |
| 1379 | sorghum\|gb161. xeno\|BE344582_ T1 | sorghum | 2725 | 52 | 80 | 85.3881 279 | 74.8 | blastp |
| 1380 | sorghum\|gb161. xeno\|AI855280_ T1 | sorghum | 2726 | 52 | 82 | 96.8036 53 | 73.3564 014 | blastp |
| 1381 | spikemoss\|gb165 \|DN838148_T1 | spikemoss | 2727 | 52 | 83 | 91.7808 219 | 71.5302 491 | blastp |
| 1382 | spikemoss\|gb165 \|DN838057_T1 | spikemoss | 2728 | 52 | 83 | 91.7808 219 | 71.5302 491 | blastp |
| 1383 | sugarcane\|gb157 .2\|CA139573_T 1 | sugarcane | 2729 | 52 | 82 | 90.4109 589 | 71.8978 102 | blastp |
| 1384 | sugarcane\|gb157 .2\|CA145403_T 1 | sugarcane | 2730 | 52 | 90 | 57.0776 256 | 99.2063 492 | blastp |
| 1385 | sunflower\|gb162\| CF083179_T1 | sunflower | 2731 | 52 | 80 | 57.0776 256 | 93.2835 821 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1386 | sunflower\|gb162\| BU035823_T1 | sunflower | 2732 | 52 | 86 | 56.6210 046 | 89.2086 331 | blastp |
| 1387 | switchgrass\|gb16 5\|DN140790_T1 | switchgrass | 2733 | 52 | 83 | 61.6438 356 | 99.2647 059 | blastp |
| 1388 | switchgrass\|gb16 5\|FE631354_T1 | switchgrass | 2734 | 52 | 81 | 66.2100 457 | 96.6666 667 | blastp |
| 1389 | tobacco\|gb162\|D V159802_T1 | tobacco | 2735 | 52 | 81 | 87.6712 329 | 48.7722 269 | tblastn |
| 1390 | wheat\|gb164\|AF 139815_T1 | wheat | 2736 | 52 | 82 | 96.8036 53 | 74.1258 741 | blastp |
| 1391 | wheat\|gb164\|BE 406301_T1 | wheat | 2737 | 52 | 81 | 96.8036 53 | 73.4482 759 | blastp |
| 1392 | wheat\|gb164\|BE 404904_T1 | wheat | 2738 | 52 | 81 | 96.8036 53 | 73.4482 759 | blastp |
| 1393 | wheat\|gb164\|BE 400219_T1 | wheat | 2739 | 52 | 81 | 96.8036 53 | 73.4482 759 | blastp |
| 1394 | wheat\|gb164\|CA 619093_T1 | wheat | 2740 | 52 | 81 | 54.7945 205 | 90.1515 152 | blastp |
| 1395 | wheat\|gb164\|BE 605056_T1 | wheat | 2741 | 52 | 88 | 56.6210 046 | 93.2330 827 | blastp |
| 1396 | wheat\|gb164\|BQ 245211_T1 | wheat | 2742 | 52 | 82 | 96.8036 53 | 74.1258 741 | blastp |
| 1397 | wheat\|gb164\|BE 497487_T1 | wheat | 2743 | 52 | 81 | 96.8036 53 | 73.4482 759 | blastp |
| 1398 | wheat\|gb164\|BQ 295206_T1 | wheat | 2744 | 52 | 89 | 64.8401 826 | 100 | blastp |
| 1399 | wheat\|gb164\|BE 499954_T1 | wheat | 2745 | 52 | 80 | 98.6301 37 | 74.8275 862 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 1400 | wheat\|gb164\|BE 405794_T1 | wheat | 2746 | 52 | 81 | 96.8036 53 | 73.4482 759 | blastp |
| 6 | tomato\|gb164\| AW934056_T1 | tomato | 32 | 31 | 82 | 100 | 100 | blastp |
| 21 | barley\|gb157.3\|A L501410_T1 | barley | 47 | 46 | 87 | 98.3935 743 | 98.7951 8072 | blastp |
| 2844 | antirrhinum\|gb16 6\|AJ559427_T1 | antirrhinum | 3052 | 25 | 88 | 100 | 100 | blastp |
| 2845 | antirrhinum\|gb16 6\|AJ791214_T1 | antirrhinum | 3053 | 25 | 85 | 100 | 100 | blastp |
| 2846 | bruguiera\|gb166\| BP939664_T1 | bruguiera | 3054 | 25 | 82 | 61.2903 2258 | 100 | blastp |
| 2847 | centauxea\|gb166\| EL931601_T1 | centaurea | 3055 | 25 | 84 | 98.7903 2258 | 100 | blastp |
| 2848 | eucalyptus\|gb16 6\|CD668425_T1 | eucalyptus | 3056 | 25 | 85 | 98.7903 2258 | 98.7903 2258 | blastp |
| 2849 | kiwi\|gb166\|FG4 09998_T1 | kiwi | 3057 | 25 | 85 | 100 | 100 | blastp |
| 2850 | kiwi\|gb166\|FG4 53166_T1 | kiwi | 3058 | 25 | 86 | 100 | 100 | blastp |
| 2851 | kiwi\|gb166\|FG4 01585_T1 | kiwi | 3059 | 25 | 87 | 99.5967 7419 | 100 | blastp |
| 2852 | kiwi\|gb166\|FG4 19790_T1 | kiwi | 3060 | 25 | 85 | 100 | 100 | blastp |
| 2853 | liriodendron\|gb1 66\|FD495170_T 1 | liriodend ron | 3061 | 25 | 82 | 99.1935 4839 | 98.7951 8072 | blastp |
| 2854 | poppy\|gb166\|FG 607362_T1 | poppy | 3062 | 25 | 82 | 82.6612 9032 | 99.5145 6311 | blastp |
| 2855 | soybean\|gb167\|A A660186_T1 | soybean | 3063 | 25 | 83 | 100 | 100 | blastp |
| 2856 | soybean\|gb167\|C A990807_T1 | soybean | 3064 | 25 | 82 | 95.5645 1613 | 100 | blastp |
| 2857 | walnuts\|gb166\|C V196664_T1 | walnuts | 3065 | 25 | 83 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2858 | antirrhinum\|gb16 6\|X70417_T1 | antirrhinum | 3066 | 26 | 90 | 100 | 100 | blastp |
| 2859 | banana\|gb167\|FF 560721_T1 | banana | 3067 | 26 | 86 | 77.6 | 99.4871 7949 | blastp |
| 2860 | centaurea\|gb166\| EL932548_T1 | centaurea | 3068 | 26 | 86 | 100 | 100 | blastp |
| 2861 | antirrhinum\|gb16 6\|AJ789802_T1 | antirrhinum | 3069 | 27 | 86 | 90.9090 9091 | 100 | blastp |
| 2862 | bruguiera\|gb166\| BP938735_T1 | bruguiera | 3070 | 27 | 81 | 69.9604 7431 | 100 | blastp |
| 2863 | eucalyptus\|gb16 6\|ES589574_T1 | eucalyptus | 3071 | 27 | 82 | 90.9090 9091 | 100 | blastp |
| 2864 | kiwi\|gb166\|FG4 27735_T1 | kiwi | 3072 | 27 | 86 | 50.1976 2846 | 99.2187 5 | blastp |
| 2865 | kiwi\|gb166\|FG4 06415_T1 | kiwi | 3073 | 27 | 81 | 54.1501 9763 | 100 | blastp |
| 2866 | kiwi\|gb166\|FG4 06885_T1 | kiwi | 3074 | 27 | 84 | 99.2094 8617 | 99.6031 746 | blastp |
| 2867 | liriodendron\|gb1 66\|CK744430_T 1 | liriodendron | 3075 | 27 | 81 | 99.2094 8617 | 99.6031 746 | blastp |
| 2868 | poppy\|gb166\|FG 608493_T1 | poppy | 3076 | 27 | 84 | 83.0039 5257 | 99.5260 6635 | blastp |
| 2869 | soybean\|gb167\|E V269611_T1 | soybean | 3077 | 27 | 86 | 60.0790 5138 | 96.2025 3165 | blastp |
| 2870 | amborella\|gb166\| CD482678_T1 | amborella | 3078 | 28 | 82 | 100 | 100 | blastp |
| 2871 | cenchrus\|gb166\| BM084541_T1 | cenchrus | 3079 | 28 | 81 | 100 | 100 | blastp |
| 2872 | leymus\|gb166\|E G376267_T1 | leymus | 3080 | 28 | 80 | 100 | 100 | blastp |
| 2873 | leymus\|gb166\|E G386149_T1 | leymus | 3081 | 28 | 80 | 100 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2874 | walnuts\|gb166\|E L895384_T1 | walnuts | 3082 | 28 | 83 | 82 | 94.4954 1284 | blastp |
| 2875 | amborella\|gb166\| CD481950_T1 | amborella | 3083 | 30 | 88 | 98.2638 8889 | 98.9547 0383 | blastp |
| 2876 | antirrhinum\|gb16 6\|AJ796874_T1 | antirrhinum | 3084 | 30 | 86 | 93.4027 7778 | 100 | blastp |
| 2877 | antirrhinum\|gb16 6\|AJ798039_T1 | antirrhinum | 3085 | 30 | 84 | 73.2638 8889 | 99.5260 6635 | blastp |
| 2878 | antirrhinum\|gb16 6\|AJ792331_T1 | antirrhinum | 3086 | 30 | 88 | 85.0694 4444 | 100 | blastp |
| 2879 | antirrhinum\|gb16 6\|AJ558770_T1 | antirrhinum | 3087 | 30 | 86 | 98.2638 8889 | 99.3006 993 | blastp |
| 2880 | banana\|gb167\|FF 558844_T1 | banana | 3088 | 30 | 88 | 98.2638 8889 | 98.9510 4895 | blastp |
| 2881 | bean\|gb167\|CA8 98412_T1 | bean | 3089 | 30 | 81 | 98.2638 8889 | 99.3031 3589 | blastp |
| 2882 | bruguiera\|gb166\| BP941115_T1 | bruguiera | 3090 | 30 | 86 | 52.7777 7778 | 95 | blastp |
| 2883 | bruguiera\|gb166\| BP939033_T1 | bruguiera | 3091 | 30 | 85 | 97.9166 6667 | 99.3031 3589 | blastp |
| 2884 | cenchrus\|gb166\| EB656428_T1 | cenchrus | 3092 | 30 | 85 | 98.2638 8889 | 98.9583 3333 | blastp |
| 2885 | centauxea\|gb166\| EH767475_T1 | centaurea | 3093 | 30 | 86 | 98.2638 8889 | 98.9547 0383 | blastp |
| 2886 | centauxea\|gb166\| EL935569_T1 | centaurea | 3094 | 30 | 86 | 98.2638 8889 | 98.9583 3333 | blastp |
| 2887 | cycas\|gb166\|CB 089724_T1 | cycas | 3095 | 30 | 84 | 98.2638 8889 | 98.9547 0383 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2888 | cycas\|gb166\|CB 088798_T1 | cycas | 3096 | 30 | 83 | 98.2638 8889 | 98.2698 9619 | blastp |
| 2889 | eucalyptus\|gb16 6\|CD668044_T1 | eucalyptus | 3097 | 30 | 84 | 98.2638 8889 | 99.3055 5556 | blastp |
| 2890 | eucalyptus\|gb16 6\|AW191311_T 1 | eucalyptus | 3098 | 30 | 87 | 98.2638 8889 | 98.9547 0383 | blastp |
| 2891 | kiwi\|gb166\|FG4 03284_T1 | kiwi | 3099 | 30 | 85 | 98.2638 8889 | 99.3006 993 | blastp |
| 2892 | kiwi\|gb166\|FG4 04130_T1 | kiwi | 3100 | 30 | 86 | 98.2638 8889 | 99.3006 993 | blastp |
| 2893 | kiwi\|gb166\|FG3 96354_T1 | kiwi | 3101 | 30 | 90 | 98.2638 8889 | 98.9510 4895 | blastp |
| 2894 | kiwi\|gb166\|FG4 04890_T1 | kiwi | 3102 | 30 | 85 | 72.2222 2222 | 99.5215 311 | blastp |
| 2895 | kiwi\|gb166\|FG4 17962_T1 | kiwi | 3103 | 30 | 87 | 62.1527 7778 | 99.4444 4444 | blastp |
| 2896 | kiwi\|gb166\|FG4 03188_T1 | kiwi | 3104 | 30 | 89 | 98.2638 8889 | 96.9178 0822 | blastp |
| 2897 | kiwi\|gb166\|FG4 03647_T1 | kiwi | 3105 | 30 | 85 | 68.0555 5556 | 99.4923 8579 | blastp |
| 2898 | kiwi\|gb166\|FG3 97405_T1 | kiwi | 3106 | 30 | 85 | 98.2638 8889 | 99.3006 993 | blastp |
| 2899 | leymus\|gb166\|E G384635_T1 | leymus | 3107 | 30 | 85 | 99.3055 5556 | 99.6551 7241 | blastp |
| 2900 | leymus\|gb166\|C N466016_T1 | leymus | 3108 | 30 | 83 | 98.2638 8889 | 98.9726 0274 | blastp |
| 2901 | leymus\|gb166\|C N466006_T1 | leymus | 3109 | 30 | 83 | 98.2638 8889 | 98.9726 0274 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2902 | leymus\|gb166\|E G376500_T1 | leymus | 3110 | 30 | 83 | 98.2638 8889 | 98.9726 0274 | blastp |
| 2903 | liriodendron\|gb1 66\|CK749885_T 1 | liriodendron | 3111 | 30 | 89 | 98.2638 8889 | 98.9547 0383 | blastp |
| 2904 | liriodendron\|gb1 66\|CV002697_T 1 | liriodendron | 3112 | 30 | 88 | 98.2638 8889 | 98.9547 0383 | blastp |
| 2905 | lovegrass\|gb167\| DN480914_T1 | lovegrass | 3113 | 30 | 85 | 98.2638 8889 | 98.9619 3772 | blastp |
| 2906 | nuphar\|gb166\|C D472824_T1 | nuphar | 3114 | 30 | 86 | 98.2638 8889 | 98.9473 6842 | blastp |
| 2907 | nuphar\|gb166\|C D472574_T1 | nuphar | 3115 | 30 | 86 | 98.2638 8889 | 98.9473 6842 | blastp |
| 2908 | nuphar\|gb166\|C D473614_T1 | nuphar | 3116 | 30 | 84 | 51.7361 1111 | 98.0263 1579 | blastp |
| 2909 | peanut\|gb167\|ES 759056_T1 | peanut | 3117 | 30 | 83 | 64.2361 1111 | 100 | blastp |
| 2910 | poppy\|gb166\|FE 966578_T1 | poppy | 3118 | 30 | 83 | 98.6111 1111 | 98.6206 8966 | blastp |
| 2911 | pseudoroegneria\| gb167\|FF344096 _T1 | pseudoroegneria | 3119 | 30 | 82 | 98.2638 8889 | 98.9726 0274 | blastp |
| 2912 | pseudoroegneria\| gb167\|FF346975 _T1 | pseudoroegneria | 3120 | 30 | 85 | 98.6111 1111 | 98.6254 2955 | blastp |
| 2913 | pseudoroegneria\| gb167\|FF342094 _T1 | pseudoroegneria | 3121 | 30 | 83 | 98.2638 8889 | 98.9726 0274 | blastp |
| 2914 | soybean\|gb167\|C A898412_T1 | soybean | 3122 | 30 | 84 | 94.4444 4444 | 95.0877 193 | blastp |
| 2915 | switchgrass\|gb16 7\|FE621985_T1 | switchgrass | 3123 | 30 | 87 | 52.4305 5556 | 98.6928 1046 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2916 | switchgrass\|gb16 7\|DN141371_T1 | switchgrass | 3124 | 30 | 86 | 98.2638 8889 | 98.9583 3333 | blastp |
| 2917 | tamarix\|gb166\|C F199714_T1 | tamarix | 3125 | 30 | 88 | 78.4722 2222 | 99.1228 0702 | blastp |
| 2918 | tamarix\|gb166\|C F226851_T1 | tamarix | 3126 | 30 | 85 | 98.2638 8889 | 99.3031 3589 | blastp |
| 2919 | walnuts\|gb166\|C B303847_T1 | walnuts | 3127 | 30 | 84 | 98.2638 8889 | 99.3103 4483 | blastp |
| 2920 | walnuts\|gb166\|C V194951_T1 | walnuts | 3128 | 30 | 86 | 98.2638 8889 | 99.3031 3589 | blastp |
| 2921 | walnuts\|gb166\|C V196162_T1 | walnuts | 3129 | 30 | 85 | 98.2638 8889 | 99.3150 6849 | blastp |
| 2922 | zamia\|gb166\|FD 765004_T1 | zamia | 3130 | 30 | 84 | 98.2638 8889 | 98.9547 0383 | blastp |
| 2923 | antirrhinum\|gb16 6\|AJ799752_T1 | antirrhinum | 3131 | 31 | 80 | 98.7854 251 | 100 | blastp |
| 2924 | kiwi\|gb166\|FG4 00670_T1 | kiwi | 3132 | 31 | 82 | 74.0890 6883 | 100 | blastp |
| 2925 | kiwi\|gb166\|FG4 18275_T1 | kiwi | 3133 | 31 | 83 | 98.7854 251 | 98.3870 9677 | blastp |
| 2926 | centauxea\|gb166\| EL931588_T1 | centaurea | 3134 | 34 | 83 | 88.5714 2857 | 32.7402 1352 | blastp |
| 2927 | soybean\|gb167\|A W119586_T1 | soybean | 3135 | 34 | 83 | 94.2857 1429 | 36.2637 3626 | blastp |
| 2928 | soybean\|gb167\|A W573764_T1 | soybean | 3136 | 34 | 83 | 94.2857 1429 | 36.6666 6667 | blastp |
| 2929 | amborella\|gb166\| FD440187_T1 | amborella | 3137 | 35 | 81 | 65.7627 1186 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2930 | centauxea\|gb166\| EL931433_T1 | centaurea | 3138 | 35 | 82 | 97.62711864 | 96.61016949 | blastp |
| 2931 | eucalyptus\|gb16 166\|CD668486_T1 | eucalyptus | 3139 | 35 | 85 | 73.89830508 | 100 | blastp |
| 2932 | petunia\|gb166\|D C243166_T1 | petunia | 3140 | 36 | 86 | 100 | 100 | blastp |
| 2933 | amborella\|gb166\| CD482946_T1 | amborella | 3141 | 39 | 82 | 99.64157706 | 98.93992933 | blastp |
| 2934 | antirrhinum\|gb16 6\|AJ559435_T1 | antirrhinum | 3142 | 39 | 88 | 99.28315412 | 98.57651246 | blastp |
| 2935 | antirrhinum\|gb16 6\|AJ793990_T1 | antirrhinum | 3143 | 39 | 89 | 71.32616487 | 100 | blastp |
| 2936 | antirrhinum\|gb16 6\|AJ559760_T1 | antirrhinum | 3144 | 39 | 83 | 61.29032258 | 87.24489796 | blastp |
| 2937 | antirrhinum\|gb16 6\|AJ558545_T1 | antirrhinum | 3145 | 39 | 88 | 88.53046595 | 100 | blastp |
| 2938 | bean\|gb167\|FE6 82762_T1 | bean | 3146 | 39 | 86 | 100 | 100 | blastp |
| 2939 | bean\|gb167\|CV5 31088_T1 | bean | 3147 | 39 | 86 | 99.64157706 | 98.94736842 | blastp |
| 2940 | bean\|gb167\|CA9 07460_T1 | bean | 3148 | 39 | 86 | 99.64157706 | 98.94736842 | blastp |
| 2941 | cenchrus\|gb166\| EB655519_T1 | cenchrus | 3149 | 39 | 83 | 97.49103943 | 95.86206897 | blastp |
| 2942 | centauxea\|gb166\| EL934360_T1 | centaurea | 3150 | 39 | 88 | 53.76344086 | 96.15384615 | blastp |
| 2943 | centauxea\|gb166\| EH751120_T1 | centaurea | 3151 | 39 | 80 | 88.53046595 | 100 | blastp |
| 2944 | centauxea\|gb166\| EH752971_T1 | centaurea | 3152 | 39 | 87 | 83.5125448 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2945 | centauxea\|gb166\| EH768434_T1 | centaurea | 3153 | 39 | 80 | 91.7562 724 | 99.6168 5824 | blastp |
| 2946 | centauxea\|gb166\| EH767287_T1 | centaurea | 3154 | 39 | 83 | 83.1541 2186 | 100 | blastp |
| 2947 | cichorium\|gb166 \|DT212008_T1 | cichorium | 3155 | 39 | 82 | 89.9641 5771 | 100 | blastp |
| 2948 | cichorium\|gb166 \|EL354583_T1 | cichorium | 3156 | 39 | 84 | 86.7383 5125 | 97.6190 4762 | blastp |
| 2949 | eucalyptus\|gb16 6\|CD668553_T1 | eucalyptus | 3157 | 39 | 87 | 83.8709 6774 | 100 | blastp |
| 2950 | eucalyptus\|gb16 6\|CD668534_T1 | eucalyptus | 3158 | 39 | 80 | 69.8924 7312 | 100 | blastp |
| 2951 | eucalyptus\|gb16 6\|CD668523_T1 | eucalyptus | 3159 | 39 | 88 | 100 | 99.3006 993 | blastp |
| 2952 | eucalyptus\|gb16 6\|CD669942_T1 | eucalyptus | 3160 | 39 | 83 | 100 | 100 | blastp |
| 2953 | kiwi\|gb166\|FG4 05216_T1 | kiwi | 3161 | 39 | 84 | 100 | 99.2932 8622 | blastp |
| 2954 | kiwi\|gb166\|FG4 95821_T1 | kiwi | 3162 | 39 | 86 | 50.1792 1147 | 100 | blastp |
| 2955 | kiwi\|gb166\|FG4 17997_T1 | kiwi | 3163 | 39 | 86 | 64.5161 2903 | 100 | blastp |
| 2956 | kiwi\|gb166\|FG4 03725_T1 | kiwi | 3164 | 39 | 83 | 73.1182 7957 | 100 | blastp |
| 2957 | kiwi\|gb166\|FG3 97310_T1 | kiwi | 3165 | 39 | 88 | 100 | 100 | blastp |
| 2958 | kiwi\|gb166\|FG4 08531_T1 | kiwi | 3166 | 39 | 83 | 100 | 99.3031 3589 | blastp |
| 2959 | leymus\|gb166\|E G381236_T1 | leymus | 3167 | 39 | 81 | 55.5555 5556 | 97.4683 5443 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2960 | leymus\|gb166\|E G376087_T1 | leymus | 3168 | 39 | 80 | 96.7741 9355 | 96.5517 2414 | blastp |
| 2961 | leymus\|gb166\|C D808804_T1 | leymus | 3169 | 39 | 81 | 100 | 100 | blastp |
| 2962 | leymus\|gb166\|E G378918_T1 | leymus | 3170 | 39 | 86 | 51.2544 8029 | 100 | blastp |
| 2963 | liriodendron\|gb1 66\|CK761396_T 1 | liriodendron | 3171 | 39 | 81 | 99.6415 7706 | 98.9619 3772 | blastp |
| 2964 | nuphar\|gb166\|ES 730700_T1 | nuphar | 3172 | 39 | 81 | 61.2903 2258 | 98.2758 6207 | blastp |
| 2965 | poppy\|gb166\|FE 965621_T1 | poppy | 3173 | 39 | 84 | 88.5304 6595 | 100 | blastp |
| 2966 | pseudoroegneria\| gb167\|FF340233_ T1 | pseudoroegneria | 3174 | 39 | 81 | 100 | 100 | blastp |
| 2967 | switchgrass\|gb16 7\|FE638368_T1 | switchgrass | 3175 | 39 | 80 | 100 | 100 | blastp |
| 2968 | switchgrass\|gb16 7\|FE657460_T1 | switchgrass | 3176 | 39 | 80 | 99.2831 5412 | 98.9583 3333 | blastp |
| 2969 | switchgrass\|gb16 7\|FE641178_T1 | switchgrass | 3177 | 39 | 82 | 78.8530 4659 | 86.5900 3831 | blastp |
| 2970 | switchgrass\|gb16 7\|FE619224_T1 | switchgrass | 3178 | 39 | 82 | 94.2652 3297 | 95.7295 3737 | blastp |
| 2971 | switchgrass\|gb16 7\|FE657460_T2 | switchgrass | 3179 | 39 | 81 | 100 | 100 | blastp |
| 2972 | tamarix\|gb166\|E H051524_T1 | tamarix | 3180 | 39 | 82 | 56.2724 0143 | 98.7421 3836 | blastp |
| 2973 | walnuts\|gb166\|C B304207_T1 | walnuts | 3181 | 39 | 84 | 100 | 99.3031 3589 | blastp |
| 2974 | walnuts\|gb166\|C B303561_T1 | walnuts | 3182 | 39 | 84 | 100 | 100 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2975 | walnuts\|gb166\|E L892579_T1 | walnuts | 3183 | 39 | 84 | 89.6057 3477 | 98.8235 2941 | blastp |
| 2976 | zamia\|gb166\|DY 032141_T1 | zamia | 3184 | 39 | 80 | 96.0573 4767 | 94.3262 4113 | blastp |
| 2977 | zamia\|gb166\|FD 764669_T1 | zamia | 3185 | 39 | 80 | 99.6415 7706 | 98.9247 3118 | blastp |
| 2978 | zamia\|gb166\|DY 034152_T1 | zamia | 3186 | 39 | 80 | 94.6236 5591 | 92.2535 2113 | blastp |
| 2979 | antirrhinum\|gb16 6\|AJ568195_T1 | antirrhinum | 3187 | 40 | 87 | 100 | 100 | blastp |
| 2980 | antirrhinum\|gb16 6\|AJ568110_T1 | antirrhinum | 3188 | 40 | 87 | 100 | 100 | blastp |
| 2981 | banana\|gb167\|FL 657842_T1 | banana | 3189 | 40 | 81 | 83.0388 6926 | 92.4901 1858 | blastp |
| 2982 | bruguiera\|gb166\| EF126757_T1 | bruguiera | 3190 | 40 | 84 | 100 | 100 | blastp |
| 2983 | centaurea\|gb166\| EH765776_T1 | centaurea | 3191 | 40 | 84 | 92.5795 053 | 100 | blastp |
| 2984 | eucalyptus\|gb16 6\|AJ627837_T1 | eucalyptus | 3192 | 40 | 85 | 100 | 100 | blastp |
| 2985 | kiwi\|gb166\|FG4 11924_T1 | kiwi | 3193 | 40 | 86 | 100 | 100 | blastp |
| 2986 | kiwi\|gb166\|FG4 00706_T1 | kiwi | 3194 | 40 | 85 | 100 | 100 | blastp |
| 2987 | kiwi\|gb166\|FG4 18898_T1 | kiwi | 3195 | 40 | 84 | 71.0247 3498 | 98.5 | blastp |
| 2988 | kiwi\|gb166\|FG4 20187_T1 | kiwi | 3196 | 40 | 85 | 72.7915 1943 | 100 | blastp |
| 2989 | kiwi\|gb166\|FG3 98010_T1 | kiwi | 3197 | 40 | 86 | 100 | 100 | blastp |
| 2990 | petunia\|gb166\|A F452012_T1 | petunia | 3198 | 40 | 88 | 100 | 100 | blastp |
| 2991 | tamarix\|gb166\|C V121772_T1 | tamarix | 3199 | 40 | 83 | 100 | 100 | blastp |
| 2992 | walnuts\|gb166\|E L893208_T1 | walnuts | 3200 | 40 | 84 | 100 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 2993 | bean\|gb167\|FD7 86218_T1 | bean | 3201 | 41 | 83 | 58.60927152 | 100 | blastp |
| 2994 | citrus\|gb166\|CX 074333_T2 | citrus | 3202 | 41 | 81 | 72.51655629 | 99.5412844 | blastp |
| 2995 | citrus\|gb166\|CX 074333_T1 | citrus | 3203 | 41 | 83 | 96.02649007 | 91.42857143 | blastp |
| 2996 | kiwi\|gb166\|FG4 20468_T2 | kiwi | 3204 | 41 | 84 | 87.08609272 | 97.04797048 | blastp |
| 2997 | kiwi\|gb166\|FG4 20468_T1 | kiwi | 3205 | 41 | 83 | 58.94039735 | 95.69892473 | blastp |
| 2998 | tamarix\|gb166\|E G972096_T1 | tamarix | 3206 | 42 | 82 | 60.97560976 | 95.23809524 | blastp |
| 2999 | pseudoroegneria\| gb167\|FF353501_ T1 | pseudoroegneria | 3207 | 43 | 86 | 98.3935743 | 98.79518072 | blastp |
| 3000 | switchgrass\|gb16 7\|FE646459_T1 | switchgrass | 3208 | 43 | 86 | 98.7951 8072 | 99.1967 8715 | blastp |
| 3001 | switchgrass\|gb16 7\|FL887003_T1 | switchgrass | 3209 | 43 | 84 | 71.4859 4378 | 93.2291 6667 | blastp |
| 3002 | wheat\|gb164\|BE 403397_T1 | wheat | 3210 | 43 | 86 | 98.3935 743 | 98.7951 8072 | blastp |
| 3003 | leymus\|gb166\|E G381168_T1 | leymus | 3211 | 44 | 96 | 52.0161 2903 | 100 | blastp |
| 3004 | pseudoroegneria\| gb167\|FF341567_ T1 | pseudoro egneria | 3212 | 44 | 97 | 100 | 100 | blastp |
| 3005 | switchgrass\|gb16 7\|FE618822_T1 | switchgrass | 3213 | 44 | 91 | 100 | 100 | blastp |
| 3006 | switchgrass\|gb16 7\|FE633786_T1 | switchgrass | 3214 | 44 | 92 | 78.2258 0645 | 100 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 3007 | eucalyptus\|gb16 6\|CB967586_T1 | eucalyptus | 3215 | 46 | 84 | 97.5155 2795 | 60.7843 1373 | blastp |
| 3008 | liriodendron\|gb1 66\|CK762443_T 1 | liriodendron | 3216 | 46 | 80 | 74.5341 6149 | 100 | blastp |
| 3009 | switchgrass\|gb16 7\|FE615387_T1 | switchgrass | 3217 | 46 | 90 | 100 | 61.3899 6139 | blastp |
| 3010 | walnuts\|gb166\|E L893973_T1 | walnuts | 3218 | 46 | 82 | 97.5155 2795 | 55.4744 5255 | blastp |
| 3011 | bean\|gb167\|FD7 82805_T1 | bean | 3219 | 47 | 91 | 88.1720 4301 | 79.6116 5049 | blastp |
| 3012 | bean\|gb167\|EY4 57935_T1 | bean | 3220 | 47 | 82 | 100 | 38.5892 1162 | blastp |
| 3013 | cichorium\|gb166 \|EH685648_T2 | cichorium | 3221 | 47 | 80 | 100 | 67.3913 0435 | blastp |
| 3014 | cichorium\|gb166 \|EH685648_T1 | cichorium | 3222 | 47 | 80 | 100 | 32.1799 308 | blastp |
| 3015 | citrus\|gb166\|CK 701147_T1 | citrus | 3223 | 47 | 90 | 100 | 76.8595 0413 | blastp |
| 3016 | citrus\|gb166\|CX 544905_T1 | citrus | 3224 | 47 | 81 | 79.5698 9247 | 88.0952 381 | blastp |
| 3017 | cycas\|gb166\|CB 088978_T1 | cycas | 3225 | 47 | 82 | 87.0967 7419 | 30.6818 1818 | blastp |
| 3018 | cycas\|gb166\|EX 920749_T1 | cycas | 3226 | 47 | 88 | 100 | 76.2295 082 | blastp |
| 3019 | kiwi\|gb166\|FG4 29765_T1 | kiwi | 3227 | 47 | 84 | 98.9247 3118 | 77.9661 0169 | blastp |
| 3020 | leymus\|gb166\|C N466394_T1 | leymus | 3228 | 47 | 87 | 100 | 79.4871 7949 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 3021 | leymus\|gb166\|E G376019_T1 | leymus | 3229 | 47 | 88 | 100 | 32.7464 7887 | blastp |
| 3022 | leymus\|gb166\|E G390723_T1 | leymus | 3230 | 47 | 81 | 100 | 31.9587 6289 | blastp |
| 3023 | leymus\|gb166\|E G387193_T1 | leymus | 3231 | 47 | 81 | 100 | 32.1799 308 | blastp |
| 3024 | nuphar\|gb166\|C D473277_T1 | nuphar | 3232 | 47 | 91 | 100 | 75.6097 561 | blastp |
| 3025 | nuphar\|gb166\|F D384794_T1 | nuphar | 3233 | 47 | 86 | 96.7741 9355 | 81.0810 8108 | blastp |
| 3026 | nuphar\|gb166\|C D475538_T1 | nuphar | 3234 | 47 | 89 | 100 | 69.9248 1203 | blastp |
| 3027 | nuphar\|gb166\|C D472711_T1 | nuphar | 3235 | 47 | 91 | 100 | 48.9473 6842 | blastp |
| 3028 | petunia\|gb166\|D C240378_T1 | petunia | 3236 | 47 | 92 | 58.0645 1613 | 98.1818 1818 | blastp |
| 3029 | pseudoroegneria\|gb167\|FF344283_ T1 | pseudoroegneria | 3237 | 47 | 87 | 100 | 32.7464 7887 | blastp |
| 3030 | sorghum\|gb161.c rp\|AI724931_T1 | sorghum | 3238 | 47 | 92 | 100 | 32.5174 8252 | blastp |
| 3031 | switchgrass\|gb16 7\|FL923354_T1 | switchgrass | 3239 | 47 | 82 | 100 | 33.6956 5217 | blastp |
| 3032 | switchgrass\|gb16 7\|FE657461_T1 | switchgrass | 3240 | 47 | 92 | 98.9247 3118 | 74.7967 4797 | blastp |
| 3033 | switchgrass\|gb16 7\|FL765830_T1 | switchgrass | 3241 | 47 | 82 | 100 | 72.2222 2222 | blastp |
| 3034 | switchgrass\|gb16 7\|FL915169_T1 | switchgrass | 3242 | 47 | 83 | 97.8494 6237 | 87.5 | blastp |

(continued)

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 3035 | tamarix\|gb166\|E H054604_T1 | tamarix | 3243 | 47 | 83 | 100 | 65.6470 5882 | tblastn |
| 3036 | walnuts\|gb166\|C B303798_T1 | walnuts | 3244 | 47 | 91 | 100 | 80.1724 1379 | blastp |
| 3037 | bruguiera\|gb166\| BP942548_T1 | bruguiera | 3245 | 48 | 82 | 52.0547 9452 | 100 | blastp |
| 3038 | bruguiera\|gb166\| BP938825_T1 | bruguiera | 3246 | 48 | 89 | 56.6210 0457 | 91.1764 7059 | blastp |
| 3039 | centauxea\|gb166\| EH739326_T1 | centaurea | 3247 | 48 | 83 | 87.2146 1187 | 50 | tblastn |
| 3040 | eucalyptus\|gb16 6\|CD669176_T1 | eucalyptus | 3248 | 48 | 87 | 56.1643 8356 | 91.7910 4478 | blastp |
| 3041 | leymus\|gb166\|E G382428_T1 | leymus | 3249 | 48 | 80 | 52.0547 9452 | 90.4761 9048 | blastp |
| 3042 | liriodendron\|gb1 66\|CK743477_T 1 | liriodendron | 3250 | 48 | 81 | 94.0639 2694 | 54.5454 5455 | tblastn |
| 3043 | marchantia\|gb16 6\|BJ840587_T1 | marchantia | 3251 | 48 | 83 | 94.0639 2694 | 72.5352 1127 | blastp |
| 3044 | marchantia\|gb16 6\|C96070_T1 | marchantia | 3252 | 48 | 83 | 93.1506 8493 | 71.5789 4737 | blastp |
| 3045 | nuphar\|gb166\|C K748374_T1 | nuphar | 3253 | 48 | 82 | 65.7534 2466 | 99.3103 4483 | blastp |
| 3046 | pseudoroegneria\| gb167\|FF340047_ T1 | pseudoroegneria | 3254 | 48 | 81 | 96.8036 5297 | 73.4482 7586 | blastp |
| 3047 | pseudoroegneria\| gb167\|FF340899_ T1 | pseudoroegneria | 3255 | 48 | 81 | 96.8036 5297 | 73.4482 7586 | blastp |
| 3048 | pseudoroegneria\| gb167\|FF352644_ T1 | pseudoroegneria | 3256 | 48 | 82 | 96.8036 5297 | 74.1258 7413 | blastp |

| Polynuc. SEQ ID NO: | Cluster name | Organism | Polypep. SEQ ID NO: | Hom. of SEQ ID NO: | % Ident. | Query cover. | % Subject cover. | Algorithm |
|---|---|---|---|---|---|---|---|---|
| 3049 | sorghum\|gb161.c rp\|SBGWP0301 88_T1 | sorghum | 3257 | 48 | 80 | 96.8036 5297 | 74.1258 7413 | blastp |
| 3050 | switchgrass\|gb16 7\|FL718379_T1 | switchgrass | 3258 | 48 | 81 | 96.8036 5297 | 74.1258 7413 | blastp |
| 3051 | switchgrass\|gb16 7\|FE639195_T1 | switchgrass | 3259 | 48 | 82 | 96.8036 5297 | 73.1034 4828 | blastp |

**Table 3:** Homologues and orthologues of the AQP proteins are provided. Homology was calculated as % of identity over the aligned sequences. Polynuc. = Polynucleotide; Polypep. = Polypeptide; Hom. = Homologues/Orthologues; % Ident. = percent identity; Cover. = coverage.

*EXAMPLE 2*

*mRNA EXPRESSION OF IN SILICO EXPRESSED POLYNUCLEOTIDES*

Messenger RNA levels were determined using reverse transcription assay followed by quantitative Real-Time PCR (qRT-PCR) analysis. RNA levels were compared between leaves of 20 days old seedlings of tomato plants grown under salinity water. A correlation analysis between mRNA levels in different experimental conditions/genetic backgrounds was performed in order to determine the role of the gene in the plant.

*Materials and Experimental Methods*

*Quantitative Real Time RT-PCR (qRT-PCR) -* To verify the level of expression, specificity and trait-association, Reverse Transcription followed by quantitative Real-Time PCR (qRTPCR) was performed on total RNA extracted from leaves of 2 tomato varieties namely YO361 (salt tolerant variety) and FA191 (salt sensitive variety). Messenger RNA (mRNA) levels were determined for AQP genes, expressed under normal and stressed conditions.

Twenty days-old tomato seedlings were grown in soil and soaked with 300 mM NaCl for 0, 1, 6, 24, 118 hours. Leaves were harvested and frozen in liquid nitrogen and then kept at -80 °C until RNA extraction. Total RNA was extracted from leaves using RNeasy plant mini kit (Qiagen, Hilden, Germany) and by using the protocol provided by the manufacturer. Reverse transcription was performed using 1 μg total RNA, using 200 U Super Script II Reverse Transcriptase enzyme (Invitrogen), 150 ng random deoxynucleotide hexamers (Invitrogen), 500 μM deoxynucleotide tri-phosphates (dNTPs) mix (Takara, Japan), 0.2 volume of x5 reverse transcriptase (RT) buffer (Invitrogen), 0.01 M dithiothreitol (DTT), 40 U RNAsin (Promega), diethylpyrocarbonate (DEPC) treated double distilled water (DDW) was added up to 24 μl.

Mix of RNA, random deoxynucleotide hexamers, dNTPs mix and DEPC treated DDW was incubated at 65 °C for 5 minutes, followed by 4 °C for 5 minutes. Mix of reverse transcriptase (RT) buffer, dithiothreitol (DTT) and RNAsin was added to the RT reactions followed by incubation at 25 °C for 10 minutes and at 42 °C for 2 minutes afterwards. Finally, Super Script II Reverse Transcriptase enzyme was added to the RT reactions that were further incubated for 50 minutes at 42 °C, followed by 70 °C for 15 minutes.

cDNA was diluted 1:20 in Tris EDTA, pH = 7.5 for MAB69 and housekeeping genes. For MAB58 and MAB59 cDNA was diluted 1:2 due to very weak expression and consequently for housekeeping genes cDNA was diluted 1:8 in order to insert the Ct values in calibration curve range. 5 μL of the diluted cDNA was used for qPCR.

For qPCR amplification, primers of the AQP genes were designed, as summarized in Table 4 below. The expression level of the housekeeping genes: Actin (SEQ ID NO: 2841), GAPDH (SEQ ID NO: 2842) and RPL19 (SEQ ID NO: 2747) was determined in order to normalize the expression level between the different tissues.

*Table 4*

| Primers for qPCR amplification | | | | |
|---|---|---|---|---|
| Gene | Forward primer SEQ ID NO: | Forward primer sequence (5'→3') | Reverse primer SEQ ID NO: | Reverse primer sequence (5'→3') |
| MAB58 | 2829 | CTTTTGGTAGGGCCGATGAAG | 2830 | CGAAGATGAAGGTGGATAAGAGCT |
| MAB59 | 2831 | CAGTATGAACGTCTCCGGTGG | 2832 | CAACAGCACCTAGCAACTGACC |
| MAB69 | 2833 | TGTCTTGGATTCCATTGAGCACT | 2834 | GTTTGAGCTGCTGTCCCCA |
| Actin (SEQ ID NO: 2841) | 2835 | CCACATGCCATTCTCCGTCT | 2836 | GCTTTTCTTTCACGTCCCTGA |
| GAPD H (SEQ ID NO: 2842) | 2837 | TTGTTGTGGGTGTCAACGAGA | 2838 | ATGGCGTGGACAGTGGTCA |

(continued)

| Primers for qPCR amplification | | | | |
|---|---|---|---|---|
| Gene | Forward primer SEQ ID NO: | Forward primer sequence (5'→3') | Reverse primer SEQ ID NO: | Reverse primer sequence (5'→3') |
| RPL19 (SEQ ID NO: 2747) | 2839 | CACTCTGGATATGGTAAGC GTAAGG | 2840 | TTCTTGGACTCCCTGTA CTTACGA |
| Table 4. | | | | |

### Experimental Results

### Changes in mRNA levels of AQP genes in leaves of plants under salt tolerance

- Steady state levels of tomato AQP genes in leaves of tolerant versus sensitive lines, under salinity conditions are summarized in Table 5 below. In all 3 cases, aquaporin gene expression was increased after plant was exposed to salt stress. Gene peak expression was higher in the salt tolerance tomato line (YO361) versus the sensitive line (FA191). The elevated gene expression demonstrates the involvement of the tested AQP genes in tomato plants tolerating high salinity.

*Table 5*

| Expression levels of tomato AQP genes | | | |
|---|---|---|---|
| Well name (cDNA) | MAB58 | MAB59 | MAB69* |
| leaf FA191 0 h | 2.86 | 5.97 | 875 |
| leaf FA191 1 h | 4.56 | 5.73 | 597 |
| leaf FA191 6 h | 5.34 | 8.2 | 945 |
| leaf FA191 24 h | 42.7 | 62.4 | 613 |
| leaf FA191 118 h | 55.4 | 22.2 | 1800 |
| leaf Y0361 0 h | 1.96 | 2.81 | 638 |
| leaf Y0361 1 h | 2.33 | 0.517 | 583 |
| leaf Y0361 6 h | 2.88 | 5.66 | 464 |
| leaf Y0361 24 h | 75.4 | 139 | 513 |
| leaf Y0361 118 h | 26.3 | Not determined | 2300 |
| Table 5: Provided are the steady state levels of tomato AQP genes under salinity conditions [the incubation periods in the salt solution are provided in hours (h)]. Different dilutions of cDNA were used (1:20 for MAB69 and 1:2 for MAB58 and MAB59). Numbers are given after normalization for each sample. | | | |

### EXAMPLE 3

### GENE CLONING AND GENERATION OF BINARY VECTORS FOR PLANT EXPRESSION

To validate their role in improving ABST and yield, the AQP genes were over-expressed in plants, as follows.

### Cloning strategy

Selected genes from those presented in Example 1 were cloned into binary vectors for the generation of transgenic plants. For cloning, the full-length open reading frames (ORFs) were identified. EST clusters and in some cases mRNA sequences were analyzed to identify the entire open reading frame by comparing the results of several translation algorithms to known proteins from other plant species. In case where the entire coding sequence is not found, RACE kits from Ambion or Clontech (RACE = Rapid Access to cDNA Ends) were used to prepare RNA from the plant samples to thereby access the full cDNA transcript of the gene.

In order to clone the full-length cDNAs, Reverse Transcription followed by PCR (RT-PCR) was performed on total RNA extracted from leaves, roots or other plant tissues, growing under either normal or nutrient deficient conditions. Total RNA extraction, production of cDNA and PCR amplification was performed using standard protocols described elsewhere (Sambrook J., E.F. Fritsch, and T. Maniatis. 1989. Molecular Cloning. A Laboratory Manual., 2nd Ed. Cold Spring Harbor Laboratory Press, New York.), and are basic for those skilled in the art. PCR products were purified using PCR purification kit (Qiagen) and sequencing of the amplified PCR products was performed, using ABI 377 sequencer (Applied Biosystems).

Usually, 2 sets of primers were ordered for the amplification of each gene, via nested PCR (meaning first amplifying the gene using external primers and then using the produced PCR product as a template for a second PCR reaction, where the internal set of primers are used). Alternatively, one or two of the internal primers were used for gene amplification, both in the first and the second PCR reactions (meaning only 2-3 primers were designed for a gene). To facilitate further cloning of the cDNAs, a 8-12 bp extension is added to the 5' primer end of each internal primer. The primer extension includes an endonuclease restriction site. The restriction sites are selected using two parameters: (a) The restriction site does not exist in the cDNA sequence; and (b) The restriction sites in the forward and reverse primers are designed so the digested cDNA is inserted in the sense formation into the binary vector utilized for transformation. In Table 6 below, primers used for cloning tomato and barley AQPs are provided.

**Table 6**

| MAB gene No. | Forward external primer sequence from 5'→3' (SEQ ID NO:) | Forward internal primer sequence from 5'→3' (SEQ ID NO:) | Reverse external primer sequence from 5'→3 (SEQ ID NO:) | Reverse internal primer sequence from 5'→3'(SEQ ID NO:) |
|---|---|---|---|---|
| | | Primers used for cloning tomato and barley AQP genes | | |
| 55 | GGAGTCGACGACCAT CAAGTTTTAAGTGAC TTC (2770) | GGAGTCGACTT AAGTACATTCTT TAGTGAGAGCC (2771) | TGAGCTCACTTC AAAACCATCCG TTGTC (2772) | TGAGCTCCCATCC GTTGTCAAAATGA AC (2773) |
| 56 | | GGAGTCGACGT AAGAAACAATA ATGCCAATTTC (2774) | CGAGCTCGTAA AGCCAAGTTTTG AAAGAC (2775) | CGAGCTCAAGAC AAACAAAGAGAA GAGGG (2776) |
| 57 | | GTTAAAAATGC CGATCAACC (2777) | GCGATATCTAA ATAACAAAAGC CGTCCG (2778) | GCGATATCAGCCG TCCGAATAAACAA AG (2779) |
| 58 | AATGTCGACCGAATT GATCTCCTTCTTGATC (2780) | TATGTCGACTTC ATTTCTTGGGTC ACTCG (2781) | TTTCTAGAGGTC TGGGATTATCGT CTTG (2782) | TTTCTAGAGATGT GCAGGCAGCTAC ATAC (2783) |
| 59 | AATGTCGACTTTAAG CGGTGTGTTTTGTG (2784) | AATGTCGACTC ACAATTATGCA GCCACG (2785) | | AATCTAGATTAGA CCCAAACATACAA ACTTCAC (2786)) |
| 69 | TAAGTCGACACAAAC CTTATCCTGGTCTCAT C (2787) | AATGTCGACCTT GGATTCCATTGA GCACTC (2788) | | TGAGCTCTGGAGA AAGAAAACTTTAG ATACA (2789) |
| 70 | AACTGCAGAGCTGTA CATGGTCCTCCTCC (2790) | AACTGCAGTGT ACATGGTCCTCC TCCG (2791) | TCCCGGGCCAG ACAAAACTTCA ATTTCATC (2792) | TCCCGGGCTTCAA TTTCATCTTCTGA TTTC (2793) |
| 71 | AAAGTCGACGGAAAA TGCATTAAAACCTTA AG (2794) | TTTGTCGACCTT AGTTTTCTCCCA CATATGG (2795) | AATCTAGACAA GTAGAGGTACT AGGTAGGGAC (2796) | AATCTAGAGTACT AGGTAGGGACAA TATGATATG (2797) |

| | Primers used for cloning tomato and barley AQP genes | | | |
|---|---|---|---|---|
| MAB gene No. | Forward external primer sequence from 5'→3' (SEQ ID NO:) | Forward internal primer sequence from 5'→3' (SEQ ID NO:) | Reverse external primer sequence from 5'→3 (SEQ ID NO:) | Reverse internal primer sequence from 5'→3'(SEQ ID NO:) |
| 72 | AATGTCGACGTGGAG GAGGAGTCTTTGATA C (2798) | AATGTCGACCTC CAACACTCTTAT CAATTACCA (2799) | | TATCTAGAGGATG CAACTACAAAGA AATTG (2800) |
| 74 | | TTCTGCAGGTTT GGGAGTTATTG ATCTAAGATG (2801) | | TCCCGGGGCATAG TTCACACAGAGCA AATC (2802) |
| 76 | AATGTCGACCTGTAT CCTCTTAAGTATGAA TCG (2803) | AATGTCGACGT CGTCTTGTATGT ATTTGTACTACT G (2804) | TTTCTAGACTAG TGGTATAGATC ATTTTATGGTGA C (2805) | AATCTAGATTAGA GCTGGAGAATGA ACTGAAGC (2806) |
| 77 | AACTGCAGCTTCTTTC ACCGAGTGGGAG (2807) | AACTGCAGCTTT CACCGAGTGGG AGAG (2808) | ACCCGGGAATT CCAACTAGCTGT TATGATTC (2809) | TCCCGGGTCCAAC TAGCTGTTATGAT TCTG (2810) |
| 79 | AAGATATCAAAAAAA TGTCGAAGGACGTG (2811) | AAGATATCAAC AATGTCGAAGG ACGTGATTGA (2812) | | AAGATATCGACCA CCAACTCTAGTCT CATACC (2813) |
| 115 | AGATTCGAATCTTTA GCCTG (2814) | AATCTAGAGAA GTCACAGAAA AACAGTCGAG (2815) | | AGAGCTCTTAAGG GAAATTCATCACA CAAGG (2816) |
| 116 | AAAGTCGACCTCATC AGTGTTAAAGCCATA AG (2817) | TTTGTCGACCAT AAGCCCTCTTTG AGTGTG (2818) | TATCTAGAATTG AATCGAAAGGG AAACAC (2819) | TTTCTAGAGACCG TGACACACCATTT GTAC (2820) |

(continued)

| MAB gene No. | Forward external primer sequence from 5'→3' (SEQ ID NO:) | Forward internal primer sequence from 5'→3' (SEQ ID NO:) | Reverse external primer sequence from 5'→3 (SEQ ID NO:) | Reverse internal primer sequence from 5'→3'(SEQ ID NO:) |
|---|---|---|---|---|
| | | Primers used for cloning tomato and barley AQP genes | | |
| 117 | | TTTCTAGACTCA GCGACAACATT TCATCTC (2821) | | TGAGCTCCAGATA GAGAAGCATGCA TCATC (2822) |

Table 6.

PCR products were purified (PCR Purification Kit, Qiagen, Germany) and digested with the restriction endonucleases (Roche, Switzerland) according to the sites design in the primers (Tables 8 and 9 below). Each of the digested PCR products was cloned first into high copy plasmid pBlue-script KS [Hypertext Transfer Protocol://World Wide Web (dot) stratagene (dot) com/manuals/212205 (dot) pdf] which was digested with the same restriction enzymes. In some cases (Table 8, below) the Nopaline Synthase (NOS) terminator originated from the binary vector pBI101.3 [nucleotide coordinates 4417-4693 in GenBank Accession No. U12640 (SEQ ID NO:2824)] was already cloned into the pBlue-script KS, between the restriction endonuclease sites *Sac*I and *Eco*RI, so the gene is introduced upstream of the terminator. In other cases (Table 9, below) the At6669 promoter (SEQ ID NO: 2823) is already cloned into the pBlue-script KS, so the gene is introduced downstream of the promoter. The digested PCR products and the linearized plasmid vector were ligated using T4 DNA ligase enzyme (Roche, Switzerland). Sequencing of the inserted genes was conducted, using ABI 377 sequencer (Applied Biosystems). Sequences of few of the cloned AQP genes, as well as their encoded proteins are listed in Table 7, below.

*Table 7: Cloned sequences*

| Serial No | Gene Name | Polynucleotide SEQ ID NO: | Polypeptide SEQ ID NO: |
|---|---|---|---|
| 1 | MAB115 | 2748 | 2765 |
| 2 | MAB116 | 2749 | 47 |
| 3 | MAB117 | 2750 | 48 |
| 4 | MAB54 | 2751 | 27 |
| 5 | MAB55 | 2752 | 28 |
| 6 | MAB56 | 2753 | 29 |
| 7 | MAB57 | 2754 | 30 |
| 8 | MAB58 | 2755 | 2766 |
| 9 | MAB59 | 2756 | 2767 |
| 10 | MAB69 | 2757 | 33 |
| 11 | MAB70 | 2758 | 34 |
| 12 | MAB71 | 2759 | 2768 |
| 13 | MAB72 | 2760 | 2769 |
| 14 | MAB72 (Optimized for expression in Arabidopsis,Tomato and Maize) | 2843 | 2769 |
| 15 | MAB74 | 2761 | 38 |
| 16 | MAB76 | 2762 | 40 |
| 17 | MAB77 | 2763 | 41 |
| 18 | MAB79 | 2764 | 43 |
| Table 7. | | | |

The genes were digested again and ligated into pPI or pGI binary plasmids, harboring the At6669 promoter (between the *Hind*III and *Sal*I restriction endonucleases site) (Table 8). In other cases the At6669 promoter together with the gene are digested out of the pBlue-script KS plasmid and ligated into pPI or pGI binary plasmids, using restriction endonucleases as given in Table 8.

**Table 8**

| MAB gene No. (SEQ ID NO:) | Restriction enzymes used for cloning into high copy plasmid-FORWARD | Restriction enzymes used for cloning into high copy plasmid-REVERSE | Restriction enzymes used for cloning into binary vector-FORWARD | Restriction enzymes used for cloning into binary vector-REVERSE | Restriction enzymes used for digesting the binary vector |
|---|---|---|---|---|---|
| **Restriction enzyme sites used to clone the MAB AQP genes into pKS+NOS terminator high copy plasmid, followed by cloning into the binary vector pGI+At6669 promoter** | | | | | |
| 54 (SEQ ID NO:2751) | XbaI | SacI | SalI | EcoRI | SalI/EcoRI |
| 55 (SEQ ID NO:2752) | SalI | SacI | SalI | EcoRI | SalI/EcoRI |
| 56 (SEQ ID NO:2753) | SalI | SacI | SalI | EcoRI | SalI/EcoRI |
| 58 (SEQ ID NO:2755) | SalI | XbaI | SalI | EcoRI | SalI/EcoRI |
| 59 (SEQ ID NO:2756) | SalI | XbaI | SalI | EcoRI | SalI/EcoRI |
| 69 (SEQ ID NO:2757) | SalI | SacI | SalI | EcoRI | SalI/EcoRI |
| 71 (SEQ ID NO:2759) | SalI | XbaI | SalI | EcoRI | SalI/EcoRI |
| 72 (SEQ ID NO:2760) | SalI | XbaI | SalI | EcoRI | SalI/EcoRI |
| 76 (SEQ ID NO:2762) | SalI | XbaI | SalI | EcoRI | SalI/EcoRI |

(continued)

*Restriction enzyme sites used to clone the MAB AQP genes into pKS+NOS terminator high copy plasmid, followed by cloning into the binary vector pGI+At6669 promoter*

| MAB gene No. (SEQ ID NO:;) | Restriction enzymes used for cloning into high copy plasmid-FORWARD | Restriction enzymes used for cloning into high copy plasmid-REVERSE | Restriction enzymes used for cloning into binary vector-FORWARD | Restriction enzymes used for cloning into binary vector-REVERSE | Restriction enzymes used for digesting the binary vector |
|---|---|---|---|---|---|
| 115 (SEQ ID NO:2748) | XbaI | SacI | SalI | EcoRI | SalI/EcoRI |
| 116 (SEQ ID NO:2749) | SalI | XbaI | SalI | EcoRI | SalI/EcoRI |
| 117 (SEQ ID NO:2750) | XbaI | SacI | SalI | EcoRI | SalI/EcoRI |

**Table 8:** MAB AQP genes cloned into pKS+NOS terminator high copy plasmid, followed by cloning into the binary vector pGI+At6669 promoter.

*Table 9*

| Restriction enzyme sites used to clone the MAB AQP genes into pKS+At6669 promoter high copy plasmid, followed by cloning promoter+gene into pGI binary vector | | | | | |
|---|---|---|---|---|---|
| MAB gene No. (SEQ ID NO:) | Restriction enzymes used for cloning into high copy plasmid-FORWARD | Restriction enzymes used for cloning into high copy plasmid-REVERSE | Restriction enzymes used for cloning into binary vector-FORWARD | Restriction enzymes used for cloning into binary vector-REVERSE | Restriction enzymes used for digesting the binary vector |
| 57 (SEQ ID NO:2754 ) | Blunt | EcoRV | SalI | EcoRV | SalI /Ec1136 II |
| 70 (SEQ ID NO:2758 ) | PstI | SmaI | BamHI | SmaI | BamHI /Ec1136II |
| 74 (SEQ ID NO:2761 ) | PstI | SmaI | BamHI | SmaI | BamHI /Ec1136II |
| 77 (SEQ ID NO:2763 ) | PstI | SmaI | BamHI | SmaI | BamHI /Ec1136II |
| 79 (SEQ ID NO:2764 ) | EcoRI | EcoRV | SalI | SmaI | SalI /Ec1136 II |
| **Table 9:** MAB AQP genes cloned into pKS+At6669 promoter high copy plasmid, followed by cloning promoter+gene into pGI binary vector. | | | | | |

The pPI plasmid vector was constructed by inserting a synthetic poly-(A) signal sequence, originating from pGL3 basic plasmid vector (Promega, Acc. No. U47295; bp 4658-4811) into the *Hind*III restriction site of the binary vector pBI101.3 (Clontech, Acc. No. U12640). pGI (Figure 1) is similar to pPI, but the original gene in the back bone is GUS-Intron, rather than GUS. The cloned genes were sequenced.

Synthetic sequences (such as of MAB54, nucleotide SEQ ID NO: 2751, which encodes protein SEQ ID NO: 27; or MAB72 SEQ ID NO:2843, which encodes SEQ ID NO:2769) of some of the cloned polynucleotides were ordered from a commercial supplier (GeneArt, GmbH). To optimize the coding sequence, codon-usage Tables calculated from plant transcriptomes were used [example of such Tables can be found in the Codon Usage Database available online at Hypertext Transfer Protocol://World Wide Web (dot) kazusa (dot) or (dot) jp/codon/]. The optimized coding sequences were designed in a way that no changes were introduced in the encoded amino acid sequence while using codons preferred for expression in dicotyledonous plants mainly tomato and *Arabidopsis*; and monocotyledonous plants such as maize. Such optimized sequences promote better translation rate and therefore higher protein expression levels. To the optimized sequences flanking additional unique restriction enzymes sites were added to facilitate cloning genes in binary vectors.

Promoters used: CaMV 35S promoter (SEQ ID NO: 2825) and Arabidopsis At6669 promoter (SEQ ID NO: 2823; which is SEQ ID NO:61 of WO04081173 to Evogene Ltd.).

## EXAMPLE 4

### GENERATION OF TRANSGENIC PLANTS EXPRESSING THE AQP GENES

#### Experimental Results

*Arabidopsis transformation-* Arabidopsis transformation of the following MAB genes and orthologues: MAB115, MAB54, MAB55, MAB56, MAB57, MAB58, MAB59 (ortholog of MAB58), MAB69, MAB70, MAB71, MAB72, MAB74, MAB76, MAB77, MAB79, MAB116 (ortholog of MAB115 and MAB55), and MAB117 (the sequence identifiers of the cloned polynucleotides and their expressed polypeptides are provided in Table 7 above) was performed according to Clough SJ, Bent AF. (1998) "Floral dip: a simplified method for Agrobacterium-mediated transformation of Arabidopsis thaliana." Plant J. 16(6): 735-43; and Desfeux C, Clough SJ, Bent AF. (2000) "Female reproductive tissues are the primary targets of Agrobacterium-mediated transformation by the Arabidopsis floral-dip method." Plant Physiol. 123(3): 895-904; with minor modifications. Briefly, *Arabidopsis thaliana* Columbia (Co10) To Plants were sown in 250 ml pots filled with wet peat-based growth mix. The pots were covered with aluminum foil and a plastic dome, kept at 4 °C for 3-4 days, then uncovered and incubated in a growth chamber at 18-24 °C under 16/8 hours light/dark cycles. The To plants were ready for transformation six days prior to anthesis. Single colonies of *Agrobacterium* carrying the binary vectors harboring the AQP genes are cultured in LB medium supplemented with kanamycin (50 mg/L) and gentamycin (50 mg/L). The cultures were incubated at 28 °C for 48 hours under vigorous shaking and centrifuged at 4000 rpm for 5 minutes. The pellets comprising *Agrobacterium* cells were resuspended in a transformation medium which contained half-strength (2.15 g/L) Murashige-Skoog (Duchefa); 0.044 $\mu$M benzylamino purine (Sigma); 112 $\mu$g/L B5 Gambourg vitamins (Sigma); 5 % sucrose; and 0.2 ml/L Silwet L-77 (OSI Specialists, CT) in double-distilled water, at pH of 5.7.

Transformation of To plants was performed by inverting each plant into an *Agrobacterium* suspension such that the flowering stem was submerged for 3-5 seconds. Each inoculated To plant was immediately placed in a plastic tray, then covered with clear plastic dome to maintain humidity and kept in the dark at room temperature for 18 hours to facilitate infection and transformation. Transformed (transgenic) plants were then uncovered and transferred to a greenhouse for recovery and maturation. The transgenic To plants were grown in the greenhouse for 3-5 weeks until siliques maturation, and then seeds were harvested and kept at room temperature until sowing.

For generating T1 and $T_2$ transgenic plants harboring the genes, seeds collected from transgenic To plants are surface-sterilized by soaking in 70 % ethanol for 1 minute, followed by soaking in 5 % sodium hypochlorite and 0.05 % Triton X-100 for 5 minutes. The surface-sterilized seeds are thoroughly washed in sterile distilled water then placed on culture plates containing half-strength Murashige-Skoog (Duchefa); 2 % sucrose; 0.8 % plant agar; 50 mM kanamycin; and 200 mM carbenicylin (Duchefa). The culture plates are incubated at 4 °C for 48 hours then transferred to a growth room at 25 °C for an additional week of incubation. Vital $T_1$ *Arabidopsis* plants are transferred to fresh culture plates for another week of incubation. Following incubation the $T_1$ plants are removed from culture plates and planted in growth mix contained in 250 ml pots. The transgenic plants were allowed to grow in a greenhouse to maturity. Seeds harvested from $T_1$ plants are cultured and grown to maturity as $T_2$ plants under the same conditions as used for culturing and growing the $T_1$ plants. At least 10 independent transformation events are created from each construct for which T2 seeds are collected. The introduction of the gene is determined for each event by PCR performed on genomic DNA extracted from each event produced.

*Transformation of tomato (Var M82) plants with putative cotton genes-*Tomato (Solanum esculentum, var M82) transformation and cultivation of transgenic plants is effected according to: "Curtis I.S, Davey M.R, and Power J.B. 1995. "Leaf disk transformation". Methods Mol. Biol. 44, 59-70 and Meissner R, Chague V, Zhu Q, Emmanuel E, Elkind Y, Levy A.A. 2000. "Technical advance: a high throughput system for transposon tagging and promoter trapping in tomato". Plant J. 22, 265-74; with slight modifications.

## EXAMPLE 5

### EVALUATING TRANSGENICARABIDOPSIS PLANT GROWTH UNDER ABIOTIC STRESS AS WELL AS UNDER FAVORABLE CONDITIONS IN TISSUE CULTURE ASSAY

*Assay 1: plant growth under osmotic stress [poly (ethylene glycol) (PEG)] in tissue culture conditions* - One of the consequences of drought is the induction of osmotic stress in the area surrounding the roots; therefore, in many scientific studies, PEG (e.g., 25 % PEG8000) is used to simulate the osmotic stress conditions resembling the high osmolarity found during drought stress.

Surface sterilized seeds were sown in basal media [50 % Murashige-Skoog medium (MS) supplemented with 0.8 % plant agar as solidifying agent] in the presence of Kanamycin (for selecting only transgenic plants). After sowing, plates were transferred for 2-3 days for stratification at 4 °C and then grown at 25 °C under 12-hour light 12-hour dark daily cycles for 7 to 10 days. At this time point, seedlings randomly chosen were carefully transferred to plates containing 25 % PEG: 0.5 MS media or Normal growth conditions (0.5 MS media). Each plate contained 5 seedlings of the same transgenic event, and 3-4 different plates (replicates) for each event. For each polynucleotide of the disclosure at least four independent transformation events were analyzed from each construct. Plants expressing the polynucleotides of the disclosure were compared to the average measurement of the control plants (empty vector or GUS reporter gene under the same promoter) used in the same experiment.

*Digital imaging -* A laboratory image acquisition system, which consists of a digital reflex camera (Canon EOS 300D) attached with a 55 mm focal length lens (Canon EF-S series), mounted on a reproduction device (Kaiser RS), which included 4 light units (4x150 Watts light bulb) and located in a darkroom, was used for capturing images of plantlets sawn in agar plates.

The image capturing process was repeated every 2-5 days starting at day 1 till day 10-15 (see for example the images in Figures 2A-B)

An image analysis system was used, which consists of a personal desktop computer (Intel P4 3.0 GHz processor) and a public domain program - ImageJ 1.39 (Java based image processing program which was developed at the U.S. National Institutes of Health and freely available on the internet at Hypertext Transfer Protocol://rsbweb (dot) nih (dot) gov/). Images were captured in resolution of 10 Mega Pixels (3888 x 2592 pixels) and stored in a low compression JPEG (Joint Photographic Experts Group standard) format. Next, analyzed data was saved to text files and processed using the JMP statistical analysis software (SAS institute).

*Seedling analysis -* Using the digital analysis seedling data was calculated, including leaf area, root coverage and root length.

The relative growth rate for the various seedling parameters was calculated according to the following formulas II, III and IV.

### Formula II:

$$\text{Relative growth rate of leaf area} = (\Delta \text{ rosette area} / \Delta t) * (1/ \text{ rosette area } t_1)$$

$\Delta$ rosette area is the interval between the current rosette area (measured at $t_2$) and the rosette area measured at the previous day (Area $t_1$)
$\Delta t$ is the time interval ($t_2$-$t_1$, in days) between the current analyzed image day ($t_2$) and the previous day ($t_1$).

Thus, the relative growth rate of leaf area is in units of 1/day.

### Formula III:

$$\text{Relative growth rate of root coverage} = (\Delta \text{ root coverage area} / \Delta t) * (1/ \text{ root coverage area } t_1)$$

$\Delta$ root coverage area is the interval between the current root coverage area (measured at $t_2$) and the root coverage area measured at the previous day (Area $t_1$)

$\Delta t$ is the time interval ($t_2$-$t_1$, in days) between the current analyzed image day ($t_2$) and the previous day ($t_1$).

Thus, the relative growth rate of root coverage area is in units of 1/day.

## Formula IV:

$$\text{Relative growth rate of root length} = (\Delta \text{ root length} / \Delta t) * (1/ \text{ root length } t_1)$$

$\Delta$ root length is the interval between the current root length (measured at $t_2$) and the root length measured at the previous day (Area $t_1$)

$\Delta t$ is the time interval ($t_2$-$t_1$, in days) between the current analyzed image day ($t_2$) and the previous day ($t_1$).

Thus, the relative growth rate of root length is in units of 1/day.

At the end of the experiment, plantlets were removed from the media and weighed for the determination of plant fresh weight. Plantlets were then dried for 24 hours at 60 °C, and weighed again to measure plant dry weight for later statistical analysis. Growth rate was determined by comparing the leaf area coverage, root coverage and root length, between each couple of sequential photographs, and results were used to resolve the effect of the gene introduced on plant vigor, under osmotic stress, as well as under optimal conditions. Similarly, the effect of the gene introduced on biomass accumulation, under osmotic stress as well as under optimal conditions, was determined by comparing the plants' fresh and dry weight to that of control plants (containing an empty vector or the GUS reporter gene under the same promoter). From every construct created, 3-5 independent transformation events were examined in replicates.

*Statistical analyses -* To identify genes conferring significantly improved tolerance to abiotic stresses or enlarged root architecture, the results obtained from the transgenic plants were compared to those obtained from control plants. To identify outperforming genes and constructs, results from the independent transformation events tested were analyzed separately. To evaluate the effect of a gene event over a control the data was analyzed by Student's t-test and the p value was calculated. Results wer considered significant if $p \leq 0.1$. The JMP statistics software package was used (Version 5.2.1, SAS Institute Inc., Cary, NC, USA).

*Experimental Results -* The polynucleotide sequences of the disclosure were assayed for a number of desired traits.

Tables 10-14 depict analyses of the above mentioned growth parameters of seedlings overexpressing the polynucleotides of the disclosure under the regulation of the At6669 promoter (SEQ ID NO:2823) when grown under osmotic stress (25 % PEG) conditions.

*Table 10*

| MAB70-25 % PEG | | | | | | | |
|---|---|---|---|---|---|---|---|
| Event No. | Control | 7971.3 | | 7972.1 | | 7974.1 | |
| | | A | P | A | P | A | P |
| RGR of Roots Coverage between day 5 and 10 | 0.16 | 0.29 | 0.00 | 0.31 | 0.00 | 0.30 | 0.02 |
| RGR of Roots Length between day 1 and 5 | 0.07 | 0.12 | 0.05 | 0.13 | 0.03 | | |

Table 10: Provided are the growth and biomass parameters of transgenic or control plants as measured in Tissue Calture growth under 25 % PEG. A = average; P = p value; RGR = Relative Growth Rate. The indicated days refer to days from planting.

*Table 11*

| MAB76 - 25 % PEG | | | | | |
|---|---|---|---|---|---|
| Event No. | Control | 7635.4 | | 7635.1 | |
| | | A | | A | P |

(continued)

| MAB76 - 25 % PEG | | | | | |
|---|---|---|---|---|---|
| Event No. | Control | 7635.4 | | 7635.1 | |
| RGR of Roots Coverage between day 5 and 10 | 0.16 | 0.22 | 0.02 | 0.21 | 0.09 |
| **Table 11:** Provided are the growth and biomass parameters of transgenic or control plants as measured in Tissue Calture growth under 25 % PEG. A = average; P = p value; RGR = Relative Growth Rate. The indicated days refer to days from planting. | | | | | |

**Table 12**

| MAB79 - 25 % PEG | | | | | |
|---|---|---|---|---|---|
| Event No. | Control | 7324.1 | | 7961.1 | |
| | | A | P | A | P |
| Dry Weight [gr] | 0.01 | 0.01 | 0.06 | | |
| Fresh Wight [gr] | 0.08 | | | 0.13 | 0.07 |
| Leaf Area on day 5 | 0.15 | | | 0.19 | 0.05 |
| RGR of Roots Coverage between day 5 and 10 | 0.16 | | | 0.32 | 0.05 |
| RGR of Roots Length between day 1 and 5 | 0.07 | | | 0.11 | 0.02 |
| **Table 12:** Provided are the growth and biomass parameters of transgenic or control plants as measured in Tissue Calture growth under 25 % PEG. A = average; P = p value; RGR = Relative Growth Rate. The indicated days refer to days from planting. | | | | | |

**Table 13**

| MAB56 - 25 % PEG | | | |
|---|---|---|---|
| Event No. | Control | 6802.10 | |
| | | A | P |
| Roots Coverage on day 7 | 2.46 | 3.38 | 0.09 |
| Roots Length on day 7 | 2.81 | 3.43 | 0.07 |
| **Table 13:** Provided are the growth and biomass parameters of transgenic or control plants as measured in Tissue Calture growth under 25 % PEG. A = average; P = p value; RGR = Relative Growth Rate. The indicated days refer to days from planting. | | | |

**Table 14**

| MAB58 - 25 % PEG | | | |
|---|---|---|---|
| Event No. | Control | 6783.30 | |
| | | A | P |
| Leaf Area on day 7 | 0.31 | 0.41 | 0.03 |
| Leaf Area on day 14 | 0.80 | 1.09 | 0.02 |
| Fresh Weight | 0.18 | 0.31 | 0.00 |

(continued)

| MAB58 - 25 % PEG | | | |
|---|---|---|---|
| **Event No.** | **Control** | **6783.30** | |
| Dry Weight [gr] | 0.01 | 0.013 | 0.01 |

**Table 14:** Provided are the growth and biomass parameters of transgenic or control plants as measured in Tissue Calture growth under 25 % PEG. A = average; P = p value; RGR = Relative Growth Rate. The indicated days refer to days from planting.

Tables 15-29 depict analyses of the above mentioned growth parameters of seedlings overexpressing the polynucleotides of the disclosure under the regulation of the At6669 promoter in *Normal Growth* conditions (0.5 MS medium).

*Table 15*

| MAB70 - Normal Growth Conditions | | | | | | | | | |
|---|---|---|---|---|---|---|---|---|---|
| **Event No.** | **Control** | **7971.3** | | **7972.1** | | **7974.1** | | **7974.3** | |
| | | A | P | A | P | A | P | A | P |
| Dry Weight [gr] | 0.01 | 0.01 | 0.03 | 0.01 | 0.05 | 0.02 | 0.04 | 0.01 | 0.07 |
| Fresh Wight [gr] | 0.14 | | | | | 0.24 | 0.04 | 0.22 | 0.10 |
| Leaf Area on day 10 | 0.46 | 0.59 | 0.00 | | | | | | |
| Leaf Area on day 5 | 0.21 | | | | | 0.30 | 0.10 | | |
| RGR of Roots Coverage between day 5 and 10 | 0.18 | 0.42 | 0.00 | 0.37 | 0.00 | 0.32 | 0.01 | | |
| RGR of Roots Length between day 1 and 5 | 0.06 | 0.15 | 0.01 | 0.16 | 0.00 | 0.15 | 0.00 | | |
| RGR of Roots Length between day 5 and 10 | 0.22 | | | 0.32 | 0.09 | | | | |

**Table 15:** Provided are the growth and biomass parameters of transgenic or control plants as measured in Tissue Calture growth under normal growth conditions. A = average; P = p value; RGR = Relative Growth Rate. The indicated days refer to days from planting.

*Table 16*

| MAB71 - Normal Growth Conditions | | | | | | | |
|---|---|---|---|---|---|---|---|
| **Event No.** | **Control** | **7331.5** | | **7332.2** | | **7333.5** | |
| | | A | P | A | P | A | P |
| Dry Weight [gr] | 0.01 | 0.01 | 0.05 | | | | |
| Fresh Wight [gr] | 0.14 | 0.20 | 0.08 | | | | |
| RGR of Roots Coverage between day 5 and 10 | 0.18 | | | 0.28 | 0.04 | 0.26 | 0.07 |
| RGR of Roots Length between day 1 and 5 | 0.06 | | | 0.11 | 0.02 | 0.11 | 0.01 |

**Table 16:** Provided are the growth and biomass parameters of transgenic or control plants as measured in Tissue Calture growth under normal growth conditions. A = average; P = p value; RGR = Relative Growth Rate. The indicated days refer to days from planting.

*Table 17*

| MAB74 - Normal Growth Conditions | | | | | | | |
|---|---|---|---|---|---|---|---|
| *Event No.* | *Control* | *7981.1* | | *7982.4* | | *7983.9* | |
| | | A | P | A | P | A | P |
| Dry Weight [gr] | 0.01 | 0.01 | 0.09 | 0.02 | 0.05 | 0.01 | 0.06 |
| Fresh Wight [gr] | 0.14 | | | | | 0.21 | 0.01 |
| Leaf Area on day 10 | 0.46 | 0.67 | 0.01 | | | | |
| Leaf Area on day 5 | 0.21 | 0.29 | 0.04 | 0.29 | 0.01 | 0.27 | 0.00 |
| RGR of Roots Coverage between day 5 and 10 | 0.18 | | | 0.31 | 0.07 | | |
| RGR of Roots Coverage between day 1 and 5 | 0.73 | | | 1.70 | 0.07 | | |
| RGR of Roots Length between day 1 and 5 | 0.06 | | | 0.12 | 0.09 | 0.14 | 0.03 |
| RGR of Roots Length between day 5 and 10 | 0.22 | 0.45 | 0.05 | 0.58 | 0.00 | | |

**Table 17:** Provided are the growth and biomass parameters of transgenic or control plants as measured in Tissue Calture growth under normal growth conditions. A = average; P = p value; RGR = Relative Growth Rate. The indicated days refer to days from planting.

*Table 18*

| MAB76 - Normal Growth Conditions | | | | | | | |
|---|---|---|---|---|---|---|---|
| *Event No.* | *Control* | *7633.3* | | *7635.4* | | *7635.1* | |
| | | A | P | A | P | A | P |
| RGR Leaf Area between dav 5 and 10 | 0.24 | 0.34 | 0.04 | 0.33 | 0.07 | | |
| RGR of Roots Coverage between day 5 and 10 | 0.18 | | | | | 0.38 | 0.08 |
| RGR of Roots Length between day 1 and 5 | 0.06 | | | | | 0.13 | 0.02 |

**Table 18:** Provided are the growth and biomass parameters of transgenic or control plants as measured in Tissue Calture growth under normal growth conditions. A = average; P = p value; RGR = Relative Growth Rate. The indicated days refer to days from planting.

*Table 19*

| MAB77 -Normal Growth Conditions | | | | | | | |
|---|---|---|---|---|---|---|---|
| *Event No.* | *Control* | *7931.11* | | *8211.2* | | *8212.2* | |
| | | A | P | A | P | A | P |
| Dry Weight [gr] | 0.01 | 0.01 | 0.08 | | | 0.01 | 0.10 |
| Roots Coverage on day 10 | 2.77 | | | | | 4.33 | 0.07 |
| RGR Leaf Area between day 5 and 10 | 0.24 | 0.38 | 0.10 | 0.35 | 0.04 | | |
| RGR of Roots Coverage between day 5 and 10 | 0.18 | 0.27 | 0.09 | 0.29 | 0.09 | | |
| RGR of Roots Length between day 1 and 5 | 0.06 | | | 0.10 | 0.03 | | |

**Table 19:** Provided are the growth and biomass parameters of transgenic or control plants as measured in Tissue Calture growth under normal growth conditions. A = average; P = p value; RGR = Relative Growth Rate. The indicated days refer to days from planting.

*Table 20*

| MAB79 - Normal Growth Conditions | | | | | | | | | |
|---|---|---|---|---|---|---|---|---|---|
| *Event No.* | *Control* | 7323.3 | | 7324.1 | | 7961.1 | | 7962.2 | |
| | | A | P | A | P | A | P | A | P |
| Dry Weight [gr] | 0.01 | 0.02 | 0.03 | 0.02 | 0.08 | 0.01 | 0.00 | | |
| Fresh Wight [gr] | 0.14 | 0.34 | 0.04 | 0.31 | 0.09 | | | | |
| Leaf Area on day 10 | 0.46 | 0.92 | 0.01 | 0.90 | 0.01 | 0.81 | 0.00 | | |
| Leaf Area on day 5 | 0.21 | | | | | 0.29 | 0.02 | 0.28 | 0.00 |
| Roots Coverage on day 10 | 2.77 | 5.31 | 0.02 | 4.59 | 0.07 | 3.81 | 0.00 | 3.71 | 0.01 |
| RGR Leaf Area between day 5 and 10 | 0.24 | | | | | 0.36 | 0.02 | | |
| RGR Leaf Area between day 1 and 5 | 0.82 | | | | | 1.34 | 0.00 | | |
| RGR of Roots Coverage between day 5 and 10 | 0.18 | | | | | 0.45 | 0.06 | 0.39 | 0.02 |
| RGR of Roots Length between day 1 and 5 | 0.06 | 0.12 | 0.02 | 0.17 | 0.06 | | | 0.14 | 0.00 |

**Table 20:** Provided are the growth and biomass parameters of transgenic or control plants as measured in Tissue Calture growth under normal growth conditions. A = average; P = p value; RGR = Relative Growth Rate. The indicated days refer to days from planting.

*Table 21*

| MAB115 - Normal Growth Conditions | | | | | | | | | |
|---|---|---|---|---|---|---|---|---|---|
| *Event No.* | *Control* | 8561.2.. | | 8564.1.. | | 8564.2.. | | 8565.1.. | |
| | | A | P | A | P | A | P | A | P |
| Dry Weight [gr] | 0.005 | 0.006 | 0.00 | | | | | 0.009 | 0.00 |
| Leaf Area on day 10 | 0.24 | | | | | | | 0.27 | 0.00 |
| Leaf Area on day 5 | 0.35 | | | | | | | 0.38 | 0.01 |
| Roots Coverage on day 10 | 1.67 | | | | | | | 2.13 | 0.02 |
| Roots Coverage on day 5 | 3.38 | | | | | | | 4.80 | 0.03 |
| Roots Length on day 10 | 2.39 | | | | | | | 2.74 | 0.00 |
| Roots Length on day 5 | 3.46 | | | | | | | 4.22 | 0.02 |
| RGR Leaf Area between day 5 and 10 | 0.37 | 0.43 | 0.00 | | | | | 0.48 | 0.00 |
| RGR Leaf Area between day 1 and 5 | 0.16 | 0.19 | 0.00 | | | | | | |
| RGR of Roots Coverage between day 5 and 10 | 1.89 | 3.21 | 0.00 | 2.24 | 0.02 | 2.23 | 0.02 | 3.47 | 0.01 |
| RGR of Roots Coverage between day 1 and 5 | 0.33 | 0.35 | 0.00 | 0.41 | 0.00 | 0.43 | 0.00 | 0.44 | 0.00 |
| RGR of Roots Length between day 1 and 5 | 0.37 | 0.56 | 0.02 | | | 0.53 | 0.06 | 0.68 | 0.00 |
| RGR of Roots Length between day 5 and 10 | 0.15 | | | 0.18 | 0.00 | 0.17 | 0.00 | 0.18 | 0.00 |

**Table 21:** Provided are the growth and biomass parameters of transgenic or control plants as measured in Tissue Calture growth under normal growth conditions. A = average; P = p value; RGR = Relative Growth Rate. The indicated days refer to days from planting.

EP 2 240 510 B1

*Table 22*

| MAB54 - Normal Growth Conditions | | | | | | | | | |
|---|---|---|---|---|---|---|---|---|---|
| Event No. | Control | 8181.2.. | | 8182.2.. | | 8184.3.. | | 8185.3.. | |
| | | A | P | A | P | A | P | A | P |
| Dry Weight [gr] | 0.005 | 0.009 | 0.00 | 0.008 | 0.00 | 0.008 | 0.00 | 0.007 | 0.00 |
| Roots Coverage on day 10 | 1.67 | 1.80 | 0.04 | | | | | | |
| Roots Coverage on day 5 | 3.38 | 4.27 | 0.02 | 3.40 | 0.00 | | | | |
| Roots Length on day 10 | 2.39 | 2.52 | 0.01 | | | | | | |
| Roots Length on day 5 | 3.46 | 4.11 | 0.02 | 3.50 | 0.00 | | | | |
| RGR Leaf Area between day 5 and 10 | 0.37 | 0.45 | 0.00 | | | | | | |
| RGR Leaf Area between day 1 and 5 | 0.16 | 0.17 | 0.04 | | | | | 0.19 | 0.00 |
| RGR of Roots Coverage between day 5 and 10 | 1.89 | 3.59 | 0.00 | 3.60 | 0.01 | 2.28 | 0.01 | 2.20 | 0.01 |
| RGR of Roots Coverage between day 1 and 5 | 0.33 | 0.52 | 0.00 | 0.55 | 0.00 | 0.39 | 0.00 | 0.36 | 0.00 |
| RGR of Roots Length between day 1 and 5 | 0.37 | 0.70 | 0.00 | 0.73 | 0.00 | 0.48 | 0.08 | 0.51 | 0.02 |
| RGR of Roots Length between day 5 and 10 | 0.15 | 0.21 | 0.01 | 0.22 | 0.01 | 0.17 | 0.00 | 0.17 | 0.00 |
| **Table 22:** Provided are the growth and biomass parameters of transgenic or control plants as measured in Tissue Culture growth under normal growth conditions. A = average; P = p value; RGR = Relative Growth Rate. The indicated days refer to days from planting. | | | | | | | | | |

*Table 23*

| MAB55 - Normal Growth Conditions | | | | | | | | | |
|---|---|---|---|---|---|---|---|---|---|
| Event No. | | 6802.1 | | 6802.11 | | 6802.8 | | 6805.3 | |
| | | A | P | A | P | A | P | A | P |
| Roots Coverage on day 7 | 3.67 | 5.93 | 0.04 | | | | | | |
| Roots Coverage on day 14 | 7.40 | 13.26 | 0.04 | | | | | | |
| Roots Length on day 7 | 3.99 | 5.32 | 0.01 | | | | | | |
| Roots Length on day 14 | 6.14 | 7.80 | 0.04 | | | | | 7.65 | 0.04 |
| RGR of Roots Coverage between day 1 and 7 | 0.53 | 1.30 | 0.00 | 1.11 | 0.02 | 0.93 | 0.02 | | |
| RGR of Roots Length between day 1 and 7 | 0.29 | 0.48 | 0.00 | 0.45 | 0.03 | 0.43 | 0.02 | | |
| Fresh Weight | 0.19 | | | | | 0.10 | 0.00 | 0.12 | 0.01 |
| Dry Weight [gr] | 0.01 | | | | | 0.01 | 0.00 | 0.01 | 0.00 |
| **Table 23:** Provided are the growth and biomass parameters of transgenic or control plants as measured in Tissue Culture growth under normal growth conditions. A = average; P = p value; RGR = Relative Growth Rate. The indicated days refer to days from planting. | | | | | | | | | |

*Table 24*

| MAB56 - Normal Growth Conditions | | | | | | | | | |
|---|---|---|---|---|---|---|---|---|---|
| Event No. | | 6691.2 | | 6691.3 | | 6693.2 | | 6693.5 | |
| | | A | P | A | P | A | P | A | P |
| Roots Coverage on day 7 | 3.67 | 5.81 | 0.00 | | | | | 5.67 | 0.01 |

(continued)

| MAB56 - Normal Growth Conditions | | | | | | | | | |
|---|---|---|---|---|---|---|---|---|---|
| Event No. | | | 6691.2 | | 6691.3 | | 6693.2 | | 6693.5 | |
| Roots Length on day 7 | 3.99 | 6.21 | 0.00 | | | | | 5.39 | 0.00 |
| Roots Length on day 14 | 6.14 | 8.32 | 0.01 | | | | | 8.01 | 0.02 |
| RGR of Roots Length between day 7 and 14 | 0.09 | 0.05 | 0.01 | 0.05 | 0.06 | 0.06 | 0.08 | | |
| Fresh Weight | 0.19 | 0.14 | 0.03 | 0.14 | 0.03 | | | | |
| Dry Weight [gr] | 0.01 | 0.01 | 0.02 | 0.01 | 0.02 | 0.00 | 0.00 | | |

**Table 24:** Provided are the growth and biomass parameters of transgenic or control plants as measured in Tissue Calture growth under normal growth conditions. A = average; P = p value; RGR = Relative Growth Rate. The indicated days refer to days from planting.

*Table 25*

| MAB57 - Normal Growth Conditions | | | | | | | | | |
|---|---|---|---|---|---|---|---|---|---|
| Event No. | | | 6912.14 | | 6912.2 | | 6912.6 | | 6914.1 | |
| | | A | P | A | P | A | P | A | P |
| Roots Coverage on day 7 | 3.67 | 6.40 | 0.00 | 6.71 | 0.00 | | | | |
| Roots Coverage on day 14 | 7.40 | 17.33 | 0.00 | 15.27 | 0.05 | 12.30 | 0.02 | | |
| Roots Length on day 7 | 3.99 | 5.54 | 0.00 | 6.12 | 0.00 | 6.34 | 0.00 | | |
| Roots Length on day 14 | 6.14 | 8.76 | 0.00 | 8.48 | 0.01 | 7.97 | 0.02 | 7.85 | 0.04 |
| RGR of Roots Length between day 1 and 7 | 0.29 | 0.39 | 0.06 | | | | | | |
| RGR of Roots Length between day 7 and 14 | 0.09 | | | | | 0.04 | 0.09 | | |
| Fresh Weight | 0.19 | 0.24 | 0.09 | | | 0.11 | 0.01 | 0.11 | 0.01 |
| Dry Weight [gr] | 0.01 | | | | | 0.01 | 0.01 | 0.01 | 0.01 |

**Table 25:** Provided are the growth and biomass parameters of transgenic or control plants as measured in Tissue Calture growth under normal growth conditions. A = average; P = p value; RGR = Relative Growth Rate. The indicated days refer to days from planting.

*Table 26*

| MAB58 - Normal Growth Conditions | | | | | | | | |
|---|---|---|---|---|---|---|---|---|
| Event No. | | | 6783.1 | | 6783.2 | | 6783.3 | |
| | | A | P | A | P | A | P |
| Roots Coverage on day 7 | 3.67 | | | | | 6.18 | 0.00 |
| Roots Coverage on day 14 | 7.40 | | | 12.65 | 0.02 | 12.00 | 0.09 |
| Roots Length on day 7 | 3.99 | | | | | 5.20 | 0.02 |
| Roots Length on day 14 | 6.14 | 7.38 | 0.09 | 7.51 | 0.07 | 7.59 | 0.08 |
| RGR of Roots Length between day 1 and 7 | 0.29 | 0.35 | 0.07 | | | | |
| Fresh Weight | 0.19 | 0.13 | 0.03 | | | | |

(continued)

| MAB58 - Normal Growth Conditions | | | | | | | |
|---|---|---|---|---|---|---|---|
| Event No. | | 6783.1 | | 6783.2 | | 6783.3 | |
| Dry Weight [gr] | 0.01 | 0.01 | 0.00 | | | | |

Table 26: Provided are the growth and biomass parameters of transgenic or control plants as measured in Tissue Calture growth under normal growth conditions. A = average; P = p value; RGR = Relative Growth Rate. The indicated days refer to days from planting.

Table 27

| MAB59 - Normal Growth Condition | | | | | | | |
|---|---|---|---|---|---|---|---|
| Event No. | | 6791.4 | | 6793.4 | | 6794.4 | |
| | | A | P | A | P | A | P |
| Roots Coverage on day 7 | 3.67 | | | 5.61 | 0.04 | 5.23 | 0.09 |
| Roots Coverage on day 14 | 7.40 | | | 9.65 | 0.09 | 10.28 | 0.09 |
| Roots Length on day 7 | 3.99 | | | 5.47 | 0.08 | 4.95 | 0.09 |
| Roots Length on day 14 | 6.14 | | | | | 7.70 | 0.07 |
| RGR of Roots Coverage between day 1 and 7 | 0.53 | | | | | 0.80 | 0.09 |
| RGR of Roots Length between day 7 and 14 | 0.09 | | | 0.05 | 0.10 | | |
| Fresh Weight | 0.19 | 0.09 | 0.00 | | | | |
| Dry Weight [gr] | 0.01 | 0.004 | 0.00 | 0.005 | 0.02 | | |

Table 27: Provided are the growth and biomass parameters of transgenic or control plants as measured in Tissue Calture growth under normal growth conditions. A = average; P = p value; RGR = Relative Growth Rate. The indicated days refer to days from planting.

Table 28

| MAB69 - Normal Growth Conditions | | | | | | | | | |
|---|---|---|---|---|---|---|---|---|---|
| Event No. | | 6651.1 | | 6651.12 | | 6651.13 | | 6651.8 | |
| | | A | P | A | P | A | P | A | P |
| Roots Length on day 7 | 3.99 | 4.97 | 0.10 | | | | | | |
| Roots Length on day 14 | 6.14 | 8.01 | 0.02 | | | | | | |
| RGR of Roots Length between day 1 and 7 | 0.29 | | | | | | | 0.35 | 0.09 |
| Fresh Weight | 0.19 | | | | | 0.12 | 0.02 | 0.12 | 0.01 |
| Dry Weight [gr] | 0.01 | | | 0.007 | 0.09 | 0.005 | 0.00 | 0.006 | 0.00 |

Table 28: Provided are the growth and biomass parameters of transgenic or control plants as measured in Tissue Calture growth under normal growth conditions. A = average; P = p value; RGR = Relative Growth Rate. The indicated days refer to days from planting.

*Table 29*

| MAB72 - Normal Growth Conditions | | | | | | | |
|---|---|---|---|---|---|---|---|
| Event No. | Control | 8552.1.. | | 8552.4.. | | 8553.2.. | |
| | | A | P | A | P | A | P |
| Dry Weight [gr] | 0.005 | 0.008 | 0.00 | 0.008 | 0.00 | 0.007 | 0.00 |
| Leaf Area on day 10 | 0.24 | 0.24 | 0.01 | | | | |
| Roots Coverage on day 10 | 1.67 | 1.84 | 0.01 | 1.93 | 0.02 | 1.89 | 0.03 |
| Roots Coverage on day 5 | 3.38 | 3.60 | 0.04 | 3.90 | 0.06 | 4.50 | 0.00 |
| Roots Length on day 10 | 2.39 | 2.48 | 0.01 | | | | |
| Roots Length on day 5 | 3.46 | 3.70 | 0.04 | 3.65 | 0.04 | 3.84 | 0.00 |
| RGR Leaf Area between day 5 and 10 | 0.37 | 0.47 | 0.00 | 0.39 | 0.00 | | |
| RGR Leaf Area between day 1 and 5 | 0.16 | | | 0.20 | 0.09 | | |
| RGR of Roots Coverage between day 5 and 10 | 1.89 | 1.93 | 0.03 | 2.29 | 0.06 | 3.04 | 0.01 |
| RGR of Roots Coverage between day 1 and 5 | 0.33 | | | 0.35 | 0.00 | 0.52 | 0.00 |
| RGR of Roots Length between day 1 and 5 | 0.37 | | | | | 0.55 | 0.01 |
| RGR of Roots Length between day 5 and 10 | 0.15 | 0.16 | 0.00 | 0.18 | 0.00 | 0.22 | 0.01 |

**Table 29:** Provided are the growth and biomass parameters of transgenic or control plants as measured in Tissue Calture growth under normal growth conditions. A = average; P = p value; RGR = Relative Growth Rate. The indicated days refer to days from planting.

## EXAMPLE 6

### EVALUATING TRANSGENIC ARABIDOPSIS PLANT GROWTH UNDER ABIOTIC STRESS AS WELL AS FAVORABLE CONDITIONS IN GREENHOUSE ASSAY

*ABS tolerance: Yield and plant growth rate at high salinity concentration under greenhouse conditions -* This assay followed the rosette area growth of plants grown in the greenhouse as well as seed yield at high salinity irrigation. Seeds were sown in agar media supplemented only with a selection agent (Kanamycin) and Hoagland solution under nursery conditions. The $T_2$ transgenic seedlings were then transplanted to 1.7 trays filled with peat and perlite. The trays were irrigated with tap water (provided from the pots' bottom). Half of the plants were irrigated with a salt solution (40-80 mM NaCl and 5 mM $CaCl_2$) so as to induce salinity stress (stress conditions). The other half of the plants was irrigated with tap water (normal conditions). All plants were grown in the greenhouse until mature seeds, then harvested (the above ground tissue) and weighted (immediately or following drying in oven at 50 °C for 24 hours). High salinity conditions were achieved by irrigating with a solution containing 40-80 mM NaCl ("ABS" growth conditions) and compared to regular growth conditions.

Each construct was validated at its T2 generation. Transgenic plants transformed with a construct including the uidA reporter gene (GUS) under the AT6669 promoter or with an empty vector including the AT6669 promoter were used as control.

The plants were analyzed for their overall size, growth rate, flowering, seed yield, weight of 1,000 seeds, dry matter and harvest index (HI- seed yield/dry matter). Transgenic plants performance was compared to control plants grown in parallel under the same conditions. Mock- transgenic plants expressing the uidA reporter gene (GUS-Intron) or with no gene at all, under the same promoter were used as control.

The experiment was planned in nested randomized plot distribution. For each gene of the disclosure three to five independent transformation events were analyzed from each construct.

*Digital imaging -* A laboratory image acquisition system, which consists of a digital reflex camera (Canon EOS 300D) attached with a 55 mm focal length lens (Canon EF-S series), mounted on a reproduction device (Kaiser RS), which included 4 light units (4 x 150 Watts light bulb) was used for capturing images of plant samples.

The image capturing process was repeated every 2 days starting from day 1 after transplanting till day 16. Same camera, placed in a custom made iron mount, was used for capturing images of larger plants sawn in white tubs in an environmental controlled greenhouse. The tubs were square shape include 1.7 liter trays. During the capture process, the tubs were placed beneath the iron mount, while avoiding direct sun light and casting of shadows.

An image analysis system was used, which consists of a personal desktop computer (Intel P4 3.0 GHz processor) and a public domain program - ImageJ 1.39 (Java based image processing program which was developed at the U.S National Institutes of Health and freely available on the internet at Hypertext Transfer Protocol://rsbweb (dot) nih (dot) gov/). Images were captured in resolution of 10 Mega Pixels (3888 x 2592 pixels) and stored in a low compression JPEG (Joint Photographic Experts Group standard) format. Next, analyzed data was saved to text files and processed using the JMP statistical analysis software (SAS institute).

*Leaf analysis -* Using the digital analysis leaves data was calculated, including leaf number, area, perimeter, length and width.

*Vegetative growth rate:* the relative growth rate (RGR) of leaf number and rosette area were calculated formulas V and VI, respectively.

## Formula V:

$$\text{Relative growth rate of leaf number} = (\Delta \text{ leaf number} / \Delta t) * (1/ \text{ leaf number } t_1)$$

$\Delta$ leaf number is the interval between the current leaf number (measured at $t_2$) and the leaf number measured at the previous day (Area $t_1$)
$\Delta t$ is the time interval ($t_2$-$t_1$, in days) between the current analyzed image day ($t_2$) and the previous day ($t_1$).

Thus, the relative growth rate of leaf number is in units of 1/day.

## Formula VI:

$$\text{Relative growth rate of rosette area} = (\Delta \text{ rosette area} / \Delta t) * (1/ \text{ rosette area } t_1)$$

$\Delta$ rosette area is the interval between the current rosette area (measured at $t_2$) and the rosette area measured at the previous day (Area $t_1$)
$\Delta t$ is the time interval ($t_2$-$t_1$, in days) between the current analyzed image day ($t_2$) and the previous day ($t_1$).

Thus, the relative growth rate of rosette area is in units of 1/day.

*Seeds average weight -* At the end of the experiment all seeds were collected. The seeds were scattered on a glass tray and a picture was taken. Using the digital analysis, the number of seeds in each sample was calculated.

*Dry weight and seed yield -* On about day 80 from sowing, the plants were harvested and left to dry at 30 °C in a drying chamber. The biomass and seed weight of each plot were measured and divided by the number of plants in each plot. Dry weight = total weight of the vegetative portion above ground (excluding roots) after drying at 30 °C in a drying chamber; Seed yield per plant = total seed weight per plant (gr). 1000 seed weight (the weight of 1000 seeds) (gr.).

*Harvest Index (HI) -* The harvest index was calculated using Formula VII.

## Formula VII:

$$\text{Harvest Index} = \text{Average seed yield per plant/ Average dry weight}$$

*Statistical analyses -* To identify genes conferring significantly improved tolerance to abiotic stresses, the results obtained from the transgenic plants were compared to those obtained from control plants. To identify outperforming genes and constructs, results from the independent transformation events tested were analyzed separately. Data was analyzed using Student's t-test and results were considered significant if the p value was less than 0.1. The JMP statistics software package is used (Version 5.2.1, SAS Institute Inc., Cary, NC, USA).

### Experimental Results

Tables 30-44 depict analyses of plant parameters as describe above overexpressing the polynucleotides of the disclosure under the regulation of the At6669 promoter under salinity irrigation conditions [NaCl 40-80 mM; NaCl Electrical conductivity (E.C.) of 7-10].

**Table 30**

| MAB115 - Salt irrigation (40-80 mM NaCl) | | | | | |
|---|---|---|---|---|---|
| Event No. | Control | 8564.1 | | 8565.1 | |
| | | A | P | A | P |
| Rosette Diameter on day 3* | 1.70 | 1.80 | 0.09 | 1.75 | 0.04 |
| Rosette Diameter on day 5 | 2.36 | | | | |
| Rosette Diameter on day 8 | 3.77 | | | 4.00 | 0.09 |
| Rosette Area on day 3 | 0.90 | | | | |
| Rosette Area on day 5 | 1.56 | | | 1.70 | 0.09 |
| Rosette Area on day 8 | 4.06 | | | 4.38 | 0.06 |
| Plot Coverage on day 5 | 12.30 | | | 13.61 | 0.06 |
| Plot Coverage on day 8 | 31.90 | | | 35.07 | 0.02 |
| Leaf Number on day 3 | 5.08 | | | 5.94 | 0.00 |
| Leaf Number on day 5 | 6.86 | | | 7.38 | 0.05 |
| RGR of Leaf Number between day 3 and 5 | 0.18 | | | | |
| RGR of Leaf Number between day 5 and 8 | 0.09 | | | | |
| RGR of Rosette Area between day 5 and 8 | 0.53 | 0.62 | 0.01 | | |
| Biomass DW [gr] | 3.24 | | | | |
| Harvest Index | 0.11 | 0.15 | 0.07 | 0.16 | 0.03 |

**Table 30:** Provided are the growth, biomass and yield parameters of transgenic or control plants as measured in Green House under salinity irrigation. A = average; P = p value; RGR = Relative Growth Rate. The indicated days refer to days from planting.

**Table 31**

| MAB54 - Salt irrigation (40-80 mM NaCl) | | | | | | | | | |
|---|---|---|---|---|---|---|---|---|---|
| Event No. | Control | 8181.2 | | 8182.2 | | 8183.2 | | 8185.4 | |
| | | A | P | A | P | A | P | A | P |
| Rosette Diameter on day 3 * | 1.70 | 1.95 | 0.04 | | | | | | |
| Rosette Diameter on day 5 | 2.36 | | | 2.70 | 0.02 | 2.64 | 0.00 | | |
| Rosette Diameter on day 8 | 3.77 | | | 4.00 | 0.08 | | | | |
| Rosette Area on day 3 | 0.90 | | | | | 1.19 | 0.04 | | |
| Plot Coverage on day 3 | 7.04 | | | | | 9.52 | 0.04 | 7.49 | 0.08 |
| Leaf Number on day 3 | 5.08 | | | 6.19 | 0.06 | | | | |
| Leaf Number on day 8 | 8.66 | 9.31 | 0.00 | 9.38 | 0.00 | | | | |
| RGR of Leaf Number between day 3 and 5 | 0.18 | | | | | | | | |
| RGR of Rosette Area between day 1 and 3 | 0.45 | | | 0.52 | 0.01 | | | | |
| 1000 Seeds weight [gr] | 0.02 | | | | | 0.02 | 0.00 | 0.02 | 0.00 |
| Yield [gr]/Plant | 0.04 | 0.06 | 0.05 | | | | | | |

(continued)

| MAB54 - Salt irrigation (40-80 mM NaCl) | | | | | | | | |
|---|---|---|---|---|---|---|---|---|
| Event No. | Control | 8181.2 | | 8182.2 | | 8183.2 | | 8185.4 |
| Harvest Index | 0.11 | 0.17 | 0.01 | | | | | |

Table 31: Provided are the growth, biomass and yield parameters of transgenic or control plants as measured in Green House under salinity irrigation. A = average; P = p value; RGR = Relative Growth Rate. The indicated days refer to days from planting.

### Table 32

| MAB55 - Salt irrigation (40-80 mM NaCl) | | | | | | | |
|---|---|---|---|---|---|---|---|
| Event No. | Control | 6802.10 | | 6805.3 | | 6805.4 | |
| | | A | P | A | P | A | P |
| Rosette Diameter on day 5 | 2.36 | 2.81 | 0.03 | | | | |
| Rosette Diameter on day 8 | 3.77 | 4.29 | 0.05 | | | | |
| Rosette Area on day 3 | 0.90 | 1.35 | 0.01 | | | | |
| Rosette Area on day 5 | 1.56 | | | 1.70 | 0.03 | | |
| Rosette Area on day 8 | 4.06 | 5.91 | 0.05 | | | | |
| Plot Coverage on day 3 | 7.04 | 10.76 | 0.01 | | | | |
| Plot Coverage on day 5 | 12.30 | | | 13.60 | 0.02 | | |
| Plot Coverage on day 8 | 31.90 | 47.28 | 0.06 | | | | |
| Leaf Number on day 3 | 5.08 | 6.25 | 0.00 | | | | |
| Leaf Number on day 5 | 6.86 | 7.94 | 0.03 | | | | |
| Leaf Number on day 8 | 8.66 | 9.50 | 0.01 | | | | |
| RGR of Rosette Area between day 1 and 3 | 0.45 | 0.51 | 0.05 | | | | |
| Yield [gr]/Plant | 0.04 | | | | | 0.06 | 0.03 |
| Harvest Index | 0.11 | | | 0.15 | 0.05 | | |

Table 32: Provided are the growth, biomass and yield parameters of transgenic or control plants as measured in Green House under salinity irrigation. A = average; P = p value; RGR = Relative Growth Rate. The indicated days refer to days from planting.

### Table 33

| MAB56 - Salt irrigation (40-80 mM NaCl) | | | | | | | | |
|---|---|---|---|---|---|---|---|---|
| Event No. | Control | 6691.2 | | 6691.3 | | 6693.2 | | 6695.6 |
| | | A | P | A | P | A | P | A | P |
| Rosette Diameter on day 3 | 1.70 | | | | | 1.89 | 0.05 | 1.97 | 0.07 |
| Rosette Diameter on day 5 | 2.36 | | | | | 2.55 | 0.09 | 2.58 | 0.01 |
| Rosette Area on day 3 | 0.90 | | | 1.06 | 0.00 | 1.15 | 0.02 | 1.23 | 0.08 |
| Rosette Area on day 5 | 1.56 | | | | | 1.94 | 0.02 | 1.94 | 0.03 |
| Rosette Area on day 8 | 4.06 | 4.63 | 0.02 | | | | | | |
| Plot Coverage on day 3 | 7.04 | | | 8.45 | 0.00 | 9.21 | 0.01 | 9.82 | 0.07 |

(continued)

| MAB56 - Salt irrigation (40-80 mM NaCl) | | | | | | | | |
|---|---|---|---|---|---|---|---|---|
| Event No. | Control | 6691.2 | | 6691.3 | | 6693.2 | | 6695.6 |
| Plot Coverage on day 5 | 12.30 | | | | | 15.49 | 0.01 | 15.53 | 0.02 |
| Plot Coverage on day 8 | 31.90 | 37.03 | 0.01 | | | | | 34.82 | 0.05 |
| Leaf Number on day 3 | 5.08 | | | 5.50 | 0.00 | 5.81 | 0.00 | 6.00 | 0.02 |
| Leaf Number on day 5 | 6.86 | | | | | 7.38 | 0.01 | 7.50 | 0.02 |
| 1000 Seeds weight [gr] | 0.02 | | | 0.02 | 0.08 | | | | |
| Harvest Index | 0.11 | | | 0.18 | 0.01 | | | | |

**Table 33:** Provided are the growth, biomass and yield parameters of transgenic or control plants as measured in Green House under salinity irrigation. A = average; P = p value; RGR = Relative Growth Rate. The indicated days refer to days from planting.

**Table 34**

| MAB57 - Salt irrigation (40-80 mM NaCl) | | | | | | | |
|---|---|---|---|---|---|---|---|
| Event No. | Control | 6912.1 | | 6912.13 | | 6914.5 | |
| | | A | P | A | P | A | P |
| Leaf Number on day 3 | 5.08 | | | | | 5.69 | 0.00 |
| Leaf Number on day 5 | 6.86 | | | 8.13 | 0.06 | | |
| Leaf Number on day 8 | 8.66 | | | | | 9.56 | 0.06 |
| RGR of Leaf Number between day 5 and 8 | 0.09 | 0.14 | 0.01 | | | | |
| RGR of Rosette Area between day 5 and 8 | 0.53 | 0.62 | 0.02 | | | 0.58 | 0.10 |
| 1000 Seeds weight [gr] | 0.02 | | | | | 0.02 | 0.00 |
| Yield [gr]/Plant | 0.04 | 0.06 | 0.01 | 0.06 | 0.03 | 0.06 | 0.01 |
| Harvest Index | 0.11 | | | 0.19 | 0.06 | 0.23 | 0.00 |

**Table 34:** Provided are the growth, biomass and yield parameters of transgenic or control plants as measured in Green House under salinity irrigation. A = average; P = p value; RGR = Relative Growth Rate. The indicated days refer to days from planting.

**Table 35**

| MAB58 - Salt irrigation (40-80 mM NaCl) | | | | | | | | | |
|---|---|---|---|---|---|---|---|---|---|
| Event No. | Control | 6783.3 | | 7522.10 | | 7522.3 | | 7523.6 | |
| | | A | P | A | P | A | P | A | P |
| Rosette Diameter on day 3 | 1.70 | | | | | | | 2.11 | 0.00 |
| Rosette Diameter on day 5 | 2.36 | 2.57 | 0.00 | | | | | | |
| Rosette Diameter on day 8 | 3.77 | | | | | 4.06 | 0.07 | 4.54 | 0.00 |
| Rosette Area on day 3 | 0.90 | | | | | | | 1.36 | 0.00 |
| Rosette Area on day 5 | 1.56 | | | | | | | 2.29 | 0.00 |
| Rosette Area on day 8 | 4.06 | | | | | | | 5.98 | 0.00 |
| Plot Coverage on day 3 | 7.04 | | | | | | | 10.90 | 0.00 |

(continued)

| MAB58 - Salt irrigation (40-80 mM NaCl) | | | | | | | | | |
|---|---|---|---|---|---|---|---|---|---|
| Event No. | Control | 6783.3 | | 7522.10 | | 7522.3 | | 7523.6 | |
| Plot Coverage on day 5 | 12.30 | | | | | | | 18.32 | 0.00 |
| Plot Coverage on day 8 | 31.90 | | | | | | | 47.88 | 0.00 |
| Leaf Number on dav 3 | 5.08 | | | 5.63 | 0.06 | | | 6.06 | 0.00 |
| Leaf Number on dav 8 | 8.66 | 9.25 | 0.04 | | | | | 9.81 | 0.04 |
| RGR of Leaf Number between day 5 and 8 | 0.09 | 0.12 | 0.03 | | | | | | |
| 1000 Seeds weight [gr] | 0.02 | 0.02 | 0.08 | | | | | | |
| Yield [gr]/Plant | 0.04 | 0.06 | 0.09 | | | | | | |

**Table 35:** Provided are the growth, biomass and yield parameters of transgenic or control plants as measured in Green House under salinity irrigation. A = average; P = p value; RGR = Relative Growth Rate. The indicated days refer to days from planting.

**Table 36**

| MAB59 - Salt irrigation (40-80 mM NaCl) | | | | | | | |
|---|---|---|---|---|---|---|---|
| Event No. | Control | 6791.6 | | 6794.4 | | 6794.5 | |
| | | A | P | A | P | A | P |
| Rosette Diameter on day 3 | 1.70 | | | | | 2.38 | 0.05 |
| Rosette Diameter on day 5 | 2.36 | | | 2.73 | 0.06 | | |
| Rosette Area on day 3 | 0.90 | | | 1.27 | 0.06 | 1.65 | 0.03 |
| Plot Coverage on day 3 | 7.04 | | | 10.15 | 0.06 | 13.19 | 0.03 |
| Leaf Number on dav 3 | 5.08 | 6.44 | 0.05 | 6.00 | 0.02 | 6.75 | 0.01 |
| Leaf Number on day 5 | 6.86 | 8.38 | 0.00 | 7.69 | 0.05 | 8.00 | 0.07 |
| Leaf Number on day 8 | 8.66 | | | | | 9.38 | 0.02 |
| Yield [gr]/Plant | 0.04 | 0.06 | 0.06 | | | | |
| Harvest Index | 0.11 | 0.16 | 0.04 | | | | |

**Table 36:** Provided are the growth, biomass and yield parameters of transgenic or control plants as measured in Green House under salinity irrigation. A = average; P = p value; RGR = Relative Growth Rate. The indicated days refer to days from planting.

**Table 37**

| MAB69 - Salt irrigation (40-80 mM NaCl) | | | | | | | | | |
|---|---|---|---|---|---|---|---|---|---|
| Event No. | Control | 6651.11 | | 6651.12 | | 6651.2 | | 8342.1 | |
| | | A | P | A | P | A | P | A | P |
| Rosette Diameter on day 3 | 1.70 | | | 1.92 | 0.01 | | | | |
| Rosette Area on day 3 | 0.90 | | | 1.09 | 0.00 | | | | |
| Rosette Area on day 8 | 4.06 | | | 5.05 | 0.04 | | | | |
| Plot Coverage on day 3 | 7.04 | | | 8.74 | 0.00 | | | | |
| Plot Coverage on day 8 | 31.90 | | | 40.41 | 0.04 | | | | |

(continued)

| MAB69 - Salt irrigation (40-80 mM NaCl) | | | | | | | | |
|---|---|---|---|---|---|---|---|---|
| Event No. | Control | 6651.11 | | 6651.12 | | 6651.2 | | 8342.1 |
| Leaf Number on day 3 | 5.08 | | | 5.69 | 0.00 | | | | |
| Leaf Number on day 8 | 8.66 | | | | | | | 8.94 | 0.08 |
| RGR of Rosette Area between day 1 and 3 | 0.45 | 0.49 | 0.02 | 0.51 | 0.04 | | | | |
| 1000 Seeds weight [gr] | 0.02 | 0.02 | 0.00 | | | 0.02 | 0.01 | | |
| Yield [gr]/Plant | 0.04 | 0.05 | 0.09 | 0.06 | 0.02 | | | 0.07 | 0.01 |
| Harvest Index | 0.11 | | | 0.17 | 0.09 | 0.22 | 0.01 | | |

**Table 37:** Provided are the growth, biomass and yield parameters of transgenic or control plants as measured in Green House under salinity irrigation. A = average; P = p value; RGR = Relative Growth Rate. The indicated days refer to days from planting.

*Table 38*

| MAB70 - Salt irrigation (40-80 mM NaCl) | | | | | | | |
|---|---|---|---|---|---|---|---|
| Event No. | Control | 7971.3 | | 7972.1 | | 7972.3 | |
| | | A | P | A | P | A | P |
| Rosette Diameter on day 3 | 1.70 | | | | | 1.94 | 0.00 |
| Rosette Diameter on day 5 | 2.36 | | | | | 2.62 | 0.04 |
| Rosette Diameter on day 8 | 3.77 | | | 4.40 | 0.00 | | |
| Rosette Area on day 3 | 0.90 | | | 1.20 | 0.07 | 1.12 | 0.01 |
| Rosette Area on day 5 | 1.56 | | | 2.06 | 0.05 | 1.87 | 0.00 |
| Rosette Area on day 8 | 4.06 | | | 5.86 | 0.06 | | |
| Plot Coverage on day 3 | 7.04 | | | 9.61 | 0.06 | 9.00 | 0.01 |
| Plot Coverage on day 5 | 12.30 | | | 16.46 | 0.04 | 14.93 | 0.00 |
| Plot Coverage on day 8 | 31.90 | | | 46.87 | 0.06 | | |
| Leaf Number on day 3 | 5.08 | 5.94 | 0.09 | | | | |
| Leaf Number on day 8 | 8.66 | 9.31 | 0.00 | | | 9.75 | 0.09 |
| RGR of Rosette Area between day 5 and 8 | 0.53 | | | 0.62 | 0.01 | | |
| Yield [gr]/Plant | 0.04 | 0.08 | 0.00 | 0.07 | 0.01 | | |
| Harvest Index | 0.11 | 0.25 | 0.00 | | | | |

**Table 38:** Provided are the growth, biomass and yield parameters of transgenic or control plants as measured in Green House under salinity irrigation. A = average; P = p value; RGR = Relative Growth Rate. The indicated days refer to days from planting.

*Table 39*

| MAB71 - Salt irrigation (40-80 mM NaCl) | | | | | | | | | |
|---|---|---|---|---|---|---|---|---|---|
| Event No. | Control | 7331.4 | | 7331.5 | | 7333.5 | | 7334.5 | |
| | | A | P | A | P | A | P | A | P |
| Rosette Diameter on day 5 | 2.36 | 3.03 | 0.00 | | | 2.52 | 0.02 | | |

(continued)

| MAB71 - Salt irrigation (40-80 mM NaCl) | | | | | | | | | |
|---|---|---|---|---|---|---|---|---|---|
| Event No. | Control | 7331.4 | | 7331.5 | | 7333.5 | | 7334.5 | |
| Rosette Diameter on day 8 | 3.77 | 5.01 | 0.05 | 4.33 | 0.01 | | | | |
| Plot Coverage on day 5 | 12.30 | 19.90 | 0.09 | | | 14.25 | 0.08 | | |
| Leaf Number on day 3 | 5.08 | 6.44 | 0.05 | | | 5.47 | 0.06 | | |
| Leaf Number on day 5 | 6.86 | 8.13 | 0.00 | 7.56 | 0.00 | | | | |
| Leaf Number on day 8 | 8.66 | 10.38 | 0.05 | | | | | | |
| RGR of Leaf Number between day 5 and 8 | 0.09 | | | | | | | 0.12 | 0.01 |
| RGR of Rosette Area between day 5 and 8 | 0.53 | 0.61 | 0.03 | | | | | 0.61 | 0.04 |
| Yield [gr]/Plant | 0.04 | | | | | | | 0.05 | 0.10 |
| Harvest Index | 0.11 | 0.21 | 0.06 | | | | | | |

Table 39: Provided are the growth, biomass and yield parameters of transgenic or control plants as measured in Green House under salinity irrigation. A = average; P = p value; RGR = Relative Growth Rate. The indicated days refer to days from planting.

## Table 40

| MAB72 - Salt irrigation (40-80 mM NaCl) | | | | | | | | | |
|---|---|---|---|---|---|---|---|---|---|
| Event No. | Control | 8552.1 | | 8553.2 | | 8553.3 | | 8555.3 | |
| | | A | P | A | P | A | P | A | P |
| Rosette Diameter on day 3 | 1.70 | | | 1.90 | 0.05 | | | 1.83 | 0.00 |
| Rosette Diameter on day 5 | 2.36 | | | 2.67 | 0.00 | | | 2.55 | 0.01 |
| Rosette Diameter on day 8 | 3.77 | | | 4.06 | 0.08 | 4.15 | 0.10 | | |
| Rosette Area on day 3 | 0.90 | | | 1.20 | 0.00 | 1.02 | 0.00 | 1.08 | 0.02 |
| Rosette Area on dav 5 | 1.56 | 1.88 | 0.07 | 2.00 | 0.00 | | | 1.87 | 0.00 |
| Rosette Area on dav 8 | 4.06 | 4.68 | 0.00 | 5.05 | 0.00 | | | 4.82 | 0.00 |
| Plot Coverage on day 3 | 7.04 | | | 9.64 | 0.00 | 8.13 | 0.00 | 8.67 | 0.02 |
| Plot Coverage on day 5 | 12.30 | 15.05 | 0.05 | 16.04 | 0.00 | | | 14.98 | 0.00 |
| Plot Coverage on day 8 | 31.90 | 37.48 | 0.00 | 40.39 | 0.00 | | | 38.57 | 0.00 |
| Leaf Number on dav 3 | 5.08 | 5.81 | 0.00 | 5.88 | 0.00 | 5.56 | 0.00 | 5.50 | 0.00 |
| Leaf Number on day 8 | 8.66 | | | | | 9.38 | 0.02 | | |
| RGR of Leaf Number between day 1 and 3 | 0.12 | 0.17 | 0.01 | | | | | | |

Table 40: Provided are the growth, biomass and yield parameters of transgenic or control plants as measured in Green House under salinity irrigation. A = average; P = p value; RGR = Relative Growth Rate. The indicated days refer to days from planting.

## Table 41

| MAB74 - Salt irrigation (40-80 mM NaCl) | | | | | | | |
|---|---|---|---|---|---|---|---|
| Event No. | Control | 7982.1 | | 7983.6 | | 7983.9 | |
| | | A | P | A | P | A | P |

(continued)

| MAB74 - Salt irrigation (40-80 mM NaCl) | | | | | | |
|---|---|---|---|---|---|---|
| Event No. | Control | 7982.1 | | 7983.6 | | 7983.9 |
| Rosette Diameter on day 3 | 1.70 | | | 2.06 | 0.10 | 1.94 | 0.00 |
| Rosette Diameter on day 5 | 2.36 | | | | | 2.62 | 0.06 |
| Rosette Diameter on day 8 | 3.77 | | | | | 4.05 | 0.02 |
| Rosette Area on day 3 | 0.90 | | | 1.14 | 0.02 | | |
| Rosette Area on day 5 | 1.56 | | | 1.83 | 0.05 | 1.85 | 0.10 |
| Rosette Area on day 8 | 4.06 | | | | | 4.46 | 0.03 |
| Plot Coverage on day 3 | 7.04 | | | 9.13 | 0.02 | | |
| Plot Coverage on day 5 | 12.30 | | | 14.62 | 0.03 | 14.79 | 0.08 |
| Plot Coverage on day 8 | 31.90 | | | | | 35.66 | 0.01 |
| Leaf Number on day 3 | 5.08 | | | 5.75 | 0.04 | 5.31 | 0.07 |
| Leaf Number on day 5 | 6.86 | | | | | 7.25 | 0.04 |
| RGR of Rosette Area between day 3 and 5 | 0.37 | 0.42 | 0.07 | | | | |
| 1000 Seeds weight [gr] | 0.02 | 0.02 | 0.02 | | | 0.02 | 0.05 |
| Yield [gr]/Plant | 0.04 | 0.06 | 0.05 | | | | |
| Harvest Index | 0.11 | 0.20 | 0.06 | | | | |

**Table 41:** Provided are the growth, biomass and yield parameters of transgenic or control plants as measured in Green House under salinity irrigation. A = average; P = p value; RGR = Relative Growth Rate. The indicated days refer to days from planting.

**Table 42**

| MAB76 - Salt irrigation (40-80 mM NaCl) | | | | | |
|---|---|---|---|---|---|
| Event No. | Control | 7633.1 | | 7633.2 | |
| | | A | P | A | P |
| Event No. | Control | 7633.1 | | 7633.2 | |
| | | A | P | A | P |
| Leaf Number on day 3 | 5.08 | | | 5.44 | 0.02 |
| Leaf Number on day 8 | 8.66 | 9.13 | 0.07 | | |

**Table 42:** Provided are the growth, biomass and yield parameters of transgenic or control plants as measured in Green House under salinity irrigation. A = average; P = p value; RGR = Relative Growth Rate. The indicated days refer to days from planting.

**Table 43**

| MAB77- Salt irrigation (40-80 mM NaCl) | | | | | |
|---|---|---|---|---|---|
| Event No. | Control | 7931.11 | | 8212.2 | |
| | | A | P | A | P |
| Leaf Number on day 8 | 8.66 | | | 9.75 | 0.09 |
| RGR of Rosette Area between day 1 and 3 | 0.45 | 0.52 | 0.10 | | |

(continued)

| MAB77- Salt irrigation (40-80 mM NaCl) | | | | | |
|---|---|---|---|---|---|
| Event No. | Control | 7931.11 | | 8212.2 | |
| Harvest Index | 0.11 | | | 0.15 | 0.07 |

Table 43: Provided are the growth, biomass and yield parameters of transgenic or control plants as measured in Green House under salinity irrigation. A = average; P = p value; RGR = Relative Growth Rate. The indicated days refer to days from planting.

**Table 44**

| MAB79 - Salt irrigation (40-80 mM NaCl) | | | | | | | | | |
|---|---|---|---|---|---|---|---|---|---|
| Event No. | Control | 7323.10, | | 7961.1, | | 7962.2, | | 7962.2, | |
| | | A | P | A | P | A | P | A | P |
| Rosette Diameter on day 3 | 1.70 | | | 1.84 | 0.10 | 1.93 | 0.05 | | |
| Rosette Diameter on day 5 | 2.36 | | | 2.53 | 0.01 | | | | |
| Rosette Diameter on day 8 | 3.77 | | | 4.32 | 0.03 | 4.01 | 0.04 | | |
| Rosette Area on day 3 | 0.90 | | | 1.08 | 0.09 | | | | |
| Rosette Area on day 8 | 4.06 | | | 5.29 | 0.03 | 4.78 | 0.05 | | |
| Plot Coverage on day 3 | 7.04 | | | 8.63 | 0.08 | | | | |
| Plot Coverage on day 8 | 31.90 | | | 42.32 | 0.03 | 38.27 | 0.05 | | |
| Leaf Number on day 3 | 5.08 | 5.63 | 0.00 | | | | | 5.75 | 0.04 |
| Leaf Number on day 5 | 6.86 | | | | | | | 7.44 | 0.01 |
| RGR of Leaf Number between day 5 and 8 | 0.09 | | | 0.11 | 0.01 | | | | |
| RGR of Rosette Area between day 5 and 8 | 0.53 | | | | | | | 0.59 | 0.01 |
| 1000 Seeds weight [gr] | 0.02 | | | 0.02 | 0.00 | | | | |
| Harvest Index | 0.11 | | | 0.19 | 0.00 | | | | |

Table 44: Provided are the growth, biomass and yield parameters of transgenic or control plants as measured in Green House under salinity irrigation. A = average; P = p value; RGR = Relative Growth Rate. The indicated days refer to days from planting.

Tables 45-59 depict analyses of plant parameters (as describe above) overexpressing the polynucleotides of the disclosure under the regulation of the 6669 promoter under Normal Growth conditions [Normal irrigation included NaCl Electrical conductivity (E.C.) of 1-2].

**Table 45**

| MAB115 - Normal Growth Conditions | | | | | | | |
|---|---|---|---|---|---|---|---|
| Event No. | Control | 8564.1 | | 8565.1 | | 8565.2 | |
| | | A | P | A | P | A | P |
| Rosette Diameter on day 3 | 1.67 | 1.80 | 0.09 | 1.75 | 0.04 | 1.95 | 0.04 |
| Rosette Diameter on day 8 | 3.60 | | | 4.00 | 0.09 | | |
| Rosette Area on day 5 | 1.54 | | | 1.70 | 0.09 | | |
| Rosette Area on day 8 | 3.98 | | | 4.38 | 0.06 | | |
| Plot Coverage on day 5 | 12.30 | | | 13.61 | 0.06 | | |

(continued)

| MAB115 - Normal Growth Conditions | | | | | | | |
|---|---|---|---|---|---|---|---|
| Event No. | Control | 8564.1 | | 8565.1 | | 8565.2 | |
| Plot Coverage on day 8 | 31.82 | | | 35.07 | 0.02 | | |
| Leaf Number on day 3 | 5.25 | | | 5.94 | 0.00 | | |
| Leaf Number on day 5 | 6.52 | | | 7.38 | 0.05 | | |
| Leaf Number on day 8 | 8.92 | | | | | 9.31 | 0.00 |
| RGR of Leaf Number between day 3 and 5 | 0.12 | | | 0.12 | 0.04 | | |
| RGR of Rosette Area between day 5 and 8 | 0.53 | 0.62 | 0.01 | | | | |

**Table 45:** Provided are the growth, biomass and yield parameters of transgenic or control plants as measured in Green House under normal irrigation. A = average; P = p value; RGR = Relative Growth Rate. The indicated days refer to days from planting.

**Table 46**

| MAB54 - Normal Growth Conditions | | | | | | | | | |
|---|---|---|---|---|---|---|---|---|---|
| Event No. | Control | 8181.2 | | 8182.2 | | 8184.3 | | 8185.4 | |
| | | A | P | A | P | A | P | A | P |
| Rosette Diameter on day 5 | 2.24 | 2.70 | 0.02 | 2.64 | 0.00 | | | 2.81 | 0.03 |
| Rosette Diameter on day 8 | 3.60 | 4.00 | 0.08 | | | | | 4.29 | 0.05 |
| Rosette Area on day 3 | 0.89 | | | 1.19 | 0.04 | | | 1.35 | 0.01 |
| Rosette Area on day 8 | 3.98 | | | | | | | 5.91 | 0.05 |
| Plot Coverage on day 3 | 7.10 | | | 9.52 | 0.04 | 7.49 | 0.08 | 10.76 | 0.01 |
| Plot Coverage on day 8 | 31.82 | | | | | | | 47.28 | 0.06 |
| Leaf Number on day 3 | 5.25 | 6.19 | 0.06 | | | | | 6.25 | 0.00 |
| Leaf Number on day 5 | 6.52 | | | | | | | 7.94 | 0.03 |
| Leaf Number on day 8 | 8.92 | 9.38 | 0.00 | | | | | 9.50 | 0.01 |
| RGR of Leaf Number between day 3 and 5 | 0.12 | 0.13 | 0.08 | | | | | | |
| RGR of Rosette Area between day 1 and 3 | 0.46 | 0.52 | 0.01 | | | | | 0.51 | 0.05 |
| 1000 Seeds weight [gr] | 0.02 | | | 0.02 | 0.00 | 0.02 | 0.00 | | |

**Table 46:** Provided are the growth, biomass and yield parameters of transgenic or control plants as measured in Green House under normal irrigation. A = average; P = p value; RGR = Relative Growth Rate. The indicated days refer to days from planting.

**Table 47**

| MAB55 - Normal Growth Conditions | | | | | |
|---|---|---|---|---|---|
| Event No. | Control | 6802.5 | | 6805.4 | |
| | | A | P | A | P |
| Rosette Area on day 5 | 1.54 | 1.70 | 0.03 | | |
| Rosette Area on day 8 | 3.98 | | | 4.63 | 0.02 |
| Plot Coverage on day 5 | 12.30 | 13.60 | 0.02 | | |

(continued)

| MAB55 - Normal Growth Conditions | | | | | |
|---|---|---|---|---|---|
| Event No. | Control | 6802.5 | | 6805.4 | |
| Plot Coverage on day 8 | 31.82 | | | 37.03 | 0.01 |

**Table 47:** Provided are the growth, biomass and yield parameters of transgenic or control plants as measured in Green House under normal irrigation. A = average; P = p value; RGR = Relative Growth Rate. The indicated days refer to days from planting.

**Table 48**

| MAB56 - Normal Growth Conditions | | | | | | | | | |
|---|---|---|---|---|---|---|---|---|---|
| Event No. | Control | 6691.2 | | 6691.3 | | 6693.2 | | 6695.7 | |
| | | A | P | A | P | A | P | A | P |
| Rosette Diameter on day 3 | 1.67 | | | 1.89 | 0.05 | 1.97 | 0.07 | | |
| Rosette Diameter on day 5 | 2.24 | | | 2.55 | 0.09 | 2.58 | 0.01 | | |
| Rosette Area on day 3 | 0.89 | 1.06 | 0.00 | 1.15 | 0.02 | 1.23 | 0.08 | | |
| Rosette Area on day 5 | 1.54 | | | 1.94 | 0.02 | 1.94 | 0.03 | | |
| Plot Coverage on day 3 | 7.10 | 8.45 | 0.00 | 9.21 | 0.01 | 9.82 | 0.07 | | |
| Plot Coverage on day 5 | 12.30 | | | 15.49 | 0.01 | 15.53 | 0.02 | | |
| Plot Coverage on day 8 | 31.82 | | | | | 34.82 | 0.05 | | |
| Leaf Number on day 3 | 5.25 | 5.50 | 0.00 | 5.81 | 0.00 | 6.00 | 0.02 | | |
| Leaf Number on day 5 | 6.52 | | | 7.38 | 0.01 | 7.50 | 0.02 | | |
| RGR of Leaf Number between day 3 and 5 | 0.12 | | | | | 0.13 | 0.06 | | |
| RGR of Leaf Number between day 5 and 8 | 0.12 | | | | | | | 0.14 | 0.01 |
| RGR of Rosette Area between day 5 and 8 | 0.53 | | | | | | | 0.62 | 0.02 |
| 1000 Seeds weight [gr] | 0.02 | 0.02 | 0.08 | | | | | | |

**Table 48:** Provided are the growth, biomass and yield parameters of transgenic or control plants as measured in Green House under normal irrigation. A = average; P = p value; RGR = Relative Growth Rate. The indicated days refer to days from planting.

**Table 49**

| MAB57 - Normal Growth Conditions | | | | | | | |
|---|---|---|---|---|---|---|---|
| Event No. | Control | 6912.1 | | 6912.6 | | 6912.9 | |
| | | A | P | A | P | A | P |
| Rosette Area on day 8 | 3.98 | | | 4.48 | 0.02 | | |
| Plot Coverage on day 8 | 31.82 | | | 35.81 | 0.01 | | |
| Leaf Number on day 3 | 5.25 | | | | | 5.69 | 0.00 |
| Leaf Number on day 5 | 6.52 | 8.13 | 0.06 | 8.13 | 0.06 | | |
| Leaf Number on day 8 | 8.92 | | | | | 9.56 | 0.06 |
| RGR of Rosette Area between day 5 and 8 | 0.53 | | | | | 0.58 | 0.10 |

(continued)

| MAB57 - Normal Growth Conditions | | | | | | | |
|---|---|---|---|---|---|---|---|
| Event No. | Control | 6912.1 | | 6912.6 | | 6912.9 | |
| 1000 Seeds weight [gr] | 0.02 | | | | | 0.02 | 0.00 |

Table 49: Provided are the growth, biomass and yield parameters of transgenic or control plants as measured in Green House under normal irrigation. A = average; P = p value; RGR = Relative Growth Rate. The indicated days refer to days from planting.

**Table 50**

| MAB58 - Normal Growth Conditions | | | | | | | | | |
|---|---|---|---|---|---|---|---|---|---|
| Event No. | Control | 6783.2 | | 7522.10 | | 7522.3 | | 7523.6 | |
| | | A | P | A | P | A | P | A | P |
| Rosette Diameter on day 3 | 1.67 | | | | | 2.11 | 0.00 | | |
| Rosette Diameter on day 5 | 2.24 | 2.57 | 0.00 | | | | | | |
| Rosette Diameter on day 8 | 3.60 | | | 4.06 | 0.07 | 4.54 | 0.00 | | |
| Rosette Area on day 3 | 0.89 | | | | | 1.36 | 0.00 | | |
| Rosette Area on day 5 | 1.54 | | | | | 2.29 | 0.00 | | |
| Rosette Area on day 8 | 3.98 | | | | | 5.98 | 0.00 | | |
| Plot Coverage on day 3 | 7.10 | | | | | 10.90 | 0.00 | | |
| Plot Coverage on day 5 | 12.30 | | | | | 18.32 | 0.00 | | |
| Plot Coverage on day 8 | 31.82 | | | | | 47.88 | 0.00 | | |
| Leaf Number on day 3 | 5.25 | | | | | 6.06 | 0.00 | 6.44 | 0.05 |
| | | A | P | A | P | A | P | A | P |
| Leaf Number on day 5 | 6.52 | | | | | | | 8.38 | 0.00 |
| Leaf Number on day 8 | 8.92 | 9.25 | 0.04 | | | 9.81 | 0.04 | | |
| RGR of Leaf Number between day 5 and 8 | 0.12 | 0.12 | 0.03 | | | | | | |
| 1000 Seeds weight [gr] | 0.02 | 0.02 | 0.08 | | | | | | |

Table 50: Provided are the growth, biomass and yield parameters of transgenic or control plants as measured in Green House under normal irrigation. A = average; P = p value; RGR = Relative Growth Rate. The indicated days refer to days from planting.

**Table 51**

| MAB59 - Normal Growth Conditions | | | | | | | |
|---|---|---|---|---|---|---|---|
| Event No. | Control | 6793.4 | | 6794.4 | | 6794.5 | |
| | | A | P | A | P | A | P |
| Rosette Diameter on day 3 | 1.67 | | | 2.38 | 0.05 | | |
| Rosette Diameter on day 5 | 2.24 | 2.73 | 0.06 | | | | |
| Rosette Area on day 3 | 0.89 | 1.27 | 0.06 | 1.65 | 0.03 | | |
| Plot Coverage on day 3 | 7.10 | 10.15 | 0.06 | 13.19 | 0.03 | | |
| Leaf Number on day 3 | 5.25 | 6.00 | 0.02 | 6.75 | 0.01 | | |

(continued)

| MAB59 - Normal Growth Conditions | | | | | | | |
|---|---|---|---|---|---|---|---|
| Event No. | Control | 6793.4 | | 6794.4 | | 6794.5 | |
| Leaf Number on day 5 | 6.52 | 7.69 | 0.05 | 8.00 | 0.07 | | |
| Leaf Number on day 8 | 8.92 | | | 9.38 | 0.02 | | |
| RGR of Rosette Area between day 1 and 3 | 0.46 | | | | | 0.49 | 0.02 |
| 1000 Seeds weight [gr] | 0.02 | | | | | 0.02 | 0.00 |

Table 51: Provided are the growth, biomass and yield parameters of transgenic or control plants as measured in Green House under normal irrigation. A = average; P = p value; RGR = Relative Growth Rate. The indicated days refer to days from planting.

**Table 52**

| MAB69 - Normal Growth Conditions | | | | | | | | | |
|---|---|---|---|---|---|---|---|---|---|
| Event No. | Control | 6651.11 | | 6651.12 | | 8341.1 | | 8342.1 | |
| | | A | P | A | P | A | P | A | P |
| Rosette Diameter on day 3 | 1.67 | 1.92 | 0.01 | | | | | | |
| Rosette Area on day 3 | 0.89 | 1.09 | 0.00 | | | | | | |
| Rosette Area on day 8 | 3.98 | 5.05 | 0.04 | | | | | | |
| Plot Coverage on day 3 | 7.10 | 8.74 | 0.00 | | | | | | |
| Plot Coverage on day 8 | 31.82 | 40.41 | 0.04 | | | | | | |
| Leaf Number on day 3 | 5.25 | 5.69 | 0.00 | | | | | 5.94 | 0.09 |
| Leaf Number on day 8 | 8.92 | | | | | 8.94 | 0.08 | 9.31 | 0.00 |
| RGR of Rosette Area between day 1 and 3 | 0.46 | 0.51 | 0.04 | | | | | | |
| 1000 Seeds weight [gr] | 0.02 | | | 0.02 | 0.01 | | | | |

Table 52: Provided are the growth, biomass and yield parameters of transgenic or control plants as measured in Green House under normal irrigation. A = average; P = p value; RGR = Relative Growth Rate. The indicated days refer to days from planting.

**Table 53**

| MAB70 - Normal Growth Conditions | | | | | | | |
|---|---|---|---|---|---|---|---|
| Event No. | Control | 7971.3 | | 7972.1 | | 7974.3 | |
| | | A | P | A | P | A | P |
| Rosette Diameter on day 3 | 1.67 | | | 1.94 | 0.00 | 2.27 | 0.00 |
| Rosette Diameter on day 5 | 2.24 | | | 2.62 | 0.04 | 3.03 | 0.00 |
| Rosette Diameter on day 8 | 3.60 | 4.40 | 0.00 | | | 5.01 | 0.05 |
| Rosette Area on day 3 | 0.89 | 1.20 | 0.07 | 1.12 | 0.01 | 1.52 | 0.04 |
| Rosette Area on day 5 | 1.54 | 2.06 | 0.05 | 1.87 | 0.00 | 2.49 | 0.10 |
| Rosette Area on day 8 | 3.98 | 5.86 | 0.06 | | | 7.03 | 0.05 |
| Plot Coverage on day 3 | 7.10 | 9.61 | 0.06 | 9.00 | 0.01 | 12.12 | 0.04 |
| Plot Coverage on day 5 | 12.30 | 16.46 | 0.04 | 14.93 | 0.00 | 19.90 | 0.09 |

(continued)

| MAB70 - Normal Growth Conditions | | | | | | | |
|---|---|---|---|---|---|---|---|
| Event No. | Control | 7971.3 | | 7972.1 | | 7974.3 | |
| Plot Coverage on day 8 | 31.82 | 46.87 | 0.06 | | | 56.23 | 0.05 |
| Leaf Number on day 3 | 5.25 | | | | | 6.44 | 0.05 |
| Leaf Number on day 5 | 6.52 | | | | | 8.13 | 0.00 |
| Leaf Number on day 8 | 8.92 | | | 9.75 | 0.09 | 10.38 | 0.05 |
| RGR of Rosette Area between day 5 and 8 | 0.53 | 0.62 | 0.01 | | | 0.61 | 0.03 |

**Table 53:** Provided are the growth, biomass and yield parameters of transgenic or control plants as measured in Green House under normal irrigation. A = average; P = p value; RGR = Relative Growth Rate. The indicated days refer to days from planting.

**Table 54**

| MAB71 - Normal Growth Conditions | | | | | | | | | |
|---|---|---|---|---|---|---|---|---|---|
| Event No. | Control | 7331.4 | | 7332.2 | | 7333.5 | | 7334.5 | |
| | | A | P | A | P | A | P | A | P |
| Rosette Diameter on day 5 | 2.24 | | | 2.52 | 0.02 | | | | |
| Rosette Diameter on day 8 | 3.60 | 4.33 | 0.01 | | | | | | |
| Rosette Area on day 5 | 1.54 | | | | | | | 1.88 | 0.07 |
| Rosette Area on day 8 | 3.98 | | | | | | | 4.68 | 0.00 |
| Plot Coverage on day 5 | 12.30 | | | 14.25 | 0.08 | | | 15.05 | 0.05 |
| Plot Coverage on day 8 | 31.82 | | | | | | | 37.48 | 0.00 |
| Leaf Number on day 3 | 5.25 | | | 5.47 | 0.06 | | | 5.81 | 0.00 |
| Leaf Number on day 5 | 6.52 | 7.56 | 0.00 | | | | | | |
| RGR of Leaf Number between day 1 and 3 | 0.16 | | | | | | | 0.17 | 0.01 |
| RGR of Leaf Number between day 3 and 5 | 0.12 | | | | | 0.12 | 0.10 | | |
| RGR of Leaf Number between day 5 and 8 | 0.12 | | | | | 0.12 | 0.01 | | |
| RGR of Rosette Area between day 5 and 8 | 0.53 | | | | | 0.61 | 0.04 | | |

**Table 54:** Provided are the growth, biomass and yield parameters of transgenic or control plants as measured in Green House under normal irrigation. A = average; P = p value; RGR = Relative Growth Rate. The indicated days refer to days from planting.

**Table 55**

| MAB72 - Normal Growth Conditions | | | | | | | |
|---|---|---|---|---|---|---|---|
| Event No. | Control | 8552.4 | | 8553.2 | | 8553.3 | |
| | | A | P | A | P | A | P |
| Rosette Diameter on day 3 | 1.67 | 1.90 | 0.05 | | | 1.83 | 0.00 |
| Rosette Diameter on day 5 | 2.24 | 2.67 | 0.00 | | | 2.55 | 0.01 |
| Rosette Diameter on day 8 | 3.60 | 4.06 | 0.08 | 4.15 | 0.10 | | |
| Rosette Area on day 3 | 0.89 | 1.20 | 0.00 | 1.02 | 0.00 | 1.08 | 0.02 |

(continued)

| MAB72 - Normal Growth Conditions | | | | | | | |
|---|---|---|---|---|---|---|---|
| Event No. | Control | 8552.4 | | 8553.2 | | 8553.3 | |
| Rosette Area on day 5 | 1.54 | 2.00 | 0.00 | | | 1.87 | 0.00 |
| Rosette Area on day 8 | 3.98 | 5.05 | 0.00 | | | 4.82 | 0.00 |
| Plot Coverage on day 3 | 7.10 | 9.64 | 0.00 | 8.13 | 0.00 | 8.67 | 0.02 |
| Plot Coverage on day 5 | 12.30 | 16.04 | 0.00 | | | 14.98 | 0.00 |
| Plot Coverage on day 8 | 31.82 | 40.39 | 0.00 | | | 38.57 | 0.00 |
| Leaf Number on day 3 | 5.25 | 5.88 | 0.00 | 5.56 | 0.00 | 5.50 | 0.00 |
| Leaf Number on day 8 | 8.92 | | | 9.38 | 0.02 | | |

**Table 55:** Provided are the growth, biomass and yield parameters of transgenic or control plants as measured in Green House under normal irrigation. A = average; P = p value; RGR = Relative Growth Rate. The indicated days refer to days from planting.

Table 56

| MAB74 - Normal Growth Conditions | | | | | | | | | |
|---|---|---|---|---|---|---|---|---|---|
| Event No. | Control | 7981.1 | | 7982.4 | | 7983.6 | | 7983.9 | |
| | | A | P | A | P | A | P | A | P |
| Rosette Diameter on day 3 | 1.67 | | | 2.06 | 0.10 | 1.94 | 0.00 | | |
| Rosette Diameter on day 5 | 2.24 | | | | | 2.62 | 0.06 | | |
| Rosette Diameter on day 8 | 3.60 | | | | | 4.05 | 0.02 | | |
| Rosette Area on day 3 | 0.89 | | | 1.14 | 0.02 | | | | |
| Rosette Area on dav 5 | 1.54 | | | 1.83 | 0.05 | 1.85 | 0.10 | | |
| Rosette Area on dav 8 | 3.98 | | | | | 4.46 | 0.03 | | |
| Plot Coverage on day 3 | 7.10 | | | 9.13 | 0.02 | | | | |
| Plot Coverage on dav 5 | 12.30 | | | 14.62 | 0.03 | 14.79 | 0.08 | | |
| Plot Coverage on dav 8 | 31.82 | | | | | 35.66 | 0.01 | | |
| Leaf Number on dav 3 | 5.25 | | | 5.75 | 0.04 | 5.31 | 0.07 | | |
| Leaf Number on day 5 | 6.52 | 6.26 | 0.02 | | | 7.25 | 0.04 | | |
| Leaf Number on day 8 | 8.92 | | | | | | | 9.13 | 0.07 |
| RGR of Leaf Number between day 3 and 5 | 0.12 | | | 0.13 | 0.08 | | | 0.12 | 0.03 |
| RGR of Rosette Area between day 1 and 3 | 0.46 | | | | | | | 0.33 | 0.00 |
| RGR of Rosette Area between day 3 and 5 | 0.36 | 0.42 | 0.07 | | | | | | |
| Biomass DW [gr] | 3.07 | | | | | | | | |
| 1000 Seeds weight [gr] | 0.02 | 0.02 | 0.02 | | | 0.02 | 0.05 | | |

**Table 56:** Provided are the growth, biomass and yield parameters of transgenic or control plants as measured in Green House under normal irrigation. A = average; P = p value; RGR = Relative Growth Rate. The indicated days refer to days from planting.

**Table 57**

| MAB76 - Normal Growth Conditions | | | | | |
|---|---|---|---|---|---|
| Event No. | Control | 7633.1 | | 7635.16 | |
| | | A | P | A | P |
| Rosette Diameter on day 3 | 1.67 | | | 1.93 | 0.04 |
| Leaf Number on day 3 | 5.25 | 5.44 | 0.02 | | |
| Leaf Number on day 5 | 6.52 | | | 7.25 | 0.04 |

**Table 57:** Provided are the growth, biomass and yield parameters of transgenic or control plants as measured in Green House under normal irrigation. A = average; P = p value; RGR = Relative Growth Rate. The indicated days refer to days from planting.

**Table 58**

| MAB77 -Normal Growth Conditions | | | | | |
|---|---|---|---|---|---|
| Event No. | Control | 8212.1 | | 8212.2 | |
| | | A | | A | P |
| Leaf Number on day 3 | 5.25 | | | 5.63 | 0.00 |
| Leaf Number on day 8 | 8.92 | 9.75 | 0.09 | | |

**Table 58:** Provided are the growth, biomass and yield parameters of transgenic or control plants as measured in Green House under normal irrigation. A = average; P = p value; RGR = Relative Growth Rate. The indicated days refer to days from planting.

**Table 59**

| MAB79 - Normal Growth Conditions | | | | | | | |
|---|---|---|---|---|---|---|---|
| Event No. | Control | 7324.1 | | 7961.1 | | 7962.2 | |
| | | A | P | A | P | A | P |
| Rosette Diameter on day 3 | 1.67 | 1.84 | 0.10 | 1.93 | 0.05 | | |
| Rosette Diameter on day 5 | 2.24 | 2.53 | 0.01 | | | | |
| Rosette Diameter on day 8 | 3.60 | 4.32 | 0.03 | 4.01 | 0.04 | | |
| Rosette Area on day 3 | 0.89 | 1.08 | 0.09 | | | | |
| Rosette Area on day 8 | 3.98 | 5.29 | 0.03 | 4.78 | 0.05 | | |
| Plot Coverage on day 3 | 7.10 | 8.63 | 0.08 | | | | |
| Plot Coverage on day 8 | 31.82 | 42.32 | 0.03 | 38.27 | 0.05 | | |
| Leaf Number on day 3 | 5.25 | | | | | 5.75 | 0.04 |
| Leaf Number on day 5 | 6.52 | | | | | 7.44 | 0.01 |
| RGR of Rosette Area between day 5 and 8 | 0.53 | | | | | 0.59 | 0.01 |
| 1000 Seeds weight [gr] | 0.02 | 0.02 | 0.00 | | | | |

**Table 59:** Provided are the growth, biomass and yield parameters of transgenic or control plants as measured in Green House under normal irrigation. A = average; P = p value; RGR = Relative Growth Rate. The indicated days refer to days from planting.

*EXAMPLE 7*

*IMPROVED FERTILIZER USE EFFICIENCYINARABIDOPSIS TISSUE CULTURE ASSAY*

Plants transgenic to the following MAB genes were assayed for fertilizer use efficiency in a tissue culture assay: MAB115, MAB54, MAB55, MAB56, MAB57, MAB58, MAB59, MAB69, MAB70, MAB71, MAB72, MAB74, MAB76, MAB77, MAB79, MAB116, and MAB117 (the sequence identifiers of the cloned polynucleotides and their expressed polypeptides are provided in Table 7 above).

*Assay 1: plant growth at nitrogen deficiency under tissue culture conditions*

The present inventors have found the nitrogen use efficiency (NUE) assay to be relevant for the evaluation of the ABST candidate genes, since NUE deficiency encourages root elongation, increase of root coverage and allows detecting the potential of the plant to generate a better root system under drought conditions. In addition, there are indications in the literature that biological mechanisms of NUE and drought tolerance are linked (Wesley et al., 2002 Journal of Experiment Botany Vol 53, No.366, pp. 13-25).
Surface sterilized seeds were sown in basal media [50 % Murashige-Skoog medium (MS) supplemented with 0.8 % plant agar as solidifying agent] in the presence of Kanamycin (for selecting only transgenic plants). After sowing, plates were transferred for 2-3 days for stratification at 4 °C and then grown at 25 °C under 12-hour light 12-hour dark daily cycles for 7 to 10 days. At this time point, seedlings randomly chosen were carefully transferred to plates with nitrogen-limiting conditions: 0.5 MS media in which the combined nitrogen concentration ($NH_4NO_3$ and $KNO_3$) is 0.75 mM (nitrogen deficient conditions) or 3 mM [Normall (optimal) nitrogen concentration]. Each plate contains 5 seedlings of same event, and 3-4 different plates (replicates) for each event. For each polynucleotide of the disclosure at least four independent transformation events were analyzed from each construct. Plants expressing the polynucleotides of the disclosure were compared to the average measurement of the control plants (empty vector or GUS reporter under the same promoter) used in the same experiment.
*Digital imaging and statistical analysis -* Parameters were measured and analyzed as previously described in Example 5, Assay 1 above.
Tables 60-69 depict analyses of seedling parameters (as describe above) overexpressing the polynucleotides of the disclosure under the regulation of At6669 promoter under Nitrogene Deficiency conditions.

*Table 60*

| MAB70 - Nitrogene Deficiency (0.75 mM Nitrogen) | | | | | | | | | |
|---|---|---|---|---|---|---|---|---|---|
| **Event No.** | **Control** | **7971.3** | | **7972.1** | | **7974.1** | | **7974.3** | |
| | | A | P | A | P | A | P | A | P |
| Dry Weight [gr] | 0.01 | | | | | | | 0.01 | 0.00 |
| Fresh Wight [gr] | 0.10 | | | | | | | 0.18 | 0.04 |
| Leaf Area on day 10 | 0.36 | | | | | 0.47 | 0.04 | 0.55 | 0.00 |
| Leaf Area on day 5 | 0.14 | | | | | | | 0.24 | 0.04 |
| Roots Coverage on day 10 | 5.58 | 7.93 | 0.01 | 7.45 | 0.06 | | | | |
| Roots Coverage on day 5 | 1.75 | 2.41 | 0.00 | | | | | 2.58 | 0.06 |
| Roots Length on day 10 | 5.19 | 6.16 | 0.00 | | | | | | |
| Roots Length on day 5 | 2.86 | 3.16 | 0.05 | | | | | | |
| RGR of Roots Coverage between day 5 and 10 | 0.45 | | | | | 0.67 | 0.05 | | |
| RGR of Roots Coverage between day 1 and 5 | 0.81 | 2.35 | 0.02 | 2.01 | 0.06 | 2.10 | 0.05 | 2.23 | 0.02 |
| RGR of Roots Length between day 1 and 5 | 0.16 | | | | | 0.24 | 0.04 | | |
| RGR of Roots Length between day 5 and 10 | 0.20 | 0.50 | 0.00 | 0.48 | 0.00 | 0.56 | 0.00 | 0.58 | 0.00 |

**Table 60:** Provided are the growth and biomass parameters of transgenic or control plants as measured in Tissue Calture growth under Nitrogene Deficiency (0.75 mM N). A = average; P = p value; RGR = Relative Growth Rate. The indicated days refer to days from planting.

*Table 61*

| MAB71 - Nitrogene Deficiency (0.75 mM Nitrogen) | | | | | | | | | |
|---|---|---|---|---|---|---|---|---|---|
| Event No. | Control | 7331.5 | | 7332.2 | | 7333.5 | | 7334.4 | |
| | | A | P | A | P | A | P | A | P |
| Dry Weight [gr] | 0.01 | 0.01 | 0.00 | 0.01 | 0.01 | 0.01 | 0.01 | 0.01 | 0.01 |
| Fresh Wight [gr] | 0.10 | 0.22 | 0.00 | 0.18 | 0.02 | 0.13 | 0.09 | | |
| Leaf Area on day 10 | 0.36 | 0.55 | 0.00 | | | | | 0.52 | 0.00 |
| Leaf Area on day 5 | 0.14 | 0.28 | 0.00 | | | | | 0.21 | 0.00 |
| Roots Coverage on day 10 | 5.58 | | | | | 8.19 | 0.05 | 9.47 | 0.03 |
| Roots Coverage on day 5 | 1.75 | 2.33 | 0.10 | | | | | 2.99 | 0.02 |
| Roots Length on day 10 | 5.19 | | | | | | | 6.69 | 0.03 |
| Roots Length on day 5 | 2.86 | | | | | | | 3.84 | 0.01 |
| RGR of Roots Length between day 1 and 5 | 0.16 | | | 0.20 | 0.07 | | | | |
| RGR of Roots Length between day 5 and 10 | 0.20 | 0.66 | 0.05 | | | 0.26 | 0.08 | | |

**Table 61:** Provided are the growth and biomass parameters of transgenic or control plants as measured in Tissue Calture growth under Nitrogene Deficiency (0.75 mM N). A = average; P = p value; RGR = Relative Growth Rate. The indicated days refer to days from planting.

*Table 62*

| MAB74 - Nitrogene Deficiency (0.75 mM Nitrogen) | | | | | |
|---|---|---|---|---|---|
| Event No. | Control | 7982.4 | | 7983.9 | |
| | | A | P | A | P |
| Roots Coverage on day 10 | 5.58 | | | 9.76 | 0.06 |
| Roots Length on day 10 | 5.19 | | | 6.70 | 0.00 |
| RGR Leaf Area between day 5 and 10 | 0.30 | | | 0.44 | 0.09 |
| RGR of Roots Coverage between day 5 and 10 | 0.45 | 0.63 | 0.08 | | |

**Table 62:** Provided are the growth and biomass parameters of transgenic or control plants as measured in Tissue Calture growth under Nitrogene Deficiency (0.75 mM N). A = average; P = p value; RGR = Relative Growth Rate. The indicated days refer to days from planting.

*Table 63*

| MAB76 - Nitrogene Deficiency (0.75 mM Nitrogen) | | | |
|---|---|---|---|
| Event No. | Control | 7635.4 | |
| | | A | P |
| RGR of Roots Coverage between day 5 and 10 | 0.45 | 0.70 | 0.07 |
| RGR of Roots Length between day 1 and 5 | 0.16 | 0.26 | 0.07 |

**Table 63:** Provided are the growth and biomass parameters of transgenic or control plants as measured in Tissue Calture growth under Nitrogene Deficiency (0.75 mM N). A = average; P = p value; RGR = Relative Growth Rate. The indicated days refer to days from planting.

**Table 64**

| MAB77 - Nitrogene Deficiency (0.75 mM Nitrogen) | | | | | | | |
|---|---|---|---|---|---|---|---|
| Event No. | Control | 7931.11 | | 8211.8 | | 8212.2 | |
| | | A | P | A | P | A | P |
| Dry Weight [gr] | 0.01 | | | | | 0.01 | 0.00 |
| Fresh Wight [gr] | 0.10 | | | 0.13 | 0.03 | 0.14 | 0.01 |
| Roots Coverage on day 5 | 1.75 | 2.26 | 0.03 | | | | |
| RGR of Roots Coverage between day 5 and 10 | 0.45 | | | 0.71 | 0.01 | 0.75 | 0.05 |
| RGR of Roots Length between day 1 and 5 | 0.16 | | | 0.24 | 0.03 | | |
| RGR of Roots Length between day 5 and 10 | 0.20 | 0.31 | 0.05 | 0.99 | 0.04 | 0.86 | 0.08 |

**Table 64:** Provided are the growth and biomass parameters of transgenic or control plants as measured in Tissue Culture growth under Nitrogene Deficiency (0.75 mM N). A = average; P = p value; RGR = Relative Growth Rate. The indicated days refer to days from planting.

**Table 65**

| MAB79 - Nitrogene Deficiency (0.75 mM Nitrogen) | | | | | | | |
|---|---|---|---|---|---|---|---|
| Event No. | Control | 7323.3 | | 7324.1 | | 7961.1 | |
| | | A | P | A | P | A | P |
| Dry Weight [gr] | 0.01 | 0.01 | 0.00 | 0.01 | 0.03 | 0.01 | 0.01 |
| Fresh Wight [gr] | 0.10 | 0.16 | 0.00 | | | 0.11 | 0.10 |
| RGR of Roots Coverage between dav 5 and 10 | 0.45 | 0.71 | 0.09 | | | | |
| RGR of Roots Coverage between dav 1 and 5 | 0.81 | 3.11 | 0.00 | | | | |
| RGR of Roots Length between day 5 and 10 | 0.20 | 0.67 | 0.00 | 0.49 | 0.09 | | |

**Table 65:** Provided are the growth and biomass parameters of transgenic or control plants as measured in Tissue Culture growth under Nitrogene Deficiency (0.75 mM N). A = average; P = p value; RGR = Relative Growth Rate. The indicated days refer to days from planting.

**Table 66**

| MAB115 - Nitrogene Deficiency (0.75 mM Nitrogen) | | | | | | | |
|---|---|---|---|---|---|---|---|
| Event No. | Control | 8561.2 | | 8564.2 | | 8565.1 | |
| | | A | P | A | P | A | P |
| Dry Weight [gr] | 0.005 | 0.006 | 0.00 | 0.010 | 0.00 | 0.009 | 0.00 |
| Leaf Area on day 10 | 0.24 | | | | | 0.27 | 0.00 |
| Leaf Area on day 5 | 0.35 | | | | | 0.38 | 0.01 |
| Roots Coverage on day 10 | 1.67 | | | | | 2.13 | 0.02 |
| Roots Coverage on day 5 | 3.38 | | | | | 4.80 | 0.03 |
| Roots Length on day 10 | 2.39 | | | | | 2.74 | 0.00 |
| Roots Length on day 5 | 3.46 | | | | | 4.22 | 0.02 |
| RGR Leaf Area between day 5 and 10 | 0.37 | 0.43 | 0.00 | | | 0.48 | 0.00 |
| RGR Leaf Area between day 1 and 5 | 0.16 | 0.19 | 0.00 | | | | |

(continued)

| MAB115 - Nitrogene Deficiency (0.75 mM Nitrogen) | | | | | | | |
|---|---|---|---|---|---|---|---|
| Event No. | Control | 8561.2 | | 8564.2 | | 8565.1 | |
| RGR of Roots Coverage between day 5 and 10 | 1.89 | 3.21 | 0.00 | 2.23 | 0.02 | 3.47 | 0.01 |
| RGR of Roots Coverage between day 1 and 5 | 0.33 | 0.35 | 0.00 | 0.43 | 0.00 | 0.44 | 0.00 |
| RGR of Roots Length between day 1 and 5 | 0.37 | 0.56 | 0.02 | 0.53 | 0.06 | 0.68 | 0.00 |
| RGR of Roots Length between day 5 and 10 | 0.15 | | | 0.17 | 0.00 | 0.18 | 0.00 |

**Table 66:** Provided are the growth and biomass parameters of transgenic or control plants as measured in Tissue Calture growth under Nitrogene Deficiency (0.75 mM N). A = average; P = p value; RGR = Relative Growth Rate. The indicated days refer to days from planting.

*Table 67*

| MAB54 -Nitrogene Deficiency (0.75 mM Nitrogen) | | | | | | | |
|---|---|---|---|---|---|---|---|
| Event No. | Control | 8181.2 | | 8182.2 | | 8185.3 | |
| | | A | P | A | P | A | P |
| Dry Weight [gr] | 0.005 | 0.009 | 0.00 | 0.008 | 0.00 | 0.007 | 0.00 |
| Roots Coverage on day 10 | 1.67 | 1.80 | 0.04 | | | | |
| Roots Coverage on day 5 | 3.38 | 4.27 | 0.02 | 3.40 | 0.00 | | |
| Roots Length on day 10 | 2.39 | 2.52 | 0.01 | | | | |
| Roots Length on day 5 | 3.46 | 4.11 | 0.02 | 3.50 | 0.00 | | |
| RGR Leaf Area between day 5 and 10 | 0.37 | 0.45 | 0.00 | | | | |
| RGR Leaf Area between day 1 and 5 | 0.16 | 0.17 | 0.04 | | | 0.19 | 0.00 |
| RGR of Roots Coverage between day 5 and 10 | 1.89 | 3.59 | 0.00 | 3.60 | 0.01 | 2.20 | 0.01 |
| RGR of Roots Coverage between day 1 and 5 | 0.33 | 0.52 | 0.00 | 0.55 | 0.00 | 0.36 | 0.00 |
| RGR of Roots Length between day 1 and 5 | 0.37 | 0.70 | 0.00 | 0.73 | 0.00 | 0.51 | 0.02 |
| RGR of Roots Length between day 5 and 10 | 0.15 | 0.21 | 0.01 | 0.22 | 0.01 | 0.17 | 0.00 |

**Table 67:** Provided are the growth and biomass parameters of transgenic or control plants as measured in Tissue Calture growth under Nitrogene Deficiency (0.75 mM N). A = average; P = p value; RGR = Relative Growth Rate. The indicated days refer to days from planting.

*Table 68*

| MAB57 - Nitrogene Deficiency (0.75 mM Nitrogen) | | | | | | | |
|---|---|---|---|---|---|---|---|
| Event No. | | 6912.14 | | 6912.20 | | 6912.60 | |
| | Control | A | P | A | P | A | P |
| Roots Coverage on day 7 | 5.55 | | | 6.71 | 0.10 | | |
| Roots Coverage on day 14 | 15.02 | 17.33 | 0.05 | | | | |
| Roots Length on day 7 | 4.93 | 5.54 | 0.10 | 6.12 | 0.02 | 6.34 | 0.02 |
| Roots Length on day 14 | 7.83 | 8.76 | 0.02 | | | | |

(continued)

| MAB57 - Nitrogene Deficiency (0.75 mM Nitrogen) | | | | | | | |
|---|---|---|---|---|---|---|---|
| Event No. | | | 6912.14 | | 6912.20 | | 6912.60 |
| Fresh Weight | 0.19 | 0.24 | 0.09 | | | | |

Table 68: Provided are the growth and biomass parameters of transgenic or control plants as measured in Tissue Calture growth under Nitrogene Deficiency (0.75 mM N). A = average; P = p value; RGR = Relative Growth Rate. The indicated days refer to days from planting.

**Table 69**

| MAB72 - Nitrogene Deficiency (0.75 mM Nitrogen) | | | | | | | |
|---|---|---|---|---|---|---|---|
| Event No. | Control | 8552.1 | | 8552.4 | | 8553.2 | |
| | | A | P | A | P | A | P |
| Dry Weight [gr] | 0.005 | 0.01 | 0.00 | 0.01 | 0.00 | 0.01 | 0.00 |
| Leaf Area on day 10 | 0.24 | 0.24 | 0.01 | | | | |
| Roots Coverage on day 10 | 1.67 | 1.84 | 0.01 | 1.93 | 0.02 | 1.89 | 0.03 |
| Roots Coverage on day 5 | 3.38 | 3.60 | 0.04 | 3.90 | 0.06 | 4.50 | 0.00 |
| Roots Length on day 10 | 2.39 | 2.48 | 0.01 | | | | |
| Roots Length on day 5 | 3.46 | 3.70 | 0.04 | 3.65 | 0.04 | 3.84 | 0.00 |
| RGR Leaf Area between day 5 and 10 | 0.37 | 0.47 | 0.00 | 0.39 | 0.00 | | |
| RGR Leaf Area between day 1 and 5 | 0.16 | | | 0.20 | 0.09 | | |
| RGR of Roots Coverage between day 5 and 10 | 1.89 | 1.93 | 0.03 | 2.29 | 0.06 | 3.04 | 0.01 |
| RGR of Roots Coverage between day 1 and 5 | 0.33 | | | 0.35 | 0.00 | 0.52 | 0.00 |
| RGR of Roots Length between day 1 and 5 | 0.37 | | | | | 0.55 | 0.01 |
| RGR of Roots Length between day 5 and 10 | 0.15 | 0.16 | 0.00 | 0.18 | 0.00 | 0.22 | 0.01 |

Table 69: Provided are the growth and biomass parameters of transgenic or control plants as measured in Tissue Calture growth under Nitrogene Deficiency (0.75 mM N). A = average; P = p value; RGR = Relative Growth Rate. The indicated days refer to days from planting.

## EXAMPLE 8

### TRANSGENIC TOMATO AND ARABIDOPSIS PLANTS SHOW IMPROVED TOLERANCE TO SALT AND WATER-DEFICIENCY STRESSES UNDER FIELD CONDITIONS

To test the impact of AQP TIP2 genes on plant's stress tolerance, the present inventors have previously cloned and overexpressed a polynucleotide which comprises the nucleic acid sequence set forth by SEQ ID NO:2827 (also known as ABST36 set forth by SEQ ID NO:13 in WO2004/104162; or S1TIP2;2) and which encodes the TIP2 polypeptide set forth by SEQ ID NO:2828 (which comprises the consensus sequence TLXFXFAGVGS; SEQ ID NO:2826). The nucleic acid constructs which comprises the ABST36 polynucleotide under the regulation of the constitutive Arabidopsis At6669 promoter (SEQ ID NO: 2823) (further referred to as the At6669::ABST36 construct) was further transformed into tomato (*Solanum lycopersicum*) as a model crop plant (Tom-ABST36), as well as into *Arabidopsis thaliana.* Four independent, $T_2$ transgenic tomato genotypes, overexpressing ABST36 in heterozygous form, were evaluated for their tolerance to salt and water deficiency in two different salt-stress field trials and one water-deficiency-stress field trial consisting of two water-deficiency regimes. Transgenic genotypes in each field trial were compared to their null-segregant counterparts as controls.

*Materials and Experimental Methods*

*Tomato Salt-stress field trial* - All field trials were performed in a light soil, in an open field (net-house) near Rehovot, Israel. The F1 hybrids of four independent events of the cross between *ABST36*-transgenic MicroTom plants and M82 tomato plants were grown for the first 3 weeks in a nursery under normal irrigation conditions. The seedlings were then transplanted into rows and grown in a commercial greenhouse. The salt-stress trial was divided into four blocks. In each block, two different irrigation systems were established: a normal water regime for tomato cultivation and a continuous irrigation with saline water (addition of 180 to 200 mM NaCl). Each block consisted of a total of 60 plants divided as follows: six plants per event and six seedling null segregants were planted in the control row and a similar number of plants were planted in the salt-stressed row. At the stage of about 80 % red fruits *in planta*, fruit yield, plant fresh weight, and harvest index were calculated. Harvest index was calculated as yield/plant biomass.

*Tomato Water-deficiency-stress field trial* - All field trials were performed in a light soil, in an open field (net-house) near Rehovot, Israel. The F1 hybrids of the four independent events were initially grown as described above. Three-week-old seedlings were transplanted to a net-greenhouse. The experiment was structured in four blocks containing three rows irrigated with different water levels and intervals (WLI-0, WLI-1, WLI-2). In each block, six transgenic plants per event analyzed and six non transgenic plants were transplanted in each row. Seedlings were transplanted after 4 weeks into wet soil. The amount of water used to uniformly irrigate before transplanting reached maximum water capacity [20 % weight per weight (w/w)] at 60 cm depth, but without the creation of water overload. Each plant was transplanted near a dripper, with a 30-cm distance between plants, giving a total density of 2,600 plants per 1,000 $m^2$, according to a commercial growth protocol. Soil water capacity was measured using the standard procedures by sampling soil from the following three depths: 0 to 20 cm, 20 to 40 cm, and 40 to 60 cm. The water content in these soil layers was measured routinely every week. The soil contained 5 % hygroscopic water while the maximum water capacity of the soil was 20 %. All fertilizers were applied in the soil prior to plant transplantation. The amount of both phosphorus and potassium was calculated to be sufficient for all seasons. Nitrogen was applied as recommended, equally to all treatments, through the irrigation system. Each row contained three dripping irrigation lines creating a coverage of nine drippers per 1 $m^2$. The water control was performed separately for each treatment. The soil was dried completely before the beginning of the experiment. The different water regimes were begun only 4 weeks after transplanting when plants initiated the flowering stage. The amount of water supplied every week during the assay was calculated at the beginning of every week following the recommendations of standard growth protocols. WLI-0 treatment (control) received the recommended total weekly irrigation volume divided into three irrigations. WLI-1 was irrigated three times a week, but the amount of water supplied was half that supplied to WLI-0. At the end of every week, WLI-1 plants received the amount of water required to reach maximum soil water capacity. WLI-2 plants were irrigated only once a week, at the beginning of the week. The water-stress experiment lasted throughout the flowering period (23 days), corresponding to four cycles of the above-described stresses. Afterwards, all treatments received the recommended amount of water. The calculated water amount was equal to the difference between the water contents in dry soil and in soil with maximum water capacity. At the end of each stress cycle, the water amounts were compared between treatments according to actual water content in the soil (S3). During the stress period, treatments WLI-1 and WLI-2 received a total of 75 % less water than the controls (WLI-0).

*Experimental Results*

*Transgenic plants exhibit increased tolerance to salt stress* - To induce salt-stress, transgenic and control tomato plants were continuously irrigated in field trials with 180 to 200 mM NaCl. As shown in Figures 3a-c, 3g-j and Table 70 below, Tom-ABST36 plants appeared to be more vigorous in all of the experiments than the control plants, which were smaller and showed severe symptoms of leaf and shoot necrosis (see for example, Figure 3j). This was also associated with higher fruit yield in Tom-ABST36 plants relative to controls (Figure 3a).

*Table 70*

| Salt-stress field trial | | | | | | |
|---|---|---|---|---|---|---|
| | Control | | | 180 mM NaCl | | | |
| | Plant FW (tn/acre) | Fruit yield (tn/acre) | Harvest index | Plant FW (tn/acre) | Fruit yield (tn/acre) | %* | Harvest index |
| SITIP2;2 | ND | 24.0 | ND | 2.8[a] | 8.0[a] | 110% | 2.8 |

(continued)

| Salt-stress field trial | | | | | | | |
|---|---|---|---|---|---|---|---|
| | Control | | | 180 mM NaCl | | | |
| | Plant FW (tn/acre) | Fruit yield (tn/acre) | Harvest index | Plant FW (tn/acre) | Fruit yield (tn/acre) | %* | Harvest index |
| WT | ND | 24.0 | ND | 1.4[b] | 3.8[b] | 0% | 2.7 |

**Table 70:** Total yield (ton fruit/acre), plant fresh weight (FW), and harvest index were calculated for TOM *ABST36* vs. control plants growing in the field under salt-stress conditions (180 mM NaCl). Results are the average of four independent events. a, b- Values in a column followed by different superscript letters are significantly different.

*Transgenic plants exhibit increased tolerance to water-deficiency stress -* Transgenic plants subjected to water-deficiency stress exhibited a significantly higher (26 %, $p \leq 0.05$) plant biomass compared to control plants (Figure 3e). Moreover the Tom-ABST36 plants showed a significant (up to 21 %, $p \leq 0.05$) increment of fruit yield under water-deficient regimes (water level intervals WLI-1), while under normal irrigation, the yield improvement was even higher (27 %, $p \leq 0.05$; Figure 3d). The harvest index of the Tom-ABST36 plants was also higher when plants grew under regular and WLI-1 conditions while it remained similar to control when the water-deficient regime consisted of once-a-week irrigation (WLI-2) (Figure 3f).

The results from the three field trials provided strong evidence that the tomato Tom-ABST36 plants show improved tolerance to salt and water-deficiency stress relative to the control plants, which is translated into significant increments in plant biomass and more importantly, fruit yield.

*Arabidopsis Salt-stress green house trial -* A complementary experiment with transgenic Arabidopsis plants expressing the ABST36 construct showed increased tolerance to a salt stress of 150 mM NaCl compared to control plants, as reflected in 42 % higher fresh biomass and 60 % higher dry biomass (Table 71 below).

*In-vitro salt-stress assay -* Seeds of transgenic *Arabidopsis* plants harboring the At6669::ABST36 construct or 35S::GUS construct (which was used as control) were sown in 1/2 MS media containing 40 mg/l kanamycin for selection. Selected seedlings were sub-cultured to 1/2 MS media with 0 or 150 mM NaCl. Plants were grown for a period of 3 weeks. Results are the average of four independent events that were analyzed in four repeats. For the determination of shoot dry weight, shoot plants were collected and dried for 24 hours at 60 °C and then weighed.

**Table 71**

| Arabidopsis salt - stress assay | | | | | | |
|---|---|---|---|---|---|---|
| | 0 mM NaCl | | | 150 mM NaCl | | |
| | Plant FW | | Plant DW | Plant FW | | Plant DW |
| Lines | (mg) | | (mg) | (mg) | | (mg) |
| S/TIP2;2 | 408.28[a] | | 23.52[a] | 68.55[a] | | 4.4[a] |
| WT | 394.36[a] | | 22.63[a] | 48.12[b] | | 2.7[b] |

**Table 71.** *Arabidopsis* seedlings were grown in 0 and 150 mM NaCl under tissue-culture conditions. Shown are the fresh weight (FW) and total dry weight (DW) (both measured in milligrams) of *ABST36* (SEQ ID NO: 2827) transgenic or wild type controls under normal conditions (0 mM NaCl) or salinity stress (150 mM NaCl). a, b-Values in a column followed by different superscript letters are significantly different at $P < 0.05$

**Claims**

1. A method of increasing abiotic stress tolerance of a plant as compared to a non-transformed plant of the same species which is grown under the same growth conditions, comprising over-expressing within the plant an exogenous polynucleotide encoding a polypeptide comprising an amino acid sequence at least 85 % homologous to the amino acid sequence set forth in SEQ ID NO: 33, wherein said polypeptide is capable of transporting water in a plant, thereby increasing the abiotic stress tolerance of the plant as compared to the non-transformed plant of the same species which is grown under the same growth conditions, wherein the abiotic stress condition is salinity stress.

**2.** The method of claim 1, wherein said exogenous polynucleotide comprising the nucleic acid sequence set forth in SEQ ID NO: 7 or 2757.

**3.** The method of claim 1, wherein said polypeptide comprising an amino acid sequence at least 90 % homologous to the amino acid sequence set forth in SEQ ID NO: 33.

**4.** The method of claim 1, wherein said polypeptide comprising an amino acid sequence at least 95 % homologous to the amino acid sequence set forth in SEQ ID NO: 33.

**5.** The method of claim 1, wherein said polypeptide comprising the amino acid sequence set forth by SEQ ID NO:33.

**6.** The method of claim 1, 2, 3, 4, or 5, further comprising growing the plant expressing said exogenous polynucleotide under the abiotic stress.

**7.** The method of claim 1, 2, 3, 4 or 5, wherein the plant is a dicotyledonous plant.

**8.** The method of claim 1, 2, 3, 4 or 5, wherein the plant is a monocotyledonous plant.


**Patentansprüche**

**1.** Verfahren zum Erhöhen der abiotischen Stresstoleranz einer Pflanze gegenüber einer nichttransformierten Pflanze der gleichen Spezies, die unter den gleichen Züchtungsbedingungen gezüchtet wird, das das Überexprimieren eines exogenen Polynukleotids innerhalb der Pflanze umfasst, das für ein Polypeptid codiert, das eine Aminosäuresequenz umfasst, die zumindest zu 85 % mit der Aminosäuresequenz gemäß SEQ ID NO:33 homolog ist, wobei das Polypeptid in der Lage ist, Wasser in einer Pflanze zu transportieren, wodurch die abiotische Stresstoleranz der Pflanze gegenüber der nichttransformierten Pflanze der gleichen Spezies, die unter den gleichen Züchtungsbedingungen gezüchtet wird, erhöht wird, wobei der abiotische Stresszustand Stress in Bezug auf den Salzgehalt ist.

**2.** Verfahren nach Anspruch 1, wobei das exogene Polynukleotid die Nukleinsäuresequenz gemäß SEQ ID NO: 7 oder 2757 umfasst.

**3.** Verfahren nach Anspruch 1, wobei das Polypeptid eine Aminosäuresequenz umfasst, die zumindest zu 90 % mit der Aminosäuresequenz gemäß SEQ ID NO: 33 homolog ist.

**4.** Verfahren nach Anspruch 1, wobei das Polypeptid eine Aminosäuresequenz umfasst, die zumindest zu 95 % mit der Aminosäuresequenz gemäß SEQ ID NO: 33 homolog ist.

**5.** Verfahren nach Anspruch 1, wobei das Polypeptid die Aminosäuresequenz gemäß SEQ ID NO: 33 umfasst.

**6.** Verfahren nach Anspruch 1, 2, 3, 4 oder 5, das ferner das Züchten der Pflanze, die das exogene Polynukleotid exprimiert, unter dem abiotischen Stress umfasst.

**7.** Verfahren nach Anspruch 1, 2, 3, 4 oder 5, wobei die Pflanze eine zweikeimblättrige Pflanze ist.

**8.** Verfahren nach Anspruch 1, 2, 3, 4 oder 5, wobei die Pflanze eine einkeimblättrige Pflanze ist.


**Revendications**

**1.** Procédé pour augmenter la tolérance d'une plante à un stress abiotique par comparaison à une plante non transformée de la même espèce qui est cultivée dans les mêmes conditions de croissance, comprenant la surexpression dans la plante d'un polynucléotide exogène codant pour un polypeptide comprenant une séquence d'acides aminés au moins 85 % homologue à la séquence d'acides aminés décrite dans SEQ ID NO: 33, où ledit polypeptide est capable de transporter de l'eau dans une plante, en augmentant ainsi la tolérance de la plante à un stress abiotique par comparaison à la plante non transformée de la même espèce qui est cultivée dans les mêmes conditions de croissance, où la condition de stress abiotique est un stress salin.

**2.** Procédé selon la revendication 1, dans lequel ledit polynucléotide exogène comprend la séquence d'acide nucléique décrite dans SEQ ID NO: 7 ou 2757.

**3.** Procédé selon la revendication 1, dans lequel ledit polypeptide comprend une séquence d'acides aminés au moins 90 % homologue à la séquence d'acides aminés décrite dans SEQ ID NO: 33.

**4.** Procédé selon la revendication 1, dans lequel ledit polypeptide comprend une séquence d'acides aminés au moins 95 % homologue à la séquence d'acides aminés décrite dans SEQ ID NO: 33.

**5.** Procédé selon la revendication 1, dans lequel ledit polypeptide comprend la séquence d'acides aminés décrite par SEQ ID NO: 33.

**6.** Procédé selon la revendication 1, 2, 3, 4, ou 5, comprenant en outre la croissance de la plante exprimant ledit polynucléotide exogène dans la condition de stress abiotique.

**7.** Procédé selon la revendication 1, 2, 3, 4 ou 5, dans lequel la plante est une plante dicotylédone.

**8.** Procédé selon la revendication 1, 2, 3, 4 ou 5, dans lequel la plante est une plante monocotylédone.

FIG. 1

FIG. 2B

FIG. 2A

**NaCl stress**

FIG. 3A — Total fruit yield

FIG. 3B — Plant fresh weight

FIG. 3C — Harvest index

**Water-deficiency stress**

FIG. 3D — Total fruit yield

FIG. 3E — Plant fresh weight

FIG. 3F — Harvest index

EP 2 240 510 B1

Standard irrigation

FIG. 3G          FIG. 3H

200 mM NaCl irrigation

FIG. 3I          FIG. 3J

EP 2 240 510 B1

REFERENCES CITED IN THE DESCRIPTION

**Patent documents cited in the description**

- US 81085510 A
- IL 2008001657 W
- US 61136238 A
- US 61009166 B
- US 20020046419 A, Choo
- US 20030233670 A, Edgerton
- US 20060179511 A, Chomet
- US 6084153 A, Good
- WO 2004104162 A
- WO 2007020638 A
- US 2003233670 A
- WO 9307278 PCT
- WO 04081173 A2
- US 5659026 A
- US 5608149 A
- US 5608144 A
- US 5604121 A
- US 5569597 A
- US 5466785 A
- US 5399680 A
- US 5268463 A
- US 5608142 A
- EP 99106056 A
- US 5187267 A
- US 5464765 A
- US 4855237 A
- EP 67553 A
- JP 63014693 A
- EP 194809 A
- EP 278667 A
- WO 8706261 A
- US 5316931 A
- US 4945050 A
- US 5693507 A
- FR 1963, Conway TF. and Earle
- WO 2001023884 A
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A
- WO 04081173 A

**Non-patent literature cited in the description**

- **YANAGISAWA et al.** *Proc. Natl. Acad. Sci. U.S.A.,* 2004, vol. 101 (20), 7833-8
- **GOOD AG et al.** *Trends Plant Sci.,* 2004, vol. 9 (12), 597-605
- **MAUREL C.** Plant aquaporins: Novel functions and regulation properties. *FEBS Lett.,* 2007, vol. 581 (12), 2227-36
- **MAUREL C.** Plant aquaporins: Novel functions and regulation properties. *FEBS Lett.,* 12 June 2007, vol. 581 (12), 2227-36
- **QUIGLEY F et al.** From genome to function: the Arabidopsis aquaporins. *Genome Biol.,* 2002, vol. 3 (1
- **CHAUMONT F et al.** Aquaporins constitute a large and highly divergent protein family in maize. *Plant Physiol,* 2001, vol. 125 (3), 1206-15
- **SAKURAI, J.** Identification of 33 rice aquaporin genes and analysis of their expression and function. *Plant Cell Physiol.,* 2005, vol. 46, 1568-1577
- **LIAN HL et al.** *Cell Res.,* 2006, vol. 16, 651-60
- **AHARON R. et al.** *Plant Cell,* 2003, vol. 15, 439-47
- **MURRAY et al.** *Nuc Acids Res.,* 1989, vol. 17, 477-498
- **ODELL et al.** *Nature,* 1985, vol. 313, 810-812
- **CHRISTENSEN et al.** *Plant Sol. Biol.,* 1992, vol. 18, 675-689
- **MCELROY et al.** *Plant Cell,* 1990, vol. 2, 163-171
- **LAST et al.** *Theor. Appl. Genet.,* 1991, vol. 81, 581-588

- **NILSSON et al.** *Physiol. Plant,* 1997, vol. 100, 456-462
- **DE PATER et al.** *Plant J Nov,* 1992, vol. 2 (6), 837-44
- **CHRISTENSEN et al.** *Plant Mol. Biol.,* 1992, vol. 18, 675-689
- **BUCHOLZ et al.** *Plant Mol Biol.,* 1994, vol. 25 (5), 837-43
- **LEPETIT et al.** *Mol. Gen. Genet.,* 1992, vol. 231, 276-285
- **AN et al.** *Plant J.,* 1996, vol. 10 (1), 107-121
- **NI et al.** *The Plant Journal,* 1995, vol. 7, 661-76
- **YAMAMOTO et al.** *Plant J.,* 1997, vol. 12, 255-265
- **KWON et al.** *Plant Physiol.,* 1994, vol. 105, 357-67
- **YAMAMOTO et al.** *Plant Cell Physiol.,* 1994, vol. 35, 773-778
- **GOTOR et al.** *Plant J.,* 1993, vol. 3, 509-18
- **OROZCO et al.** *Plant Mol. Biol.,* 1993, vol. 23, 1129-1138
- **MATSUOKA et al.** *Proc. Natl. Acad. Sci. USA,* 1993, vol. 90, 9586-9590
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 143, 323-32
- **STALBERG et al.** *Planta,* 1996, vol. 199, 515-519
- **ALBANI ET AL.** *Plant Cell,* 1997, vol. 9, 171-184
- **CUMMINS et al.** *Plant Mol. Biol.,* 1992, vol. 19, 873-876
- *Mol Gen Genet,* 1989, vol. 216, 81-90
- *EMBO,* 1984, vol. 3, 1409-15
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **NAKASE et al.** *Plant Mol. Biol.,* 1997, vol. 33, 513-S22
- *Trans Res,* 1997, vol. 6, 157-68
- *Plant J,* 1997, vol. 12, 235-46
- *PMB,* 1996, vol. 32, 1029-35
- **SATO et al.** *Proc. Nati. Acad. Sci. USA,* vol. 93, 8117-8122
- **POSTMA-HAARSMA.** *Plant Mol. Biol.,* 1999, vol. 39, 257-71
- **WU.** *J. Biochem.,* 1998, vol. 123, 386
- **VAN DER MEER et al.** *Plant Mol. Biol.,* 1990, vol. 15, 95-109
- **TWELL et al.** *Mol. Gen Genet.,* 1989, vol. 217, 240-245
- **YAMAGUCHI-SHINOZALEI et al.** *Mol. Gen. Genet.,* 1993, vol. 236, 331-340
- **PLA.** *Plant Mol. Biol.,* 1993, vol. 21, 259-266
- **BUSK.** *Plant J.,* 1997, vol. 11, 1285-1295
- **PELLESCHI.** *Plant Mol. Biol.,* 1999, vol. 39, 373-380
- **POTRYKUS, I.** *Annu. Rev. Plant. Physiol., Plant. Mol. Biol.,* 1991, vol. 42, 205-225
- **SHIMAMOTO et al.** *Nature,* 1989, vol. 338, 274-276
- **KLEE et al.** *Annu. Rev. Plant Physiol.,* 1987, vol. 38, 467-486
- Cell Culture and Somatic Cell Genetics of Plants. **KLEE ; ROGERS.** Molecular Biology of Plant Nuclear Genes. Academic Publishers, 1989, vol. 6, 2-25
- **GATENBY.** Plant Biotechnology. Butterworth Publishers, 1989, 93-112
- Cell Culture and Somatic Cell Genetics of Plants. **PASZKOWSKI et al.** Molecular Biology of Plant Nuclear Genes. Academic Publishers, 1989, vol. 6, 52-68
- **TORIYAMA, K. et al.** *Bio/Technology,* 1988, vol. 6, 1072-1074
- **ZHANG et al.** *Plant Cell Rep.,* 1988, vol. 7, 379-384
- **FROMM et al.** *Nature,* 1986, vol. 319, 791-793
- **KLEIN et al.** *Bio/Technology,* 1988, vol. 6, 559-563
- **MCCABE et al.** *Bio/Technology,* 1988, vol. 6, 923-926
- **SANFORD.** *Physiol. Plant.,* 1990, vol. 79, 206-209
- **NEUHAUS et al.** *Theor. Appl. Genet.,* 1987, vol. 75, 30-36
- **NEUHAUS ; SPANGENBERG.** *Physiol. Plant.,* 1990, vol. 79, 213-217
- **DEWET et al.** Experimental Manipulation of Ovule Tissue. W. Longman, 1985, 197-209
- **OHTA.** *Proc. Natl. Acad. Sci. USA,* 1986, vol. 83, 715-719
- **HORSCH et al.** Plant Molecular Biology Manual. Kluwer Academic Publishers, 1988, vol. A5, 1-9
- **GLUZMAN, Y. et al.** Communications in Molecular Biology: Viral Vectors. Cold Spring Harbor Laboratory, 1988, 172-189

- **KURIHARA ; WATANABE.** *Molecular Plant Pathology,* 2003, vol. 4, 259-269
- Plant Virology Protocols: From Virus Isolation to Transgenic Resistance. Methods in Molecular Biology. Humana Press, 1998, vol. 81
- **DAWSON, W. O. et al.** *Virology,* 1989, vol. 172, 285-292
- **TAKAMATSU et al.** *EMBO J.,* 1987, vol. 6, 307-311
- **FRENCH et al.** *Science,* 1986, vol. 231, 1294-1297
- **TAKAMATSU et al.** *FEBS Letters,* 1990, vol. 269, 73-76
- Methods in Virology. Academic Press, vol. 7, 1967-1984
- **HILL, S.A.** Methods in Plant Virology. Blackwell, 1984
- **WALKEY, D.G.A.** Applied Plant Virology. Wiley, 1985
- Principles and Techniques in Plant Virology. Van Nostrand-Reinhold
- **QUESDA et al.** *Plant Physiol.,* 2002, vol. 130, 951-063
- **MENG, Q. X. et al.** *Cell Physiol Biochem,* 2008, vol. 21, 123-128
- Root Growth Under Salinity Stress. **BERNSTEIN ; KAFKAFI.** Plant Roots, The Hidden Half. Marcel Dekker Inc, 2002
- **MURASHIGE ; SKOOG.** *Plant Physiology,* 1962, vol. 15, 473-497
- **YANAGISAWA et al.** *Proc Natl Acad Sci USA.,* 2004, vol. 101, 7833-8
- **PURCELL ; KING.** *Argon. J.,* 1996, vol. 88, 111-113
- **VODOVOTZ.** *Biotechniques,* 1996, vol. 20, 390-394
- **SAMONTE et al.** *Agron. J.,* 2006, vol. 98, 168-176
- Journal of the American Oil Chemists' Society. Springer
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- Recombinant DNA. **WATSON et al.** Scientific American Books
- Genome Analysis: A Laboratory Manual Series. Cold Spring Harbor Laboratory Press, 1998, 1-4
- Cell Biology: A Laboratory Handbook. 1994, vol. I-III
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. Nucleic Acid Hybridization. 1984
- Transcription and Translation. 1984
- Animal Cell Culture. 1986
- Immobilized Cells and Enzymes. IRL Press, 1986
- **PERBAL, B.** *A Practical Guide to Molecular Cloning,* 1984
- Methods in Enzymology. Academic Press, vol. 1-317
- PCR Protocols: A Guide To Methods And Applications. Academic Press, 1990
- **MARSHAK et al.** Strategies for Protein Purification and Characterization - A Laboratory Course Manual. CSHL Press, 1996
- **YELIN.** *Nature Biotechnology,* 2003, vol. 21, 379-85
- **SAMBROOK J. ; E.F. FRITSCH ; T. MANIATIS.** Molecular Cloning. A Laboratory Manual. Cold Spring Harbor Laboratory Press, 1989
- **CLOUGH SJ ; BENT AF.** Floral dip: a simplified method for Agrobacterium-mediated transformation of Arabidopsis thaliana. *Plant J.,* 1998, vol. 16 (6), 735-43
- **DESFEUX C ; CLOUGH SJ ; BENT AF.** Female reproductive tissues are the primary targets of Agrobacterium-mediated transformation by the Arabidopsis floral-dip method. *Plant Physiol.,* 2000, vol. 123 (3), 895-904
- **WESLEY et al.** *Journal of Experiment Botany,* 2002, vol. 53 (366), 13-25